# EUROPEAN PATENT SPECIFICATION

(11) **EP 2 049 517 B1**
(45) Date of publication and mention of the grant of the patent: **27.11.2013**
(21) Application number: 07786158.1
(22) Date of filing: 18.07.2007
(51) Int. Cl.: C07D 401/12, C07D 311/30, A61K 31/455, A61P 9/00, C07D 211/58, C07D 401/14, C07D 413/14, C07D 417/14

(54) **AMINO-PIPERIDINE DERIVATIVES AS CETP INHIBITORS**
AMINOPIPERIDINDERIVATE ALS CETP-HEMMER
DERIVES D'AMINO-PIPERIDINE COMME INHIBITEURS DE CETP

(30) Priority: 20.07.2006 EP 06117541; 29.01.2007 US 887058 P
(43) Date of publication of application: 22.04.2009
(73) Proprietor: Novartis AG, 4056 Basel (CH)
(72) Inventor: IMASE, Hidetomo, Tsukuba Ibaraki 300-2611 (JP); IWAKI, Yuki, Tsukuba Ibaraki 300-2611 (JP); KAWANAMI, Toshio, Tsukuba Ibaraki 300-2611 (JP); MIYAKE, Takahiro, Tsukuba Ibaraki 300-2611 (JP); MOGI, Muneto, Tsukuba Ibaraki 300-2611 (JP); OHMORI, Osamu, Tsukuba Ibaraki 300-2611 (JP); QIN, Hongbo, Tsukuba Ibaraki 300-2611 (JP); UMEMURA, Ichiro, Ibaraki 300-2611 (JP); YAMADA, Ken, Tsukuba Ibaraki 300-2611 (JP); YASOSHIMA, Kayo, Tsukuba Ibaraki 300-2611 (JP)
(74) Representative: Vögeli-Lange, Regina
(86) International application number: PCT/EP2007/006384
(87) International publication number: WO 2008/009435

(56) References cited:
- EP-A- 0 987 251
- WO-A-2006/134378
- WEIS R ET AL: "Synthesis of 2-substituted bamipine derivatives" TETRAHEDRON, ELSEVIER SCIENCE PUBLISHERS, AMSTERDAM, NL, vol. 59, no. 9, 24 February 2003 (2003-02-24), pages 1395-1402, XP004409938 ISSN: 0040-4020
- VARTANYAN R S ET AL: "4-ANILIDES OF 1-SUBSTITUTED 2,5-DIMETHYLPIPERIDINES: SYNTHESIS AND ANALGESIC ACTIVITY" KHIMIKO-FARMATSEVTICHESKII ZHURNAL, MOSCOW, RU, vol. 23, no. 5, 1989, pages 562-565, XP002937455 ISSN: 0023-1134
- N. S. PROSTAKOV ET AL: "Benzylation of gamma-(N-arylamino)piperidines by the Wallach Method" KHIMIYA GETEROTSIKLICHESKIKH SOEDINENII, vol. 8, 1988, pages 1078-1083, XP009078378
- V. V. KUZNETSOV ET AL: "1-Methyl(benzyl)-2,5-dimethyl-4-N-[aryl(a lkyl)amino]piperidines and their N-acyl derivatives" KHIMIYA GETEROTSIKLICHESKIKH SOEDINENII, vol. 7, 1987, pages 949-953, XP009078377
- E. E. STASHENKO ET AL: "Mass-spectrometric study of ring-substituted secondary and tertiary gamma-aminopiperidines" KHIMIYA GETEROTSIKLICHESKIKH SOEDINENII, vol. 3, 1990, pages 380-387, XP009078379

## Description

The present invention related to novel compound of formula (I): 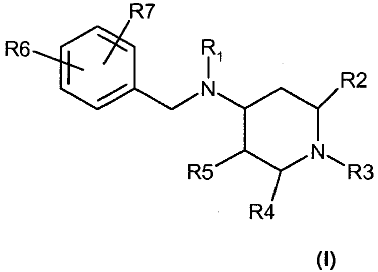
wherein R1 is cycloalkyl, heterocyclyl, aryl, alkyl-O-C(O)-, alkanoyl, or alkyl, wherein each cycloalkyl, heterocyclyl, or aryl is optionally substituted with one to three substituents selected from alkyl, aryl, haloalkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamoyl, alkyl-S-, alkyl-SO-, alkyl-SO₂-, amino, mono- or di-substituted (alkyl, cycloalkyl, aryl and/or aryl-alkyl-) amino, H₂N-SO₂-, or heterocyclyl, and wherein each alkanoyl, alkyl-O-C(O)-, alkyl, alkoxy, or heterocyclyl is further optionally substituted with one to three substituents selected from hydroxy, alkyl, halogen, nitro, carboxy, thiol, cyano, HSO₃-, cycloalkyl, alkenyl, alkoxy, cycloalkoxy, alkenyloxy, alkyl-O-C(O)-, alkanoyl, carbamoyl, alkyl-S-, alkyl-SO-, alkyl-SO₂-, amino, mono- or di-substituted (alkyl, cycloalkyl, aryl and/or aryl-alkyl-) amino, H₂N-SO₂-, or heterocyclyl;
R2 is alkyl, cycloalkyl, cycloalkyl-alkyl-, or alkoxy, wherein each alkyl, cycloalkyl or alkoxy is optionally substituted with one to three substituents selected from alkyl, alkoxy or halogen; R3 is R8-O-C(O)-, (R8)(R9)N-C(O)-, R8-C(O)-, R8-S(O)₂-, alkyl, cycloalkyl, or aryl-alkyl-, wherein each alkyl, cycloalkyl or aryl-alkyl- is optionally substituted with one to three substituents selected from alkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamimidoyl, alkyl-S-, alkyl-SO-, alkyl-SO₂-, amino, H₂N-SO₂-, heterocyclyl,
wherein R8 and R9 are independently hydrogen, alkyl, cycloalkyl, aryl, aryl-alkyl- or cycloalkyl-alkyl-, wherein each alkyl, cycloalkyl, aryl, aryl-alkyl- or cycloalkyl-alkyl- is optionally substituted with one to three substituents selected from alkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamimidoyl, alkyl-S-, alkyl-SO-, alkyl-SO₂-, amino, H₂N-SO₂-, heterocyclyl;
R4 and R5 are independently hydrogen, alkyl, alkoxy, cycloalkyl, aryl, heteroaryl, aryl-alkyl-, cycloalkyl-alkyl-, or heteroaryl-alkyl-, wherein each alkyl, cycloalkyl, aryl, heteroaryl, aryl-alkyl-, cycloalkyl-alkyl-, or heteroaryl-alkyl- is optionally substituted with one to three substituents selected from alkyl, hydroxy, halogen, haloalkyl, nitro, carboxy, thiol, cyano, HSO₃-, cycloalkyl, alkenyl, alkoxy, cycloalkoxy, haloalkoxy, alkenyloxy, alkyl-O-C(O)-, alkanoyl, carbamimidoyl, alkyl-S-, alkyl-SO-, alkyl-SO₂-, amino, mono- or di-substituted (alkyl, cycloalkyl, aryl and/or aryl-alkyl-) amino, H₂N-SO₂-, heterocyclyl, with the proviso that R4 and R5 cannot be hydrogen simultaneously;
R6 and R7 are independently hydrogen, alkyl, haloalkyl, halogen, cyano, nitro, hydroxy, amino, dialkylamino, or alkoxy, haloalkoxy; or
R6 is aryl, heteroaryl, or alkyl-S(O)₂-, wherein each aryl or heteroaryl is optionally substituted with one to three substituents selected from alkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamimidoyl, alkyl-S-, alkyl-SO-, alkyl-SO₂-, amino, H₂N-SO₂- heterocyclyl; or a pharmaceutically acceptable salt thereof; or an optical isomer thereof; or a mixture of optical isomers, with the proviso that when R2 and R5 are independently alkyl and R4 is hydrogen, R6 or R7 cannot be hydrogen or alkoxy.
a pharmaceutically acceptable salt thereof; or an optical isomer thereof; or a mixture of optical isomers.

In one embodiment, the present invention provides the compound of formula (I), wherein
R1 is alkyl-O-C(O)-, or heteroaryl that is optionally substituted by one to three substituents selected from halogen, heteroaryl, hydroxyl, alkoxy, non-aromatic heterocyclyl, alkyl, or dialkylamino, wherein each of heteroaryl, alkoxy, alkyl and non-aromatic heterocyclyl is further optionally substituted by one to three substituents selected from alkyl, hydroxyl, alkyl-O-C(O)-, carboxy, alkyl-SO₂-, alkoxy, dialkylamino, or non-aromatic heterocyclyl, or alkanoyl;
R2 is alkyl;
R3 is R8-C(O)-, or R8-O-C(O)-, wherein R8 is alkyl, non-aromatic heterocyclyl or cycloalkyl, each of alkyl, non-aromatic heterocyclyl or cycloalkyl is optionally substituted by one to three substituents selected from alkanoyl, alkyl-C(O)-O-, or hydroxyl;
R4 is aryl-alkyl-, alkyl, or heteroaryl, each of which is optionally substituted by one to three substituents selected from alkyl, halogen, or hydroxyl;
R5 is hydrogen or alkyl;
R6 and R7 are independently haloalkyl, halogen or alkoxy ; or
a pharmaceutically acceptable salt thereof; or an optical isomer thereof; or a mixture of optical isomers.

In one embodiment, the present invention provides the compound of formula (I), wherein
R1 is (C1-C4) alkyl-O-C(O)-, or 5- to 7-membered heteroaryl that is optionally substituted by one to three substituents selected from halogen, 5- to 7-membered heteroaryl, (C1-C4) alkoxy, 5- to 7-membered non-aromatic heterocyclyl, (C1-C4) alkyl, or (C1-C4) dialkylamino, wherein (C1-C4) alkyl is optionally substituted by one to three hydroxyl groups, 5- to 7-membered non-aromatic heterocyclyl is optionally substituted by one to three alkanoyl groups, and wherein each of 5- to 7-membered heteroaryl and (C1-C4) alkoxy is further optionally substituted by one to three substituents selected from (C1-C4) alkyl, hydroxy, (C1-C4) alkyl-O-C(O)-, (C1-C4) alkyl-SO₂-, (C1-C4) alkoxy, (C1-C4) dialkylamino, or 5- to 7-membered non-aromatic heterocyclyl;
R2 is (C1-C4) alkyl;
R3 is R8-C(O)-, or R8-O-C(O)-, wherein R8 is (C1-C4) alkyl, 5- to 7-membered non-aromatic heterocyclyl or (C5-C7) cycloalkyl, each of (C1-C4) alkyl, 5- to 7-membered non-aromatic heterocyclyl and (C5-C7) cycloalkyl is optionally substituted by one to three substituents selected from (C1-C4) alkanoyl, (C1-C4) alkyl-C(O)-O-, or hydroxy;
R4 is (C5-C9)aryl-(C1-C4) alkyl-, (C1-C4) alkyl, or 5- to 7-membered heteroaryl, each of which is optionally substituted by one to three substituents selected from (C1-C4) alkyl, halogen, or hydroxy;
R5 is hydrogen or (C1-C4) alkyl;
R6 and R7 are independently (C1-C4) haloalkyl, halogen or (C1-C4) alkoxy; or
a pharmaceutically acceptable salt thereof; or an optical isomer thereof; or a mixture of optical isomers.

EP0987251 (Pfizer) describes 4-carboxyamino-2-methyl-1,2,3,4-tetrahydroquinolines as CETP inhibitors.

The present invention also relates to a process for the preparation of these compounds, to the use of these compounds and to pharmaceutical preparations containing such a compound I in free form or in the form of a pharmaceutically acceptable salt.

Extensive pharmacological investigations have shown that the compounds I and their pharmaceutically acceptable salts, for example, have pronounced selectivity in inhibiting CETP (cholesteryl ester transfer protein). CETP is involved in the metabolism of any lipoprotein in living organisms, and has a major role in the reverse cholesterol transfer system. Namely, CETP has drawn attention as a mechanism for preventing accumulation of cholesterol in peripheral cells and preventing arteriosclerosis. In fact, with regard to HDL having an important role in this reverse cholesterol transfer system, a number of epidemiological researches have shown that a decrease in CE (cholesteryl ester) of HDL in blood is one of the risk factors of coronary artery diseases. It has been also clarified that the CETP activity varies depending on the animal species, wherein arteriosclerosis due to cholesterol-loading is hardly induced in animals with lower activity, and in reverse, easily induced in animals with higher activity, and that hyper-HDL-emia and hypo-LDL (low density lipoprotein)-emia are induced in the case of CETP deficiency, thus rendering the development of arteriosclerosis difficult, which in turn led to the recognition of the significance of blood HDL, as well as significance of CETP that mediates transfer of CE in HDL into blood LDL. While many attempts have been made in recent years to develop a drug that inhibits such activity of CETP, a compound having a satisfactory activity has not been developed yet.

For purposes of interpreting this specification, the following definitions will apply and whenever appropriate, terms used in the singular will also include the plural and vice versa. As used herein, the term "alkyl" refers to a fully saturated branched or unbranched hydrocarbon moiety. Preferably the alkyl comprises 1 to 20 carbon atoms, more preferably 1 to 16 carbon atoms, 1 to 10 carbon atoms, 1 to 7 carbon atoms, or 1 to 4 carbon atoms. Representative examples of alkyl include, but are not limited to, methyl, ethyl, *n*-propyl, *iso-*propyl, n-butyl, *sec*-butyl, *iso*-butyl, *tert*-butyl, n-pentyl, isopentyl, neopentyl, n-hexyl, 3-methylhexyl, 2,2- dimethylpentyl, 2,3-dimethylpentyl, n-heptyl, n-octyl, n-nonyl, n- decyl and the like. When an alkyl group includes one or more unsaturated bonds, it can be referred to as an alkenyl (double bond) or an alkynyl (triple bond) group. If the alkyl group can be substituted, it is preferably substituted by 1, 2 or 3 substituents selected from hydroxy, halogen, nitro, carboxy, thiol, cyano, HSO₃--, cycloalkyl, alkenyl, alkoxy, cycloalkoxy, alkenyloxy, alkoxycarbonyl, carbamimidoyl, alkyl-S--, alkyl-SO--, alkyl-SO₂--, amino, H₂N-SO₂--, alkanoyl, or heterocyclyl, more preferably selected from hydroxy, halogen, nitro, carboxy, thiol, cyano, alkoxy, or amino.

The term "aryl" refers to monocyclic or bicyclic aromatic hydrocarbon groups having 6-20 carbon atoms in the ring portion. Preferably, the aryl is a (C₆-C₁₀) aryl. Non-limiting examples include phenyl, biphenyl, naphthyl or tetrahydronaphthyl, most preferably phenyl, each of which may optionally be substituted by 1-4 substituents, such as alkyl, haloalkyl such as trifluoromethyl, cycloalkyl, halogen, hydroxy, alkoxy, alkyl-C(O)-O--, aryl-O--, heteroaryl-O--, amino, acyl, thiol, alkyl-S--, aryl-S--, nitro, cyano, carboxy, alkyl-O-C(O)--, carbamoyl, alkyl-S(O)--, sulfonyl, sulfonamido, heterocyclyl, alkenyl, haloalkoxy, cycloalkoxy, alkenyloxy, alkoxycarbonyl, alkyl-SO--, alkyl-SO₂--, amino, mono- or di-substituted (alkyl, cycloalkyl, aryl and/or aryl alkyl) amino or H₂N-SO₂;.

Furthermore, the term "aryl" as used herein, refers to an aromatic substituent which can be a single aromatic ring, or multiple aromatic rings that are fused together, linked covalently, or linked to a common group such as a methylene or ethylene moiety. The common linking group also can be a carbonyl as in benzophenone or oxygen as in diphenylether or nitrogen as in diphenylamine.

As used herein, the term "alkoxy" refers to alkyl-O-, wherein alkyl is defined herein above. Representative examples of alkoxy include, but are not limited to, methoxy, ethoxy, propoxy, 2-propoxy, butoxy, *tert*-butoxy, pentyloxy, hexyloxy, cyclopropyloxy-, cyclohexyloxy- and the like. Preferably, alkoxy groups have about 1-7, more preferably about 1-4 carbons.

As used herein, the term "acyl" refers to a group R-C(O)- of from 1 to 10 carbon atoms of a straight, branched, or cyclic configuration or a combination thereof, attached to the parent structure through carbonyl functionality. Such group can be saturated or unsaturated, and aliphatic or aromatic. Preferably, R in the acyl residue is alkyl, or alkoxy, or aryl, or heteroaryl. When R is alkyl then the moiety is referred to a alkanoyl. Also preferably, one or more carbons in the acyl residue may be replaced by nitrogen, oxygen or sulfur as long as the point of attachment to the parent remains at the carbonyl. Examples include but are not limited to, acetyl, benzoyl, propionyl, isobutyryl, t- butoxycarbonyl, benzyloxycarbonyl and the like. Lower acyl refers to acyl containing one to four carbons.

As used herein, the term "acylamino" refers to acyl-NH--, wherein "acyl" is defined herein.

As used herein, the term "carbamoyl" refers to H₂NC(O)-, alkyl-NHC(O)-, (alkyl)₂NC(O)-, aryl-NHC(O)-, alkyl(aryl)-NC(O)-, heteroaryl-NHC(O)-, alkyl(heteroaryl)-NC(O)-, aryl-alkyl-NHC(O)-, alkyl(aryl-alkyl)-NC(O)- and the like.

As used herein, the term "sulfonyl" refers to R-SO₂--, wherein R is hydrogen, alkyl, aryl, hereoaryl, aryl-alkyl, heteroaryl-alkyl, aryl-O--, heteroaryl-O--, alkoxy, aryloxy, cycloalkyl, or heterocyclyl.

As used herein, the term "sulfonamido" refers to alkyl-S(O)₂-NH-, aryl-S(O)₂-NH-, aryl-alkyl-S(O)₂-NH-, heteroaryl-S(O)₂-NH-, heteroaryl-alkyl-S(O)₂-NH-, alkyl-S(O)₂-N(alkyl)-, aryl-S(O)₂-N(alkyl)-, aryl-alkyl-S(O)₂-N(alkyl)-, heteroaryl-S(O)₂-N(alkyl)-, heteroarrl-alkyl-S(O)₂-N(alkyl)- and the like.

As used herein, the term "alkoxycarbonyl" or "alkyl-O-C(O)-" refers to alkoxy-C(O)--, wherein alkoxy is defined herein.

As used herein, the term "alkanoyl" refers to alkyl-C(O)--, wherein alkyl is defined herein. As used herein, the term "alkenyl" refers to a straight or branched hydrocarbon group having 2 to 20 carbon atoms and that contains at least one double bonds. The alkenyl groups preferably have about 2 to 8 carbon atoms.

As used herein, the term "alkynyl" refers to both straight-and branched-chain alkyl groups having one or more carbon-carbon triple bonds and having 2 to about 8 carbon atoms. Preferably, the term alkynyl refers to an alkyl group having 1 or 2 carbon-carbon triple bonds and having 2 to 6 carbon atoms.

As used herein, the term "alkenyloxy" refers to alkenyl-O--, wherein alkenyl is defined herein. As used herein, the term "cycloalkoxy" refers to cycloalkyl-O--, wherein cycloalkyl is defined herein.

As used herein, the term "heterocyclyl" or "heterocyclo" refers to an optionally substituted, fully saturated or unsaturated, aromatic or nonaromatic cyclic group, e.g., which is a 4- to 7-membered monocyclic, 7- to 12-membered bicyclic or 10- to 15-membered tricyclic ring system, which has at least one heteroatom in at least one carbon atom-containing ring. Each ring of the heterocyclic group containing a heteroatom may have 1, 2 or 3 heteroatoms selected from nitrogen atoms, oxygen atoms and sulfur atoms, where the nitrogen and sulfur heteroatoms may also optionally be oxidized. The heterocyclic group may be attached at a heteroatom or a carbon atom.

Exemplary monocyclic heterocyclic groups include pyrrolidinyl, pyrrolyl, pyrazolyl, oxetanyl, pyrazolinyl, imidazolyl, imidazolinyl, imidazolidinyl, triazolyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, piperazinyl, piperidinyl, 4-piperidonyl, pyridyl, pyrazinyl, pyrimidinyl, pyridazinyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, 1,1,4-trioxo-1,2,5-thiadiazolidin-2-yl and the like.

Exemplary bicyclic heterocyclic groups include indolyl, dihydroidolyl, benzothiazolyl, benzoxazinyl, benzoxazolyl, benzothienyl, benzothiazinyl, quinuclidinyl, quinolinyl, tetrahydroquinolinyl, decahydroquinolinyl, isoquinolinyl, tetrahydroisoquinolinyl, decahydroisoquinolinyl, benzimidazolyl, benzopyranyl, indolizinyl, benzofuryl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]-pyridinyl] or furo[2,3-b]pyridinyl), dihydroisoindolyl, 1,3-dioxo-1,3-dihydroisoindol-2-yl, dihydroquinazolinyl (such as 3,4-dihydro-4-oxo-quinazolinyl), phthalazinyl and the like.

Exemplary tricyclic heterocyclic groups include carbazolyl, dibenzoazepinyl, dithienoazepinyl, benzindolyl, phenanthrolinyl, acridinyl, phenanthridinyl, phenoxazinyl, phenothiazinyl, xanthenyl, carbolinyl and the like.

When heterocyclyl is aromatic, this moiety is referred to as "heteroaryl".

As used herein, the term "heteroaryl" refers to a 5-14 membered monocyclic- or bicyclic- or fused polycyclic-ring system, having 1 to 8 heteroatoms selected from N, O or S. Preferably, the heteroaryl is a 5-10 membered ring system. Typical heteroaryl groups include 2- or 3-thienyl, 2- or 3-furyl, 2- or 3-pyrrolyl, 2-, 4-, or 5-imidazolyl, 3-, 4-, or 5- pyrazolyl, 2-, 4-, or 5-thiazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2, 3-triazolyl, tetrazolyl, 2-, 3-, or 4-pyridyl, 3- or 4-pyridazinyl, 3-, 4- , or 5-pyrazinyl, 2-pyrazinyl, 2-, 4-, or 5-pyrimidinyl.

The term "heteroaryl" also refers to a group in which a heteroaromatic ring is fused to one or more aryl, cycloaliphatic, or heterocyclyl rings, where the radical or point of attachment is on the heteroaromatic ring. Nonlimiting examples include but are not limited to 1-, 2-, 3-, 5-, 6-, 7-, or 8- indolizinyl, 1-, 3-, 4-, 5-, 6-, or 7-isoindolyl, 2-, 3-, 4-, 5-, 6-, or 7-indolyl, 2-, 3-, 4-, 5-, 6-, or 7-indazolyl, 2-, 4-, 5-, 6-, 7-, or 8- purinyl, 1-, 2-, 3-, 4-, 6-, 7-, 8-, or 9-quinolizinyl, 2-, 3-, 4-, 5-, 6-, 7-, or 8-quinoliyl, 1-, 3-, 4-, 5-, 6-, 7-, or 8-isoquinoliyl, 1-, 4-, 5-, 6-, 7-, or 8-phthalazinyl, 2-, 3-, 4-, 5-, or 6-naphthyridinyl, 2-, 3- , 5-, 6-, 7-, or 8-quinazolinyl, 3-, 4-, 5-, 6-, 7-, or 8-cinnolinyl, 2-, 4-, 6-, or 7-pteridinyl, 1-, 2-, 3-, 4-, 5-, 6-, 7-, or 8-4aH carbazolyl, 1-, 2-, 3-, 4-, 5-, 6-, 7-, or 8-carbzaolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1- , 2-, 3-, 4-, 5-, 6-, 7-, 8-, or 9-acridinyl, 1-, 2-, 4-, 5-, 6-, 7-, 8-, or 9-perimidinyl, 2-, 3-, 4-, 5-, 6-, 8-, 9-, or 10-phenathrolinyl, 1-, 2- , 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenoxazinyl, 2-, 3-, 4-, 5-, 6-, or I-, 3-, 4-, 5-, 6-, 7-, 8-, 9-, or 10- benzisoqinolinyl, 2-, 3-, 4-, or thieno[2,3-b]furanyl, 2-, 3-, 5-, 6-, 7-, 8-, 9-, 10 -, or 11-7H-pyrazino[2,3-c]carbazolyl,2-, 3-, 5-, 6-, or 7-2H- furo[3,2-b]-pyranyl, 2-, 3-, 4-, 5-, 7-, or 8-5H-pyrido[2,3-d]-o-oxazinyl, 1-, 3-, or 5-1 H-pyrazolo[4,3-d]-oxazolyl, 2-, 4-, or 54H-imidazo[4,5-d] thiazolyl, 3-, 5-, or 8-pyrazino[2,3-d]pyridazinyl, 2-, 3-, 5-, or 6- imidazo[2,1-b] thiazolyl, 1-, 3-, 6-, 7-, 8-, or 9-furo[3,4-c]cinnolinyl, 1-, 2-, 3-, 4-, 5-, 6-, 8-, 9-, 10, or 11-4H-pyrido[2,3-c]carbazolyl, 2-, 3-, 6-, or 7-imidazo[1,2-b][1,2,4]triazinyl, 7-benzo[b]thienyl, 2-, 4-, 5- , 6-, or 7-benzoxazolyl, 2-, 4-, 5-, 6-, or 7-benzimidazolyl, 2-, 4-, 4-, 5-, 6-, or 7-benzothiazolyl, 1-, 2-, 4-, 5-, 6-, 7-, 8-, or 9-benzoxapinyl, 2-, 4-, 5-, 6-, 7-, or 8-benzoxazinyl, 1-, 2-, 3-, 5-, 6-, 7-, 8-, 9-, 10-, or 11-1 H-pyrrolo[1,2-b][2]benzazapinyl. Typical fused heteroaryl groups include, but are not limited to 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5- , 6-, or 7-benzoxazolyl, 2-, 4-, 5-, 6-, or 7-benzimidazolyl, 2-, 4-, 5-, 6-, or 7-benzothiazolyl.

A heteroaryl group may be mono-, bi-, tri-, or polycyclic, preferably mono-, bi-, or tricyclic, more preferably mono- or bicyclic.

The term "heterocyclyl" further refers to heterocyclic groups as defined herein substituted with 1, 2 or 3 substituents selected from the groups consisting of the following: alkyl; haloalkyl, hydroxy (or protected hydroxy); halo; oxo, i.e., =O; amino, mono- or di-substituted (alkyl, cycloalkyl, aryl and/or aryl alkyl) amino such as alkylamino or dialkylamino; alkoxy; cycloalkyl; alkenyl; carboxy; heterocyclooxy, wherein heterocyclooxy denotes a heterocyclic group bonded through an oxygen bridge; alkyl-O-C(O)--; mercapto; HSO₃; nitro; cyano; sulfamoyl or sulfonamido; aryl; alkyl-C(O)-O--; aryl-C(O)-O--; aryl-S--; cycloalkoxy; alkenyloxy; alkoxycarbonyl; aryloxy; carbamoyl; alkyl-S--; alkyl-SO--, alkyl-SO₂--; formyl, i.e., HC(O)--; aryl-alkyl--; acyl such as alkanoyl; heterocyclyl and aryl substituted with alkyl, cycloalkyl, alkoxy, hydroxy, amino, alkyl-C(O)-NH--, alkylamino, dialkylamino or halogen.

As used herein, the term "cycloalkyl" refers to optionally substituted saturated or unsaturated monocyclic, bicyclic or tricyclic hydrocarbon groups of 3-12 carbon atoms, each of which may be substituted by one or more substituents, such as alkyl, halo, oxo, hydroxy, alkoxy, alkanoyl, acylamino, carbamoyl, alkyl-NH--, (alkyl)₂N--, thiol, alkylthio, nitro, cyano, carboxy, alkyl-O-C(O)--, sulfonyl, sulfonamido, sulfamoyl, heterocyclyl and the like. Exemplary monocyclic hydrocarbon groups include, but are not limited to, cyclopropyl, cyclobutyl, cyclopentyl, cyclopentenyl, cyclohexyl and cyclohexenyl and the like. Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl,.bicyclo[2.2.2]octyl and the like. Exemplary tricyclic hydrocarbon groups include adamantyl and the like.

As used herein, the term "sulfamoyl" refers to H₂NS(O)₂-, alkyl-NHS(O)₂-, (alkyl)₂NS(O)₂-, aryl-NHS(O)₂-, alkyl(aryl)-NS(O)₂-, (aryl)₂NS(O)₂-, heteroaryl-NHS(O)₂-, aryl-alkyl-NHS(O)₂-, heteroaryl-alkyl-NHS(O)₂- and the like.

As used herein, the term "aryloxy" refers to both an --O-aryl and an --O- heteroaryl group, wherein aryl and heteroaryl are defined herein.

As used herein, the term "halogen" or "halo" refers to fluoro, chloro, bromo, and iodo.

As used herein, the term "haloalkyl" refers to an alkyl as defined herein, that is substituted by one or more halo groups as defined herein. Preferably the haloalkyl can be monohaloalkyl, dihaloalkyl or polyhaloalkyl including perhaloalkyl. A monohaloalkyl can have one iodo, bromo, chloro or fluoro within the alkyl group. Dihaloalky and polyhaloalkyl groups can have two or more of the same halo atoms or a combination of different halo groups within the alkyl. Preferably, the polyhaloalkyl contains up to 12, 10, or 8, or 6, or 4, or 3, or 2 halo groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. A perhaloalkyl refers to an alkyl having all hydrogen atoms replaced with halo atoms.

As used herein, the term "monoalkylamino" refers to an amino group which is substituted by one alkyl group.

As used herein, the term "dialkylamino" refers to an amino group which is di-substituted by alkyl, whereby the two alkyl groups can be the same or different, as defined herein. Preferably the dialkylamino can have the same alkyl substitutent. Non-limiting examples of dialkylamino include dimethylamino, diethylamino and diisopropylamino.

As used herein, the term "aryl alkyl" is interchangeable for "aryl-alkyl-", wherein aryl and alkyl are defined herein.

As used herein, the term "cycloalkyl-alkyl-" is interchangeable for "cycloalkyl alkyl", wherein cycloalkyl and alkyl are defined herein.

As used herein, the term "isomers" refers to different compounds that have the same molecular formula. Also as used herein, the term "an optical isomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non- superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and may thus give rise to enantiomers, diastereomers, and other stereoisomeric forms that may be defined, in terms of absolute stereochemistry, as (R)- or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included.

As used herein, the term "pharmaceutically acceptable salts" refers to salts that retain the biological effectiveness and properties of the compounds of this invention and, which are not biologically or otherwise undesirable. Non-limiting examples of the salts include non-toxic, inorganic and organic base or acid addition salts of compounds of the present invention. In many cases, the compounds of the present invention are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p*- toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Lists of additional suitable salts can be found, e.g., in Remington's Pharmaceutical Sciences, 20th ed., Mack Publishing Company, Easton, Pa., (1985), which is herein incorporated by reference.

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289- 1329, incorporated herein by reference). Except insofar as any conventional carrier is incompatible with the active ingredient, its use in the therapeutic or pharmaceutical compositions is contemplated.

The term "therapeutically effective amount" of a compound of the present invention refers to an amount of the compound of the present invention that will elicit the biological or medical response of a subject, or ameliorate symptoms, slow or delay disease progression, or prevent a disease, etc. In a preferred embodiment, the "effective amount" refers to the amount that inhibits or reduces expression or activity of CETP.

As used herein, the term "subject" refers to an animal. Preferably, the animal is a mammal. A subject also refers to for example, primates (e.g., humans), cows, sheep, goats, horses, dogs, cats, rabbits, rats, mice, fish, birds and the like. In a preferred embodiment, the subject is a human.

As used herein, the term "a disorder" or " a disease" refers to any derangement or abnormality of function; a morbid physical or mental state. See Dorland's Illustrated Medical Dictionary, (W.B. Saunders Co. 27th ed. 1988).

As used herein, the term "inhibition" or "inhibiting" refers to the reduction or suppression of a given condition, symptom, or disorder, or disease, or a significant decrease in the baseline activity of a biological activity or process. Preferably, the condition or symptom or disorder or disease is mediated by CETP activity or responsive to the inhibition of CETP.

As used herein, the term "treating" or "treatment" of any disease or disorder refers in one embodiment, to ameliorating the disease or disorder (i.e., arresting or reducing the development of the disease or at least one of the clinical symptoms thereof). In another embodiment "treating" or "treatment" refers to ameliorating at least one physical parameter, which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. In yet another embodiment, "treating" or "treatment" refers to preventing or delaying the onset or development or progression of the disease or disorder.

As used herein, the term "a," "an," "the" and similar terms used in the context of the present invention (especially in the context of the claims) are to be construed to cover both the singular and plural unless otherwise indicated herein or clearly contradicted by the context. Recitation of ranges of values herein are merely intended to serve as a shorthand method of referring individually to each separate value falling within the range. Unless otherwise indicated herein, each individual value is incorporated into the specification as if it are individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention. In one embodiment, the invention is related to a compound of formula I wherein
R1 is heterocyclyl, aryl, alkoxycarbonyl, alkanoyl, or alkyl, wherein each heterocyclyl or aryl is optionally substituted with one to three substituents selected from alkyl, haloalkyl, hydroxy, halogen, nitro, carboxy, thiol, cyano, HSO₃--, cycloalkyl, alkenyl, alkoxy, cycloalkoxy, alkenyloxy, alkoxycarbonyl, carbamoyl, alkyl-S--, alkyl-SO--, alkyl-SO₂--, amino, H₂N-SO₂--, alkanoyl, or heterocyclyl; and wherein each alkanoyl, alkoxycarbonyl, or alkyl is optionally substituted with one to three substituents selected from hydroxy, halogen, nitro, carboxy, thiol, cyano, HSO₃--, cycloalkyl, alkenyl, alkoxy, cycloalkoxy, alkenyloxy, alkoxycarbonyl, carbamoyl, alkyl-S--, alkyl-SO--, alkyl-SO₂--, amino, H₂N-SO₂--,alkanoyl, or heterocyclyl; R2 is alkyl;
R3 is R8-O-C(O)--, (R8)(R9)N-C(O)--, R8-C(O)-, R8-S(O)₂--, alkyl, cycloalkyl, or aryl-alkyl-; R4 or R5 are independently of each other hydrogen, aryl-alkyl-, cycloalkyl-alkyl- or heteroaryl-alkyl-, wherein each aryl, cycloalkyl or heteroaryl is optionally substituted with one to three substituents selected from alkyl, haloalkyl, hydroxy, halogen, nitro, carboxy, thiol, cyano, HSO₃--, cycloalkyl, alkenyl, alkoxy, haloalkoxy, cycloalkoxy, alkenyloxy, alkoxycarbonyl, alkyl-S--, alkyl-SO--, alkyl-SO₂--, amino, mono- or di-substituted (alkyl, cycloalkyl, aryl and/or aryl-alkyl-) amino; H₂N-SO₂--,or alkanoyl;
R6 and R7 are independently hydrogen, alkyl, haloalkyl, halogen, cyano, nitro, hydroxy, dialkylamino or alkoxy; or
R6 is aryl or heteroaryl;
R8 is hydrogen, alkyl, cycloalkyl, aryl, aryl-alkyl- or cycloalkyl-alkyl-; or
a pharmaceutically acceptable salt thereof; or an optical isomer thereof; or a mixture of optical isomers.

Preferred definitions for R1

Preferably, R1 is heterocyclyl, aryl, alkoxycarbonyl, alkanoyl, or alkyl, wherein each heterocyclyl or aryl is optionally substituted with one to three substituents selected from alkyl, haloalkyl, hydroxy, halogen, nitro, carboxy, thiol, cyano, HSO₃--, cycloalkyl, alkenyl, alkoxy, cycloalkoxy, alkenyloxy, alkoxycarbonyl, carbamoyl, alkyl-S--, alkyl-SO--, alkyl-SO₂--, amino, H₂N-SO₂--,alkanoyl, or heterocyclyl; and wherein each alkanoyl, alkoxycarbonyl, or alkyl is optionally substituted with one to three substituents selected from hydroxy, halogen, nitro, carboxy, thiol, cyano, HSO₃--, cycloalkyl, alkenyl, alkoxy, cycloalkoxy, alkenyloxy, alkoxycarbonyl, carbamoyl, alkyl-S--, alkyl-SO--, alkyl-SO₂--, amino, H₂N-SO₂--,alkanoyl, or heterocyclyl. More preferably, R1 is heterocyclyl, such as heteroaryl, alkanoyl or alkoxycarbonyl, wherein each heterocyclyl is optionally substituted with one to three substituents selected from alkyl, hydroxy, halogen, nitro, carboxy, thiol, cyano, HSO₃--, cycloalkyl, alkenyl, alkoxy, cycloalkoxy, alkenyloxy, alkoxycarbonyl, carbamoyl, alkyl-S--, alkyl-SO--, alkyl-SO₂--, amino, H₂N-SO₂--,alkanoyl, or heterocyclyl, more preferably alkyl, hydroxy, halogen, carboxy, alkoxy, amino, alkanoyl or heterocyclyl. Preferred examples for the heterocyclyl substituent of the heterocyclyl moiety for R1 is a 5- to 6-membered, preferably fully saturated ring containing at least one heteroatom seleceted from O, N or S, more preferably N, most preferably it is morpholinyl.

A preferred meaning of variable R1 is heteroaryl as preferably represented by formulae 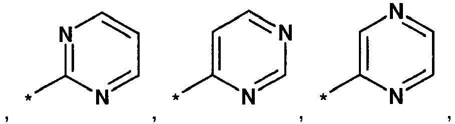 or pyridyl, especially 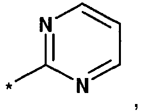 which are each unsubstituted or substituted by C₁-C₄-alkyl, especially methyl or halo, especially Br, or is C₁-C₄-alkoxy-carbonyl, C₁-C₄-alkyl-carbonyl, or is hereocyclyl such as , piperidyl, piperazinyl or morpholinyl, especially morpholinyl.

Preferred Definitions for R2

Preferably, R2 is straight chain or branched C₁-C₆ alkyl as defined herein. Examples include methyl, ethyl, isopropyl, n-propyl, isobutyl, n-butyl or sec-butyl, more preferably ethyl or isobutyl, most preferably ethyl.

Preferred Definitions for R3

Preferably R3 is alkyl-O-C(O)--, cycloalkyl-O-C(O)--, aryl-alkyl-O-C(O)--, (alkyl)₂N-C(O)--, alkanoyl, aryl-alkyl-C(O)-, alkyl-S(O)₂--, aryl-S(O)₂--, alkyl, or aryl-alkyl-.

Preferred examples of alkyl-O-C(O)-- include moieties where alkyl is selected from straight chain or branched, preferably branched, C₁-C₆ alkyl as defined herein, such as methyl, ethyl, isopropyl, isobutyl or tert-butyl, most preferably isopropyl.

Preferred examples of cycloalkyl-O-C(O)-- include moieties where cycloalkyl is selected from C₃ to C₁₂ cycloalkyl as defined herein, such as cyclohexyl or adamantyl.

Preferred examples of aryl-alkyl-O-C(O)-- include moieties where aryl is selected from C₆ to C₂₀ aryl as defined herein, such as phenyl or naphthyl, more preferably phenyl. Preferred examples of aryl-alkyl- include benzyl, phenethyl, more preferably benzyl.

Preferred examples of (alkyl)₂N-C(O)-- include moieties where alkyl is selected from straight chain or branched, preferably straight chain, C₁-C₆ alkyl as defined herein, such as methyl, ethyl, isopropyl, isobutyl or tert-butyl, most preferably methyl or ethyl.

Preferred examples of alkanoyl include moieties where alkyl is selected from straight chain or branched, preferably branched, C₁-C₆ alkyl as defined herein, such as methyl, ethyl, isopropyl, isobutyl or tert-butyl, most preferably tert-butyl.

Preferred examples of aryl-alkyl-C(O)-- include moieties where aryl is selected from C₆ to C₂₀ aryl as defined herein, such as phenyl or naphthyl, more preferably phenyl. Preferred examples of aryl-alkyl- include benzyl, phenethyl, more preferably benzyl.

Preferred examples of alkyl-S(O)₂-- include moieties where alkyl is selected from straight chain or branched, preferably branched, C₁-C₆ alkyl as defined herein, such as methyl, ethyl, isopropyl, isobutyl or tert-butyl, most preferably methyl.

Preferred examples of aryl-S(O)₂-- include moieties where aryl is selected from C₆ to C₂₀ aryl as defined herein, such as phenyl or naphthyl, more preferably phenyl.

Preferred examples of alkyl include moieties where alkyl is selected from straight chain or branched, preferably branched, C₁-C₆ alkyl as defined herein, such as methyl, ethyl, isopropyl, isobutyl or tert-butyl.

Preferred examples of aryl-alkyl- include moieties where aryl is selected from C₆ to C₂₀ aryl as defined herein, such as phenyl or naphthyl, more preferably phenyl. Preferred examples of aryl-alkyl- include benzyl, phenethyl, more preferably benzyl.

Most preferably, R3 is alkyl-O-C(O)-as defined herein.

Preferred Definitions for R4 and R5

Preferably R4 or R5 are independently of each other hydrogen, alkyl, cycloalkyl, aryl, heteroaryl, aryl-alkyl-, cycloalkyl-alkyl- or heteroaryl-alkyl-, more preferably hydrogen, aryl-alkyl-, cycloalkyl-alkyl- or heteroaryl-alkyl-, wherein each alkyl, is optionally substituted with one to three substituents selected from hydroxy, halogen, nitro, carboxy, thiol, cyano, HSO₃--, cycloalkyl, alkenyl, alkoxy, haloalkoxy, cycloalkoxy, alkenyloxy, alkoxycarbonyl, alkyl-S--, alkyl-SO--, alkyl-SO₂--, amino, mono- or di-substituted (alkyl, cycloalkyl, aryl and/or aryl-alkyl-) amino; H₂N-SO₂--, or alkanoyl, and wherein each aryl, cycloalkyl or heteroaryl is optionally substituted with one to three substituents selected from alkyl, haloalkyl, hydroxy, halogen, nitro, carboxy, thiol, cyano, HSO₃--, cycloalkyl, alkenyl, alkoxy, haloalkoxy, cycloalkoxy, alkenyloxy, alkoxycarbonyl, alkyl-S--, alkyl-SO--, alkyl-SO₂--, amino, mono- or di-substituted (alkyl, cycloalkyl, aryl and/or aryl-alkyl-) amino; H₂N-SO₂--, or alkanoyl.

More preferably R4 or R5 are independently of each other hydrogen, benzyl, or cycloalkyl-CH₂--, wherein each benzyl or cycloalkyl is optionally substituted with one to three substituents selected from alkyl, haloalkyl, hydroxy, halogen, nitro, carboxy, thiol, cyano, HSO₃--, alkoxy, haloalkoxy, amino, mono- or di-substituted (alkyl, cycloalkyl, aryl and/or aryl-alkyl-) amino; H₂N-SO₂--,or alkanoyl.

In one embodiment, one of R4 and R5, preferably R5, is hydrogen and the other, preferably R4, is a group as defined herein other than hydrogen.

In another embodiment, both R4 and R5 are hydrogen.

Most preferably, R4 is benzyl. It is also preferred that in this case R5 is hydrogen.

Preferred Definitions for R6 and R7

Preferably, R6 and R7 are independently hydrogen, alkyl, haloalkyl, halogen, or alkoxy. More preferably, R6 and R7 are independently hydrogen, alkyl or haloalkyl, such as trifluoromethyl.

In one embodiment, one of R6 and R7 is hydrogen and the other is a group as defined herein other than hydrogen.

In another preferred embodiment, both R6 and R7 are the same and are as defined herein, most preferably trifluoromethyl.

The positions of R6 and R7 on the phenyl ring are preferably as follows:

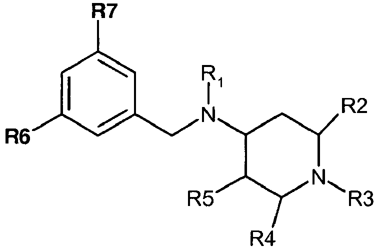

Any asymmetric carbon atom on the compounds of the present invention can be present in the *(R)-,* (S)- or (R,S)- configuration, preferably in the (R)- or (S)- configuration. Substituents at atoms with unsaturated bonds may, if possible, be present in *cis-* (Z)- or *trans-* (*E*)- form. Therefore, the compounds of the present invention can be in the form of one of the possible isomers or mixtures thereof, for example, as substantially pure geometric (*cis* or *trans)* isomers, diastereomers, optical isomers (antipodes), racemates or mixtures thereof. Preferred isomers of the compound of the present invention can be represented by the following formula: 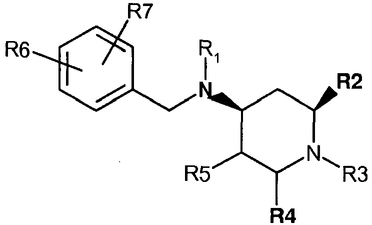 in particular:

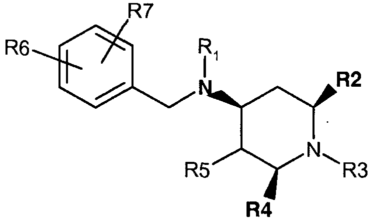

Any resulting mixtures of isomers can be separated on the basis of the physicochemical differences of the constituents, into the pure geometric or optical isomers, diastereomers, racemates, for example, by chromatography and/or fractional crystallization.

Any resulting racemates of final products or intermediates can be resolved into the optical antipodes by known methods, e.g., by separation of the diastereomeric salts thereof, obtained with an optically active acid or base, and liberating the optically active acidic or basic compound. In particular, the piperidine moiety may thus be employed to resolve the compounds of the present invention into their optical antipodes, e.g., by fractional crystallization of a salt formed with an optically active acid, e.g., tartaric acid, dibenzoyl tartaric acid, diacetyl tartaric acid, di-*O*,*O'*-*p*-toluoyl tartaric acid, mandelic acid, malic acid or camphor-10-sulfonic acid. Racemic products can also be resolved by chiral chromatography, e.g., high pressure liquid chromatography (HPLC) using a chiral adsorbent. Finally, compounds of the present invention are either obtained in the free form, as a salt thereof, or as prodrug derivatives thereof.

When a basic group is present in the compounds of the present invention, the compounds can be converted into acid addition salts thereof, in particular, acid addition salts with the imidazolyl moiety of the structure, preferably pharmaceutically acceptable salts thereof. These are formed, with inorganic acids or organic acids. Suitable inorganic acids include but are not limited to, hydrochloric acid, sulfuric acid, a phosphoric or hydrohalic acid. Suitable organic acids include but are not limited to, carboxylic acids, such as (C₁-C₄)alkanecarboxylic acids which, for example, are unsubstituted or substituted by halogen, e.g., acetic acid, such as saturated or unsaturated dicarboxylic acids, e.g., oxalic, succinic, maleic or fumaric acid, such as hydroxycarboxylic acids, e.g., glycolic, lactic, malic, tartaric or citric acid, such as amino acids, e.g., aspartic or glutamic acid, organic sulfonic acids, such as (C₁-C₄)alkylsulfonic acids, e.g., methanesulfonic acid; or arylsulfonic acids which are unsubstituted or substituted, e.g., by halogen. Preferred are salts formed with hydrochloric acid, methanesulfonic acid and maleic acid.

When an acidic group is present in the compounds of the present invention, the compounds can be converted into salts with pharmaceutically acceptable bases. Such salts include alkali metal salts, like sodium, lithium and potassium salts; alkaline earth metal salts, like calcium and magnesium salts; ammonium salts with organic bases, e.g., trimethylamine salts, diethylamine salts, *tris*(hydroxymethyl)methylamine salts, dicyclohexylamine salts and *N*-methyl-*D*-glucamine salts; salts with amino acids like arginine, lysine and the like. Salts may be formed using conventional methods, advantageously in the presence of an ethereal or alcoholic solvent, such as a lower alkanol. From the solutions of the latter, the salts may be precipitated with ethers, e.g., diethyl ether. Resulting salts may be converted into the free compounds by treatment with acids. These or other salts can also be used for purification of the compounds obtained.

When both a basic group and an acid group are present in the same molecule, the compounds of the present invention can also form internal salts.

The present invention also provides pro-drugs of the compounds of the present invention that converts *in vivo to* the compounds of the present invention. A pro-drug is an active or inactive compound that is modified chemically through *in vivo* physiological action, such as hydrolysis, metabolism and the like, into a compound of this invention following administration of the prodrug to a subject. The suitability and techniques involved in making and using pro-drugs are well known by those skilled in the art. Prodrugs can be conceptually divided into two non-exclusive categories, bioprecursor prodrugs and carrier prodrugs. See The Practice of Medicinal Chemistry, Ch. 31-32 (Ed. Wermuth, Academic Press, San Diego, Calif., 2001). Generally, bioprecursor prodrugs are compounds are inactive or have low activity compared to the corresponding active drug compound, that contains one or more protective groups and are converted to an active form by metabolism or solvolysis. Both the active drug form and any released metabolic products should have acceptably low toxicity. Typically, the formation of active drug compound involves a metabolic process or reaction that is one of the follow types:
1. Oxidative reactions, such as oxidation of alcohol, carbonyl, and acid functions, hydroxylation of aliphatic carbons, hydroxylation of alicyclic carbon atoms, oxidation of aromatic carbon atoms, oxidation of carbon-carbon double bonds, oxidation of nitrogen-containing functional groups, oxidation of silicon, phosphorus, arsenic, and sulfur, oxidative N-delakylation, oxidative O- and S-delakylation, oxidative deamination, as well as other oxidative reactions.
2. Reductive reactions, such as reduction of carbonyl groups, reduction of alcoholic groups and carbon-carbon double bonds, reduction of nitrogen-containing functions groups, and other reduction reactions.
3. Reactions without change in the state of oxidation, such as hydrolysis of esters and ethers, hydrolytic cleavage of carbon-nitrogen single bonds, hydrolytic cleavage of non-aromatic heterocycles, hydration and dehydration at multiple bonds, new atomic linkages resulting from dehydration reactions, hydrolytic dehalogenation, removal of hydrogen halide molecule, and other such reactions.

Carrier prodrugs are drug compounds that contain a transport moiety, e.g., that improve uptake and/or localized delivery to a site(s) of action. Desirably for such a carrier prodrug, the linkage between the drug moiety and the transport moiety is a covalent bond, the prodrug is inactive or less active than the drug compound, and any released transport moiety is acceptably non-toxic. For prodrugs where the transport moiety is intended to enhance uptake, typically the release of the transport moiety should be rapid. In other cases, it is desirable to utilize a moiety that provides slow release, e.g., certain polymers or other moieties, such as cyclodextrins. See, Cheng et al., US20040077595, application Ser. No. 10/656,838, incorporated herein by reference. Such carrier prodrugs are often advantageous for orally administered drugs. Carrier prodrugs can, for example, be used to improve one or more of the following properties: increased lipophilicity, increased duration of pharmacological effects, increased site-specificity, decreased toxicity and adverse reactions, and/or improvement in drug formulation (e.g., stability, water solubility, suppression of an undesirable organoleptic or physiochemical property). For example, lipophilicity can be increased by esterification of hydroxy groups with lipophilic carboxylic acids, or of carboxylic acid groups with alcohols, e.g., aliphatic alcohols. Wermuth, The Practice of Medicinal Chemistry, Ch. 31-32, Ed. Werriuth, Academic Press, San Diego, Calif., 2001.

Exemplary prodrugs are, e.g., esters of free carboxylic acids and *S*-acyl and *O*-acyl derivatives of thiols, alcohols or phenols, wherein acyl has a meaning as defined herein. Preferred are pharmaceutically acceptable ester derivatives convertible by solvolysis under physiological conditions to the parent carboxylic acid, e.g., lower alkyl esters, cycloalkyl esters, lower alkenyl esters, benzyl esters, mono- or di-substituted lower alkyl esters, such as the ω-(amino, mono- or di-lower alkylamino, carboxy, lower alkoxycarbonyl)-lower alkyl esters, the α-(lower alkanoyloxy, lower alkoxycarbonyl or di-lower alkylaminocarbonyl)-lower alkyl esters, such as the pivaloyloxymethyl ester and the like conventionally used in the art. In addition, amines have been masked as arylcarbonyloxymethyl substituted derivatives which are cleaved by esterases *in vivo* releasing the free drug and formaldehyde (Bundgaard, J. Med. Chem. 2503 (1989)). Moreover, drugs containing an acidic NH group, such as imidazole, imide, indole and the like, have been masked with N-acyloxymethyl groups (Bundgaard, Design of Prodrugs, Elsevier (1985)). Hydroxy groups have been masked as esters and ethers. EP 039,051 (Sloan and Little) discloses Mannich-base hydroxamic acid prodrugs, their preparation and use.

In view of the close relationship between the compounds, the compounds in the form of their salts and the pro-drugs, any reference to the compounds of the present invention is to be understood as referring also to the corresponding pro-drugs of the compounds of the present invention, as appropriate and expedient.

Furthermore, the compounds of the present invention, including their salts, can also be obtained in the form of their hydrates, or include other solvents used for their crystallization.

The compounds of the present invention have valuable pharmacological properties. The compounds of the present invention are useful as inhibitors for cholesteryl ester transfer protein (CETP). CETP is a 74KD glycopeptide, it is secreted by the liver and is a key player in facilitating the transfer of lipids between the various lipoproteins in plasma. The primary function of CETP is to redistribute cholesteryl esters (CE) and triglycerides between lipoproteins. See Assmann, G et al., "HDL cholesterol and protective factors in atherosclerosis," Circulation, 109: 1118-1114 (2004). Because most triglycerides in plasma originate in VLDLs and most CEs are formed in HDL particles in the reaction catalyzed by lecithin:cholesterol acyltransferase, activity of CETP results in a net mass transfer of triglycerides from VLDLs to LDLs and HDLs and a net mass transfer of CEs from HDLs to VLDLs and LDLs. Thus, CETP potentially decreases HDL-C levels, increases LDL-cholesteryl (LDL-C) levels and reduces HDL and LDL particles size, and inhibition of CETP could be a therapeutic strategy for raising HDL-cholesteryl (HDL-C), have a favorable impact on the lipoprotein profile, and reduce the risk of cardiovascular diseases. Accordingly, the compounds of the present invention as CETP inhibitors are useful for the delay of progression and/or treatment of a disorder or disease that is mediated by CETP or responsive to inhibition of CETP. Disorders, conditions and diseases that can be treated with the compounds of the present invention include but are not limited to, hyperlipidemia, arteriosclerosis, atherosclerosis, peripheral vascular disease, dyslipidemia, hyperbetalipoproteinemia, hypoalphalipoproteinemia, hypercholesterolemia, hypertriglyceridemia, familial hypercholesterolemia, cardiovascular disorder, coronary heart disease, coronary artery disease, coronary vascular disease, angina, ischemia, heart ischemia, thrombosis, cardiac infarction such as myocardial infarction, stroke, peripheral vascular disease, reperfusion injury, angioplasty restenosis, hypertension, congestive heart failure, diabetes such as type II diabetes mellitus, diabetic vascular complications, obesity, infection or egg embryonation of schistosoma, or endotoxemia etc..

Additionally, the present invention provides:
- a compound of the present invention as described herein above for use as a medicament;
- the use of a compound of the present invention as described herein above for the preparation of a pharmaceutical composition for the delay of progression and/or treatment of a disorder or disease mediated by CETP, or responsive to inhibition of CETP.
- the use of a compound of the present invention as described herein above for the preparation of a pharmaceutical composition for the delay of progression and/or treatment of a disorder or disease selected from hyperlipidemia, arteriosclerosis, atherosclerosis, peripheral vascular disease, dyslipidemia, hyperbetalipoproteinemia, hypoalphalipoproteinemia, hypercholesterolemia, hypertriglyceridemia, familial hypercholesterolemia, cardiovascular disorder, coronary heart disease, coronary artery disease, coronary vascular disease, angina, ischemia, heart ischemia, thrombosis, cardiac infarction such as myocardial infarction, stroke, peripheral vascular disease, reperfusion injury, angioplasty restenosis, hypertension, congestive heart failure, diabetes such as type II diabetes mellitus, diabetic vascular complications, obesity or endotoxemia etc.

The compounds of formula (I) can be prepared by the procedures described in the following sections.

Generally, the compounds of formula (I) can be prepared according to the following general procedures and schemes. In all these Schemes the variants R1, R2, R3, R4, R5, R6, R7 and R8 have the meaning as set forth herein unless defined otherwise.

### 1. General procedure A: using piperidone A1

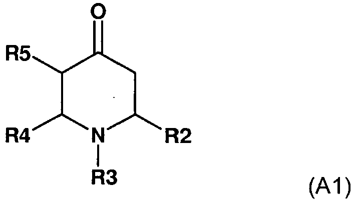

1.1. Route AI when R4 and R5 are hydrogen: 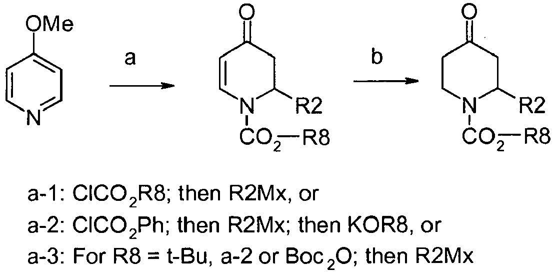 wherein R8 is as defined herein e.g. *t*-Bu, *i*-Pr, Bn, 2,2,2-trichloroethyl, allyl, Mx is e.g. MgBr, Mgl, MgCl, Li, also combination with ZnCl₂.
   In step b) standard conditions for 1,4-reductions may be employed, such as Mg, alcohol; CeCl₃, NaBH₄ or catalytic hydrogenation.
1.2. Route All when R4 and R5 are hydrogen: 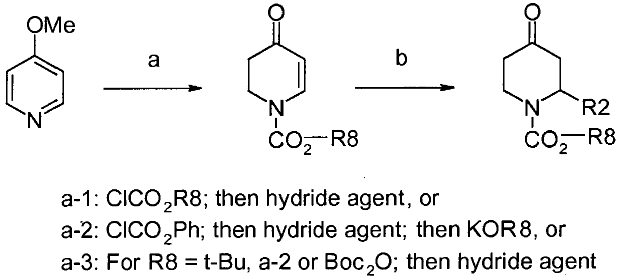 wherein R8 is as defined herein e.g. *t*-Bu, *i*-Pr, Bn, 2,2,2-trichloroethyl, allyl. Suitable hydride agents that can be used are such as NaBH(OAc)₃, NaBH₃CN, NaBH₄, or LiBH₄, K(O*i*Pr)₃BH, NaB[CH(CH₃)C₂H₅]₃H, or NaAlH(OCH₂CH₂OCH₃)₂.
   In step b) standard conditions for 1,4-additions are employed such as R2MgX (X= halo), Cul or R2₂Zn, cat. Cu species.
1.3. Route AIII when R5 is hydrogen: 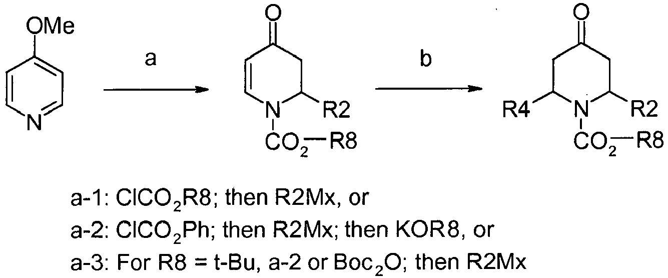 wherein R8 is as defined herein e.g. *t*-Bu, *i*-Pr, Bn, 2,2,2-trichloroethyl, allyl, Mx is e.g. MgBr, Mgl, MgCl, Li, also combination with ZnCl₂.
   In step b) standard conditions for 1,4-additions are employed such as R4MgX (X= halo), Cul or R4₂Zn, cat. Cu species.
1.4. Route AIV when R5 is hydrogen: 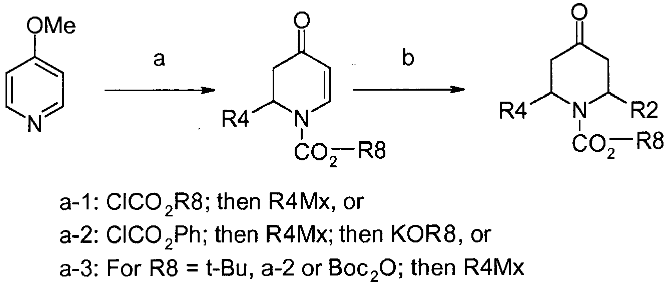 wherein R8 is as defined herein e.g. *t*-Bu, *i*-Pr, Bn, 2,2,2-trichloroethyl, allyl, Mx is e.g. MgBr, Mgl, MgCl, Li, also combination with ZnCl₂.
   In step b) standard conditions for 1,4-additions are employed such as R2MgX (X= halo), Cul or R2₂Zn, cat. Cu species.
1.5. Route AV when R4 is hydrogen: 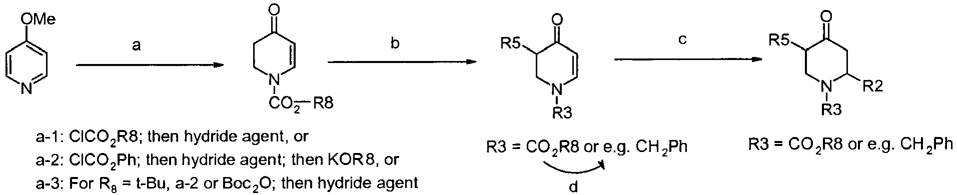 wherein R8 is as defined herein. Suitable hydride agents that can be used are such as NaBH(OAc)₃, NaBH₃ CN, NaBH₄, or LiBH₄, K(O*i*Pr)₃BH, NaB[CH(CH₃)C₂H₅]₃H, or NaAlH(OCH₂CH₂OCH₃)₂.
   In step b) standard conditions for alkylations are employed, such as strong base and a halide LDA, R5X or LHMDS or KHMDS, R5X (X = halogen or OMs, OTs, OTf).
   In step c) standard conditions for 1,4-additions are employed such as R2MgX (X= halo), Cul or R2₂Zn, cat. Cu species.
   Conversion of R3 can be effected by standard functional group manipulation as well known in the art or as specifically described herein.
1.6. Route AVI when R4 is hydrogen: 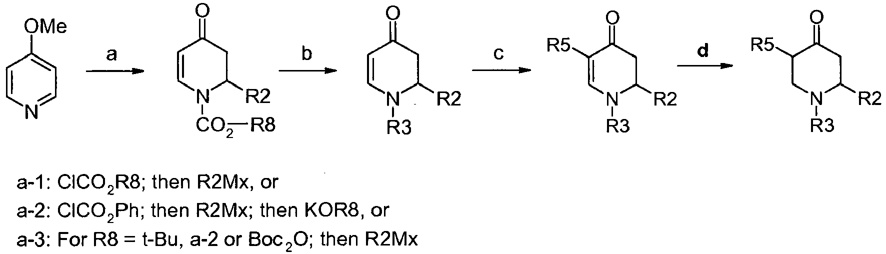 wherein R8 and R3 are as defined herein; Mx is e.g. MgBr, Mgl, MgCl, Li, also combination with ZnCl₂.
   In step b) the conversion of R3 can be effected by standard functional group manipulation as well known in the art or as specifically described herein.
   In step c) standard conditions for enamine alkylations are employed, such as R5X (X = halogen or OMs, OTs, OTf); heat; or I₂ and the use of a base to form a vinyl iodide followed by cross-coupling conditions such as Suzuki, Stille, Negishi or Kumada as described e.g. in standard textbooks.
   In step d) standard conditions for 1,4-reductions may be employed, such as Mg, alcohol; CeCl₃, NaBH₄, or catalytic hydrogenation.
1.7. Route AVII: 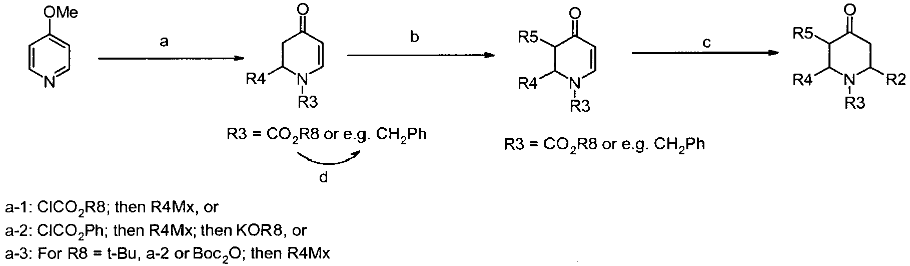 wherein R8 and R3 are as defined herein; Mx is e.g. MgBr, Mgl, MgCl, Li, also combination with ZnCl₂.
   In step b) standard conditions for alkylations are employed, such as strong base and a halide; e.g. LDA, R4X or LHMDS or KHMDS, R4X (X = halogen or OMs, OTs, OTf).
   In step c) standard conditions for 1,4-additions are employed such as R5MgX (X= halo), Cul or R5₂Zn, cat. Cu species.
   In step d) the conversion of R3 can be effected by standard functional group manipulation as well known in the art or as specifically described herein.
   Using any of the routes AI to AVII above, the piperidone A1 can be converted into the compound of formula (I) using one of the routes AVIII, AIX or AX shown below.
1.8. Route AVIII: 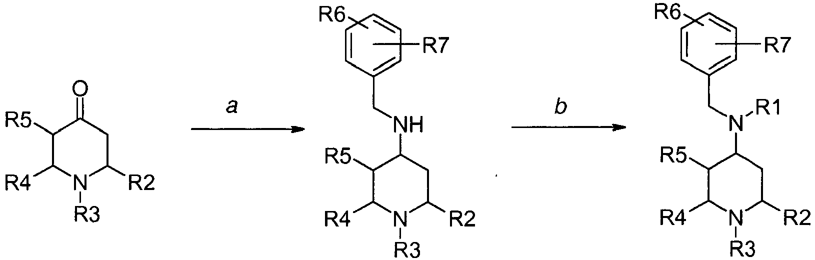 In step a) standard methods for reductive amination are employed, such as ArCH₂NH₂, hydride reagent [ex. NaBH(OAc)₃, NaBH₃CN, NaBH₄, LiBH₄, BH₃, picoline borane, borane-pyridine complex]; or Ti(OiPr)₄; then hydride reagent such as NaBH(OAc)₃, NaBH₃ CN, NaBH₄, LiBH₄, borane, picoline borane, borane-pyridine complex, LiAlH₄, 9-BBN, Alpine borane^{®}, LiB(s-Bu)₃H, LiB(Sia)₃H; or imine formation catalyzed or uncatalyzed by acid followed by reduction by hydride agents (see above).
   In step b), group R1 is introduced by usual functional group manipulation in the amine, such as alkylation, carbamate formation, urea formation, SRN1 substitution, aryl amination and reductive amination.
   The group R3 may be modified at an appropriate stage to have the desired definition as set forth in the claims be standard nitrogen protecting group chemistry as known in the art or as described herein.
1.9. Route AIX:

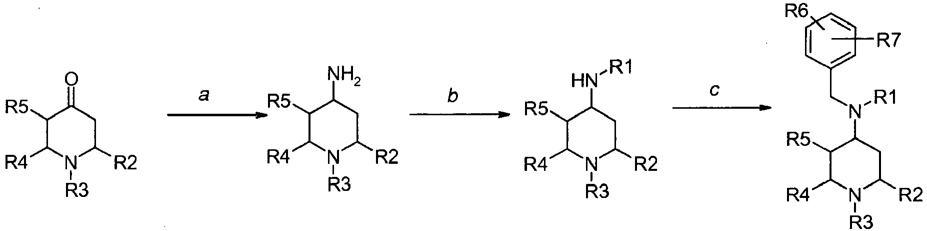

In step a) standard methods for the introduction of the primary amine are employed, such as using:
- an NH₃ equivalent [e.g. NH₃/EtOH, NH₄Cl, NH₄OH], a hydride reagent [e.g. NaBH(OAc)₃, NaBH₃ CN or a combination of Ti(OiPr)₄ with hydride agents such as NaBH₄]
- i) either simultaneous treatment with or stepwise treatment via imine formation with BnNH₂, a hydride reagent (see above), ii) cat. Hydrogenation
- a treatment with BnNH₂ under cat. Hydrogenation condition
- i) either simultaneous treatment with or stepwise treatment via imine formation with PMBNH₂, hydride reagent (see above), ii) CAN or DDQ (oxidative debenzylation) or TFA
- i) either simultaneous treatment with or stepwise treatment via imine formation with Ph₂CHNH₂ (benzhydrylamine), hydride reagent (see above), ii) deprotection with TFA/Et₃SiH or cat. Hydrogenation
- i) RONH₂ [oxime formation] ii) Na or BH₃ or cat. hydrogenation (e.g. Ra-Ni, Pd-C, Pt-C) [reduction of oxime] whereby R is for example benzyl, p-methoxybenzyl, or allyl.
- i) a hydride reagent [reduction to alcohol] ii) Mitsunobu condition using PPh₃, DEAD, N₃ anion or mesylation with MsCl and base then N₃ anion or bromination with conditions such as NBS/PPh₃, PBr₃/PPh₃,CBr₄/PPh₃ then N₃ anion or PBr₃/PPh₃ then N₃ anion iii) PR₃ or cat. Hydrogenation [reduction of azide] whereby R is for example ethyl or phenyl

In steps b) and c), group R1 or the benzyl ring, respectively, are introduced by usual functional group manipulation in the amine, such as alkylation, carbamate formation, urea formation, SRN1 substitution, aryl amination and reductive amination for step b) and preferably alkylation and reductive amination for step c).

The group R3 may be modified at an appropriate stage to have the desired definition as set forth in the claims be standard nitrogen protecting group chemistry as known in the art or as described herein.
1.10. Route AX: 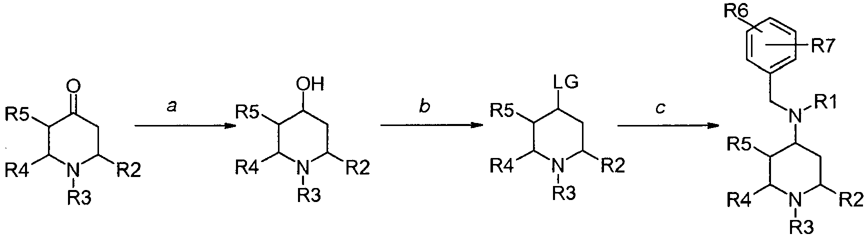 wherein LG is a leaving group such as a mesylate, tosylate, triflate or bromide.
   In step a) standard methods to reduce the carbonyl group are employed, such as the use of a hydride agent, e.g. NaBH₄ or K-Selectride.
   In step b) standard methods for the conversion of the alcohol to a leaving group (LG; e.g. a mesylate, tosylate, or bromide) are employed. The methods include the use of MsCl/base or TsCl/base or SOCl₂ or NBS/PPh₃ or CBr₄/PPh₃ or Tf₂O using conditions well known in the art.
   In step c) the amine unitis introduced using standard substitution chemistry, e.g. by employing the secondary amine and a strong base such as NaH, KO*t*Bu, LHMDS.
   The group R3 may be modified at an appropriate stage to have the desired definition as set forth in the claims be standard nitrogen protecting group chemistry as known in the art or as described herein.

### 2. General procedure B: using Ritter-type chemistry

Details for preparing benzyl-substituted piperidine B1 can be found in Bioorganic & Medical Chemistry Letters, Vol. 6, No. 24, pp.3029-3034, 1996. The methods described therein could be applied analogously obtaining substituted piperidines.

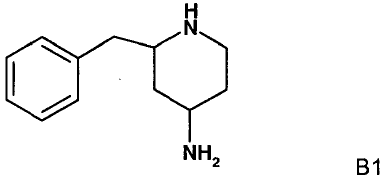

This piperidine could also be further reacted to form a compound of formula (I) by alkylation methods and nitrogen protecting group manipulations as described above in the procedure A.

### 3. General procedure C: using Dieckmann chemistry

Compounds of formula (I) can be prepared following the synthetic route outlined in Journal of Medicinal Chemistry, 2001, Vol. 44, No. 6, pp. 972-987 either directly or analogously.

### 4. General procedure D: using Mannich chemistry

Compounds of formula (I) can be prepared via intramolecular Mannich reaction either directly or analogously and converting the obtained piperidone by methods outlined in e.g. routes AVIII, AIX or AX above. An illustrative example of this chemistry is outlined in Heterocycles, 2002, Vol. 57, No. 10, pp. 1807-1830. An asymmetric variant is outlined in Organic Letters, 2001, Vol. 3, No. 20, 3169-3171.

### 5. General procedure E: using Aza-Michael chemistry

Compounds of formula (I) can be prepared via intramolecular Aza-Michael reaction either directly or analogously and converting the obtained piperidone by methods outlined in e.g. routes AVIII, AIX or AX above. An Illustrative example of this chemistry is outlined in Journal of Organic Chemistry, 2005, Vol. 70, pp. 169-174.

### 6. General procedure F: using double Aza-Michael chemistry from divinyl ketones

Compounds of formula (I) can be prepared via intramolecular double Aza-Michael reaction either directly or analogously and converting the obtained piperidone by methods outlined in e.g. routes AVIII, AIX or AX above. An Illustrative example of this chemistry is outlined in Journal of Organic Chemistry, 1992, Vol. 57, pp. 5809-5810.

### 7. General procedure G: using direct-lithiation chemistry

Compounds of formula (I) can be prepared via direct lithiation of pyperidine either directly or analogously and converting the obtained piperidone by methods outlined in e.g. routes AVIII, AIX or AX above. An illustrative example of this chemistry is outlined in Journal of Organic Chemistry, 1990, Vol. 55, pp. 2578-2580. The protected piperidone thus obtained may be readily converted to the corresponding piperidone by methods well-known in the art.

### 8. General procedure H: using Diels- Alder chemistry

Compounds of formula (I) can be prepared be following the synthetic routes outlined in Advanced Synthesis & Catalysis, 2006, Vol. 348, 2443-2448 or Journal of the American Chemical Society, 2007, ASAP (W.D. Wulff et al. "Regulation of Orthogonal Functions in a Dual Catalyst System...") either directly or analogously and converting the obtained piperidone by methods outlined in e.g. routes AVIII, AIX or AX above.

### 9. General procedure I: preparation from substituted pyrone

Compounds of formula (I) can be prepared from substituted pyrone analogously and converting the obtained pyridone by methods outlined in e.g. routes AVIII, AIX or AX above. An illustrative example of piperidinol preparation from pyrone is outlined in Journal of Organic Chemistry, 1950, Vol. 15, No. 2, pp. 337-342. The piperidinol thus obtained may be readily converted to the corresponding piperidone by methods well-known in the art.. Racemates and diastereomer mixtures obtained can be separated into the pure isomers or racemates in a known manner on the basis of the physicochemical differences of the components, for example by fractional crystallization or by chiral chromotagraphy or HPLC separation utilizing chiral stationery phases. Racemates obtained may furthermore be resolved into the optical antipodes by known methods, for example by recrystallization from an optically active solvent, chromatography on chiral adsorbents, with the aid of suitable microorganisms, by cleavage with specific immobilized enzymes, via the formation of inclusion compounds, for example using chiral crown ethers, only one enantiomer being complexed, or by conversion into diastereomeric salts, for example by reaction of a basic final substance racemate with an optically active acid, such as a carboxylic acid, for example tartaric or malic acid, or sulfonic acid, for example camphorsulfonic acid, and separation of the diastereomer mixture obtained in this manner, for example on the basis of its differing solubilities, into the diastereomers from which the desired enantiomer can be liberated by the action of suitable agents. The more active enantiomer is advantageously isolated.

In starting compounds and intermediates which are converted to the compounds of the invention in a manner described herein, functional groups present, such as amino, thiol, carboxyl and hydroxy groups, are optionally protected by conventional protecting groups that are common in preparative organic chemistry. Protected amino, thiol, carboxyl and hydroxy groups are those that can be converted under mild conditions into free amino thiol, carboxyl and hydroxy groups without the molecular framework being destroyed or other undesired side reactions taking place.

The purpose of introducing protecting groups is to protect the functional groups from undesired reactions with reaction components under the conditions used for carrying out a desired chemical transformation. The need and choice of protecting groups for a particular reaction is known to those skilled in the art and depends on the nature of the functional group to be protected (hydroxy group, amino group, etc.), the structure and stability of the molecule of which the substituent is a part and the reaction conditions.

Well-known protecting groups that meet these conditions and their introduction and removal are described, e.g., in McOmie, "Protective Groups in Organic Chemistry", Plenum Press, London, NY (1973); and Greene and Wuts, "Protective Groups in Organic Synthesis", John Wiley and Sons, Inc., NY (1999).

The above-mentioned reactions are carried out according to standard methods, in the presence or absence of diluent, preferably, such as are inert to the reagents and are solvents thereof, of catalysts, condensing or said other agents, respectively and/or inert atmospheres, at low temperatures, room temperature or elevated temperatures, preferably at or near the boiling point of the solvents used, and at atmospheric or super-atmospheric pressure. The preferred solvents, catalysts and reaction conditions are set forth in the appended illustrative Examples.

The invention further includes any variant of the present processes, in which an intermediate product obtainable at any stage thereof is used as starting material and the remaining steps are carried out, or in which the starting materials are formed *in situ* under the reaction conditions, or in which the reaction components are used in the form of their salts or optically pure antipodes.

Compounds of the invention and intermediates can also be converted into each other according to methods generally known *per se.*

In another aspect, the present invention provides a pharmaceutical composition comprising a compound of the present invention and a pharmaceutically acceptable carrier. The pharmaceutical composition can be formulated for particular routes of administration such as oral administration, parenteral administration, and rectal administration, etc. In addition, the pharmaceutical compositions of the present invention can be made up in a solid form including capsules, tablets, pills, granules, powders or suppositories, or in a liquid form including solutions, suspensions or emulsions. The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers etc.

Preferably, the pharmaceutical compositions are tablets and gelatin capsules comprising the active ingredient together with
a) diluents, e.g., lactose, dextrose, sucrose, mannitol, sorbitol, cellulose and/or glycine;
b) lubricants, e.g., silica, talcum, stearic acid, its magnesium or calcium salt and/or polyethyleneglycol; for tablets also
c) binders, e.g., magnesium aluminum silicate, starch paste, gelatin, tragacanth, methylcellulose, sodium carboxymethylcellulose and/or polyvinylpyrrolidone; if desired
d) disintegrants, e.g., starches, agar, alginic acid or its sodium salt, or effervescent mixtures; and/or
e) absorbents, colorants, flavors and sweeteners.

Tablets may be either film coated or enteric coated according to methods known in the art. Suitable compositions for oral administration include an effective amount of a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, preferably about 1-50%, of the active ingredient.

Suitable compositions for transdermal application include an effective amount of a compound of the invention with carrier. Advantageous carriers include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, e.g., to the skin and eyes, include aqueous solutions, suspensions, ointments, creams, gels or sprayable formulations, e.g., for delivery by aerosol or the like. Such topical delivery systems will in particular be appropriate for dermal application, e.g., for the treatment of skin cancer, e.g., for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

The present invention further provides anhydrous pharmaceutical compositions and dosage forms comprising the compounds of the present invention as active ingredients, since water can facilitate the degradation of some compounds. For example, the addition of water (e.g., 5%) is widely accepted in the pharmaceutical arts as a means of simulating long-term storage in order to determine characteristics such as shelf-life or the stability of formulations over time. See, e.g., Jens T. Carstensen, Drug Stability: Principles & Practice, 2d. Ed., Marcel Dekker, NY, N.Y., 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (e. g., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

The invention likewise relates to a combination of a compound of formula (I), (I A) or (I B), respectively, or a pharmaceutically acceptable salt thereof with a further active principle.

The combination may be made for example with the following active principles, selected from the group consisting of a:
(i) HMG-Co-A reductase inhibitor or a pharmaceutically acceptable salt thereof,
(ii) angiotensin II receptor antagonist or a pharmaceutically acceptable salt thereof,
(iii) angiotensin converting enzyme (ACE) Inhibitor or a pharmaceutically acceptable salt thereof,
(iv) calcium channel blocker or a pharmaceutically acceptable salt thereof,
(v) aldosterone synthase inhibitor or a pharmaceutically acceptable salt thereof,
(vi) aldosterone antagonist or a pharmaceutically acceptable salt thereof,
(vii) dual angiotensin converting enzyme/neutral endopeptidase (ACE/NEP) inhibitor or a pharmaceutically acceptable salt thereof,
(viii) endothelin antagonist or a pharmaceutically acceptable salt thereof,
(ix) renin inhibitor or a pharmaceutically acceptable salt thereof,
(x) diuretic or a pharmaceutically acceptable salt thereof;
(xi) an ApoA-I mimic; and
(Xii) a DGAT inhibitor.

An angiotensin II receptor antagonist or a pharmaceutically acceptable salt thereof is understood to be an active ingredients which bind to the AT₁-receptor subtype of angiotensin II receptor but do not result in activation of the receptor. As a consequence of the inhibition of the AT₁ receptor, these antagonists can, for example, be employed as antihypertensives or for treating congestive heart failure.

The class of AT₁ receptor antagonists comprises compounds having differing structural features, essentially preferred are the non-peptidic ones. For example, mention may be made of the compounds which are selected from the group consisting of valsartan, losartan, candesartan, eprosartan, irbesartan, saprisartan, tasosartan, telmisartan, the compound with the designation E-1477 of the following formula 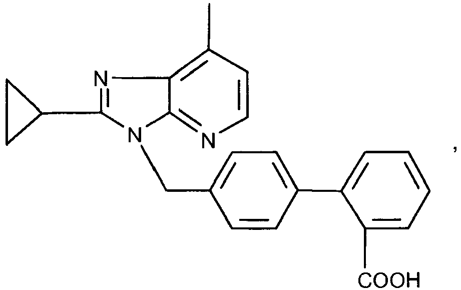 the compound with the designation SC-52458 of the following formula 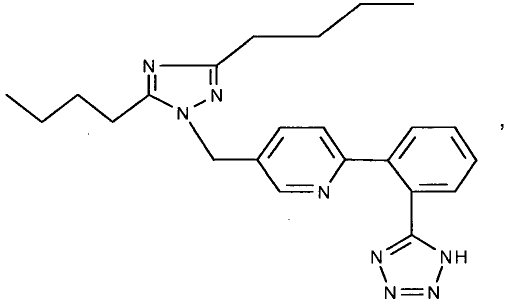 and the compound with the designation ZD-8731 of the following formula 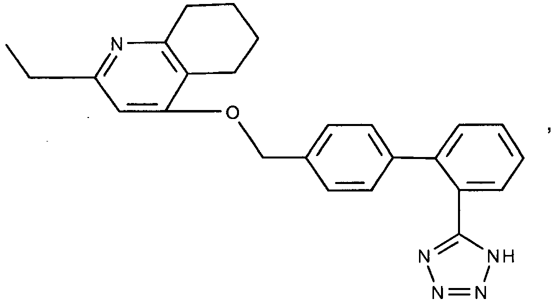 or, in each case, a pharmaceutically acceptable salt thereof.

Preferred AT₁-receptor antagonist are those agents which have been marketed, most preferred is valsartan or a pharmaceutically acceptable salt thereof.

HMG-Co-A reductase inhibitors (also called β-hydroxy-β-methylglutaryl-co-enzyme-A reductase inhibitors) are understood to be those active agents that may be used to lower the lipid levels including cholesterol in blood.

The class of HMG-Co-A reductase inhibitors comprises compounds having differing structural features. For example, mention may be made of the compounds that are selected from the group consisting of atorvastatin, cerivastatin, compactin, dalvastatin, dihydrocompactin, fluindostatin, fluvastatin, lovastatin, pitavastatin, mevastatin, pravastatin, rivastatin, simvastatin, and velostatin, or, in each case, a pharmaceutically acceptable salt thereof.

Preferred HMG-Co-A reductase inhibitors are those agents which have been marketed, most preferred is fluvastatin and pitavastatin or, in each case, a pharmaceutically acceptable salt thereof.

The interruption of the enzymatic degradation of angiotensin I to angiotensin II with so-called ACE-inhibitors (also called angiotensin converting enzyme inhibitors) is a successful variant for the regulation of blood pressure and thus also makes available a therapeutic method for the treatment of congestive heart failure.

The class of ACE inhibitors comprises compounds having differing structural features. For example, mention may be made of the compounds which are selected from the group consisting alacepril, benazepril, benazeprilat, captopril, ceronapril, cilazapril, delapril, enalapril, enaprilat, fosinopril, imidapril, lisinopril, moveltopril, perindopril, quinapril, ramipril, spirapril, temocapril, and trandolapril, or, in each case, a pharmaceutically acceptable salt thereof.

Preferred ACE inhibitors are those agents that have been marketed, most preferred are benazepril and enalapril.

The class of CCBs essentially comprises dihydropyridines (DHPs) and non-DHPs such as diltiazem-type and verapamil-type CCBs.

A CCB useful in said combination is preferably a DHP representative selected from the group consisting of amlodipine, felodipine, ryosidine, isradipine, lacidipine, nicardipine, nifedipine, niguldipine, niludipine, nimodipine, nisoldipine, nitrendipine, and nivaldipine, and is preferably a non-DHP representative selected from the group consisting of flunarizine, prenylamine, diltiazem, fendiline, gallopamil, mibefradil, anipamil, tiapamil and verapamil, and in each case, a pharmaceutically acceptable salt thereof. All these CCBs are therapeutically used, e.g. as anti-hypertensive, anti-angina pectoris or anti-arrhythmic drugs. Preferred CCBs comprise amlodipine, diltiazem, isradipine, nicardipine, nifedipine, nimodipine, nisoldipine, nitrendipine, and verapamil, or, e.g. dependent on the specific CCB, a pharmaceutically acceptable salt thereof. Especially preferred as DHP is amlodipine or a pharmaceutically acceptable salt, especially the besylate, thereof. An especially preferred representative of non-DHPs is verapamil or a pharmaceutically acceptable salt, especially the hydrochloride, thereof.

Aldosterone synthase inhibitor is an enzyme that converts corticosterone to aldosterone to by hydroxylating cortocosterone to form 18-OH-corticosterone and 18-OH-corticosterone to aldosterone. The class of aldosterone synthase inhibitors is known to be applied for the treatment of hypertension and primary aldosteronism comprises both steroidal and non-steroidal aldosterone synthase inhibitors, the later being most preferred.

Preference is given to commercially available aldosterone synthase inhibitors or those aldosterone synthase inhibitors that have been approved by the health authorities.

The class of aldosterone synthase inhibitors comprises compounds having differing structural features. For example, mention may be made of the compounds which are selected from the group consisting of the non-steroidal aromatase inhibitors anastrozole, fadrozole (including the (+)-enantiomer thereof), as well as the steroidal aromatase inhibitor exemestane, or, in each case where applicable, a pharmaceutically acceptable salt thereof. The most preferred non-steroidal aldosterone synthase inhibitor is the (+)-enantiomer of the hydrochloride of fadrozole (US patents 4617307 and 4889861) of formula

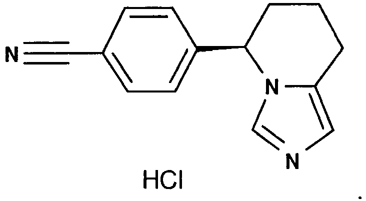

A preferred steroidal aldosterone antagonist is eplerenone of the formula 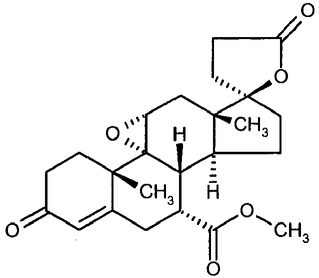 or spironolactone.

A preferred dual angiotensin converting enzyme/neutral endopetidase (ACE/NEP) inhibitor is, for example, omapatrilate (cf. EP 629627), fasidotril or fasidotrilate, or, if appropriable, a pharmaceutically acceptable salt thereof.

A preferred endothelin antagonist is, for example, bosentan (cf. EP 526708 A), furthermore, tezosentan (cf. WO 96/19459), or in each case, a pharmaceutically acceptable salt thereof. A renin inhibitor is, for example, a non-peptidic renin inhibitor such as the compound of formula 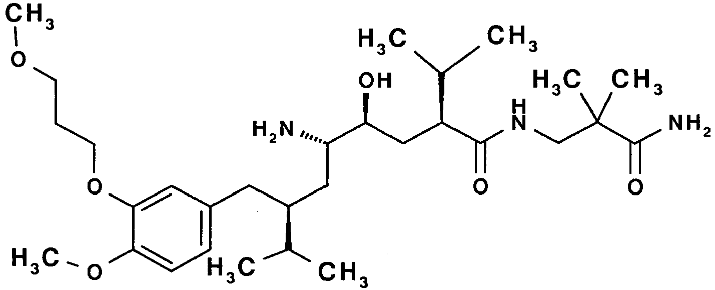 chemically defined as 2(S),4(S),5(S),7(S)-N-(3-amino-2,2-dimethyl-3-oxopropyl)-2,7-di(1-methylethyl)-4-hydroxy-5-amino-8-[4-methoxy-3-(3-methoxy-propoxy)phenyl]-octanamide. This representative is specifically disclosed in EP 678503 A. Especially preferred is the hemi-fumarate salt thereof.

A diuretic is, for example, a thiazide derivative selected from the group consisting of chlorothiazide, hydrochlorothiazide, methylclothiazide, and chlorothalidon. The most preferred is hydrochlorothiazide.

An ApoA-I mimic is, for example, D4F peptide, especially of formula D-W-F-K-A-F-Y-D-K-V-A-E-K-F-K-E-A-F.

A DGAT inhibitor is for example, one or more of the compounds described in WO2005072740, and U.S. provisional application number 60/787859.

Preferably, the jointly therapeutically effective amounts of the active agents according to the combination of the present invention can be administered simultaneously or sequentially in any order, separately or in a fixed combination.

The structure of the active agents identified by generic or tradenames may be taken from the actual edition of the standard compendium "The Merck Index" or from databases, e.g. IMS LifeCycle (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo. Furthermore, the combinations as described above can be administered to a subject via simultaneous, separate or sequential administration (use). Simultaneous administration (use) can take place in the form of one fixed combination with two or more active ingredients, or by simultaneously administering two or more compounds that are formulated independently. Sequential administration(use) preferably means administration of one (or more) compounds or active ingredients of a combination at one time point, other compounds or active ingredients at a different time point, that is, in a chronically staggered manner, preferably such that the combination shows more efficiency than the single compounds administered independently (especially showing synergism). Separate administration (use) preferably means administration of the compounds or active ingredients of the combination independently of each other at different time points, preferably meaning that two compounds are administered such that no overlap of measurable blood levels of both compounds are present in an overlapping manner (at the same time).

Also combinations of two or more of sequential, separate and simultaneous administrations are possible, preferably such that the combination compound-drugs show a joint therapeutic effect that exceeds the effect found when the combination compound-drugs are used independently at time intervals so large that no mutual effect on their therapeutic efficiency can be found, a synergistic effect being especially preferred.

Additionally, the present invention provides:
- a pharmaceutical composition or combination of the present invention for use as a medicament;
- the use of a pharmaceutical composition or combination of the present invention for the delay of progression and/or treatment of a disorder or disease mediated by CETP or responsive to the inhibition of CETP.
- the use of a pharmaceutical composition or combination of the present invention for the delay of progression and/or treatment of a disorder or disease selected from hyperlipidemia, arteriosclerosis, atherosclerosis, peripheral vascular disease, dyslipidemia, hyperbetalipoproteinemia, hypoalphalipoproteinemia, hypercholesterolemia, hypertriglyceridemia, familial hypercholesterolemia, cardiovascular disorder, coronary heart disease, coronary artery disease, coronary vascular disease, angina, ischemia, heart ischemia, thrombosis, cardiac infarction such as myocardial infarction, stroke, peripheral vascular disease, reperfusion injury, angioplasty restenosis, hypertension, congestive heart failure, diabetes such as type II diabetes mellitus, diabetic vascular complications, obesity or endotoxemia etc.

The pharmaceutical composition or combination of the present invention can be in unit dosage of about 1-1000 mg of active ingredients for a subject of about 50-70 kg, preferably about 5-500 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, e.g., mice, rats, dogs, monkeys or isolated organs, tissues and preparations thereof. The compounds of the present invention can be applied *in vitro* in the form of solutions, e.g., preferably aqueous solutions, and *in vivo* either enterally, parenterally, advantageously intravenously, e.g., as a suspension or in aqueous solution. The dosage *in vitro* may range between about 10⁻³ molar and 10⁻⁹ molar concentrations. A therapeutically effective amount *in vivo* may range depending on the route of administration, between about 0.1-500 mg/kg, preferably between about 1-100 mg/kg.

The CETP inhibitory effect of the compounds of the present invention can be determined by using the test models or assays known in the art. For example, EP1115695B1 describes both the *in vitro* and *in vivo* CETP activity assays, the contents of which are hereby incorporated by reference. In particular, the following assays are used.

### (1) CETP in vitro assay:

CETP Activity Kit (#RB-RPAK) is purchased from Roar Biochemical, Inc. (New York, NY, USA). To each well of a 96-well NBS half-area plate (costar #3686), 1.2 ng/well of the donor solution, 1 uL of the acceptor solution and 5 uL compound solution diluted in 100% DMSO are added in a 38 µL of buffer containing 10 mM Tris, 150 mM NaCl and 2 mM EDTA, pH 7.4. Then, the plate is sealed with Themowell™ Sealers (costar #6524) and followed by a mixing on a plate shaker by MICROPLATE MIXER MPX-96 (IWAKI) at power 3 for 10 sec at room temperature. After 10-min incubation at 37°C, the reaction is started by adding 5 uL of rhCETP solution (Cardiovascular Target, New York, NY, USA) and mixed on the plate shaker for 10 sec, then the fluorescence intensity at 0 min is measured by a ARVO SX (Perkin Elmerr, USA) at excitation wavelength of 465 nm and emission wavelength of 535 nm. After 120 min-incubation at 37°C, fluorescence intensity is measured again. The inhibition of rhCETP activity by a compound was calculated by the following calculation. Inhibition%= {1- (F120 - F0) / (f120 - f0)}x 100 F: measured fluorescence intensity with compound at 0 or 120 min. f: measured fluorescence intensity of without compound at 0 or 120 min.

The IC₅₀ values are determined from the dose-effect curve by Origin software. IC₅₀ values, especially from about 0.1 nM to about 50 µM, are determined for the compounds of the present invention or a pharmaceutically acceptable salt thereof.

### (2) Effects on plasma HDL levels in hamster:

Effects of compounds on HDL-cholesterol level in hamsters are investigated by the method reported previously with some modifications (Eur, J. Phamacol, 466 (2003) 147-154). In brief, male Syrian hamsters (10-11 week-old age, SLC, Shizuoka, Japan) are fed a high cholesterol diet for two weeks. Then, the animals are dosed singly with the compound suspended with carboxyl methyl cellulose solution. HDL-cholesterol levels are measured by using commercially available kit (Wako Pure Chemical, Japan) after the precipitation of apolipoprotein B (apoB)-containing lipoproteins with 13% polyethylene glycol 6000.

### (3) Preparation of human pro-apolipoprotein Al (pro-apoAl)

The cDNA of human pro-apoAl (NCBI accession number: NM_000039) is cloned from human liver Quick-Clone™ cDNA (Clontech, CA) and inserted to a pET28a vector (Novagen, Germany) for bacterial expression. Expressed protein as a fusion protein with 6xHis-tag at N-terminus in BL-21 Gold (DE3) (Strategene, CA) is purified using HiTrap Chelating (GE Healthcare, CT).

### (4) Preparation of donor microemulsion

Pro-apoAl containing microemulsion as a donor particle is prepared following previous reports (J. Biol. Chem., 280:14918-22). Glyceryl trioleate (62.5 ng, Sigma, MO), 3-sn-phosphatidylcholine (583 ng, Wako Pure Chemical Industries, Japan), and cholesteryl BODIPY^{®} FL C₁₂ (250 ng, Invitrogen, CA) are dissolved in 1 mL of chloroform. The solution is evaporated, then residual solvent is removed in vacuum for more than 1 hr. The dried lipid mixture is dissolved in 500 µL of the assay buffer (50 mM Tris-HCl (pH7.4) containing 150 mM NaCl and 2 mM EDTA) and sonicated at 50°C with a microtip (MICROSON™ ULTRASONIC CELL DISRUPTOR, Misonix, Farmingdale, NY) at output power 006 for 2 min. After sonication, the solution is cooled to 40°C, added to 100 µg of human pro-apoAl, and sonicated at output power 004 for 5 min at 40°C. The solution, BODIPY-CE microemulsion as a donor molecule is stored at 4°C after filtration through a 0.45 µm PVDF filter.

### (5) In vitro CETP activity assay in human plasma

Human EDTA plasma samples from healthy men are purchased from New Drug Development Research Center, Inc. Donor solution is prepared by a dilution of donor microemulsion with assay buffer. Human plasma (50 uL), assay buffer (35 uL) and test compound dissolved in dimethylsulfoxide (1 uL) are added to each well of 96 well half area black flat bottom plate. The reaction is started by the addition of donor solution (14 uL) into each well. Fluorescence intensities are measured every 30 min at 37°C with excitation wave length of 485 nm and emission wavelength of 535 nm. The CETP activity (FI/min) is defined as the changes of fluorescence intensity from 30 to 90 min. The IC₅₀ value is obtained by the logistic equation (Y=Bottom + (Top-Bottom)/(1+(x/IC₅₀)^Hill slope) using Origin software, version 7.5 SR3. The compounds of formula I exhibit inhibitory activity with an IC₅₀ value in the range from approximately from 0.001 to 100 µM, especially from 0.01 to 10 µM.

The compounds of the present invention or a pharmaceutically acceptable salt thereof have superior CETP inhibitory activity in mammals (e.g., human, monkey, bovine, horse, dog, cat, rabbit, rat, mouse and the like), and can be used as CETP activity inhibitors. In addition, utilizing the superior CETP inhibitory activity of a compound of the present invention or a pharmaceutically acceptable salt thereof, the compounds of the present invention are useful as pharmaceutical agents effective for the prophylaxis or treatment of or delay progression to overt to diseases in which CETP is involved (e.g., hyperlipidemia, arteriosclerosis, atherosclerosis, peripheral vascular disease, dyslipidemia, hyperbetalipoproteinemia, hypoalphalipoproteinemia, hypercholesterolemia, hypertriglyceridemia, familial hypercholesterolemia, cardiovascular disorder, coronary heart disease, coronary artery disease, coronary vascular disease, angina, ischemia, heart ischemia, thrombosis, cardiac infarction such as myocardial infarction, stroke, peripheral vascular disease, reperfusion injury, angioplasty restenosis, hypertension, congestive heart failure, diabetes such as type II diabetes mellitus, diabetic vascular complications, obesity or endotoxemia etc.), particularly as prophylactic or therapeutic agents for hyperlipidemia or arteriosclerotic diseases.

**Table 1 Inhibitory Activity of Compounds**

| **#** | | **CETP Plasma IC₅₀ (nM)** |
|---|---|---|
| 1 | 2,4,6-*cis*-2-Benzyl-4-[(5-bromo-pyrimidin-2-yl)-(3-methoxy-5-trifluoromethyl-benzyl)-amino]-6-ethyl-piperidine-1-carboxylic acid tert-butyl ester | 360 |
| 2 | 2,4,6-*cis*-2-Benzyl-4-[(3,5-bis-trifluoromethyl-benzyl)-(5-dimethylamino-pyrimidin-2-yl)-amino]-6-ethyl-piperidine-1-carboxylic acid tert-butyl ester | 110 |
| 3 | 2,4,6-*cis*-4-[[5-(4-Acetyl-piperazin-1-yl)-pyrimidin-2-yl]-(3,5-bis-trifluoromethyl-benzyl)-amino]-2-benzyl-6-ethyl-piperidine-1-carboxylic acid tert-butyl ester | 200 |
| 4 | 2,4,6-*cis*-2-Benzyl-4-[(3,5-bis-trifluoromethyl-benzyl)-(5-morpholin-4-yl-pyrimidin-2-yl)-amino]-6-ethyl-piperidine-1-carboxylic acid tert-butyl ester | 63 |
| 5 | {2,4,6-*cis*-2-Benzyl-4-[(3,5-bis-trifluoromethyl-benzyl)-(5-bromo-pyrimidin-2-yl)-amino]-6-ethyl-piperidin-1-yl}-cyclopentyl-methanone | 270 |
| 6 | 2,4,6-*cis*-2-Benzyl-4-[(3-chloro-5-trifluoromethylbenzyl)-(5-furan-2-yl-pyrimidin-2-yl)-amino]-6-ethyl-piperidine-1-carboxylic acid isopropyl ester | 152 |
| 7 | 2,4,6-*cis*-2-Benzyl-4-[(3,5-bis-trifluoromethyl-benzyl)-(5-hydroxy-pyrimidin-2-yl)-amino]-6-ethyl-piperidine-1-carboxylic acid tert-butyl ester | 143 |
| 8 | 2,4,6-*cis*-4-[(3-Chloro-5-trifluoromethyl-benzyl)-(5-methoxy-pyrimidin-2-yl)-amino]-2,6-diethyl-piperidine-1-carboxylic acid isopropyl ester | 278 |
| 9 | 2,4,6-*cis*-2-Benzyl-4-{(3,5-bis-trifluoromethyl-benzyl)-[5-(2-hydroxy-ethoxy)-pyrimidin-2-yl]-amino}-6-ethyl-piperidine-1-carboxylic acid tert-butyl ester | 56 |
| 10 | 2,4,6-*cis*-2-Benzyl-4-{(3-chloro-5-trifluoromethyl-benzyl)-[5-(1-methyl-1H-pyrazol-4-yl)-pyrimidin-2-yl]-amino}-6-ethyl-piperidine-1-carboxylic acid isopropyl ester | 60 |
| 11 | 2,4,6-*cis*-2-Benzyl-4-[(3,5-bis-trifluoromethyl-benzyl)-(5-methoxycarbonylmethoxy-pyrimidin-2-yl)-amino]-6-ethyl-piperidine-1-carboxylic acid tert-butyl ester | 83 |
| 12 | 2,4,6-*cis*-2-Benzyl-4-{(3-chloro-5-trifluoromethyl-benzyl)-[5-(2-methanesulfonyl-ethoxy)-pyrimidin-2-yl]-amino}-6-ethyl-piperidine-1-carboxylic acid isopropyl ester | 82 |
| 13 | 2,4,6-*cis*-2-Benzyl-4-{(3-chloro-5-trifluoromethyl-benzyl)-[5-(2-methoxy-ethoxy)-pyrimidin-2-yl]-amino}-6-ethyl-piperidine-1-carboxylic acid isopropyl ester | 79 |
| 14 | 2,4,6-cis-4-[(3,5-Bis-trifluoromethyl-benzyl)-methoxycarbonyl-amino]-2-ethyl-6-(2-hydroxy-3-methyl-butyl)-piperidine-1-carboxylic acid tert-butyl ester | 377 |
| 15 | 2,4,6-*cis*-4-[(3,5-Bis-trifluoromethyl-benzyl)-methoxycarbonyl-amino]-2-ethyl-6-(2-methyl-2H-pyrazol-3-ylmethyl)-piperidine-1-carboxylic acid tert-butyl ester | 138 |
| 16 | (2R,4R,6S)-4-{(3-Chloro-5-trifluoromethyl-benzyl)-[5-(3-ethoxycarbonyl-propoxy)-pyrimidin-2-yl]-amino}-2,6-diethyl-piperidine-1-carboxylic acid isopropyl ester | 340 |
| 17 | Acetic acid 4-{2,4,6-*cis*-2-benzyl-4-[(3,5-bis-trifluoromethyl-benzyl)-(5-bromo-pyrimidin-2-yl)-amino]-6-ethyl-piperidine-1-carbonyl}-cyclohexyl ester | 314 |
| 18 | {2,4,6-*cis*-2-Benzyl-4-[(3,5-bis-trifluoromethyl-benzyl)-(5-bromo-pyrimidin-2-yl)-amino]-6-ethyl-piperidin-1-yl}-(4-hydroxy-cyclohexyl)-methanone | 357 |
| 19 | 2,4,6-*cis*-4-[(3-Chloro-5-trifluoromethyl-benzyl)-methoxycarbonyl-amino]-2-ethyl-6-(2-ethyl-2H-pyrazol-3-ylmethyl)-piperidine-1-carboxylic acid isopropyl ester | 409 |
| 20 | (2S,4R,6R)-4-{(3-Chloro-5-trifluoro methyl-benzyl)-[5-(4-methyl-piperaz in-1-yl)-pyrimidin-2-yl]-amino}-2,6 -diethyl-piperidine-1-carboxylic ac id isopropyl ester | 67 |
| 21 | (2R,4R,6S)-4-[(3-Chloro-5-trifluoromethyl-benzyl)-(5-imidazol-1-yl-pyrimidin-2-yl)-amino]-2,6-diethyl-piperidine-1-carboxylic acid isopropyl ester | 31 |
| 22 | Acetic acid 4-{2,4,6-*cis*-2-benzyl-4-[(3,5-bis-trifluoromethyl-benzyl)-(5-morpholin-4-yl-pyrimidin-2-yl)-amino]-6-ethyl-piperidine-1-carbonyl}-cyclohexyl ester | 230 |

### Abbreviations

Ac: Acetyl
aq: aqueous
Ar: aromatic
BBN: borabicyclo[3.3.1]nonane
dba:dibenzylidenacetone
BINAP: bis(diphenylphosphino)-1,1'-binaphthalene
Bn: benzyl
Boc: *tert*-butoxycarbonyl
CAN: ceric ammonium nitrate
CDI: 1,1'-carbonyldiimidazole
DCM: dichloromethane
DDQ: 2,3-dichloro-5,6-dicyano-*p*-benzoquinone
DEAD: diethyl azodicarboxylate
DIPEA: *N,N*-diisopropylethylamine
DMAP: *N,N*-dimethylaminopyridine
DME: dimethoxyethane
DMME: dimethoxymethane
DMF: *N,N*-dimethylformamide
DMSO: dimethyl sulfoxide
dppf: 1,1-bis(diphenylphosphino)ferrocene
EDTA: ethylenediaminetetraacetic acid
ESI: electrospray ionization
Et: ethyl
EtOAc: ethyl acetate
h: hours
HCl: hygrogen chloride
HPLC: high pressure liquid chromatography
IPA: 2-propanol
iPr: isopropyl
IR: infrared
KHMDS: potassium hexamethyldisilamide
LAH: lithium alminum hydride
LC: liquid chromatography
LDA: lithium diisopropylamide
LHMDS: lithium hexamethyldisilamide
mCPBA: 3-chloroperbenzoic acid
Me: methyl
min: minutes
MS: mass spectrometry
Ms: mesyl
NaHMDS: sodium hexamethyldisilamide
NBS: *N*-bromosuccinimide
NMR: nuclear magnetic resonance
Ph: phenyl
PMB: *p*-methoxybenzyl
PTLC: preparative thin layer chromatography
rac: racemate
Ra-Ni: Raney Nickel
RP: reversed phase
RT: room temperature
s-Bu: sec-butyl
Sia: siamyl
SFC: supercritical fluid chromatography
TEA: triethylamine
Tf: triflate
TFA: trifluoroacetic acid
THF: tetrahydrofuran
TLC: thin layer chromatography
TMS: trimethylsilyl
Ts: tosyl
tBu: *tert*-butyl
tol: tolyl

### EXAMPLES

The following examples are intended to illustrate the invention and are not to be construed as being limitations thereon. Temperatures are given in degrees centrigrade. If not mentioned otherwise, all evaporations are performed under reduced pressure, preferably between about 15 mm Hg and 100 mm Hg (= 20-133 mbar). The structure of final products, intermediates and starting materials is confirmed by standard analytical methods, e.g., microanalysis and spectroscopic characteristics, e.g., MS, IR, NMR. Abbreviations used are those conventional in the art. The compounds in the following examples have been found to have IC₅₀ values in the range of about 0.1nM to about 10,000 nM or about 1,000 nM for CETP.

The conditions for measuring the retention times are as follows:

### Condition A (HPLC)

Column: ACQUITY UPLC™ BEH C₁₈ 1.7 µm, 50 x 2.1 mm.

Flow rate: 0.5 ml / min

Mobile phase: A) TFA / water (0.1 / 100, v / v), B) TFA / acetonitrile (0.1 *l* 100,v / v) Gradient: linear gradient from 5% B to 100% B in 2 min then 100% B in 1 min

Detection: UV at 215 nm

### Condition B (HPLC)

Column: ACQUITY UPLC™ BEH C₁₈ 1.7 µm, 50 x 2.1 mm.

Flow rate: 0.5 ml / min

Mobile phase: A) TFA / water (0.1 / 100, v / v), B) TFA / acetonitrile (0.1 / 100, v / v)

Gradient: 5% B in 0.5 min, then linear gradient from 5% B to 100% B in 5.0 min then 100% B in 1.5 min

### Detection: UV at 215 nm

### Condition C (HPLC)

Column: CombiScreen ODS-AM, 50 x 4.6 mm.

Flow rate: 2.0 ml / min

Mobile phase: A) TFA / water (0.1 / 100, v / v), B) TFA / acetonitrile (0.1 / 100, v / v) Gradient: linear gradient from 5% B to 100% B in 5 min then 100% B in 2 min Detection: UV at 215 nm

### Condition D (SFC)

Column: OCl

Flow rate: 3 ml / min

Mobile phase: CO₂ / 10% DMME in IPA 100 / 7

Gradient: isocratic

Detection: UV at 200 nm

### Condition E (HPLC)

Column: Chiralpak AD-H, 25 x 0.46 cm.

Flow rate: 1.0 ml / min

Mobile phase: n-hexane / IPA (95 / 5, v / v)

Gradient: isocratic

Detection: UV at 220 nm

### Condition F (HPLC)

Column: Chiralpak AD-H, 25 x 0.46 cm.

Flow rate: 1.5 ml / min

Mobile phase: heptane / isopropanol (90 / 10, v / v)

Gradient: isocratic

Detection: UV at 220 nm

### Example 1: Synthesis of 4-[(3,5-bis-trifluoromethyl-benzyl)-methoxycarbonyl-amino]-2-ethyl-piperidine-1-carboxylic acid tert-butyl ester

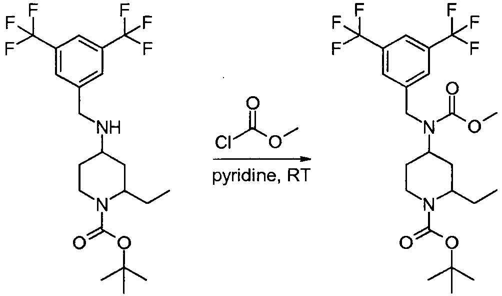

To a solution of 4-(3,5-bis-trifluoromethyl-benzylamino)-2-ethyl-piperidine-1-carboxylic acid tert-butyl ester (1.6 g, 3.1 mmol) in 10 mL of pyridine under N₂, cool to 0 °C, is added methyl chloroformate (1.7 mL, 22 mmol) dropwise over 5 minutes. The reaction mixture is warmed to room temperature and stirred for 14 h. The reaction is subsequently quenched by addition of aqueous 1 N HCl (50 mL), and the aqueous layer is extracted with ethyl acetate (2X 50 mL). The organic layers are combined and washed with brine, dried with anhydrous sodium sulfate, and the solvent removed by rotary evaporation. The residue is chromatographed to give 4-[(3,5-bis-trifluoromethyl-benzyl)-methoxycarbonyl-amino]-2-ethyl-piperidine-1-carboxylic acid tert-butyl ester; ESI-MS m/z: 513 [M+1]⁺, Retention time 2.35 min (condition A).

### Example 2: Synthesis of 4-[acetyl-(3,5-bis-trifluoromethyl-benzyl)-amino]-2-ethyl-piperidine-1-carboxylic acid tert-butyl ester

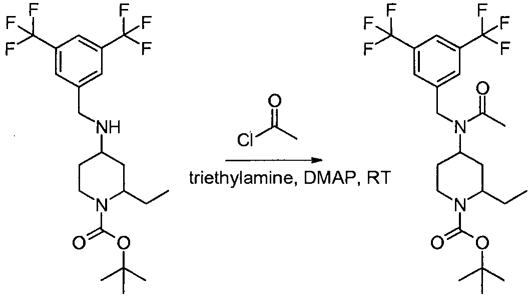

A mixture of 4-(3,5-bis-trifluoromethyl-benzylamino)-2-ethyl-piperidine-1-carboxylic acid tert-butyl ester (10 mg, 0.022 mmol) and triethylamine (9 uL, 0.066 mmol) and catalytic amount of *N,N*-dimethylaminopyridine are stirred at room temperature for 2 hours. The mixture is quenched with water, then extracted with ethyl acetate. The organic layer is concentrated under reduced pressure. The residue is purified by RP-HPLC to give 5.6 mg of 4-[acetyl-(3,5-bis-trifluoromethyl-benzyl)-amino]-2-ethyl-piperidine-1-carboxylic acid tert-butyl ester; ESI-MS m/z: 497 [M+1]⁺, Retention time 2.22 min (condition A).

The following compound is prepared following the procedure of **Example 2.**

| No. | Product | ESI-MS m/z [M+1]⁺ | Retention time (min) | Reagent | Starting Material |
|---|---|---|---|---|---|
| 1 | 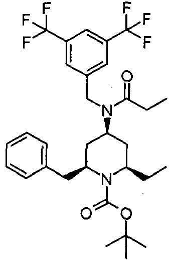 | 601 | 5.37 (condition B) | 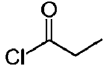 | 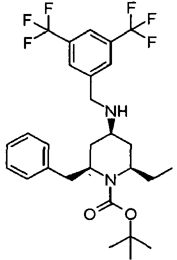 |

### Example 3: Synthesis of 4-[(3,5-bis-trifluoromethyl-benzyl)-(5-bromo-pyrimidin-2-yl)-amino]-2-ethyl-piperidine-1-carboxylic acid tert-butyl ester

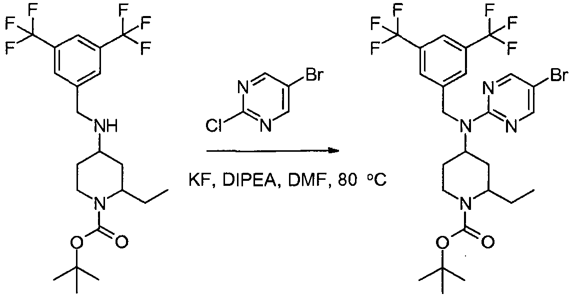

A mixture of 5-bromo-2-chloropyridine (21 mg, 0.11 mmol) and potassium fluoride (23 mg, 0.4 mmol) in *N,N*-dimethyl formamide (0.5 mL) are stirred at 80 °C for 3 hours. To the mixture is added 4-(3,5-bis-trifluoromethyl-benzylamino)-2-ethyl-piperidine-1-carboxylic acid tert-butyl ester (45 mg, 0.1 mmol) and *N,N*-diisopropylethylamine (21 mg, 0.11 mmol). The mixture is stirred at 80 °C for 15 hours, then warmed to 115 °C. After stirring for 3 hours, the mixture is cooled down to ambient temperature, then quenched with saturated aqueous sodium hydrogencarbonate. The mixture is extracted with ethyl acetate. The combined organic layer is concentrated under reduced pressure. The residue is purified by RP-HPLC to give 3 mg of 4-[(3,5-bis-trifluoromethyl-benzyl)-(5-bromo-pyrimidin-2-yl)-amino]-2-ethyl-piperidine-1-carboxylic acid tert-butyl ester; ESI-MS m/z: 611 [M+1]⁺, Retention time 2.59 min (condition A).

### Example 4: Synthesis of 4-[(3,5-bis-trifluoromethyl-benzyl)-methoxycarbonyl-amino]-2-ethyl-piperidine-1-carboxylic acid ethyl ester

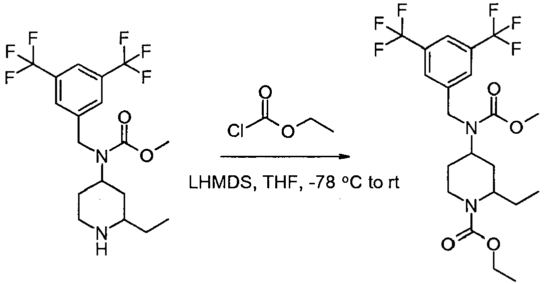

In a two-necked flask, (3,5-bis-trifluoromethyl-benzyl)-(2-ethyl-piperidin-4-yl)-carbamic acid methyl ester (0.8 g, 1.9 mmol) is dissolved in 3 mL of anhydrous THF. The solution is cooled at -78 °C, and then LHMDS (2.9 mL, 2.9 mmol) is added dropwise with stirring. After 30 min, ethyl chloroformate (0.31 mL, 3.0 mmol) is added dropwise, and the reaction mixture is allowed to room temperature. The reaction is quenched with saturated aqueous ammonium chloride (40 mL), and the mixture is extracted with ethyl acetate (2 X 50 mL). The combined organic layer is dried with anhydrous sodium sulfate and concentrated under reduced pressure. The obtained residue is purified by flash chromatography, eluting with hexane-ethyl acetate mixture to give 4-[(3,5-bis-trifluoromethyl-benzyl)-methoxycarbonyl-amino]-2-ethyl-piperidine-1-carboxylic acid ethyl ester; ESI-MS m/z: 485 [M+1]⁺, Retention time 2.21 min (condition A).

### Example 5: Synthesis of 4-[(3,5-bis-trifluoromethyl-benzyl)-(4-bromo-phenyl)-amino]-2-ethyl-piperidine-1-carboxylic acid tert-butyl ester

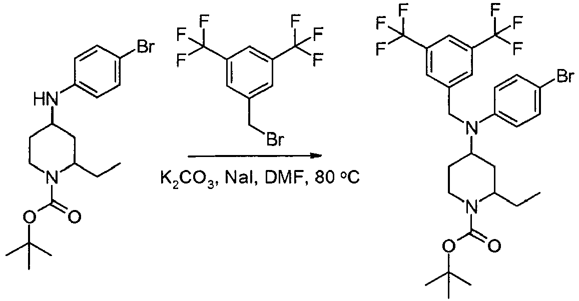

A mixture of 4-(4-bromo-phenylamino)-2-ethyl-piperidine-1-carboxylic acid tert-butyl ester (38 mg, 0.1 mmol), 1-bromomethyl-3,5-bis-trifluoromethyl-benzene (22 uL, 0.12 mmol), potassium carbonate (69 mg, 0.5 mmol) and sodium iodide (30 mg, 0.2 mmol) in *N,N-*dimethyl formamide (1 mL) is stirred at room temperature for 3 hours. The mixture is warmed to 80 °C and added 1-bromomethyl-3,5-bis-trifluoromethyl-benzene (22 uL, 0.12 mmol). After the mixture is stirred at 80 °C for 15 hours, the mixture is cooled to room temperature. The mixture is quenched with water then extracted with ethyl acetate. The organic layer is concentrated under reduced pressure. The obtained residue is purified by RP-HPLC to give 20 mg of 4-[(3,5-bis-trifluoromethyl-benzyl)-(4-bromo-phenyl)-amino]-2-ethyl-piperidine-1-carboxylic acid tert-butyl ester; ESI-MS m/z: 609 [M+1]⁺, Retention time 2.58 min (condition A).

### Example 6: Synthesis of 4-[(3,5-bis-trifluoromethyl-benzyl)-cyclohexyl-amino]-2-ethylpiperidine-1-carboxylic acid tert-butyl ester

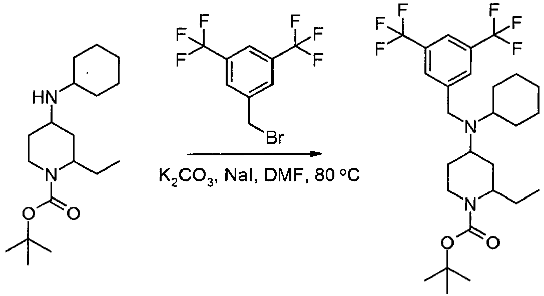

A mixture of 4-cyclohexylamino-2-ethyl-piperidine-1-carboxylic acid tert-butyl ester (31 mg, 0.1 mmol), 1-bromomethyl-3,5-bis-trifluoromethyl-benzene (22 uL, 0.12 mmol), potassium carbonate (69 mg, 0.5 mmol) and sodium iodide (30 mg, 0.2 mmol) in *N,N-*dimethyl formamide (1 mL) is stirred at room temperature for 3 hours. The mixture is warmed to 80 °C and added 1-bromomethyl-3,5-bis-trifluoromethyl-benzene (22 uL, 0.12 mmol). After the mixture is stirred at 80 °C for 15 hours, the mixture is cooled to room temperature. The mixture is quenched with water then extracted with ethyl acetate. The organic layer is concentrated under reduced pressure. The obtained residue is purified by RP-HPLC to give 7.6 mg of 4-[(3,5-bis-trifluoromethyl-benzyl)-cyclohexyl-amino]-2-ethyl-piperidine-1-carboxylic acid tert-butyl ester; ESI-MS m/z: 537 [M+1]⁺, Retention time 1.97 min (condition A).

### Example 7: Synthesis of 2-benzyl-4-[(3,5-bis-trifluoromethyl-benzyl)-methoxycarbonyl-amino]-6-ethyl-piperidine-1-carboxylic acid benzyl ester (racemic)

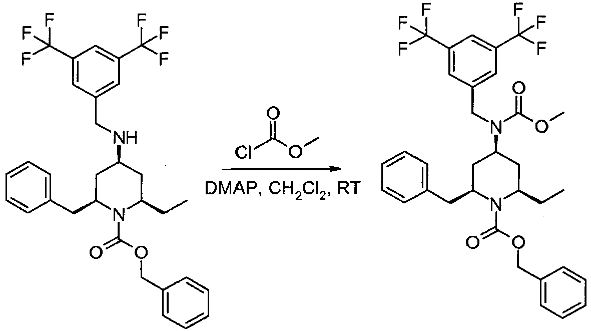

To a solution of a racemic mixture of 2-benzyl-4-(3,5-bis-trifluoromethyl-benzylamino)-6-ethyl-piperidine-1-carboxylic acid benzyl ester (0.017 mmol, 10 mg) in dry CH₂Cl₂ (0.3 ml) is added *N,N*-dimethylaminopyridine (0.034 mmol, 4.2 mg) and methylchloroformate (0.042 mmol, 3.3 mg) under N₂ at room temperature. After stirring for 1 hour, methylchloroformate (0.139 mmol, 11 mg) and CH₂Cl₂ (0.7 mL) are added. After another 1 hour, methylchloroformate (0.139 mmol, 11mg) and *N,N*-dimethylaminopyridine (0.033 mmol, 4.0 mg) are added to the mixture. After 1.5 hours, the reaction mixture is purified on preparative TLC to give a racemic mixture of 2-benzyl-4-[(3,5-bis-trifluoromethyl-benzyl)-methoxycarbonyl-amino]-6-ethyl-piperidine-1-carboxylic acid benzyl ester (82%, 8.9 mg); ESI-MS m/z: 637 [M+1]⁺, Retention time 2.45 min (condition A).

The following compounds are prepared following the procedure of **Example 7.**

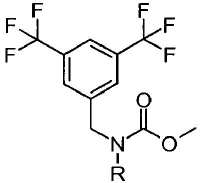

| No. | R | ESI-MS m/z [M+1]⁺ | Retention time (min) |
|---|---|---|---|
| 1 | 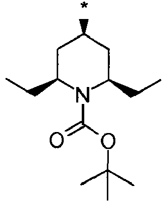 | 541 | 2.45 (condition A) |
| 2 | 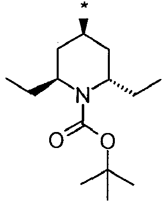 | 541 | 2.47 (condition A) |
| | racemic | | |
| 3 | 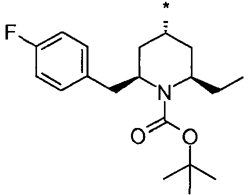 | 621 | 5.35 (condition B) |
| | racemic | | |
| 4 | 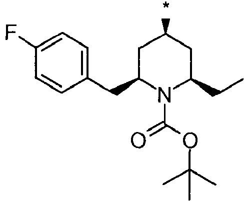 | 621 | 2.74 (condition A) |
| | racemic | | |
| 5 | 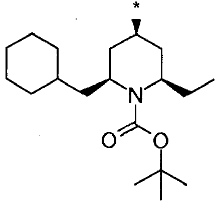 | 609 | 5.67 (condition C) |
| | racemic | | |
| 6 | 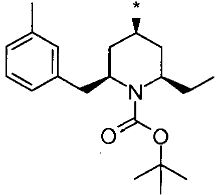 | 617 | 2.69 (condition A) |
| | racemic | | |
| 7 | 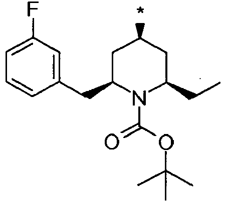 | 621 | 2.63 (condition A) |
| | racemic | | |
| 8 | 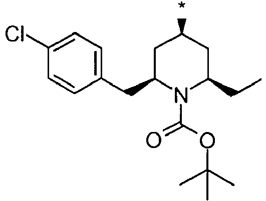 | 637 | 2.7 (condition A) |
| | racemic | | |
| 9 | 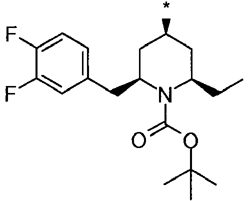 | 639 | 2.62 (condition A) |
| | racemic | | |
| 10 | 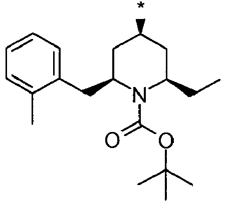 | 617 | 2.77 (condition A) |
| | racemic | | |
| 11 | 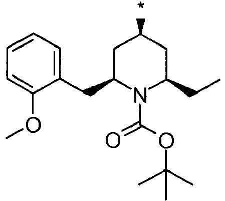 | 633 | 2.63 (condition A) |
| | racemic | | |
| 12 | 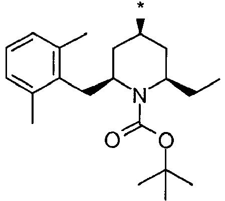 | 631 | 2.71 (condition A) |
| | racemic | | |
| 13 | 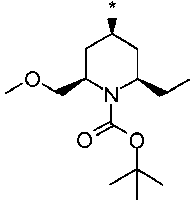 | 557 | 5.07 (condition B) |
| | racemic | | |
| 14 | 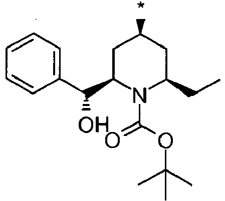 | 619 | 2.49 (condition A) |
| | racemic | | |
| 15 | 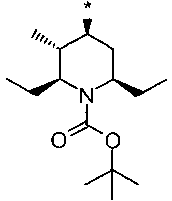 | 555 | 2.51 (condition A) |
| | racemic | | |
| | K₂CO₃ instead of DMAP, THF instead of CH₂CL₂ | | |
| 16 | 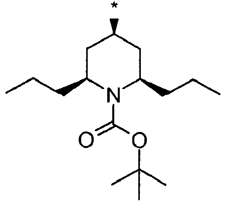 | 569 | 2.70 (condition A) |

### Example 8: Synthesis of 2-benzyl-4-[(3,5-bis-trifluoromethyl-benzyl)-(5-bromo-pyrimidin-2-yl)-amino]-6-ethyl-piperidine-1-carboxylic acid tert-butyl ester (racemic)

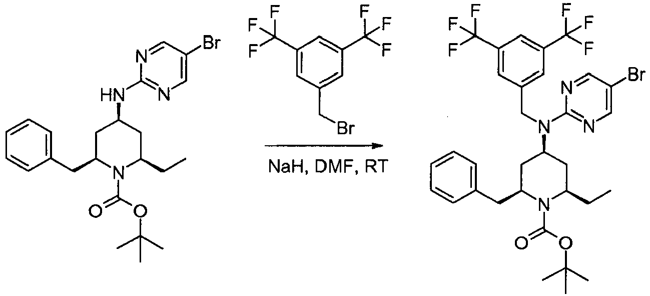

A solution of a racemic mixture of 2-benzyl-4-(5-bromo-pyrimidin-2-ylamino)-6-ethyl-piperidine-1-carboxylic acid tert-butyl ester (0.076 mmol, 36 mg) in DMF (1 ml) is cooled to 0 °C and NaH (60% oil suspension, 0.228 mmol, 5.5 mg) is added. The mixture is allowed to warm to room temperature and stirred for 30 min. The mixture is cooled to 0 °C to add 3,5-bis(trifluoromethyl)benzyl bromide (0.091 mmol, 27.9 mg). After stirring for 5 min, the solution is warmed to room temperature and stirred for 2 hours. The mixture is quenched with saturated brine and extracted with ethyl acetate. The combined organic layer is washed with brine, dried over Na₂SO₄, filtrated, and concentrated under reduced pressure. The obtained residue is purified by column chromatography (silica gel; hexane/EtOAc) to give a racemic mixture of 2-benzyl-4-[(3,5-bis-trifluoromethyl-benzyl)-(5-bromo-pyrimidin-2-yl)-amino]-6-ethyl-piperidine-1-carboxylic acid tert-butyl ester (7.0 mg, 13%); ESI-MS m/z: 701 [M+1]⁺, Retention time 2.67 min (condition A).

The following compounds are prepared following the procedure of **Example 8.**

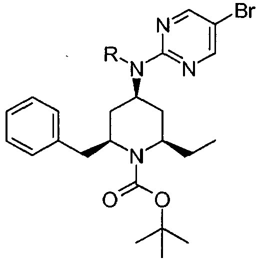

| No. | R | ESI-MS m/z [M+1]⁺ | Retention time (min) |
|---|---|---|---|
| 1 | 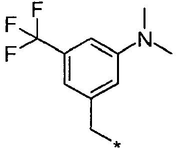 | 676 | 2.65 (condition A) |
| | racemic | | |
| 2 | 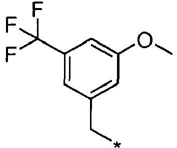 | 663 | 2.7 (condition A) |
| | racemic | | |
| 3 | 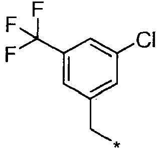 | 667 | 6.22 (condition B) |
| | racemic | | |
| 4 | 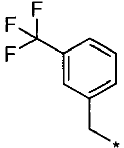 | 633 | 2.89 (condition A) |
| | racemic | | |
| 5 | 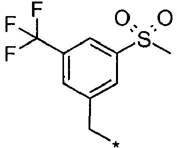 | 711 | 2.7 (condition A) |
| | racemic | | |
| 6 | 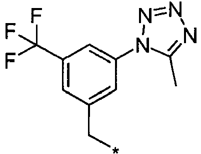 | 715 | 2.73 (condition A) |
| | racemic | | |
| 7 | 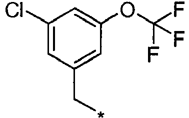 | 683 | 6.31 (condition B) |
| | racemic | | |

### Example 9: Synthesis of (2S,4R,6R)-4-[(5-bromo-pyrimidin-2-yl)-(3-chloro-5-trifluoromethyl-benzyl)-amino]-2,6-dimethyl-piperidine-1-carboxylic acid isopropyl ester

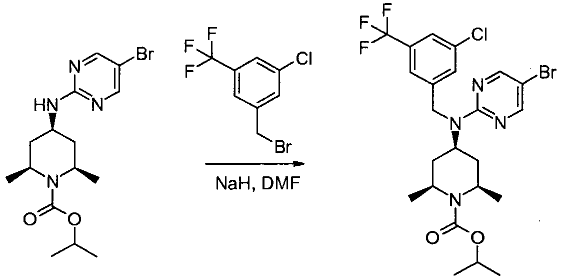

A solution of (2S,4R,6R)-4-(5-bromo-pyrimidin-2-ylamino)-2,6-dimethyl-piperidine-1-carboxylic acid isopropyl ester (102 mg, 0.275 mmol) in DMF (1.5 mL) is cooled to 0 °C and NaH (60% oil suspension, 0.228 mmol, 5.5 mg) is added. The mixture is stirred at 0 °C for 30 minutes, and 3-chloro-5-trifluoromethylbenzyl bromide (113 mg, 0.412 mmol) is added. After stirring for 1 hour, the mixture is quenched with water and extracted with ethyl acetate. The combined organic layer is washed with brine, dried over Na₂SO₄, filtrated, and concentrated under reduced pressure. The obtained residue is purified by column chromatography (silica gel; hexane/EtOAc) to give (2S,4R,6R)-4-[(5-bromo-pyrimidin-2-yl)-(3-chloro-5-trifluoromethyl-benzyl)-amino]-2,6-dimethyl-piperidine-1-carboxylic acid isopropyl ester (80 mg); ESI-MS m/z: 563 [M+1]⁺, Retention time 2.70 min (condition A).

The following compounds are prepared following the procedure of **Example 9.**

| No. | Product | ESI-MS m/z [M+1]⁺ | Retention time (min) | Starting Material |
|---|---|---|---|---|
| 1 | 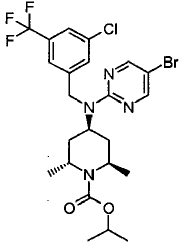 | 563 | 2.77 (condition A) | 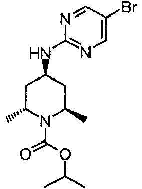 |
| 2 | 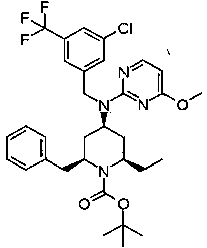 | 619 | 2.44 (condition A) | 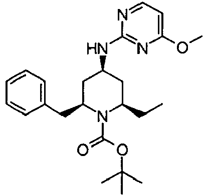 |

### Example 10: Synthesis of 2-benzyl-4-[(3,5-bis-trifluoromethyl-benzyl)-(5-morpholin-4-yl-pyrimidin-2-yl)-amino]-6-ethyl-piperidine-1-carboxylic acid tert-butyl ester (racemic)

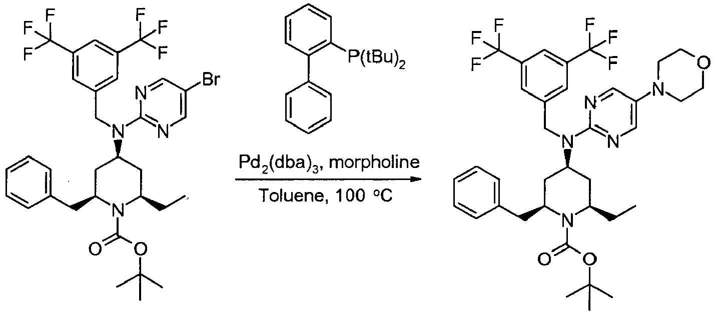

To a racemic mixture of 2-benzyl-4-[(3,5-bis-trifluoromethyl-benzyl)-(5-bromo-pyrimidin-2-yl)-amino]-6-ethyl-piperidine-1-carboxylic acid tert-butyl ester (0.038 mmol, 27 mg) in a flask purged with N₂ are added Pd₂(dba)₃ (0.0077 mmol, 7.0 mg), 2-(di-tert-butylphosphino)biphenyl (0.0154 mmol, 4.6 mg), sodium tert-butoxide (0.154 mmol, 14.8 mg) and morpholine (0.077 mmol, 0.0067 ml). The flask is purged again with N₂, toluene (0.4 ml) is added and heated at 100 °C for 4 hours. The reaction is quenched with silica and the crude product is purified by silica gel column chromatography to give a racemic mixture of 2-benzyl-4-[(3,5-bis-trifluoromethyl-benzyl)-(5-morpholin-4-yl-pyrimidin-2-yl)-amino]-6-ethyl-piperidine-1-carboxylic acid tert-butyl ester (0.0189 mmol, 13.37 mg, 50%); ESI-MS m/z: 708 [M+1]⁺, Retention time 2.55 min (condition A).

The following compounds are prepared following the procedure of **Example 10** using corresponding amines.

| No. | Product | ESI-MS m/z [M+1]⁺ | Retention time (min) | Starting Material |
|---|---|---|---|---|
| 1 | 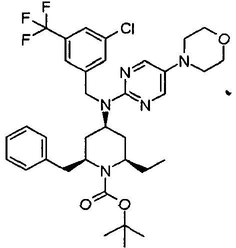 | 674 | 2.72 (condition A) | 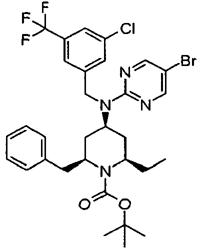 |
| | racemic | | | |
| 2 | 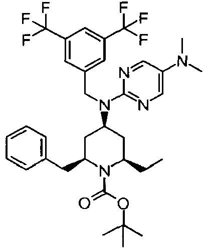 | 666 | 4.92 (condition B) | 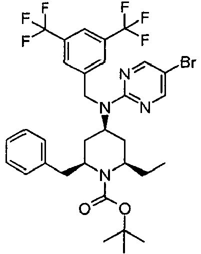 |
| | racemic | | | |
| 3 | 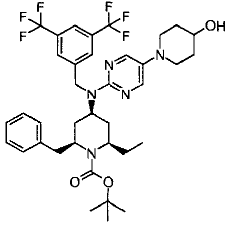 | 722 | 4.62 (condition B) | 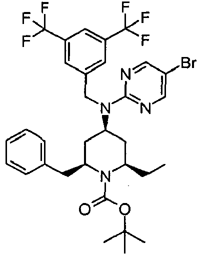 |
| | racemic | | | |
| 4 | 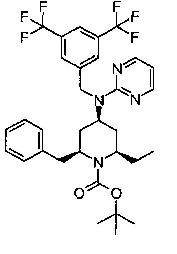 | 623 | 5.57 (condition B) | 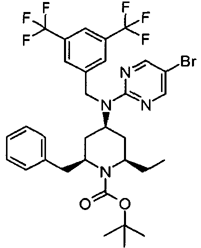 |
| | racemic | | | |
| 5 | 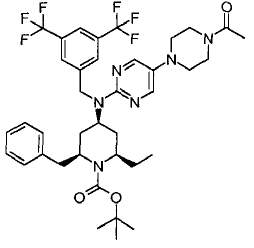 | 749 | 2.53 (condition A) | 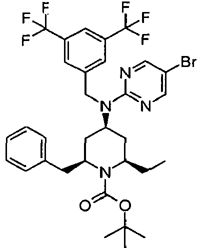 |
| | racemic | | | |
| 6 | 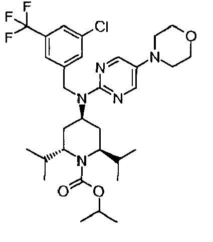 | 626 | 2.57 (condition A) | 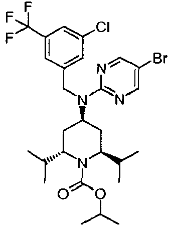 |
| | racemic | | | |
| 7 | 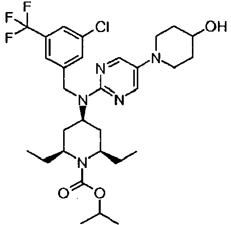 | 612 | 2.17 (condition A) | 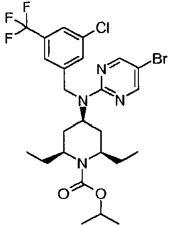 |
| | | | | (LMHDS instead of *t*BuONa; THF instead of toluene) |
| 8 | 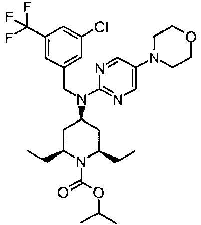 | 598 | 2.47 (condition A) | 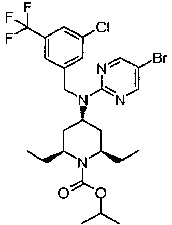 |
| 9 | 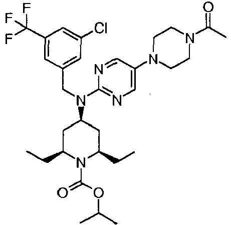 | 639 | 2.36 (condition A) | 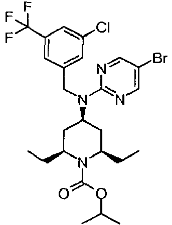 |
| 10 | 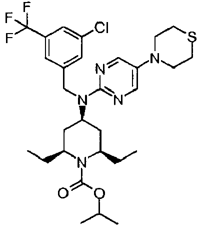 | 614 | 2.47 (condition A) | 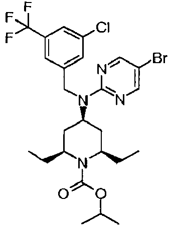 |
| 11 | 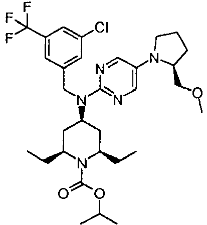 | 626 | 2.37 (condition A) | 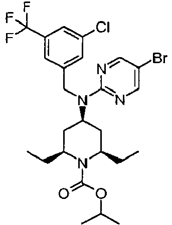 |
| 12 | 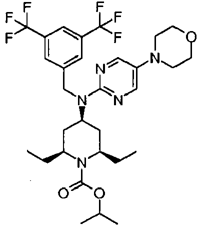 | 632 | 2.41 (condition A) | 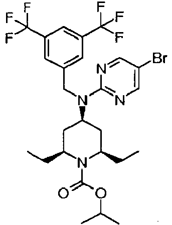 |
| 13 | 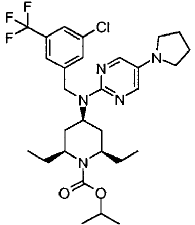 | 582 | 30.8 (condition C) | 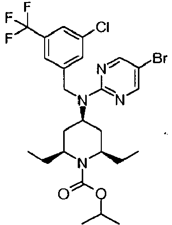 |
| 14 | 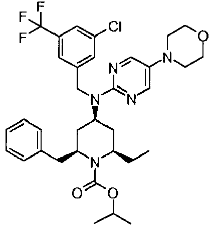 | 660 | 5.49 (condition B) | 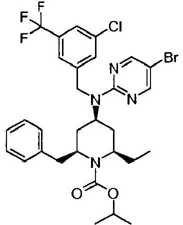 |
| 15 | 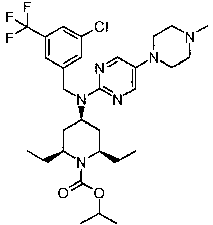 | 611 | 2.17 (condition A) | 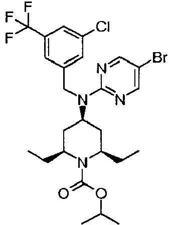 |

### Example 11: Synthesis of 2-benzyl-4-[(3,5-bis-trifluoromethyl-benzyl)-methoxycarbonyl-amino]-6-ethyl-piperidine-1-carboxylic acid tert-butyl ester (racemic)

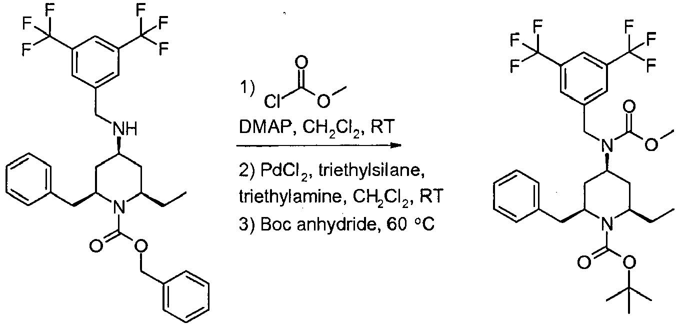

To a solution of a racemic mixture of 2-benzyl-4-(3,5-bis-trifluoromethyl-benzylamino)-6-ethyl-piperidine-1-carboxylic acid benzyl ester (0.064 mmol, 37.4 mg) in dry CH₂Cl₂ (10 ml) is added *N,N*-dimethylaminopyridine (0.128 mmol, 15.6 mg) and methylchloroformate (0.128 mmol, 10.0 mg) under N₂ at room temperature. After stirring for 14 hours, the reaction mixture is passed through silica gel pad and concentrated under reduced pressure to give 2-benzyl-4-[(3,5-bis-trifluoromethyl-benzyl)-methoxycarbonyl-amino]-6-ethyl-piperidine-1-carboxylic acid benzyl ester which is used in the next reaction without further purification. A flask containing the obtained residue and PdCl₂ (0.0064 mmol, 1.1 mg) is purged with N₂. To the mixture is added triethylsilane (0.128 mmol, 14.9 mg) and triethylamine (0.0128 mmol, 1.3 mg) and dry CH₂Cl₂ (0.5 ml). After stirring for 1 hour, additional PdCl₂ (0.0576 mmol, 9.9 mg) and triethylamine (0.128 mmol, 13.1 mg) are added. After stirring for 45 min, triethylsilane (0.250 mmol, 29 mg) is added to the mixture. The mixture is allowed to stir for 2 hours, and then concentrated under reduced pressure. The residue is purified by silica gel column chromatography (eluent: CH₂Cl₂ / Methanol = 100 / 1) to give 2-benzyl-6-ethyl-piperidin-4-yl)-(3,5-bis-trifluoromethyl-benzyl)-carbamic acid methyl ester which is used in the next reaction. Boc anhydride (0.09mmol, 19 mg) is added to the obtained residue. The mixture is allowed to stir at 60 °C for 11 hours, cooled to room temperature, and purified on preparative TLC to give a racemic mixture of 2-benzyl-4-[(3,5-bis-trifluoromethyl-benzyl)-methoxycarbonyl-amino]-6-ethyl-piperidine-1-carboxylic acid tert-butyl ester (3.5 mg, 9% in 3 steps); ESI-MS m/z: 603 [M+1]⁺, Retention time 2.50 min (condition A).

The following compound is prepared following the procedure of Example 11.

| No. | Product | ESI-MS m/z [M+1]⁺ | Retention time (min) | Starting Material |
|---|---|---|---|---|
| 1 | 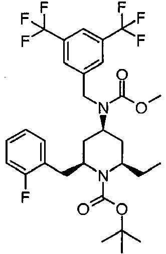 | 621 | 2.54 (condition A) | 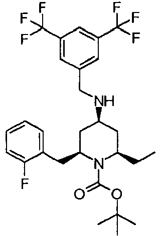 |
| | racemic | | | |

### Example 12: Synthesis of 5-benzyl-4-[(3,5-bis-trifluoromethyl-benzyl)-methoxycarbonyl-amino]-2-ethyl-piperidine-1-carboxylic acid tert-butyl ester

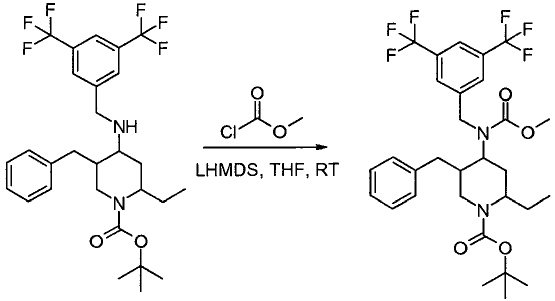

In a two-necked flask, 5-benzyl-4-(3,5-bis-trifluoromethyl-benzylamino)-2-ethyl-piperidine-1-carboxylic acid tert-butyl ester (22mg, 0.04mmol) is dissolved in 0.25 mL of anhydrous THF. The solution is cooled at -78 °C, and then LHMDS (0.05mL, 0.05 mmol) is added dropwise with stirring. After 30 min, methyl chloroformate (0.004mL, 0.048 mmol) is added dropwise, and the reaction mixture is allowed to room temperature. The reaction is quenched with saturated aqueous ammonium chloride (20 mL), and the mixture is extracted with ethyl acetate (2 X 20 mL), dried with Na₂SO₄, filtered, and evaporated to leave the crude product. The obtained residue is purified by flash chromatography, eluting with hexane-ethyl acetate mixtures to give 5-benzyl-4-[(3,5-bis-trifluoromethyl-benzyl)-methoxycarbonyl-amino]-2-ethyl-piperidine-1-carboxylic acid tert-butyl ester (40% yield); ESI-MS m/z: 603 [M+1]⁺, Retention time 2.46 min (condition A).

### Example 13: 2-benzyl-4-[(3,5-bis-trifluoromethyl-benzyl)-(5-methoxy-pyrimidin-2-yl)-amino]-6-ethyl-piperidine-1-carboxylic acid tert-butyl ester (racemic)

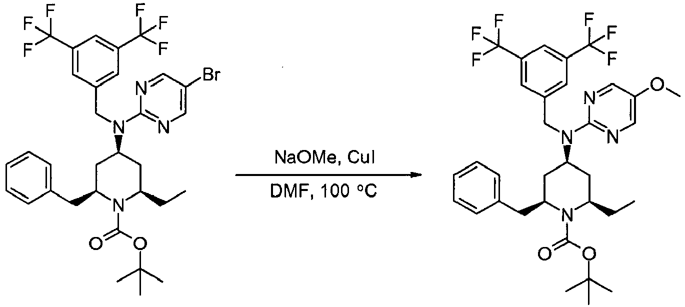

A mixture of 2-benzyl-4-[(3,5-bis-trifluoromethyl-benzyl)-(5-bromo-pyrimidin-2-yl)-amino]-6-ethyl-piperidine-1-carboxylic acid tert-butyl ester (125 mg, 0.162 mmol), sodium methoxide (25wt% in methanol, 111 uL), and copper iodide (62 mg, 0.326 mmol) in DMF (1.5 mL) is stirred at 85 °C for 2.5 hours. The mixture is cooled to room temperature and water and ethyl acetate are added. The mixture is filtered and the filtrate is washed with water and brine. The organic layer is concentrated under reduced pressure, and obtained residue is purified by preparatory HPLC to give 2-benzyl-4-[(3,5-bis-trifluoromethyl-benzyl)-(5-methoxy-pyrimidin-2-yl)-amino]-6-ethyl-piperidine-1-carboxylic acid tert-butyl ester (26 mg); ESI-MS m/z: 653 [M+1]⁺, Retention time 5.65 min (condition B).

The following compounds are prepared following the procedure of Example 13.

| No. | Product | ESI-MS m/z [M+1]⁺ | Retention time (min) | Starting Material |
|---|---|---|---|---|
| 1 | 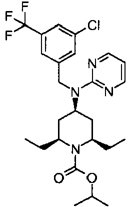 | 513 | 5.57 (condition B) | 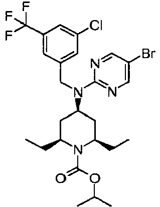 |
| 2 | 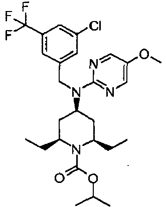 | 543 | 5.68 (condition B) | 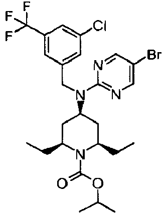 |

### Example 14: Synthesis of 1-{2-benzyl-4-[(3,5-bis-trifluoromethyl-benzyl)-(5-bromo-pyrimidin-2-yl)-amino]-6-ethyl-piperidin-1-yl}-2-cyclopentyl-ethanone (racemic)

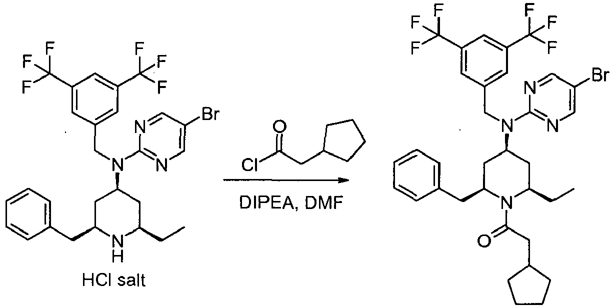

To a solution of 2-benzyl-6-ethyl-piperidin-4-yl)-(3,5-bis-trifluoromethyl-benzyl)-(5-bromo-pyrimidin-2-yl)-amine hydrochloric acid salt (50 mg, 0.078 mmol) dissolved in 1 mL of DMF is added diisopropylethylamine (54.6 uL, 0.313 mmol) and cyclopentylacetyl chloride (68 uL, 0.470 mmol) at room temperature. The solution is stirred at 60 °C for 18 hours. The mixture is purified by preparatory HPLC to give 1-{2-benzyl-4-[(3,5-bis-trifluoromethyl-benzyl)-(5-bromo-pyrimidin-2-yl)-amino]-6-ethyl-piperidin-1-yl}-2-cyclopentyl-ethanone (7.1 mg); ESI-MS m/z: 711 [M+1]⁺, Retention time 2.75 min (condition A).

The following compounds are prepared following the procedure of **Example 14.**

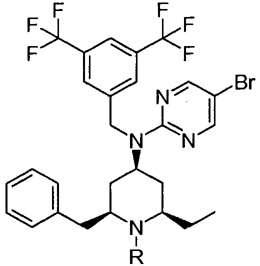

| No. | R | ESI-MS m/z [M+1]⁺ | Retention time (min) | Reagents (base/solvent) |
|---|---|---|---|---|
| 1 | 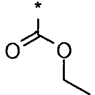 | 673 | 2.66 (condition A) | 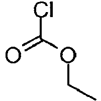 |
| | racemic | | | (Cs₂CO₃ / acetonitrile) |
| 2 | 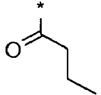 | 671 | 2.83 (condition A) | 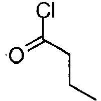 |
| | racemic | | | (DIPEA / DMF) |
| 3 | 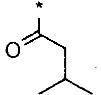 | 685 | 2.66 (condition A) | 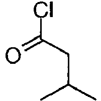 |
| | racemic | | | (DIPEA / DMF) |
| 4 | 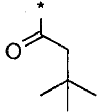 | 699 | 2.73 (condition A) | 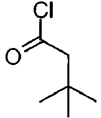 |
| | racemic | | | (DIPEA / DMF) |
| 5 | 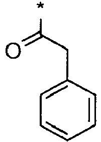 | 719 | 2.64 (condition A) | 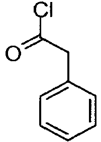 |
| | racemic | | | (DIPEA / DMF) |
| 6 | 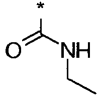 | 672 | 2.52 | 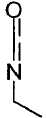 |
| | racemic | | | (DIPEA / DMF) |
| 7 | 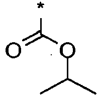 | 687 | 2.78 (condition A) | 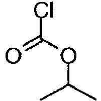 |
| | racemic | | | (DIPEA / DMF) |

### Example 15: Synthesis of 2-benzyl-4-[(3,5-bis-trifluoromethyl-benzyl)-(5-bromo-pyrimidin-2-yl)-amino]-6-ethyl-piperidin-1-yl-cyclohexyl-methanone (racemic)

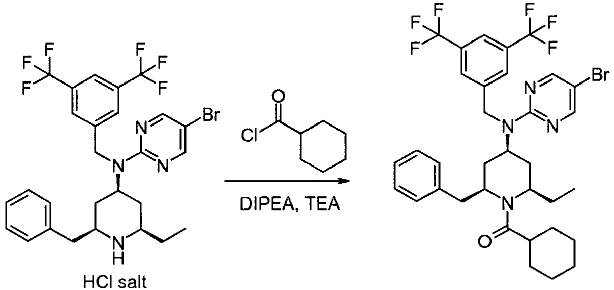

To a solution of 2-benzyl-6-ethyl-piperidin-4-yl)-(3,5-bis-trifluoromethyl-benzyl)-(5-bromo-pyrimidin-2-yl)-amine hydrochloric acid salt (50 mg, 0.078 mmol) dissolved in 1 mL of DMF is added triethylamine (110 uL, 0.78 mmol) and cyclohexanecarbonyl chloride (41 uL, 0.313 mmol) at room temperature. The solution is stirred at 150°C for 30 minutes by microwave reactor. The mixture is purified by preparatory HPLC to give 2-benzyl-4-[(3,5-bis-trifluoromethyl-benzyl)-(5-bromo-pyrimidin-2-yl)-amino]-6-ethyl-piperidin-1-yl-cyclohexyl-methanone (25 mg); ESI-MS m/z: 711 [M+1]⁺, Retention time 2.73 min (condition A).

The following compounds are prepared following the procedure of Example 15.

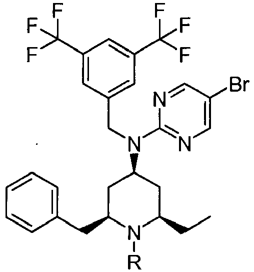

| No. | R | ESI-MS m/z [M+1]⁺ | Retention time (min) | Reagents (base/solvent) |
|---|---|---|---|---|
| 1 | 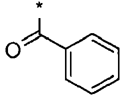 | 705 | 2.74 (condition A) | 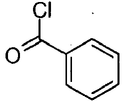 |
| | racemic | | | (TEA / DMF) |
| 2 | 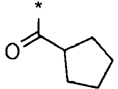 | 697 | 2.80 (condition A) | 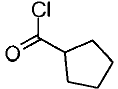 |
| | racemic | | | (TEA / DMF) |
| 3 | 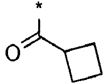 | 683 | 2.76 (condition A) | 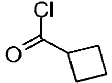 |
| | racemic | | | (TEA / DMF) |
| 4 | 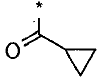 | 669 | 2.69 (condition A) | 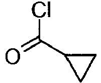 |
| | racemic | | | (TEA / DMF) |
| 5 | 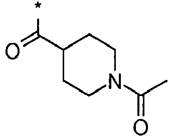 | 754 | 2.57 (condition A) | 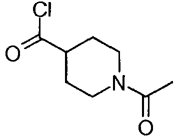 |
| | racemic | | | (TEA / DMF) |

### Example 16: Synthesis of (2S,4R,6R)-4-[(5-bromo-pyrimidin-2-yl)-(3-chloro-5-trifluoromethyl-benzyl)-amino]-2,6-diethyl-piperidine-1-carboxylic acid isopropyl ester

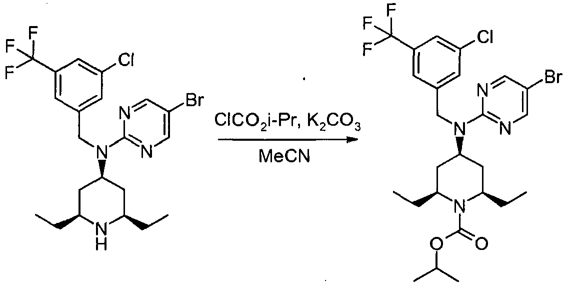

To a solution of (5-bromo-pyrimidin-2-yl)-(3-chloro-5-trifluoromethyl-benzyl)-((2S,4R,6R)-2,6-diethyl-piperidin-4-yl)-amine (386 mg, 0.74 mmol) in acetonitrile (3mL) is added potassium carbonate (138mg, 1.0mmol) and isopropyl chloroformate (0.11 mL, 1.0 mmol) at room temperature. The solution is stirred at 100 °C for 3 hours, and then saturated aq. NaHCO₃ is added. The mixture is extracted with ethyl acetate, and the organic layer is dried over Na₂SO₄, filtered, and concentrated under reduced pressure. The obtained residue is purified by silica gel column chromatography (eluent: n-hexane / ethyl acetate) to give (2S,4R,6R)-4-[(5-bromo-pyrimidin-2-yl)-(3-chloro-5-trifluoromethyl-benzyl)-amino]-2,6-diethyl-piperidine-1-carboxylic acid isopropyl ester (396 mg, 80%); ESI-MS m/z: 519 [M+1]⁺, Retention time 6.00 min (condition B).

### Example 17: Synthesis of acetic acid 4-{2-benzyl-4-[(3,5-bis-trifluoromethyl-benzyl)-(5-bromo-pyrimidin-2-yl)-amino]-6-ethyl-piperidine-1-carbonyl}-cyclohexyl ester

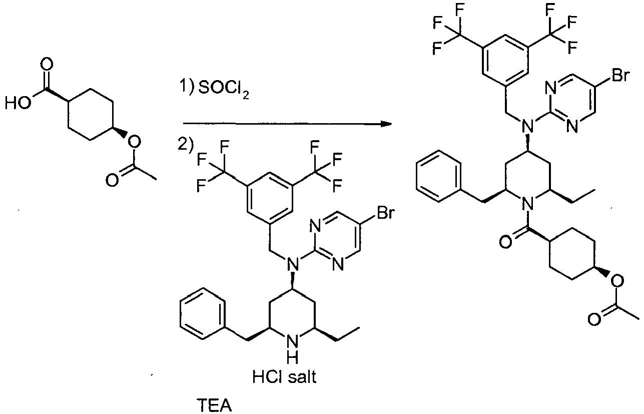

To a solution of 4-acetoxy-cyclohexanecarboxylic acid (73 mg, 0.392 mmol) dissolved in 1 mL of THF is added thionyl chloride (143 uL, 1.69 mmol) at room temperature. The solution is stirred at room temperature for 18 hours, and is concentrated under reduced pressure. To the residue is added a solution of 2-benzyl-6-ethyl-piperidin-4-yl)-(3,5-bis-trifluoromethyl-benzyl)-(5-bromo-pyrimidin-2-yl)-amine hydrochloric acid salt (50 mg, 0.078 mmol) and triethylamine (110 uL, 0.78 mmol) in 2 mL of DMF at room temperature. The solution is stirred at 150°C for 60 minutes by microwave reactor. The mixture is cooled to room temperature and water and dichloromethane are added. The organic layer is washed with water and brine, and concentrated under reduced pressure. The obtained residue is purified by preparatory HPLC to give acetic acid 4-{2-benzyl-4-[(3,5-bis-trifluoromethyl-benzyl)-(5-bromo-pyrimidin-2-yl)-amino]-6-ethyl-piperidine-1-carbonyl}-cyclohexyl ester (2.7 mg); ESI-MS m/z: 769 [M+1]⁺, Retention time 2.75 min (condition A).

The following compound is prepared following the procedure of **Example 17.**

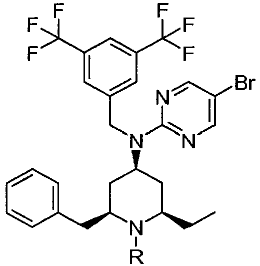

| No. | R | ESI-MS m/z [M+1]⁺ | Retention time (min) | Reagents |
|---|---|---|---|---|
| 1 | 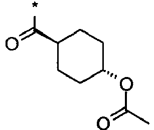 | 769 | 2.76 (condition A) | 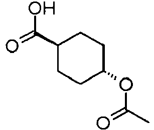 |
| | racemic | | | |

### Example 18: Synthesis of {2-benzyl-4-[(3,5-bis-trifluoromethyl-benzyl)-(5-bromo-pyrimidin-2-yl)-amino]-6-ethyl-piperidin-1-yl}-(4-hydroxy-cyclohexyl)-methanone

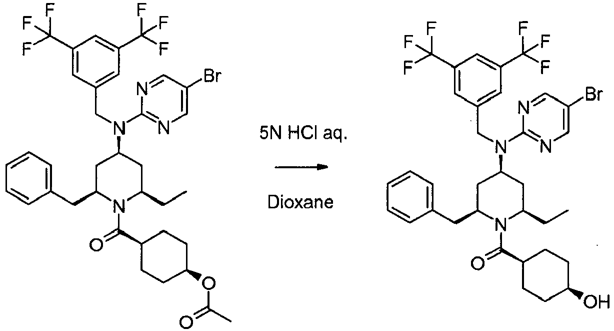

To a solution of Acetic acid 4-{2-benzyl-4-[(3,5-bis-trifluoromethyl-benzyl)-(5-bromo-pyrimidin-2-yl)-amino]-6-ethyl-piperidine-1-carbonyl}-cyclohexyl ester (4.3 mg, 0.00559 mmol) dissolved in 1 mL of 1,4-dioxane is 1 ml of 5 mol/L hydrochloric acid at room temperature. The solution is stirred at 100°C for 3 hours. The mixture is cooled to room temperature and water is added. The solvents are removed by lyophlization to give {2-benzyl-4-[(3,5-bis-trifluoromethyl-benzyl)-(5-bromo-pyrimidin-2-yl)-amino]-6-ethyl-piperidin-1-yl}-(4-hydroxy-cyclohexyl)-methanone (3.7 mg); ESI-MS m/z: 727 [M+1]⁺, Retention time 2.64 min (condition A).

The following compound is prepared following the procedure of **Example 18.**

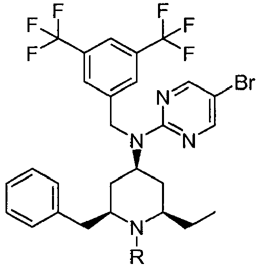

| No. | R | ESI-MS m/z [M+1]⁺ | Retention time (min) | Starting materials |
|---|---|---|---|---|
| 1 | 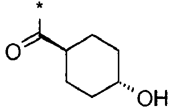 | 727 | 2.62 (condition A) | 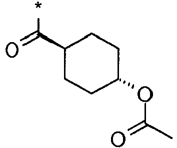 |
| | racemic | | | |

### Example 19: Synthesis acetic acid 4-{2-benzyl-4-[(3,5-bis-trifluoromethyl-benzyl)-(5-morpholin-4-yl-pyrimidin-2-yl)-amino]-6-ethyl-piperidine-1-carbonyl}-cyclohexyl ester

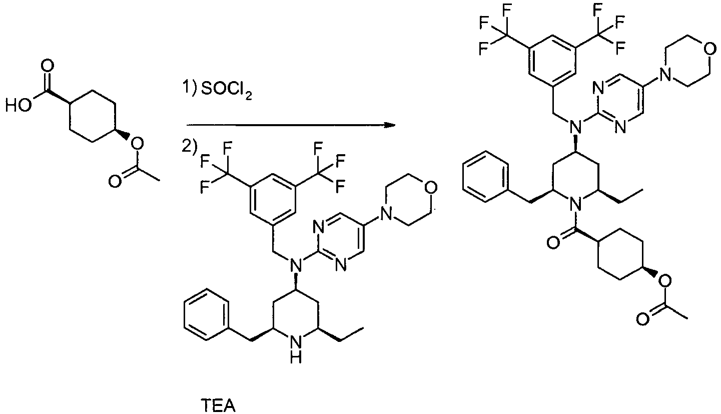

To a solution of 4-acetoxy-cyclohexanecarboxylic acid (753 mg, 4.05 mmol) dissolved in 5 mL of THF is added thionyl chloride (1.48 mL, 20.2 mmol) at room temperature. The solution is stirred at room temperature for 18 hours and then concentrated under reduced pressure. To the residue is added a solution of (2-benzyl-6-ethyl-piperidin-4-yl)-(3,5-bis-trifluoromethyl-benzyl)-(5-morpholin-4-yl-pyrimidin-2-yl)-amine (492 mg, 0.81 mmol) and triethylamine (1.70 mL, 12.2 mmol) in 10 mL of DMF at room temperature. The solution is stirred at 150°C for 60 minutes by microwave reactor. The mixture is cooled to room temperature and water and dichloromethane are added. The organic layer is washed with water and brine, and concentrated under reduced pressure. The obtained residue is purified by preparatory HPLC to give acetic acid 4-{2-benzyl-4-[(3,5-bis-trifluoromethyl-benzyl)-(5-morpholin-4-yl-pyrimidin-2-yl)-amino]-6-ethyl-piperidine-1-carbonyl}-cyclohexyl ester (163 mg); ESI-MS m/z: 776 [M+1]⁺, Retention time 2.39 min (condition A).

### Example 20: Synthesis of 2-benzyl-4-[(3,5-bis-trifluoromethyl-benzyl)-methoxycarbonyl-amino]-6-methoxymethyl-piperidine-1-carboxylic acid tert-butyl ester (racemic)

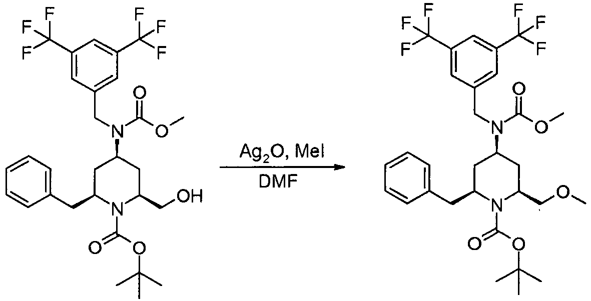

A mixture of 2-benzyl-4-[(3,5-bis-trifluoromethyl-benzyl)-methoxycarbonyl-amino]-6-hydroxymethyl-piperidine-1-carboxylic acid tert-butyl ester (10.9 mg, 0.018 mmol), Ag₂O (20.8 mg, 0.090 mmol), and iodomethane (11.2 uL, 0.180 mmol) in 0.4 mL of DMF is stirred at 60 °C for 10 hours. The obtained mixture is filtered and the filtrate is concentrated under reduced pressure. The obtained residue is purified by preparatory TLC to give 2-benzyl-4-[(3,5-bis-trifluoromethyl-benzyl)-methoxycarbonyl-amino]-6-methoxymethyl-piperidine-1-carboxylic acid tert-butyl ester (2 mg); ESI-MS m/z: 619 [M+1]⁺, Retention time 2.56 min (condition A).

### Example 21: Synthesis of 4-[(3,5-bis-trifluoromethyl-benzyl)-methoxycarbonyl-amino]-2-ethyl-6-methylcarbamoylmethyl-piperidine-1-carboxylic acid tert-butyl ester (racemic). methoxycarbonyl-amino]-6-methoxymethyl-piperidine-1-carboxylic acid tert-butyl ester (racemic)

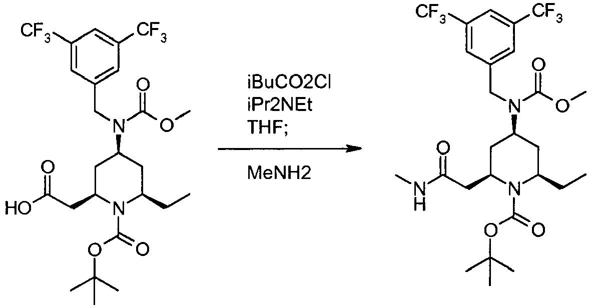

To a solution of 4-[(3,5-bis-trifluoromethyl-benzyl)-methoxycarbonyl-amino]-2-carboxymethyl-6-ethyl-piperidine-1-carboxylic acid tert-butyl ester (60 mg, 0.105 mmol) in THF (1 ml) is added iPr₂NEt (0.02 ml, 0.116 mmol). To the solution cooled to 0 °C are added ClCO₂*i*Bu (0.015 ml, 0.116 mmol) and *i*Pr₂NEt (0.02 ml, 116 mmol), followed by 2M MeNH₂ in THF (0.5 ml, 0.525 mmol). The mixture is stirred at rt for 1 h, quenched with water, extracted twice with ethyl acetate, and combined organic layers is washed with brine, dried over MgSO₄ and concentrated in vacuo to give a pale yellow oil. The crude oil is purified by silica gel column chromatography (EtOAc) to give 4-[(3,5-bis-trifluoromethyl-benzyl)-methoxycarbonyl-amino]-2-ethyl-6-methylcarbamoylmethyl-piperidine-1-carboxylic acid tert-butyl ester as a colorless oil (40 mg, 65% yield). ESI-MS m/z: 584 [M+1]⁺, Retention time 2.40 min (condition A).

### Example 22: Synthesis of 4-[(3,5-bis-trifluoromethyl-benzyl)-methoxycarbonyl-amino]-2-ethyl-6-(2-oxo-2-pyrrolidin-1-yl-ethyl)-piperidine-1-carboxylic acid tert-butyl ester (racemic).

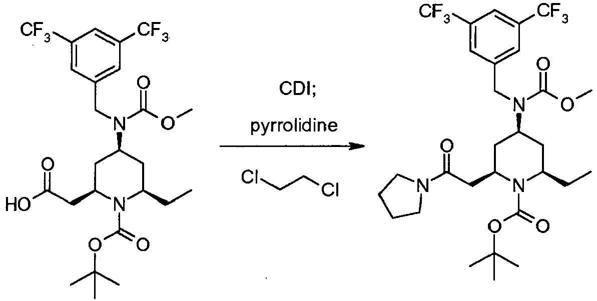

To a solution of 4-[(3,5-bis-trifluoromethyl-benzyl)-methoxycarbonyl-amino]-2-carboxymethyl-6-ethyl-piperidine-1-carboxylic acid tert-butyl ester (15 mg, 0.026 mmol) in 1,2-dichloroethane is added 1,1'-carbonyldiimidazole (6 mg, 0.034 mmol). After 1 h, pyrrolidine (0.011 ml, 0.13 mmol) is added and the mixture is stirred overnight at rt. The reaction mixture is concentrated in vacuo and directly purified by preparative TLC (4:1 EtOAc-Hex) to give 4-[(3,5-bis-trifluoromethyl-benzyl)-methoxycarbonyl-amino]-2-ethyl-6-(2-oxo-2-pyrrolidin-1-yl-ethyl)-piperidine-1-carboxylic acid tert-butyl ester as a colorless oil (16 mg, 97% yield). ESI-MS m/z: 624 [M+1]⁺, Retention time 2.56 min (condition A).

The following compounds are prepared following the procedure of **Example 22.**

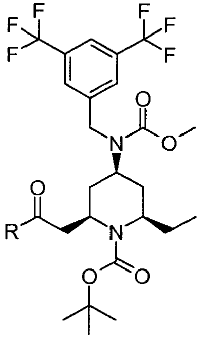

| No. | R | ESI-MS m/z [M+1]⁺ | Retention time (min) | Reagents |
|---|---|---|---|---|
| 1 | 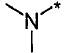 | 598 | 2.53 (condition A) | 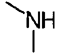 |
| | racemic | | | |
| 2 | 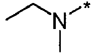 | 612 | 2.53 (condition A) | 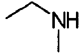 |
| | racemic | | | |
| 3 | 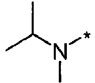 | 626 | 2.60 (condition A) | 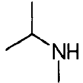 |
| | racemic | | | |

### Example 23: Synthesis of 4-[(3,5-bis-trifluoromethyl-benzyl)-methoxycarbonyl-amino]-2-ethyl-6-(2-hydroxy-3-methyl-butyl)-piperidine-1-carboxylic acid tert-butyl ester (single diastereomers, racemic).

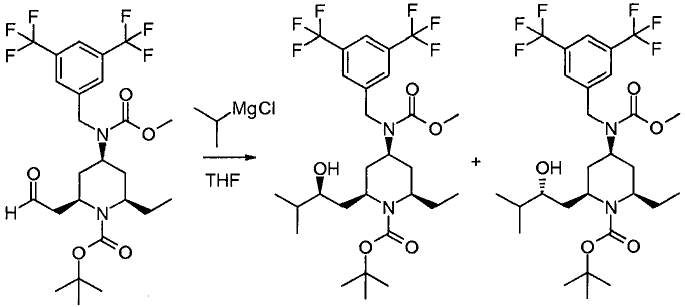

To a solution of 4-[(3,5-bis-trifluoromethyl-benzyl)-methoxycarbonyl-amino]-2-ethyl-6-(2-oxo-ethyl)-piperidine-1-carboxylic acid tert-butyl ester (22 mg, 0.040 mmol) in THF (1.5 ml) at -40 °C is added 2M iPrMgCl (0.22 ml, 0.436 mmol) and stirred at -40 °C for 1 h. The mixture is quenched with saturated aqueous ammonium chloride, extracted twice with ethyl acetate, washed with brine, dried over MgSO₄ and concentrated in vacuo. The crude product mixture is purified by preparative TLC (3:1 Hexane-EtOAc, twice) to give two diastereomers of 4-[(3,5-bis-trifluoromethyl-benzyl)-methoxycarbonyl-amino]-2-ethyl-6-(2-hydroxy-3-methyl-butyl)-piperidine-1-carboxylic acid tert-butyl ester (more polar 8 mg, less polar 3 mg). Polar isomer: ESI-MS m/z: 599 [M+1]⁺, retention time 2.61 min (condition A). Less polar isomer: ESI-MS m/z: 599 [M+1]⁺, retention time 2.72 min (condition A).

The following compound is prepared following the procedure of **Example 23.**

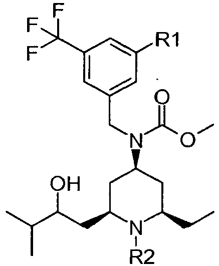

| No. | R1 | R2 | ESI-MS m/z [M+1]⁺ | Retention time (min) |
|---|---|---|---|---|
| 1 | 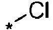 | 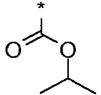 | 551 | 2.4 (condition A) |
| | racemic | | | |

### Example 24: Synthesis of 4-[(3,5-bis-trifluoromethyl-benzyl)-methoxycarbonyl-amino]-2-ethyl-6-(1-methyl-1H-imidazol-2-ylmethyl)-piperidine-1-carboxylic acid tert-butyl ester (racemic).

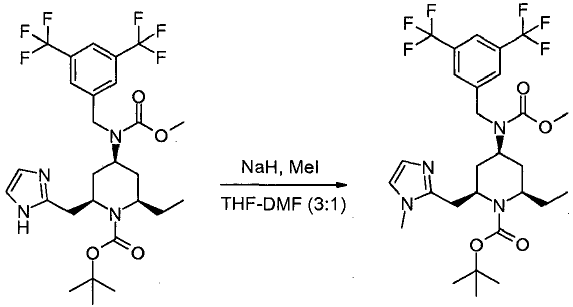

To a solution of 4-[(3,5-bis-trifluoromethyl-benzyl)-methoxycarbonyl-amino]-2-ethyl-6-(1 H-imidazol-2-ylmethyl)-piperidine-1-carboxylic acid tert-butyl ester (9 mg, 0.015 mmol) in THF (0.3 ml) and DMF (0.1 ml) is added NaH (1 mg, 0.015 mmol) followed by iodomethane (0.001 ml, 0.018 mmol). The mixture is stirred at rt for 2 h then quenched with water, extracted twice with ethyl acetate, washed with brine, dried over magnesium sulfate and concentrated. Preparative TLC (EtOAc 100%) give 4-[(3,5-bis-trifluoromethyl-benzyl)-methoxycarbonyl-amino]-2-ethyl-6-(1-methyl-1H-imidazol-2-ylmethyl)-piperidine-1-carboxylic acid tert-butyl as a colorless oil (5 mg, 54% yield). ESI-MS m/z: 607 [M+1]⁺, retention time 2.23 min (condition A).

### Example 25: Synthesis of 4-[(3,5-bis-trifluoromethyl-benzyl)-methoxycarbonyl-amino]-2-ethyl-6-(1H-imidazol-2-ylmethyl)-piperidine-1-carboxylic acid tert-butyl ester (racemic).

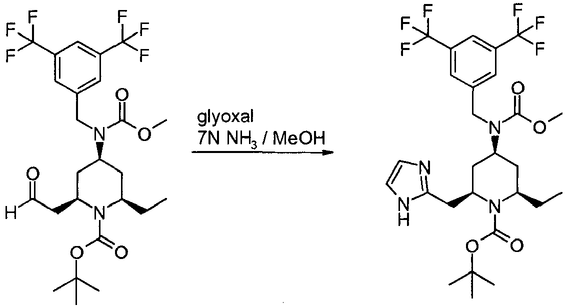

To a mixture of 4-[(3,5-Bis-trifluoromethyl-benzyl)-methoxycarbonyl-amino]-2-ethyl-6-(2-oxo-ethyl)-piperidine-1-carboxylic acid tert-butyl ester (18 mg, 0.0324 mmol) and 40% glyoxal in water (0.017 ml, 0.117 mmol) is added 7N ammonia in methanol (1.5 ml). The mixture is stirred 18 h at rt in a sealed tube. An additional amount of glyoxal (0.005 ml, 0.034 mmol) is added and the mixture is stirred for another 1 h. The mixture is then concentrated in vacuo and directly purified by preparative TLC (3% MeOH-CH₂Cl₂) to give 4-[(3,5-bis-trifluoromethyl-benzyl)-methoxycarbonyl-amino]-2-ethyl-6-(1H-imidazol-2-ylmethyl)-piperidine-1-carboxylic acid tert-butyl ester (23 mg, quant). ESI-MS m/z: 593 [M+1]⁺, retention time 2.25 min (condition A).

### Example 26: Synthesis of 4-[(3,5-bis-trifluoromethyl-benzyl)-methoxycarbonyl-amino]-2-ethyl-6-(2-hydroxy-but-3-ynyl)-piperidine-1-carboxylic acid tert-butyl ester (single diastereomers, racemic).

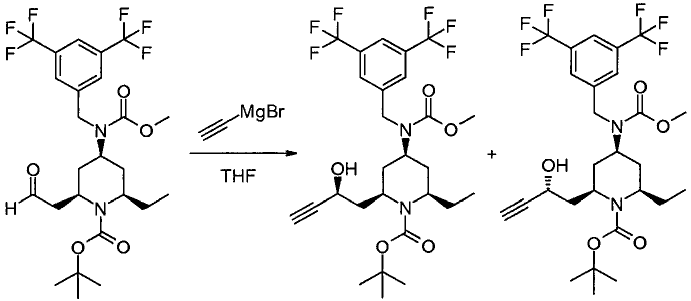

To a solution of ethynylmagnesium bromide in THF (1 ml, 0.5 mmol) and THF (1 ml) at 0 °C is added 4-[(3,5-bis-trifluoromethyl-benzyl)-methoxycarbonyl-amino]-2-ethyl-6-(2-oxo-ethyl)-piperidine-1-carboxylic acid tert-butyl ester in THF (2 ml). The mixture is stirred at rt for 1 h, then cooled to 0 °C again and another portion of ethylnylmagnesium bromide (1 ml, 0.5 mmol) is added. The mixture is stirred for 15 min, then quenched with sat. aq. NH₄Cl, extracted twice with ethyl acetate, the combined organic layer is washed with brine, dried over sodium sulfate and concentrated in vacuo to give a diastereomeric mixture of 4-[(3,5-bis-trifluoromethyl-benzyl)-methoxycarbonyl-amino]-2-ethyl-6-(2-hydroxy-but-3-ynyl)-piperidine-1-carboxylic acid tert-butyl as a colorless form (255 mg, quant). A small portion is then purified by preparative TLC to give two diastereomers (polar diastereomer: 7 mg, less polar diastereomer: 8 mg). Polar diastereomer: ESI-MS m/z: 581 [M+1]⁺, retention time 2.48 min (condition A). Less polar diastereomer: ESI-MS m/z: 581 [M+1]⁺, retention time 2.50 min (condition A).

The following compounds are prepared following the procedure of **Example 26.**

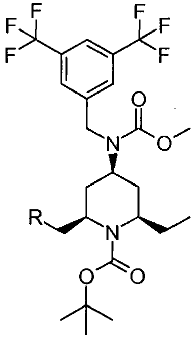

| No. | R1 | ESI-MS m/z [M+1]⁺ | Retention time (min) | Reagent |
|---|---|---|---|---|
| 1 | 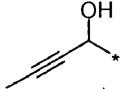 | 595 | 2.57 (condition A) | 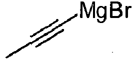 |
| | single diastereomer A | | | |
| 2 | 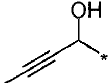 | 595 | 2.55 (condition A) | 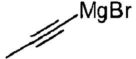 |
| | single diastereomer B | | | |

### Example 27: Synthesis of 4-[(3,5-bis-trifluoromethyl-benzyl)-methoxycarbonyl-amino]-2-ethyl-6-isoxazol-3-ylmethyl-piperidine-1-carboxylic acid tert-butyl ester and 4-[(3,5-Bis-trifluoromethyl-benzyl)-methoxycarbonyl-amino]-2-ethyl-6-isoxazol-5-ylmethyl-piperidine-1-carboxylic acid tert-butyl ester (mixture of two racemic compounds).

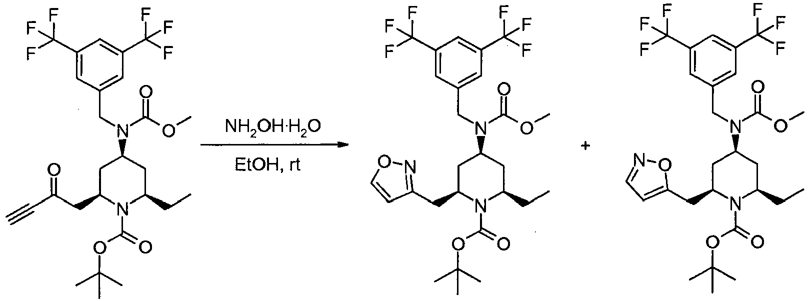

To a solution of 4-[(3,5-bis-trifluoromethyl-benzyl)-methoxycarbonyl-amino]-2-ethyl-6-(2-oxo-but-3-ynyl)-piperidine-1-carboxyfic acid tert-butyl ester (68 mg, 0.117 mmol) in ethanol (1 ml) is added 50% aqueous hydroxylamine (0.155 ml, 2.35 mmol). The mixture is stirred at rt for 10 min, then extracted twice with methylene chloride, combined organic phase is washed with brine, dried over magnesium sulfate and concentrated. The crude oil is purified by preparative TLC (2:1 Hex-Acetone) to give a 1:1 mixture of 4-[(3,5-bis-trifluoromethyl-benzyl)-methoxycarbonyl-amino]-2-ethyl-6-isoxazol-3-ylmethyl-piperidine-1-carboxylic acid tert-butyl ester and 4-[(3,5-bis-trifluoromethyl-benzyl)-methoxycarbonyl-amino]-2-ethyl-6-isoxazol-5-ylmethyl-piperidine-1-carboxylic acid tert-butyl ester (48 mg, 69% yield). ESI-MS m/z: 594 [M+1]⁺, retention time 4.28 min (single peak, condition C).

### Example 28: Synthesis of 4-[(3,5-bis-trifluoromethyl-benzyl)-methoxycarbonyl-amino]-2-ethyl-6-(2-methyl-2H-py razol-3-ylmethyl)-piperidine-1-carboxylic acid tert-butyl ester (racemic).

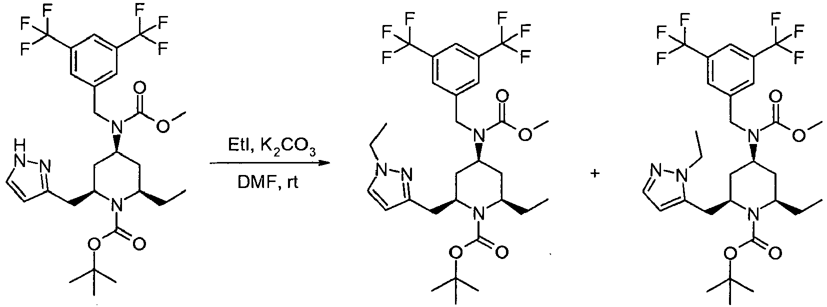

To a solution of 4-[(3,5-bis-trifluoromethyl-benzyl)-methoxycarbonyl-amino]-2-ethyl-6-(1 H-pyrazol-3-ylmethyl)-piperidine-1-carboxylic acid tert-butyl ester (20 mg, 0.0337 mmol) and potassium carbonate (9 mg, 0.0674 mmol) in DMF (0.2 ml) is added iodoethane (0.008 ml, 0.101 mmol) and stirred at rt for 20 h. To the mixture is again added iodoethane (0.1 ml, 1.25 mmol). The mixture is heated at 60 °C for 8 h, then water is added. The mixture is extracted with methylene chloride using phase separator. Purification by preparative TLC (1:1 EtOAc-Hex) give two regioisomers: 4-[(3,5-bis-trifluoromethyl-benzyl)-methoxycarbonyl-amino]-2-ethyl-6-(1-ethyl-1H-pyrazol-3-ylmethyl)-piperidine-1-carboxylic acid tert-butyl ester (9.3 mg, 44% yield). ESI-MS m/z: 621 [M+1]⁺, retention time 2.59 min (condition A); 4-[(3,5-bis-trifluoromethyl-benzyl)-methoxycarbonyl-amino]-2-ethyl-6-(2-ethyl-2H-pyrazol-3-ylmethyl)-piperidine-1-carboxylic acid tert-butyl ester (1.1 mg, 5% yield). ESI-MS m/z: 621 [M+1]⁺, retention time 2.57 min (condition A).

The following compounds are prepared following the procedure of **Example 28.**

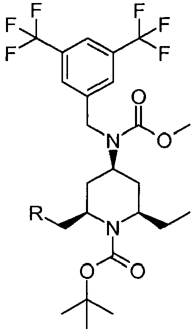

| No. | R1 | ESI-MS m/z [M+1]⁺ | Retention time (min) |
|---|---|---|---|
| 1 | 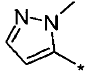 | 607 | 2.51 (condition A) |
| 2 | 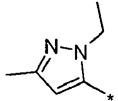 | 635 | 2.4 (condition A) |

### Example 29: Synthesis of 4-[(3,5-bis-trifluoromethyl-benzyl)-methoxycarbonyl-amino]-2-ethyl-6-(1H-pyrazol-3-ylmethyl)-piperidine-1-carboxylic acid tert-butyl ester (racemic).

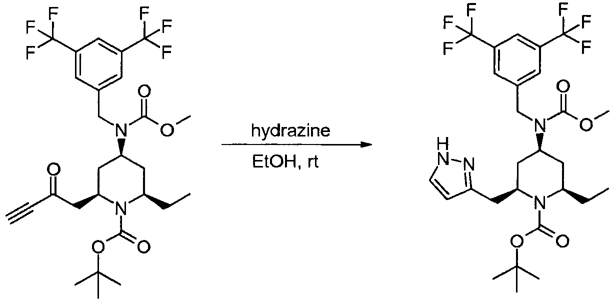

To a solution of 4-[(3,5-bis-trifluoromethyl-benzyl)-methoxycarbonyl-amino]-2-ethyl-6-(2-oxo-but-3-ynyl)-piperidine-1-carboxylic acid tert-butyl ester (117 mg, 0.202 mmol) in ethanol (2 ml) is added hydrazine monohydrate (0.196 ml, 4.04 mmol) and the mixture is stirred at rt for 2 h, then water is added. The mixture is extracted twice with methylene chloride, dried over magnesium sulfate and concentrated to give 4-[(3,5-bis-trifluoromethylbenzyl)-methoxycarbonyl-amino]-2-ethyl-6-(1H-pyrazol-3-ylmethyl)-piperidine-1-carboxylic acid tert-butyl ester as a colorless foam (120 mg, quant). ESI-MS m/z: 593 [M+1]⁺, retention time 2.36 min (condition A).

### Example 30: Synthesis of 4-[(3-chloro-5-trifluoromethyl-benzyl)-methoxycarbonyl-amino]-2-ethyl-6-(1-ethyl-1H-pyrazol-3-ylmethyl)-piperidine-1-carboxylic acid isopropyl ester (racemic) and 4-[(3-chloro-5-trifluoromethyl-benzyl)-methoxycarbonyl-amino]-2-ethyl-6-(2-ethyl-2H-pyrazol-3-ylmethyl)-piperidine-1-carboxylic acid isopropyl ester (racemic).

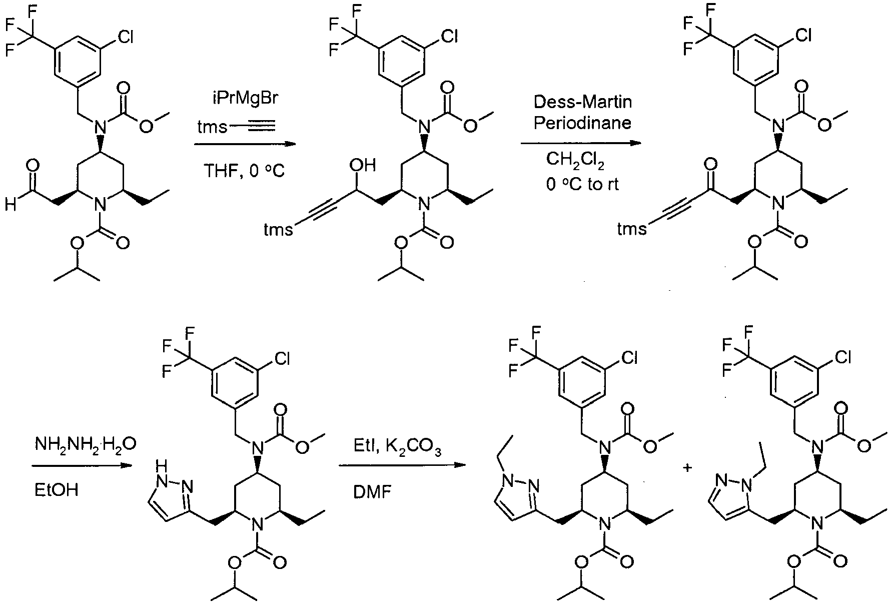

To a solution of trimethylsilylacetylene (0.236 ml, 1.70 mmol) in THF (3 ml) at -78 °C is added 0.8M iPrMgBr (2.1 ml, 1.70 mmol), followed by 4-[(3-chloro-5-trifluoromethyl-benzyl)-methoxycarbonyl-amino]-2-ethyl-6-(2-oxo-ethyl)-piperidine-1-carboxylic acid isopropyl ester (430 mg, 0.848 mmol) in THF (3 ml). The mixture is warmed to rt and stirred for 3 h. The reaction is quenched with sat. aq. ammonium chloride, extracted twice with ethyl acetate and combined organic layer is washed with brine, dried over magnesium sulfate and concentrated to give crude alcohol. To a solution of the crude alcohol in methylene chloride (10 ml) at 0 °C is added Dess-Martin periodinane (1.45 g, 3.42 mmol) and stirred for 3 h. The reaction mixture is diluted with diethyl ether, quenched with sat. aq. Na₂S₂O₄ then extracted twice with diethyl ether, combined organic layer is washed with sat. aq. NaHCO₃, brine, dried over sodium sulfate and concentrated in vacuo. Purification by silica gel column chromatography (EtOAc-Hexane) give 4-[(3-chloro-5-trifluoromethyl-benzyl)-methoxycarbonyl-amino]-2-ethyl-6-(2-oxo-4-trimethylsilanyl-but-3-ynyl)-piperidine-1-carboxylic acid isopropyl ester as a colorless foam (298 mg, 58% yield). ESI-MS m/z: 603 [M+1]⁺, retention time 2.51 min (condition A).

To a solution of 4-[(3-chloro-5-trifluoromethyl-benzyl)-methoxycarbonyl-amino]-2-ethyl-6-(2-oxo-4-trimethylsilanyl-but-3-ynyl)-piperidine-1-carboxylic acid isopropyl ester (264 mg, 0.438 mmol) in ethanol (3.5 ml) is added hydrazine monohydrate (0.425 ml, 8.76 mmol). The mixture is stirred for 2.5 h then quenched with water, extracted twice with ethyl acetate, combined organic layer is washed with brine, dried over magnesium sulfate and concentrated in vacuo to give 4-[(3-chloro-5-trifluoromethyl-benzyl)-methoxycarbonyl-amino]-2-ethyl-6-(1 H-pyrazol-3-ylmethyl)-piperidine-1-carboxylic acid isopropyl ester as a colorless foam (243 mg, quant). ESI-MS m/z: 545 [M+1]⁺, retention time 2.10 min (condition A).

To a solution of 4-[(3-chloro-5-trifluoromethyl-benzyl)-methoxycarbonyl-amino]-2-ethyl-6-(1 H-pyrazol-3-ylmethyl)-piperidine-1-carboxylic acid isopropyl ester (243 mg, 0.446 mmol) in THF (4.5 ml) and DMF (1.5 ml) at 0 °C is added NaH (21 mg, ca. 0.147 mmol). The mixture is heated to 60 °C then iodoethane (0.071 ml, 0.892 mmol) is added and stirred for 70 min then quenched with water. The mixture is extracted twice with ethyl acetate, washed twice with 5% aq. NaCl, once with brine, dried over magnesium sulfate and concentrated. The crude product is purified first with silica gel column chromatography followed by preparative TLC (3% MeOH-CH₂Cl₂) to give two regioisomers: 4-[(3-chloro-5-trifluoromethylbenzyl)-methoxycarbonyl-amino]-2-ethyl-6-(1-ethyl-1H-pyrazol-3-ylmethyl)-piperidine-1-carboxylic acid isopropyl ester (279 mg). ESI-MS m/z: 573 [M+1]⁺, retention time 4.49 min (condition C). 4-[(3-Chloro-5-trifluoromethyl-benzyl)-methoxycarbonyl-amino]-2-ethyl-6-(2-ethyl-2H-pyrazol-3-ylmethyl)-piperidine-1-carboxylic acid isopropyl ester (18.2 mg). ESI-MS m/z: 573 [M+1]⁺, retention time 4.38 min (condition C).

### Example 31: Synthesis of 4-(3,5-ditrifluoromethyl-benzyl-methoxycarbonyl-amino)-2,6-trans-diisopropyl-piperidine-1-carboxylic acid tert-butyl ester (racemic)

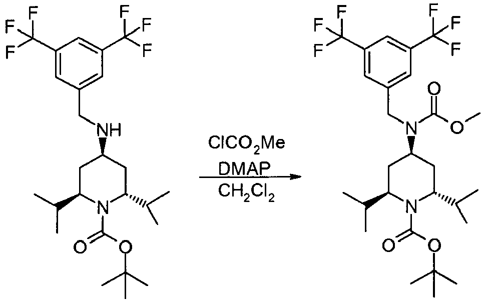

To a mixture of 4-(3,5-ditrifluoromethyl-benzylamino)-2,6-diisopropyl-piperidine-1-carboxylic acid tert-butyl ester (0.034 mmol, 18.6 mg) and DMAP (0.068 mmol, 8.2 mg) in CH₂Cl₂ (0.5 mL) is added methylchloroformate (0.068 mmol, 5 uL) at room temperature. After stirred for 5 hours at room temperature, reaction mixture is purified by PTLC (eluent: n-hexane / ethyl acetate) to give 4-(3,5-ditrifluoromethyl-benzyl-methoxycarbonyl-amino)-2,6-*trans*-diisopropyl-piperidine-1-carboxylic acid tert-butyl ester (17.3 mg, 57%); ESI-MS m/z: 569.00 [M+1]⁺, Retention time 2.71 min (condition A).

### Example 32: Synthesis of 4-{(3-chloro-5-trifluoromethyl-benzyl)-[5-(1-methyl-1H-pyrazol-4-yl)-pyrimidin-2-yl]-amino}-2,6-trans-diisopropyl-piperidine-1-carboxylic acid isopropyl ester (racemic)

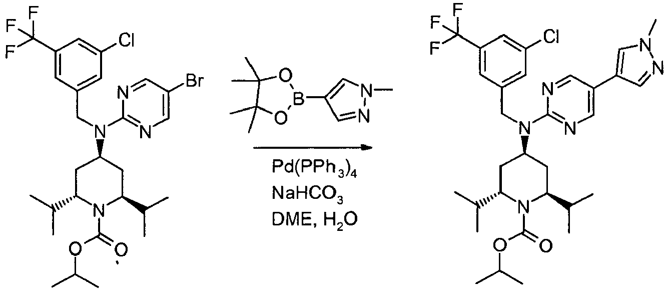

To a mixture of 4-[(3,5-bis-trifluoromethyl-benzyl)-(5-bromo-pyrimidin-2-yl)-amino]-2,6-trans -diisopropyl-piperidine-1-carboxylic acid isopropyl ester (0.24 mmol, 148 mg) in a flask purged with N₂ are added Pd(PPh₃)₄ (0.05 mmol,75 mg), 1-Methylpyrazole-4-boronic acid pinacol ester (0.36 mmol, 75 mg), sodium hydrogen carbonate (0.46 mmol, 40 mg) in DME and H₂O. The reaction mixture is heated at 95 °C for 2 hrs. After separating aqueous layer, organic layer is filtered and washed with brine, dried over Na₂SO₄. The obtained residue is purified by silica gel column chromatography (eluent: *n*-hexane / ethyl acetate) to give 4-{(3-chloro-5-trifluoromethyl-benzyl)-[5-(1-methyl-1H-pyrazol-4-yl)-pyrimidin-2-yl]-amino}-2,6-*trans*-diisopropyl-piperidine-1-carboxylic acid isopropyl ester (107 mg, 72%); ESI-MS m/z: 621.94 [M+1]⁺, Retention time 2.59 min (condition A).

### Example 33: Synthesis of 4-[(3-chloro-5-trifluoromethyl-benzyl)-(5-furan-3-yl-pyrimidin-2-yl)-amino]-2,6-diethyl-piperidine-1-carboxylic acid isopropyl ester.

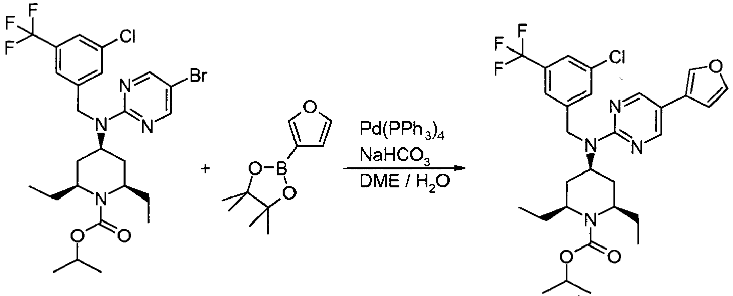

A mixture of 4-[(5-bromo-pyrimidin-2-yl)-(3-chloro-5-trifluoromethyl-benzyl)-amino]-2,6-diethyl-piperidine-1-carboxylic acid isopropyl ester (0.1 mmol, 59 mg), 2-furan-3-yl-4,4,5,5-tetramethyl-[1,3,2]dioxaborolane (0.15 mmol, 29 mg), tetrakis(triphenylphosphine)palladium(0) (0.01 mmol, 11.5 mg) and sodium hydrogen carbonate (0.2 mmol, 17 mg) in 1,2-dimethoxy-ethane (1 mL) and water (0.4 mL) is allowed to warm to 95 °C and stirred for 3 hours. The mixture is cooled to room temperature and then added water. The mixture is extracted with CH₂Cl₂. The combined organic layer is washed with brine, dried over Na₂SO₄, filtrated, and concentrated under reduced pressure. The obtained residue is purified by silica gel column chromatography (eluent: hexane / EtOAc) to give 4-[(3-Chloro-5-trifluoromethyl-benzyl)-(5-furan-3-yl-pyrimidin-2-yl)-amino]-2,6-diethyl-piperidine-1-carboxylic acid isopropyl ester (34 mg, 59%); ESI-MS m/z: 579 [M+1]⁺, Retention time 2.60 min (condition A).

The following compounds are prepared following the procedure of **Example 33.**

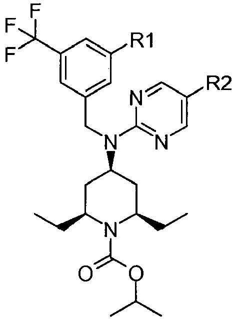

| No. | R1 | R2 | ESI-MS m/z [M+1]⁺ | Retention time (min) | Reagents |
|---|---|---|---|---|---|
| 1 | 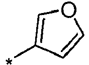 | 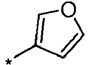 | 611 | 2.54 (condition A) | 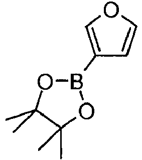 |
| 2 | 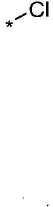 | 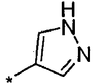 | 579 | 2.42 (condition A) | 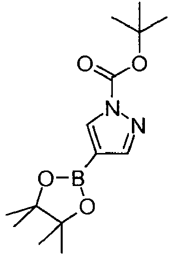 |
| 3 | 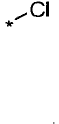 | 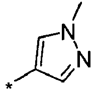 | 593 | 2.49 (condition A) | 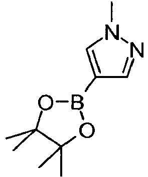 |
| 4 | 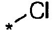 | 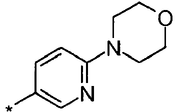 | 675 | 2.21 (condition A) | 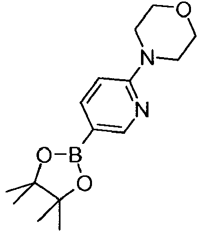 |
| 5 | 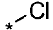 | 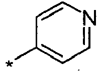 | 590 | 2.20 (condition A) | 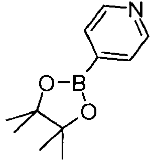 |
| 6 | 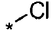 | 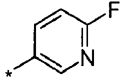 | 608 | 2.56 (condition A) | 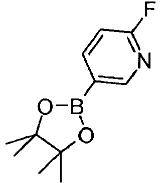 |
| 7 | 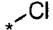 | 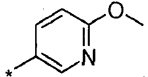 | 620 | 2.73 (condition A) | 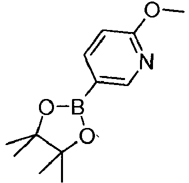 |
| 8 | 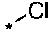 | 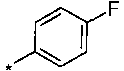 | 607 | 2.62 (condition A) | 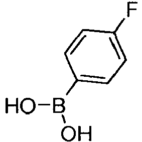 |
| 9 | 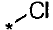 | 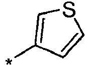 | 595 | 2.60 (condition A) | 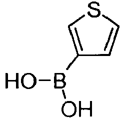 |
| 10 | 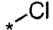 | 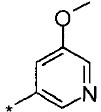 | 620 | 2.24 (condition A) | 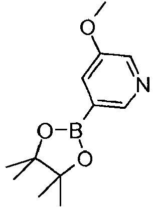 |
| 11 | 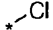 | 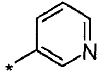 | 590 | 2.20 (condition A) | 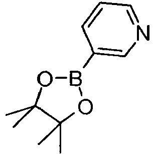 |
| 12 | 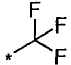 | 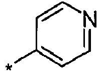 | 624 | 2.12 (condition A) | 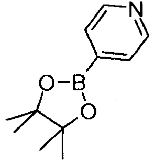 |

The following compounds are prepared following the procedure of Example 33.

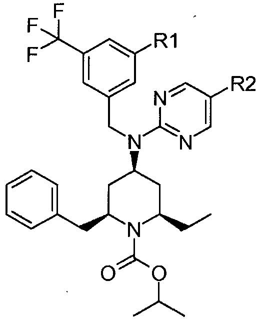

| No. | R1 | R2 | ESI-MS m/z [M+1]⁺ | Retention time (min) | Reagents |
|---|---|---|---|---|---|
| 1 | 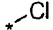 | 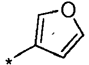 | 641 | 5.78 (condition B) | 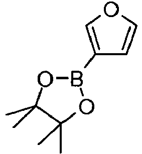 |
| 2 | 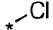 | 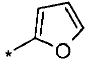 | 641 | 5.97 (condition B) | 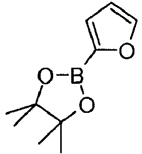 |
| 3 | 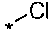 | 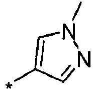 | 655 | 5.52 (condition B) | 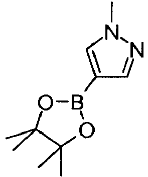 |

### Example 34: Synthesis of (2R,4R,6S)-4-[(3-chloro-5-trifluoromethyl-benzyl)-(5-thiazol-2-yl-pyrimidin-2-yl)-amino]-2,6-diethyl-piperidine-1-carboxylic acid isopropyl ester

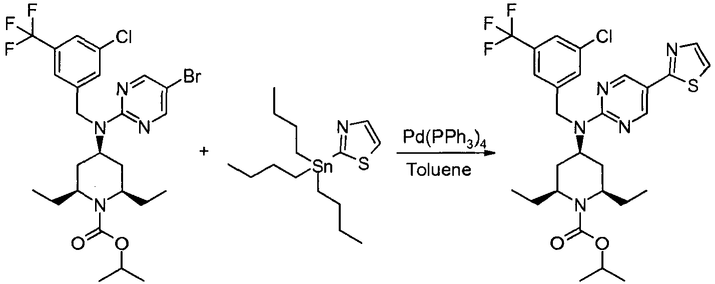

A mixture of (2S,4R,6R)-4-[(5-bromo-pyrimidin-2-yl)-(3-chloro-5-trifluoromethyl-benzyl)-amino]-2,6-diethyl-piperidine-1-carboxylic acid isopropyl ester (100 mg, 0.17 mmol), 2-tributylstannanyl-thiazole (64 uL, 0.20 mmol), and Pd(PPh₃)₄ (20 mg, 0.017 mmol) in toluene (2 mL) under nitrogen is stirred for 2 hours at 110 °C then 3 hours at 130 °C. The mixture is cooled to room temperature, and the obtained residue is purified by PTLC to give (2R,4R,6S)-4-[(3-chloro-5-trifluoromethyl-benzyl)-(5-thiazol-2-yl-pyrimidin-2-yl)-amino]-2,6-diethyl-piperidine-1-carboxylic acid isopropyl ester (63 mg); ESI-MS m/z: 596 [M+1]⁺, Retention time 2.59 min (condition A).

The following compounds are prepared following the procedure of **Example 34.**

| No. | Product | ESI-MS m/z [M+1]⁺ | Retention time (min) | Reagent |
|---|---|---|---|---|
| 1 | 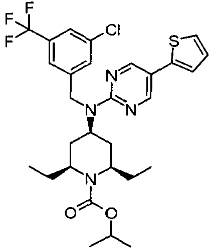 | 595 | 2.65 (condition A) | 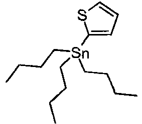 |
| 2 | 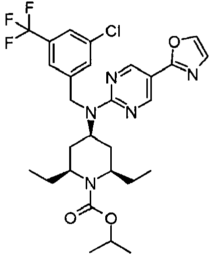 | 580 | 2.56 (condition A) | 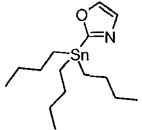 |

### Example 35: Synthesis of (2R,4R,6S)-4-{(3-chloro-5-trifluoromethyl-benzyl)-[5-(1H-pyrazol-4-yl)-pyrimidin-2-yl]-amino}-2,6-dimethyl-piperidine-1-carboxylic acid isopropyl ester

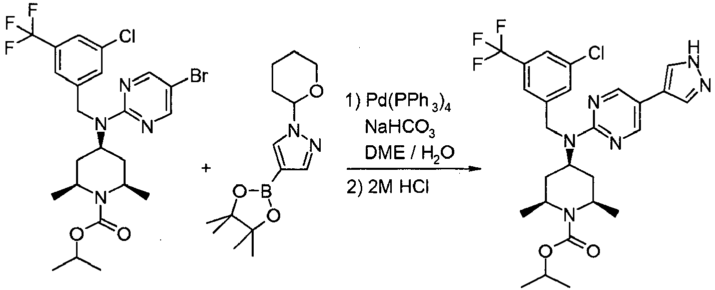

A mixture of (2R,4R,6S)-4-[(5-bromo-pyrimidin-2-yl)-(3-chloro-5-trifluoromethyl-benzyl)-amino]-2,6-dimethyl-piperidine-1-carboxylic acid isopropyl ester (54 mg, 0.0958 mmol), 1-(tetrahydro-pyran-2-yl)-4-(4,4,5,5-tetramethyl-[1,3,2]dioxaborolan-2-yl)-1H-pyrazole (0.144 mmol), tetrakis(triphenylphosphine)palladium(0) (0.01 mmol, 11 mg) and sodium hydrogen carbonate (0.2 mmol, 20 mg) in 1,2-dimethoxy-ethane (1 mL) and water (0.2 mL) is allowed to warm to 90 °C and stirred for 6 hours. The mixture is cooled to room temperature and then added 2M HCl in methanol. After stirred for 1 hour, the mixture is basified with saturated sodium bicarbonate and extracted with ethyl acetate. The combined organic layer is washed with brine, dried over Na₂SO₄, filtrated, and concentrated under reduced pressure. The obtained residue is purified by PTLC (eluent: hexane / EtOAc) to give (2R,4R,6S)-4-{(3-chloro-5-trifluoromethyl-benzyl)-[5-(1H-pyrazol-4-yl)-pyrimidin-2-yl]-amino}-2,6-dimethyl-piperidine-1-carboxylic acid isopropyl ester (6 mg); ESI-MS m/z: 551 [M+1]⁺, Retention time 2.29 min (condition A).

The following compounds are prepared following the procedure of **Example 35.**

| No. | Product | ESI-MS m/z [M+1]⁺ | Retention time (min) | Starting Material |
|---|---|---|---|---|
| 1 | 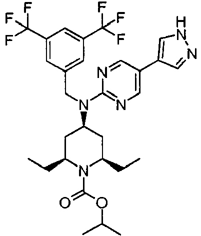 | 613 | 2.34 | 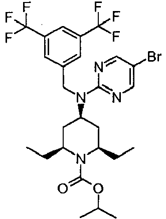 |
| 2 | 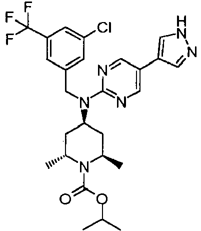 | 551 | 2.23 (condition A) | 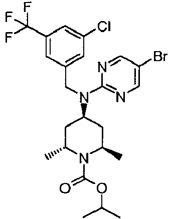 |

### Example 36: Synthesis of (2R,4R,6S)-4-[(5-amino-pyrimidin-2-yl)-(3-chloro-5-trifluoromethyl-benzyl)-amino]-2,6-diethyl-piperidine-1-carboxylic acid isopropyl ester

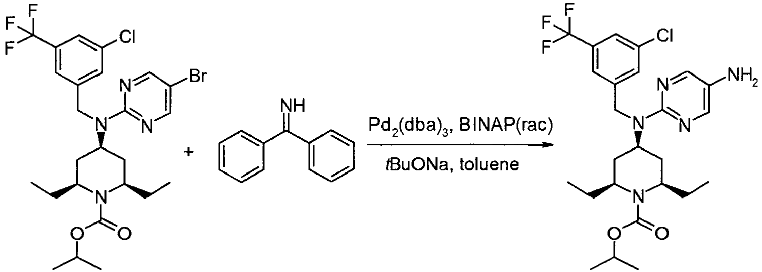

A mixture of (2R,4R,6S)-4-[(5-bromo-pyrimidin-2-yl)-(3-chloro-5-trifluoromethyl-benzyl)-amino]-2,6-dimethyl-piperidine-1-carboxylic acid isopropyl ester (365 mg, 0.617 mmol), benzhydrylideneamine (124 uL, 0.740 mmol), Pd₂(dba)₃ (56 mg, 0.0617 mmol) and sodium *t*-butoxide (89 mg, 0.925 mmol) in toluene (4 mL) is stirred at 110 °C for 3 hours. After the mixture is cooled to room temperature, water is added and extracted with ethyl acetate. The organic layer is washed with brine, dried over Na₂SO₄, filtrated, and concentrated under reduced pressure. The obtained residue is dissolved in THF (4 mL) and aqueous 2M HCl (4 mL) is added. The mixture is stirred for 30 minutes, and cooled to 0 °C. To the mixture is added aqueous 5M NaOH, and extracted with ethyl acetate. The organic layer is washed with brine, dried over Na₂SO₄, filtrated, and concentrated under reduced pressure. The obtained residue is purified by silica gel column chromatography (eluent: hexane / EtOAc) to give (2R,4R,6S)-4-[(5-amino-pyrimidin-2-yl)-(3-chloro-5-trifluoromethyl-benzyl)-amino]-2,6-diethyl-piperidine-1-carboxylic acid isopropyl ester (253 mg); ESI-MS m/z: 528 [M+1]⁺, Retention time 2.16 min (condition A).

The following compound is prepared following the procedure of **Example 36.**

| No. | Product | ESI-MS m/z [M+1]⁺ | Retention time (min) | Starting Material |
|---|---|---|---|---|
| 1 | 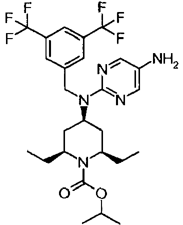 | 562 | 2.09 (condition A) | 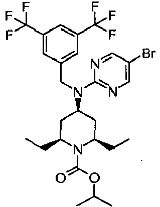 |

### Example 37: Synthesis of (2R,4R,6S)-4-[(3-chloro-5-trifluoromethyl-benzyl)-(5-tetrazol-1-yl-pyrimidin-2-yl)-amino]-2,6-diethyl-piperidine-1-carboxylic acid isopropyl ester

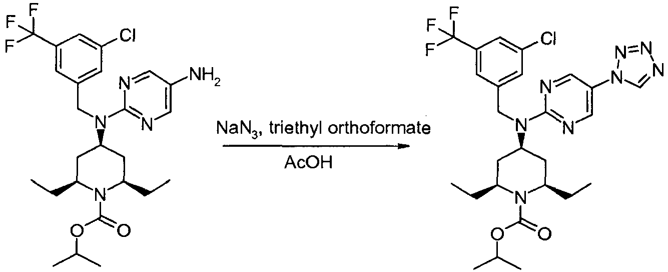

To a mixture of (2R,4R,6S)-4-[(5-amino-pyrimidin-2-yl)-(3-chloro-5-trifluoromethyl-benzyl)-amino]-2,6-diethyl-piperidine-1-carboxylic acid isopropyl ester (74 mg, 0.140 mmol) in acetic acid (1 mL) is added triethyl orthoformate (35 uL, 0.2 mmol). After the mixture is stirred at 75 °C for 30 minutes, sodium azide (27 mg, 0.420 mmol) is added and stirred at 80 °C for 3 hours. After the mixture is cooled to room temperature, water is added and extracted with ethyl acetate. The organic layer is washed with brine, dried over Na₂SO₄, filtrated, and concentrated under reduced pressure. The obtained residue is purified by silica gel column chromatography (eluent: hexane / EtOAc) to give (2R,4R,6S)-4-[(3-chloro-5-trifluoromethyl-benzyl)-(5-tetrazol-1-yl-pyrimidin-2-yl)-amino]-2,6-diethyl-piperidine-1-carboxylic acid isopropyl ester (70 mg); ESI-MS m/z: 581 [M+1]⁺, Retention time 2.42 min (condition A).

The following compound is prepared following the procedure of **Example 37.**

| No. | Product | ESI-MS m/z [M+1]⁺ | Retention time (min) | Starting Material |
|---|---|---|---|---|
| 1 | 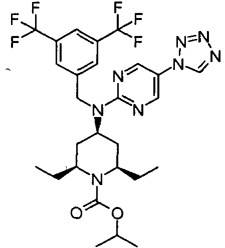 | 615 | 2.35 (condition A) | 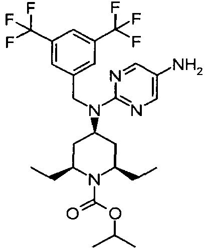 |

### Example 38: Synthesis of (2R,4R,6S)-4-[(3-chloro-5-trifluoromethyl-benzyl)-(5-[1,2,4]triazol-4-yl-pyrimidin-2-yl)-amino]-2,6-diethyl-piperidine-1-carboxylic acid isopropyl ester

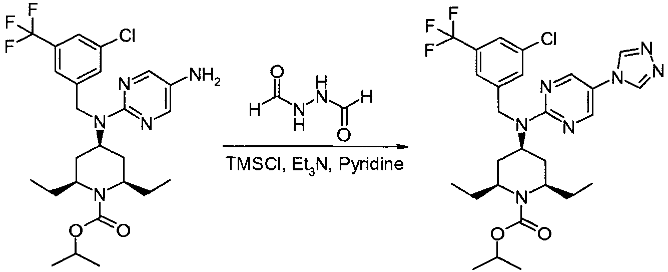

A mixture of (2R,4R,6S)-4-[(5-amino-pyrimidin-2-yl)-(3-chloro-5-trifluoromethyl-benzyl)-amino]-2,6-diethyl-piperidine-1-carboxylic acid isopropyl ester (65 mg, 0.123 mmol), 1,2-diformylhydrazine (33 mg, 0.369 mmol), TMSCI (156 uL, 1.23 mmol), and triethylamine (103 uL, 0.739 mmol) in pyridine (1 mL) is stirred at 95 °C for 16 hours. After the mixture is cooled to room temperature, water is added and extracted with ethyl acetate. The organic layer is washed with brine, dried over Na₂SO₄, filtrated, and concentrated under reduced pressure. The obtained residue is purified by silica gel column chromatography (eluent: methanol / dichloromethane) to give (2R,4R,6S)-4-[(3-chloro-5-trifluoromethyl-benzyl)-(5-[1,2,4]triazol-4-yl-pyrimidin-2-yl)-amino]-2,6-diethyl-piperidine-1-carboxylic acid isopropyl ester (51 mg); ESI-MS m/z: 580 [M+1]⁺, Retention time 2.24 min (condition A).

### Example 39: Synthesis of (2R,4R,6S)-4-{(3-chloro-5-trifluoromethyl-benzyl)-[5-(2-oxo-imidazolidin-1-yl)-pyrimidin-2-yl]-amino}-2,6-diethyl-piperidine-1-carboxylic acid isopropyl ester

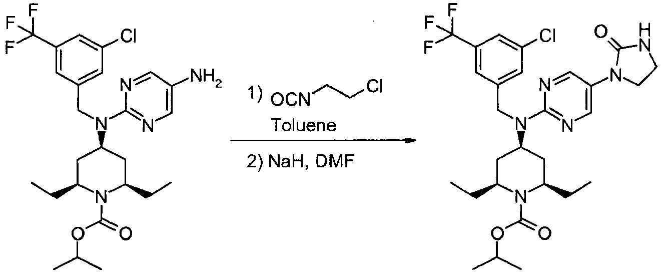

A mixture of (2R,4R,6S)-4-[(5-amino-pyrimidin-2-yl)-(3-chloro-5-trifluoromethyl-benzyl)-amino]-2,6-diethyl-piperidine-1-carboxylic acid isopropyl ester (74 mg, 0.140 mmol) and 2-chloroethyl isocyanate (34 uL) in toluene (1 mL) is stirred at room temperature for 1 hour. To the mixture is added water and extracted with ethyl acetate. The organic layer is washed with brine, dried over Na₂SO₄, filtrated, and concentrated under reduced pressure. The obtained residue passed through a pad of silica gel and the filtrate is concentrated under reduced pressure. The obtained is dissolved in DMF (1 mL) and added sodium hydride (60% in oil: 6 mg, 0.147 mmol). After stirred for 1.5 hours, water is added and extracted with ethyl acetate. The organic layer is washed with brine, dried over Na₂SO₄, filtrated, and concentrated under reduced pressure to give (2R,4R,6S)-4-{(3-chloro-5-trifluoromethyl-benzyl)-[5-(2-oxo-imidazolidin-1-yl)-pyrimidin-2-yl]-amino}-2,6-diethyl-piperidine-1-carboxylic acid isopropyl ester; ESI-MS m/z: 597 [M+1]⁺, Retention time 2.33 min (condition A).

### Example 40: Synthesis of 4-{(3-chloro-5-trifluoromethyl-benzyl)-[5-(2-oxo-oxazolidin-3-yl)-pyrimidin-2-yl]-amino}-2,6-diethyl-piperidine-1-carboxylic acid isopropyl ester.

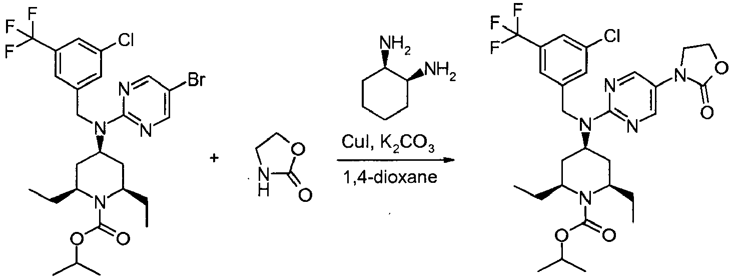

A mixture of 4-[(5-bromo-pyrimidin-2-yl)-(3-chloro-5-trifluoromethyl-benzyl)-amino]-2,6-diethyl-piperidine-1-carboxylic acid isopropyl ester (0.05 mmol, 30 mg), oxazolidin-2-one (0.05 mmol, 5 mg), cupper iodide (0.05 mmol, 10 mg), *trans*-cyclohexane-1,2-diamine (0.05 mmol, 6 mg) and potassium carbonate (0.1 mmol, 14 mg) in 1,4-dioxane (0.3 mL) is allowed to warm to 110 °C and stirred for 19 hours. The mixture is cooled to room temperature and then added aq. ammonia. The mixture is extracted with CH₂Cl₂. The combined organic layer is washed with brine, dried over Na₂SO₄, filtrated, and concentrated under reduced pressure. The obtained residue is purified by silica gel column chromatography (eluent: n-hexane / EtOAc) to give 4-{(3-chloro-5-trifluoromethyl-benzyl)-[5-(2-oxo-oxazolidin-3-yl)-pyrimidin-2-yl]-amino}-2,6-diethyl-piperidine-1-carboxylic acid isopropyl ester (26 mg, 87%); ESI-MS m/z: 598 [M+1]⁺, Retention time 2.44 min (condition A).

The following compounds are prepared following the procedure of Example 40.

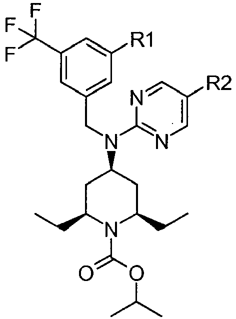

| No. | R1 | R2 | ESI-MS m/z [M+1]⁺ | Retention time (min) | Reagents |
|---|---|---|---|---|---|
| 1 | 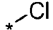 | 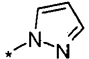 | 579 | 2.56 (condition A) | 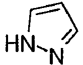 |
| | | | | | L-Proline instead of *trans-*cyclohexane-1,2-diamine |
| 2 | 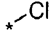 | 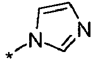 | 579 | 2.18 (condition A) | 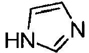 |
| | | | | | dimethylamino-acetic acid instead of *trans-*cyclohexane-1,2-diamine |
| 3 | 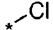 | 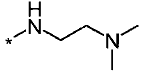 | 599 | 2.1 (condition A) | 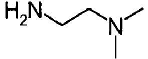 |
| 4 | 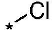 | 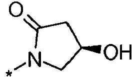 | 612 | 2.33 (condition A) | 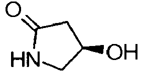 |
| 5 | 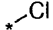 | 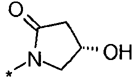 | 612 | 2.31 (condition A) | 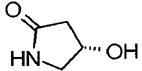 |
| 6 | 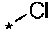 | 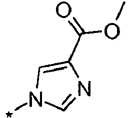 | 637 | 2.31 (condition A) | 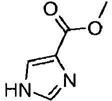 |
| 7 | 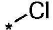 | 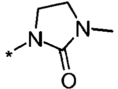 | 611 | 2.43 (condition A) | 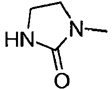 |
| 8 | 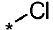 | 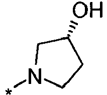 | - | Rf = 0.44 (Hexane:EtOAc = 1:2) | 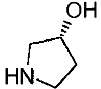 |
| 9 | 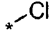 | 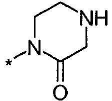 | 612 | 2.20 (condition A) | 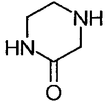 |

### Example 41: Synthesis of 2-benzyl-4-{(3,5-bis-trifluoromethyl-benzyl)-[5-(2-hydroxy-ethoxy)-pyrimidin-2-yl]-amino}-6-ethyl-piperidine-1-carboxylic acid tert-butyl ester (Racemic).

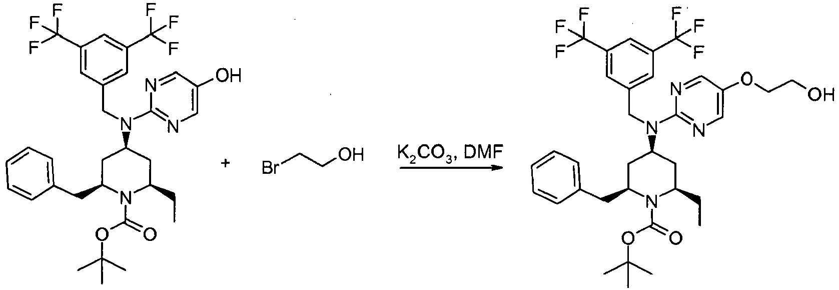

A mixture of 2-benzyl-4-[(3,5-bis-trifluoromethyl-benzyl)-(5-hydroxy-pyrimidin-2-yl)-amino]-6-ethyl-piperidine-1-carboxylic acid tert-butyl ester (0.05 mmol, 32 mg), 2-bromo-ethanol (0.15 mmol, 12 uL) and potassium carbonate (0.3 mmol, 40 mg) in DMF (0.2 mL) is stirred at 60 °C for 20 hours, cooled to room temperature. The mixture is added water and is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-{(3-chloro-5-trifluoromethyl-benzyl)-[5-(2-hydroxy-ethoxy)-pyrimidin-2-yl]-amino}-6-ethyl-piperidine-1-carboxylic acid isopropyl ester (23 mg, 67%); ESI-MS m/z: 683 [M+1]⁺, Retention time 2.61 min (condition A).

### Example 42: Synthesis of 2-benzyl-4-[(3,5-bis-trifluoromethyl-benzyl)-(5-methoxycarbonylmethoxy-pyrimidin-2-yl)-amino]-6-ethyl-piperidine-1-carboxylic acid tert-butyl ester (Racemic).

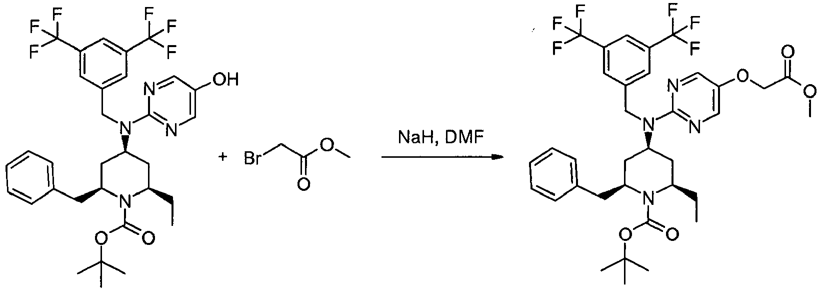

To a solution of 2-benzyl-4-[(3,5-bis-trifluoromethyl-benzyl)-(5-hydroxy-pyrimidin-2-yl)-amino]-6-ethyl-piperidine-1-carboxylic acid tert-butyl ester (0.05 mmol, 32 mg) in DMF (0.2 mL) is added sodium hydride (60% oil suspension, 0.06 mmol, 24 mg) at 0 °C and stirred at room temperature for 15 min. To the mixture is added bromo-acetic acid methyl ester (0.15 mmol, 12 uL) at 0 °C and stirred at room temperature for 2 hours. The mixture is added saturated aq. citric acid and is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-[(3,5-bis-trifluoromethyl-benzyl)-(5-methoxycarbonylmethoxy-pyrimidin-2-yl)-amino]-6-ethyl-piperidine-1-carboxylic acid tert-butyl ester (25 mg, 70%); ESI-MS m/z: 711 [M+1]⁺, Retention time 2.67 min (condition A).

### Example 43: Synthesis of 2-benzyl-4-[(3,5-bis-trifluoromethyl-benzyl)-(5-carboxymethoxy-pyrimidin-2-yl)-amino]-6-ethyl-piperidine-1-carboxylic acid tert-butyl ester (Racemic).

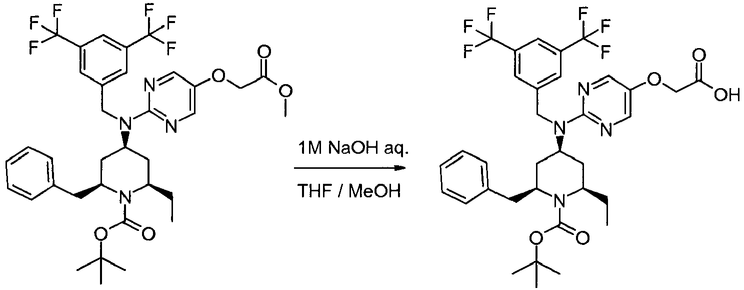

To a solution of 2-benzyl-4-[(3,5-bis-trifluoromethyl-benzyl)-(5-methoxycarbonylmethoxy-pyrimidin-2-yl)-amino]-6-ethyl-piperidine-1-carboxylic acid tert-butyl ester (0.028 mmol, 20 mg) in THF (0.5 mL) and methanol (0.5 mL) is added aq. sodium hydroxide (1M, 0.085 mmol, 85 uL) at room temperature and stirred at room temperature for 15 hours. The mixture is added aq. hydrogen chloride (1 N) and is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-[(3,5-bis-trifluoromethyl-benzyl)-(5-carboxymethoxy-pyrimidin-2-yl)-amino]-6-ethyl-piperidine-1-carboxylic acid tert-butyl ester (25 mg, 70%); ESI-MS m/z: 697 [M+1]⁺, Retention time 2.58 min (condition A).

The following compound is prepared following the procedure of **Example 43.**

| No. | Product | ESI-MS m/z [M+1]⁺ | Retention time (min) | Starting Material |
|---|---|---|---|---|
| 1 | 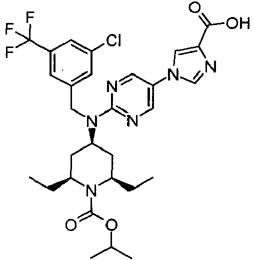 | 623 | 2.18 (condition A) | 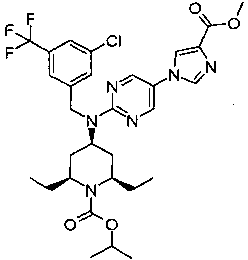 |

### Example 44: Synthesis of 2-benzyl-4-{(3-chloro-5-trifluoromethyl-benzyl)-[5-(2-hydroxy-ethoxy)-pyrimidin-2-yl]-amino}-6-ethyl-piperidine-1-carboxylic acid isopropyl ester (Racemic).

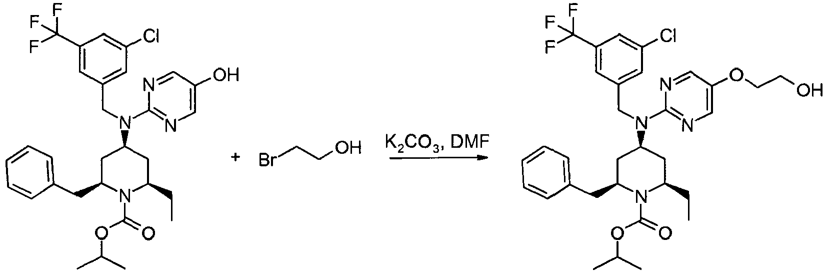

A mixture of 2-benzyl-4-[(3-chloro-5-trifluoromethyl-benzyl)-(5-hydroxy-pyrimidin-2-yl)-amino]-6-ethyl-piperidine-1-carboxylic acid isopropyl ester (0.12 mmol, 70 mg), 2-bromo-ethanol (0.72 mmol, 50 uL) and potassium carbonate (0.72 mmol, 100 mg) in DMF (0.5 mL) is stirred at 60 °C for 13 hours, cooled to room temperature. The mixture is added water and is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-{(3-chloro-5-trifluoromethyl-benzyl)-[5-(2-hydroxy-ethoxy)-pyrimidin-2-yl]-amino}-6-ethyl-piperidine-1-carboxylic acid isopropyl ester (56 mg, 74%); ESI-MS m/z: 635 [M+1]⁺, Retention time 2.56 min (condition A).

### Example 45: Synthesis of 2-benzyl-4-{(3-chloro-5-trifluoromethyl-benzyl)-[5-(2-methanesulfonyl-ethoxy)-pyrimidin-2-yl]-amino}-6-ethyl-piperidine-1-carboxylic acid isopropyl ester (Racemic).

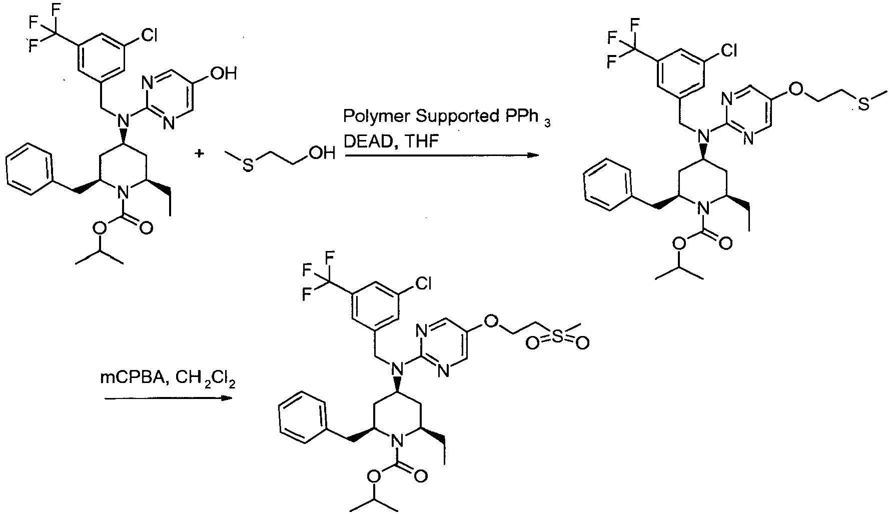

To a mixture of 2-benzyl-4-[(3-chloro-5-trifluoromethyl-benzyl)-(5-hydroxy-pyrimidin-2-yl)-amino]-6-ethyl-piperidine-1-carboxylic acid isopropyl ester (0.05 mmol, 30 mg), 2-Methylsulfanyl-ethanol (0.075 mmol, 7 uL) and polymer supported triphenylphosphine (1.41 mmol/g, 0.1 mmol, 71 mg) in THF (0.5 mL) is added DEAD (0.075 mmol, 34 uL) at room temperature and then stirred for 15 hours. The mixture is filtrated, and the filtrate is concentrated under reduced pressure. The obtained residue is purified by silica gel column chromatography (eluent: hexane / EtOAc) to give 2-benzyl-4-{(3-chloro-5-trifluoromethyl-benzyl)-[5-(2-methylsulfanyl-ethoxy)-pyrimidin-2-yl]-amino}-6-ethyl-piperidine-1-carboxylic acid isopropyl ester (22 mg, 66%)

To a solution of 2-benzyl-4-{(3-chloro-5-trifluoromethyl-benzyl)-[5-(2-methylsulfanyl-ethoxy)-pyrimidin-2-yl]-amino}-6-ethyl-piperidine-1-carboxylic acid isopropyl ester (0.033 mmol, 22 mg) in CH₂Cl₂ (1 mL) is cooled to 0 °C and 3-chloro-benzenecarboperoxoic acid (0.13 mmol, 23 mg) is added. The mixture is allowed to warm to room temperature and stirred for 2 hours. The mixture is added saturated aq. sodium hydrogen carbonate and extracted with CH₂Cl₂. The combined organic layer is concentrated under reduced pressure. The obtained residue is purified by silica gel column chromatography (eluent: hexane / EtOAc) to give 2-benzyl-4-{(3-chloro-5-trifluoromethyl-benzyl)-[5-(2-methanesulfonyl-ethoxy)-pyrimidin-2-yl]-amino}-6-ethyl-piperidine-1-carboxylic acid isopropyl ester (21 mg, 92%); ESI-MS m/z: 697 [M+1]⁺, Retention time 2.58 min (condition A).

### Example 46: Synthesis of 2-benzyl-4-{(3-chloro-5-trifluoromethyl-benzyl)-[5-(2-methoxy-ethoxy)-pyrimidin-2-yl]-amino}-6-ethyl-piperidine-1-carboxylic acid isopropyl ester (Racemic).

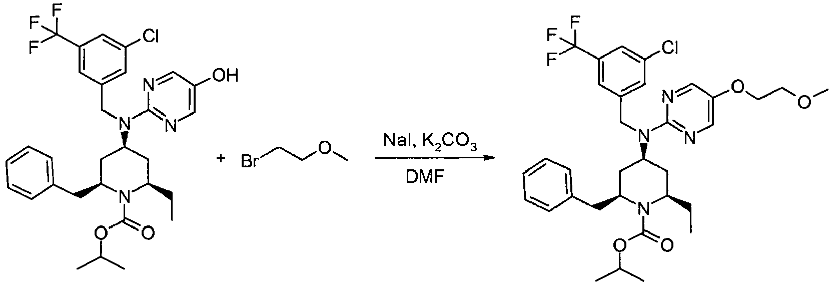

A mixture of 2-benzyl-4-[(3-chloro-5-trifluoromethyl-benzyl)-(5-hydroxy-pyrimidin-2-yl)-amino]-6-ethyl-piperidine-1-carboxylic acid isopropyl ester (0.12 mmol, 70 mg), 1-bromo-2-methoxy-ethane (0.72 mmol, 50 uL), sodium iodide (0.12 mmol, 18 mg) and potassium carbonate (0.72 mmol, 100 mg) in DMF (0.5 mL) is stirred for 15 hours. The mixture is added water and is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-{(3-chloro-5-trifluoromethyl-benzyl)-[5-(2-methoxy-ethoxy)-pyrimidin-2-yl]-amino}-6-ethyl-piperidine-1-carboxylic acid isopropyl ester (7 mg, 9%); ESI-MS m/z: 649 [M+1]⁺, Retention time 2.72 min (condition A).

### Example 47: Synthesis of 4-{(3-chloro-5-trifluoromethyl-benzyl)-[5-(2-hydroxy-ethoxy)-pyrimidin-2-yl]-amino}-2,6-diethyl-piperidine-1-carboxylic acid isopropyl ester.

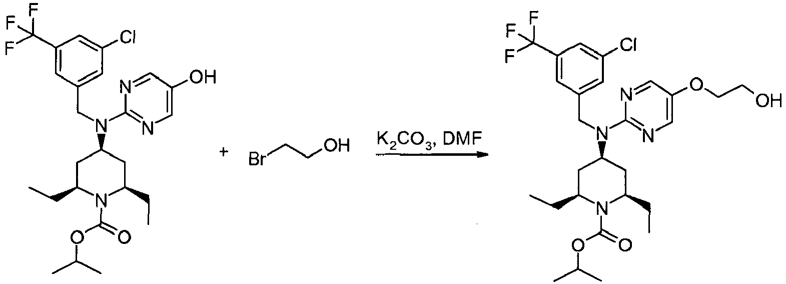

A mixture of 4-[(3-chloro-5-trifluoromethyl-benzyl)-(5-hydroxy-pyrimidin-2-yl)-amino]-2,6-diethyl-piperidine-1-carboxylic acid isopropyl ester (0.12 mmol, 64 mg), 2-bromo-ethanol (0.72 mmol, 50 uL) and potassium carbonate (0.72 mmol, 100 mg) in DMF (1 mL) ) is stirred at 60 °C for 16 hours, cooled to room temperature. The mixture is added water and is extracted with CH₂Cl₂. The combined organic layer is concentrated under reduced pressure. The obtained residue is purified by silica gel column chromatography (eluent: hexane / EtOAc) to give 4-{(3-chloro-5-trifluoromethyl-benzyl)-[5-(2-hydroxy-ethoxy)-pyrimidin-2-yl]-amino}-2,6-diethyl-piperidine-1-carboxylic acid isopropyl ester (61 mg, 89%); ESI-MS m/z: 573 [M+1]⁺, Retention time 2.60 min (condition A).

The following compounds are prepared following the procedure of Example 47.

| No. | Product | ESI-MS m/z [M+1]⁺ | Retention time (min) | Reagent |
|---|---|---|---|---|
| 1 | 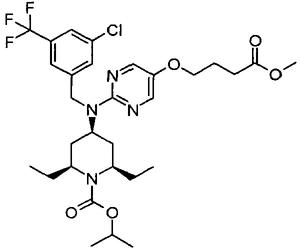 | 643 | 5.83 (condition B) | 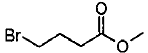 |
| 2 | 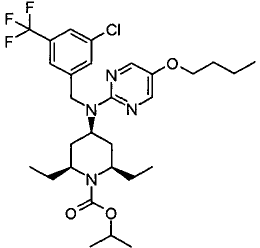 | 585 | 5.98 (condition B) | 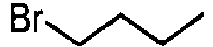 |
| 3 | 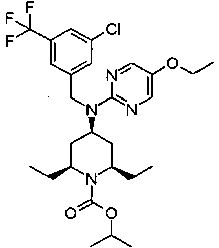 | 557 | 5.78 (condition B) | 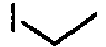 |
| | | | | NaH instead of K₂CO₃ |

### Example 48: Synthesis of 4-{(3-chloro-5-trifluoromethyl-benzyl)-[5-(2-methoxy-ethoxy)-pyrimidin-2-yl]-amino}-2,6-diethyl-piperidine-1-carboxylic acid isopropyl ester.

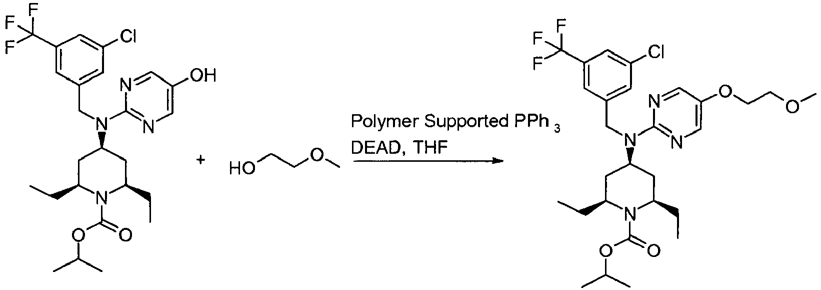

To a mixture of 4-[(3-chloro-5-trifluoromethyl-benzyl)-(5-hydroxy-pyrimidin-2-yl)-amino]-2,6-diethyl-piperidine-1-carboxylic acid isopropyl ester (0.1 mmol, 53 mg), 2-methoxy-ethanol (0.15 mmol, 12 uL) and polymer supported triphenylphosphine (1.41 mmol/g, 0.2 mmol, 142 mg) in THF (1 mL) is added DEAD (0.15 mmol, 68 uL) at room temperature and then stirred for 15 hours. The mixture is filtrated, and the filtrate is concentrated under reduced pressure. The obtained residue is purified by silica gel column chromatography (eluent: hexane / EtOAc) to give 4-{(3-chloro-5-trifluoromethyl-benzyl)-[5-(2-methoxy-ethoxy)-pyrimidin-2-yl]-amino}-2,6-diethyl-piperidine-1-carboxylic acid isopropyl ester (51 mg, 88%); ESI-MS m/z: 587 [M+1]⁺, Retention time 2.70 min (condition A).

### Example 49: Synthesis of 4-{(3-chloro-5-trifluoromethyl-benzyl)-[5-(2-morpholin-4-yl-ethoxy)-pyrimidin-2-yl]-amino}-2,6-diethyl-piperidine-1-carboxylic acid isopropyl ester.

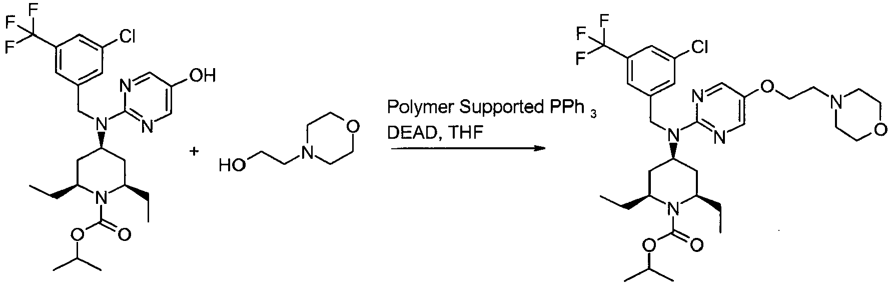

To a mixture of 4-[(3-chloro-5-trifluoromethyl-benzyl)-(5-hydroxy-pyrimidin-2-yl)-amino]-2,6-diethyl-piperidine-1-carboxylic acid isopropyl ester (0.1 mmol, 53 mg), 2-morpholin-4-yl-ethanol (0.15 mmol, 18 uL) and polymer supported triphenylphosphine (1.41 mmol/g, 0.2 mmol, 142 mg) in THF (1 mL) is added DEAD (0.15 mmol, 68 uL) at room temperature and then stirred for 15 hours. The mixture is filtrated, and the filtrate is concentrated under reduced pressure. The obtained residue is purified by silica gel column chromatography (eluent: DCM / EtOH) to give 4-{(3-Chloro-5-trifluoromethyl-benzyl)-[5-(2-morpholin-4-yl-ethoxy)-pyrimidin-2-yl]-amino}-2,6-diethyl-piperidine-1-carboxylic acid isopropyl ester (45 mg, 70%); ESI-MS m/z: 642 [M+1]⁺, Retention time 2.18 min (condition A).

The following compounds are prepared following the procedure of **Example 49.**

| No. | Product | ESI-MS m/z [M+1]⁺ | Retention time (min) | Reagent |
|---|---|---|---|---|
| 1 | 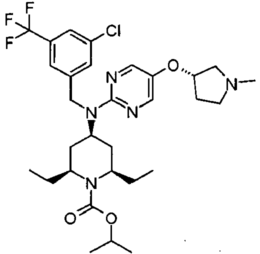 | 612 | 2.19 (condition A) | 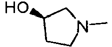 |
| 2 | 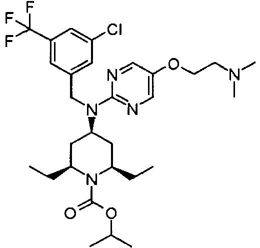 | 600 | 4.91 (condition B) | 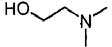 |
| 3 | 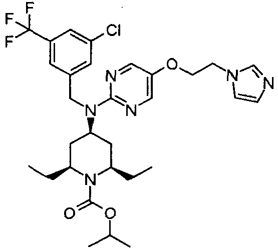 | 623 | 2.16 (condition A) | 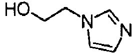 |
| 4 | 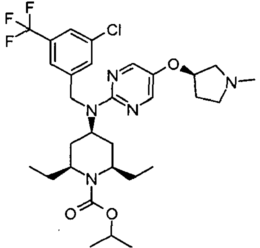 | 612 | 2.13 (condition A) | 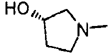 |

### Example 50: Synthesis of 2-benzyl-4-[(3-chloro-5-trifluoromethyl-benzyl)-(4-ethoxycarbonyl-oxazol-2-yl)-amino]-6-ethyl-piperidine-1-carboxylic acid tert-butyl ester

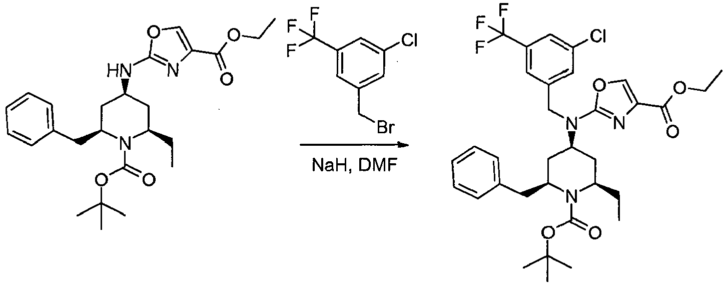

To a solution of 2-benzyl-4-(4-ethoxycarbonyl-oxazol-2-ylamino)-6-ethyl-piperidine-1-carboxylic acid tert-butyl ester (0.63 mmol, 288 mg) in DMF (3 mL) is added sodium hydride (60% oil suspension, 1.26 mmol, 50 mg) at 0 °C and stirred at room temperature for 20 min. To the mixture is added 1-bromomethyl-3-chloro-5-trifluoromethyl-benzene (0.94 mmol, 155 uL) at 0 °C and stirred at room temperature for 3 hours. The mixture is added water and is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-[(3-chloro-5-trifluoromethyl-benzyl)-(4-ethoxycarbonyl-oxazol-2-yl)-amino]-6-ethyl-piperidine-1-carboxylic acid tert-butyl ester (178 mg, 43%); ESI-MS m/z: 650 [M+1]⁺, Retention time 2.75 min (condition A).

The following compound is prepared following the procedure of Example 50.

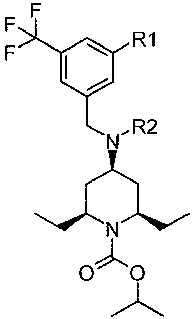

| No. | R1 | R2 | ESI-MS m/z [M+1]⁺ | Retention time (min) |
|---|---|---|---|---|
| 1 | 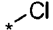 | 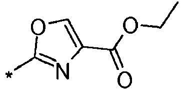 | 574 | 2.69 (condition A) |

### Example 51: Synthesis of 2-benzyl-4-[(3-chloro-5-trifluoromethyl-benzyl)-(4-hydroxymethyl-oxazol-2-yl)-amino]-6-ethyl-piperidine-1-carboxylic acid tert-butyl ester

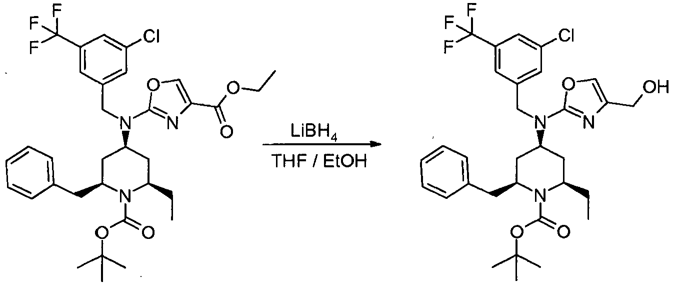

To a solution of 2-benzyl-4-[(3-chloro-5-trifluoromethyl-benzyl)-(4-ethoxycarbonyl-oxazol-2-yl)-amino]-6-ethyl-piperidine-1-carboxylic acid tert-butyl ester (0.03 mmol, 20 mg) in THF (0.3 mL) and ethanol (20 uL) is added lithium borohydride solution (2M, THF solution, 0.15 mmol, 75 uL) at 0 °C and stirred at room temperature for 3.5 hours. To the mixture is added EtOAc at room temperature and stirred for 10 min. The mixture is cooled down until 0 °C and then added dropwise aq. hydrogen chloride (1 N). The mixture is extracted with dichloromethane. The combined organic layer is concentrated under reduced pressure. The obtained residue is purified by preparative TLC (eluent: hexane / EtOAc) to give 2-benzyl-4-[(3-chloro-5-trifluoromethyl-benzyl)-(4-hydroxymethyl-oxazol-2-yl)-amino]-6-ethyl-piperidine-1-carboxylic acid tert-butyl ester (11 mg, 63%); ESI-MS m/z: 608 [M+1]⁺, Retention time 2.46 min (condition A).

The following compound is prepared following the procedure of **Example 51.**

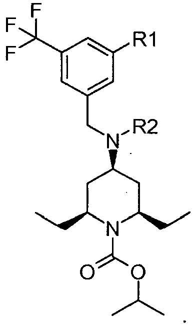

| No. | R1 | R2 | ESI-MS m/z [M+1]⁺ | Retention time (min) |
|---|---|---|---|---|
| 1 | 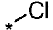 | 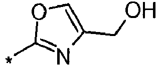 | 532 | 2.44 (condition A) |

### Example 52: Synthesis of 4-[(3-chloro-5-trifluoromethyl-benzyl)-(4-methyl-oxazol-2-yl)-amino]-2,6-diethyl-piperidine-1-carboxylic acid isopropyl ester

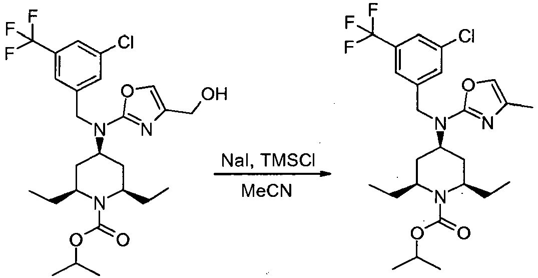

To a mixture of 4-[(3-chloro-5-trifluoromethyl-benzyl)-(4-hydroxymethyl-oxazol-2-yl)-amino]-2,6-diethyl-piperidine-1-carboxylic acid isopropyl ester (0.05 mmol, 27 mg) and sodium iodide (0.5 mmol, 75 mg) in acetonitrile (0.2 ml) is added trimethylsilyl chloride (0.5 mmol, 63 uL) and stirred for 24 hours. The mixture is added saturated aq. sodium thiosulfate. The mixture is extracted with EtOAc. The combined organic layer is dried over Na₂SO₄, filtrated, and concentrated under reduced pressure. The obtained residue is purified by preparative TLC (eluent: hexane / EtOAc) to give 4-[(3-chloro-5-trifluoromethyl-benzyl)-(4-methyl-oxazol-2-yl)-amino]-2,6-diethyl-piperidine-1-carboxylic acid isopropyl ester (5 mg, 21%); ESI-MS m/z: 516 [M+1]⁺, Retention time 2.12 min (condition A).

### Example 53: Synthesis of 4-[(3,5-bis-trifluoromethyl-benzyl)-(2H-tetrazol-5-yl)-amino]-2,6-diethyl-piperidine-1-carboxylic acid isopropyl ester

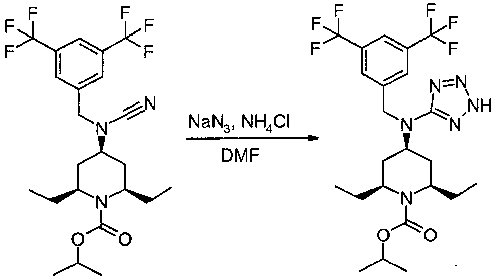

To a mixture of 4-[(3,5-bis-trifluoromethyl-benzyl)-cyano-amino]-2,6-diethyl-piperidine-1-carboxylic acid isopropyl ester (0.365 mmol, 180 mg) and ammonium chloride (1.83 mmol, 100 mg) in DMF is added sodium azide (1.825 mmol, 120 mg) at room temperature. The mixture is warmed to 60 °C and stirred for 12 hours. The mixture is added ammonium chloride (1.83 mmol, 100 mg) and sodium azide (1.825 mmol, 120 mg) and stirred at 60 °C for 9 hours. The mixture is cooled down until room temperature and then added water. The mixture is extracted with EtOAc. The combined organic layer is dried over Na₂SO₄, filtrated, and concentrated under reduced pressure. The obtained residue is purified by silica gel column chromatography (eluent: DCM / MeOH) to give 4-[(3,5-bis-trifluoromethyl-benzyl)-(2H-tetrazol-5-yl)-amino]-2,6-diethyl-piperidine-1-carboxylic acid isopropyl ester (85 mg, 43%); ESI-MS m/z: 537 [M+1]⁺, Retention time 2.49 min (condition A).

### Example 54: Synthesis of 4-[(3,5-bis-trifluoromethyl-benzyl)-(5-methyl-[1,3,4]oxadiazol-2-yl)-amino]-2,6-diethyl-piperidine-1-carboxylic acid isopropyl ester

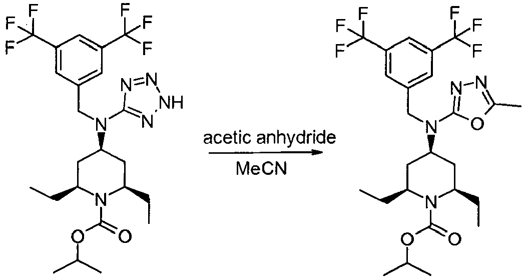

A solution of 4-[(3,5-bis-trifluoromethyl-benzyl)-(2H-tetrazol-5-yl)-amino]-2,6-diethyl-piperidine-1-carboxylic acid isopropyl ester (0.056 mmol, 30 mg) and acetic anhydride (0.28 mmol, 27 uL in acetonitrile (0.5 mL) is stirred at 120 °C for 1 hour by microwave reactor. The solution is concentrated under reduced pressure. The obtained residue is purified by silica gel column chromatography (eluent: hexane / EtOAc) to give 4-[(3,5-bis-trifluoromethyl-benzyl)-(5-methyl-[1,3,4]oxadiazol-2-yl)-amino]-2,6-diethyl-piperidine-1-carboxylic acid isopropyl ester (11 mg, 36%); ESI-MS m/z: 551 [M+1]⁺, Retention time 2.51 min (condition A).

### Example 55: Synthesis of 4-{(3,5-bis-trifluoromethyl-benzyl)-[2-(2-methoxy-ethyl)-2H-tetrazol-5-yl]-amino}-2,6-diethyl-piperidine-1-carboxylic acid isopropyl ester

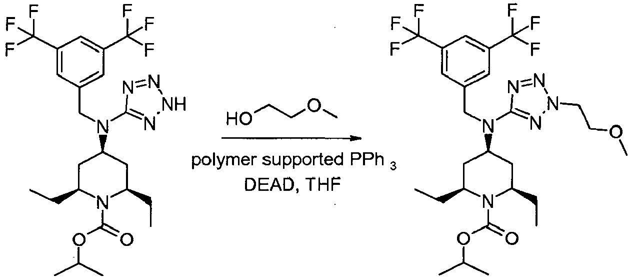

To a mixture of 4-[(3,5-bis-trifluoromethyl-benzyl)-(2H-tetrazol-5-yl)-amino]-2,6-diethyl-piperidine-1-carboxylic acid isopropyl ester (0.1 mmol, 54 mg), 2-methoxy-ethanol (0.15 mmol, 12 uL) and polymer supported triphenylphosphine (1.41 mmol/g, 0.2 mmol, 142 mg) in THF (1 mL) is added DEAD (0.15 mmol, 68 uL) at room temperature and then stirred for 15 hours. The mixture is filtrated and the filtrate is concentrated under reduced pressure. The obtained residue is purified by silica gel column chromatography (eluent: hexane / EtOAc) to give 4-{(3,5-bis-trifluoromethyl-benzyl)-[2-(2-methoxy-ethyl)-2H-tetrazol-5-yl]-amino}-2,6-diethyl-piperidine-1-carboxylic acid isopropyl ester (41 mg, 91%); ESI-MS m/z: 595 [M+1]⁺, Retention time 2.63 min (condition A).

### Example 56: Synthesis of 4-{(3,5-bis-trifluoromethyl-benzyl)-[2-(2-hydroxy-ethyl)-2H-tetrazol-5-yl]-amino}-2,6-diethyl-piperidine-1-carboxylic acid isopropyl ester

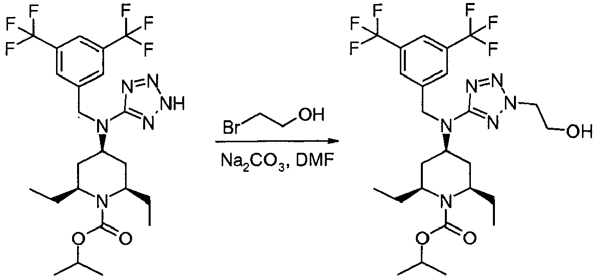

A mixture of 4-[(3,5-bis-trifluoromethyl-benzyl)-(2H-tetrazol-5-yl)-amino]-2,6-diethyl-piperidine-1-carboxylic acid isopropyl ester (0.1 mmol, 54 mg), 2-bromo-ethanol (0.5 mmol, 35 uL) and potassium carbonate (0.5 mmol, 70 mg) in DMF (1 mL) ) is stirred at 60 °C for 2 hours, then cooled to room temperature. The mixture is added and extracted with EtOAc. The combined organic layer is dried over Na₂SO₄, and then concentrated under reduced pressure. The obtained residue is purified by silica gel column chromatography (eluent: hexane / EtOAc) to give 4-{(3,5-bis-trifluoromethyl-benzyl)-[2-(2-hydroxy-ethyl)-2H-tetrazol-5-yl]-amino}-2,6-diethyl-piperidine-1-carboxylic acid isopropyl ester (49 mg, 84%); ESI-MS m/z: 581 [M+1]⁺, Retention time 2.51 min (condition A).

### Example 57: Synthesis of 4-[(3,5-bis-trifluoromethyl-benzyl)-(1-methyl-1H-[1,2,4]triazol-3-yl)-amino]-2,6-diethyl-piperidine-1-carboxylic acid isopropyl ester

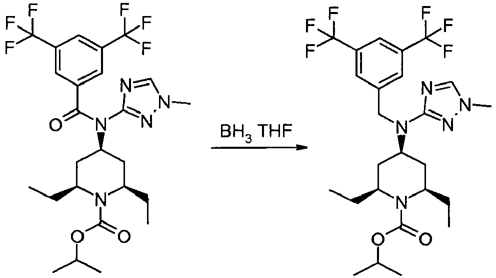

To a solution of 4-[(3,5-bis-trifluoromethyl-benzoyl)-(1-methyl-1H-[1,2,4]triazol-3-yl)-amino]-2,6-diethyl-piperidine-1-carboxylic acid isopropyl ester (0.28 mmol, 160 mg) in THF is added borane tetrahydrofuran complex in THF (1.17M, 1.4 mmol, 1.2 mL) at room temperature. The mixture is warmed to 50 °C and stirred for 5 hours. The mixture is cooled down until room temperature, and then added methanol at 0 °C and stirred for 30 min at 40 °C. The mixture is concentrated under reduced pressure. The obtained residue is purified by silica gel column chromatography (eluent: dichloromethane / MeOH) to give 4-[(3,5-bis-trifluoromethyl-benzyl)-(1-methyl-1H-[1,2,4]triazol-3-yl)-amino]-2,6-diethyl-piperidine-1-carboxylic acid isopropyl ester (42 mg, 27%); ESI-MS m/z: 550 [M+1]⁺, Retention time 2.53 min (condition A).

The following compound is prepared following the procedure of **Example 57.**

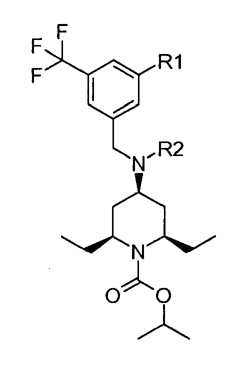

| No. | R1 | R2 | ESI-MS m/z [M+1]⁺ | Retention time (min) |
|---|---|---|---|---|
| 1 | | 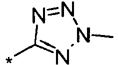 | 551 | 2.63 (condition A) |
| 2 | | 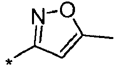 | 550 | 2.69 (condition A) |

### Example 58: Synthesis of 2-benzyl-4-[(3,5-bis-trifluoromethyl-benzyl)-(5-hydroxy-pyrimidin-2-yl)-amino]-6-ethyl-piperidine-1-carboxylic acid tert-butyl ester

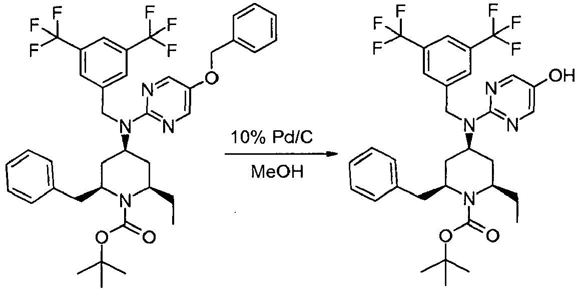

A mixture of 2-benzyl-4-[(5-benzyloxy-pyrimidin-2-yl)-(3,5-bis-trifluoromethyl-benzyl)-amino]-6-ethyl-piperidine-1-carboxylic acid tert-butyl ester (0.41 mmol, 300 mg) and 10% palladium on carbon (30 mg) in methanol is stirred at room temperature for 1 hour under hydrogen atmosphere. The mixture is filtered and the resulting solution is concentrated under reduced pressure to give 2-benzyl-4-[(3,5-bis-trifluoromethyl-benzyl)-(5-hydroxy-pyrimidin-2-yl)-amino]-6-ethyl-piperidine-1-carboxylic acid tert-butyl ester (250 mg, 95%); ESI-MS m/z: 639 [M+1]⁺, Retention time 2.59 min (condition A).

### Example 59: Synthesis of 2-benzyl-4-[(3-chloro-5-trifluoromethyl-benzyl)-(5-hydroxy-pyrimidin-2-yl)-amino]-6-ethyl-piperidine-1-carboxylic acid isopropyl ester

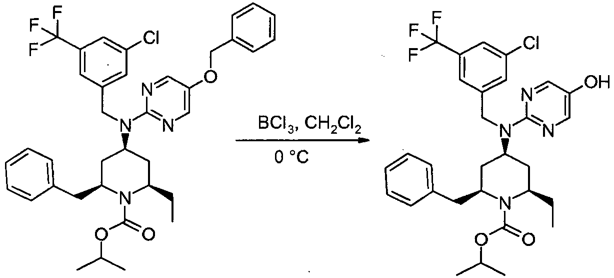

To a solution of 2-benzyl-4-[(5-benzyloxy-pyrimidin-2-yl)-(3-chloro-5-trifluoromethyl-benzyl)-amino]-6-ethyl-piperidine-1-carboxylic acid isopropyl ester (0.72 mmol, 490 mg) in dichloromethane is added boron trichloride dichloromethane solution (1M, 1.1 mmol, 1.1 mL) at 0 °C and stirred at 0 °C for 4 hours. The mixture is added boron trichloride dichloromethane solution (1M, 1.1 mmol, 1.1 mL) at 0 °C and stirred at 0 °C for 1 hours. To the mixture is added methanol (1 mL) dropwise at 0 °C. The mixture is added water and extracted with dichloromethane. The combined organic layer is dried over Na₂SO₄, filtrated, and concentrated under reduced pressure. The obtained residue is purified by silica gel column chromatography (eluent: hexane / EtOAc) to give 2-benzyl-4-[(3-chloro-5-trifluoromethyl-benzyl)-(5-hydroxy-pyrimidin-2-yl)-amino]-6-ethyl-piperidine-1-carboxylic acid isopropyl ester (417 mg, 98%); ESI-MS m/z: 591 [M+1]⁺, Retention time 5.34 min (condition B).

The following compound is prepared following the procedure of **Example 59.**

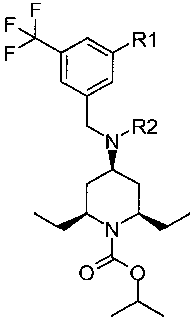

| No. | R1 | R2 | ESI-MS m/z [M+1]⁺ | Retention time (min) |
|---|---|---|---|---|
| 1 | 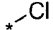 | 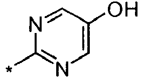 | 529 | 2.60 (condition A) |

### Example 60: Synthesis of 2-benzyl-4-[(3,5-bis-trifluoromethyl-benzyl)-methoxycarbonyl-amino]-6-cyclopropyl-piperidine-1-carboxylic acid tert-butyl ester

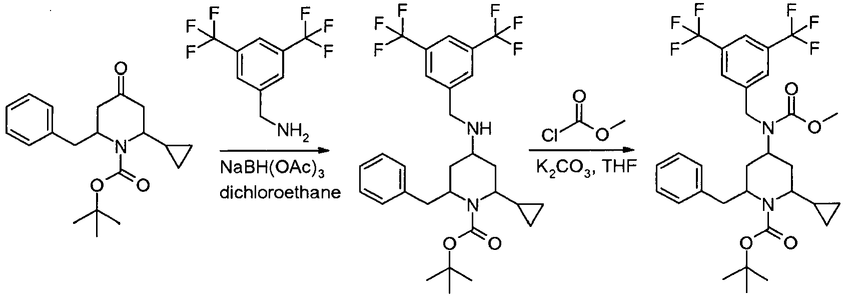

To a mixture of 2-benzyl-6-cyclopropyl-4-oxo-piperidine-1-carboxylic acid tert-butyl ester (50 mg, 0.152mmol) and 3,5-bis(-trifluoromethyl)benzylamine (45mg, 0.167mmol) in 1,2-dichloroethane (0.5 ml) is added acetic acid (0.1 mL, 1.74 mmol) and NaBH(OAc)₃ (64 mg, 0.30mmol) under N₂ at room temperature. The mixture is allowed to stir for 18 hours and then this mixture is quenched with saturated aqueous NaHCO₃. The layers are separated and the aqueous layer is extracted with EtOAc. The combined organic layer is washed with brine, dried over Na₂SO₄, filtrated, and concentrated under reduced pressure and crude mixture is used for next step without purification.

To a solution of above crude mixture and potassium carbonate (41.4mg, 0.3mmol) in THF (2.0 ml) is added methyl chloroformate (0.023mL, 0.3 mmol) under N₂ at room temperature. The mixture is allowed to stir for 1 day and then quenched with water. The layers are separated, and the aqueous layer is extracted with EtOAc. The combined organic layer is washed with brine, dried over Na₂SO₄, filtrated, and concentrated under reduced pressure. The residue is purified by column chromatography to give 2-benzyl-4-[(3,5-bis-trifluoromethyl-benzyl)-methoxycarbonyl-amino]-6-cyclopropyl-piperidine-1-carboxylic acid tert-butyl ester (23mg) in 25% yields (2steps); ESI-MS m/z: 615 [M+1]⁺, Retention time 5.63 min (condition B).

### Example 61: Synthesis of (2S,4R,6R)-4-[(3-chloro-5-trifluoromethyl-benzyl)-(5-cyano-pyrimidin-2-yl)-amino]-2,6-diethyl-piperidine-1-carboxylic acid isopropyl ester

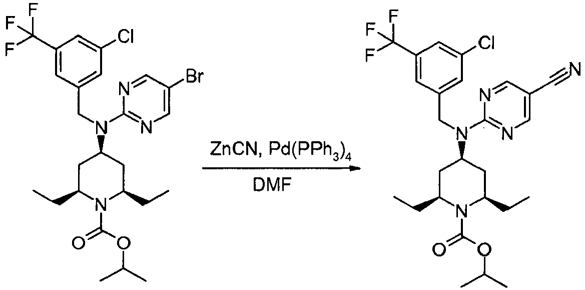

A mixture of (2S,4R,6R)-4-[(5-bromo-pyrimidin-2-yl)-(3-chloro-5-trifluoromethyl-benzyl)-amino]-2,6-diethyl-piperidine-1-carboxylic acid isopropyl ester (120 mg, 0.20 mmol), zinc cyanide (23.5 mg, 0.20 mmol), and Pd(PPh₃)₄ (12 mg, 0.01 mmol) in DMF (2 mL) under nitrogen is stirred for 3.5 hours at 110 °C. The mixture is cooled to room temperature quenched with saturated sodium bicarbonate solution. The mixture is extracted with ethyl acetate, and the organic layer is washed with brine, dried over Na₂SO₄, filtered, and concentrated under reduced pressure. The obtained residue is purified by silica gel column chromatography (hexane-ethyl acetate) to give (2S,4R,6R)-4-[(3-chloro-5-trifluoromethyl-benzyl)-(5-cyano-pyrimidin-2-yl)-amino]-2,6-diethyl-piperidine-1-carboxylic acid isopropyl ester (100 mg); ESI-MS m/z: 537 [M+1]⁺, Retention time 5.05 min (condition B).

### Example 62: Synthesis of (2S,4R,6R)-4-{(3-chloro-5-trifluoromethyl-benzyl)-[5-(2H-tetrazol-5-yl)-pyrimidin-2-yl]-amino}-2,6-diethyl-piperidine-1-carboxylic acid isopropyl ester

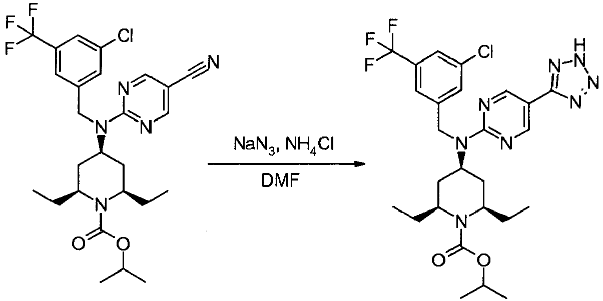

To a mixture of (2S,4R,6R)-4-[(3-chloro-5-trifluoromethyl-benzyl)-(5-cyano-pyrimidin-2-yl)-amino]-2,6-diethyl-piperidine-1-carboxylic acid isopropyl ester (87 mg, 0.16 mmol) and ammonium chloride (86 mg, 1.6 mmol) in DMF (1 mL) is added sodium azide (104 mg, 1.6 mmol). The mixture is stirred for 5 hours at 100 °C under nitrogen and cooled to room temperature. To the mixture is added water, and filtered and washed with diethyl ether and hexane (1:1) mixture. The obtained residue is purified by silica gel column chromatography (hexane-ethyl acetate) to give (2S,4R,6R)-4-{(3-chloro-5-trifluoromethyl-benzyl)-[5-(2H-tetrazol-5-yl)-pyrimidin-2-yl]-amino}-2,6-diethyl-piperidine-1-carboxylic acid isopropyl ester (82 mg); ESI-MS m/z: 580 [M+1]⁺, Retention time 4.9 min (condition B).

### Example 63: Synthesis of (2S,4R,6R)-4-{(3-chloro-5-trifluoromethyl-benzyl)-[5-(1H-imidazol-4-yl)-pyrimidin-2-yl]-amino}-2,6-diethyl-piperidine-1-carboxylic acid isopropyl ester

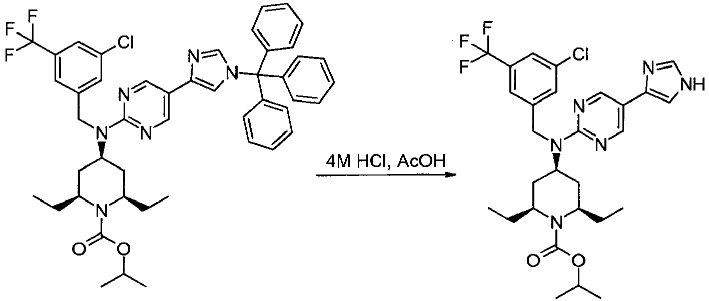

To a mixture of (2S,4R,6R)-4-{(3-chloro-5-trifluoromethyl-benzyl)-[5-(1-trityl-1H-imidazol-4-yl)-pyrimidin-2-yl]-amino}-2,6-diethyl-piperidine-1-carboxylic acid isopropyl ester (170 mg, 0.20 mmol) in acetic acid (3 mL) is added 4M HCl in ethyl acetate at 0 °C. The mixture is stirred at room temperature for 2.5 hours, and quenched with saturated sodium bicarbonate solution. The mixture is extracted with ethyl acetate and dichloromethane, and the organic layer is washed with brine, dried over Na₂SO₄, filtered, and concentrated under reduced pressure. The obtained residue is purified by silica gel column chromatography (methanol / dichloromethane) to give (2S,4R,6R)-4-{(3-chloro-5-trifluoromethyl-benzyl)-[5-(1 H-imidazol-4-yl)-pyrimidin-2-yl]-amino}-2,6-diethyl-piperidine-1-carboxylic acid isopropyl ester (30 mg); ESI-MS m/z: 579 [M+1]⁺, Retention time 4.11 min (condition B).

### Example 64: Synthesis of (2S,4R,6R)-4-{(3-chloro-5-trifluoromethyl-benzyl)-[5-(1-isopropyl-1H-pyrazol-4-yl)-pyrimidin-2-yl]-amino}-2,6-diethyl-piperidine-1-carboxylic acid isopropyl ester

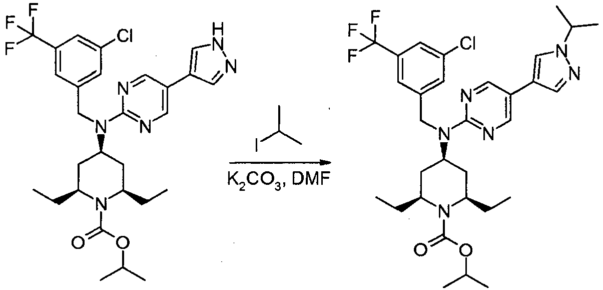

A mixture of (2S,4R,6R)-4-{(3-Chloro-5-trifluoromethyl-benzyl)-[5-(1H-pyrazol-4-yl)-pyrimidin-2-yl]-amino}-2,6-diethyl-piperidine-1-carboxylic acid isopropyl ester (30 mg, 0.05 mmol), isopropyl iodide (15 uL, 0.15 mmol), and potassium carbonate (14 mg, 0.10 mmol) in DMF (0.5 mL) is stirred at room temperature for 16 hours. To mixture is added water, and extracted with dichloromethane. After concentrating under reduced pressure, the residue is purified by silica gel column chromatography (hexane / ethyl acetate) to give (2S,4R,6R)-4-{(3-chloro-5-trifluoromethyl-benzyl)-[5-(1-isopropyl-1H-pyrazol-4-yl)-pyrimidin-2-yl]-amino}-2,6-diethyl-piperidine-1-carboxylic acid isopropyl ester (8.4 mg); ESI-MS m/z: 621 [M+1]⁺, Retention time 2.59 min (condition A).

The following compounds are prepared following the procedure of **Example 64.**

| No. | Product | ESI-MS m/z [M+1]⁺ | Retention time (min) | Reagent | Starting Material |
|---|---|---|---|---|---|
| 1 | 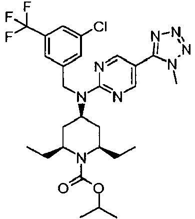 | 595 | 5.02 (condition B) | Methyl iodide | 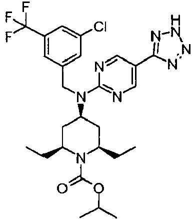 |
| 2 | 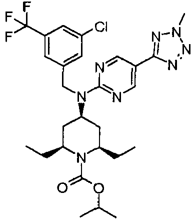 | 595 | 5.33 (condition B) | Methyl iodide | 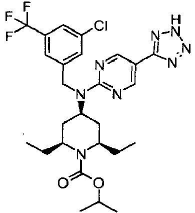 |
| 3 | 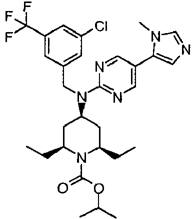 | 593 | 4.05 (condition B) | Methyl iodide (NaH instead of K₂CO₃) | 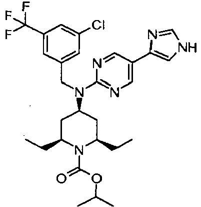 |
| 4 | 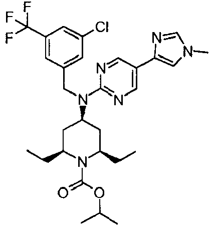 | 593 | 4.05 (condition B) | Methyl iodide (NaH instead of K₂CO₃) | 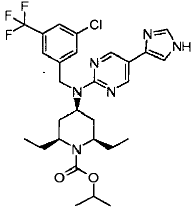 |

### Example 65: Synthesis of (2S,4R,6R)-4-{(3-chloro-5-trifluoromethyl-benzyl)-[5-(1,1-dioxo-1-thiomorpholin-4-yl)-pyrimidin-2-yl]-amino}-2,6-diethyl-piperidine-1-carboxylic acid isopropyl ester

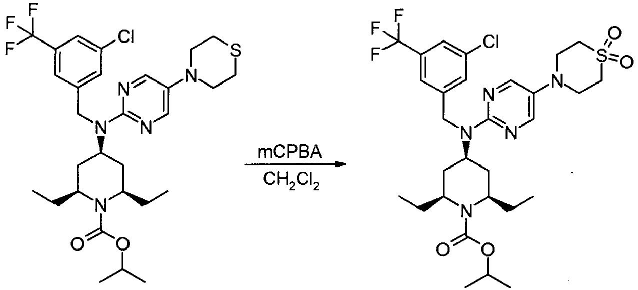

To a solution of (2S,4R,6R)-4-[(3-chloro-5-trifluoromethyl-benzyl)-(5-thiomorpholin-4-yl-pyrimidin-2-yl)-amino]-2,6-diethyl-piperidine-1-carboxylic acid isopropyl ester (80 mg, 0.13 mmol) in dichloromethane (4 mL) is added mCPBA (2.4 g, 0.31 mmol) at room temperature and stirred for 16 hours. The mixture is washed with saturated sodium bicarbonate solution and brine. The organic layer is dried over MgSO₄, filtered, and concentrated under reduced pressure. The obtained residue is purified by PTLC (NH silica) to give (2S,4R,6R)-4-{(3-chloro-5-trifluoromethyl-benzyl)-[5-(1,1-dioxo-1-thiomorpholin-4-yl)-pyrimidin-2-yl]-amino}-2,6-diethyl-piperidine-1-carboxylic acid isopropyl ester (3.87 mg); ESI-MS m/z: 646 [M+1]⁺, Retention time 2.02 min (condition A).

### Example 66: Synthesis of 2-benzyl-4-{(3,5-bis-trifluoromethyl-benzyl)-[5-(4-carboxy-piperidin-1-yl)-pyrimidin-2-yl]-amino}-6-ethyl-piperidine-1-carboxylic acid tert-butyl ester (racemic)

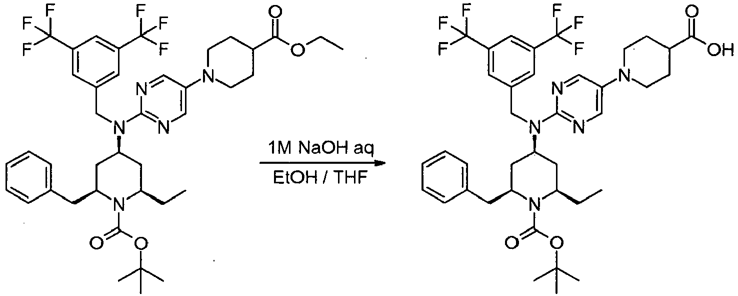

A mixture of 2-benzyl-4-{(3,5-bis-trifluoromethyl-benzyl)-[5-(4-ethoxycarbonyl-piperidin-1-yl)-pyrimidin-2-yl]-amino}-6-ethyl-piperidine-1-carboxylic acid tert-butyl ester (35 mg, 0.05 mmol) and aqueous 1M NaOH solution (125 uL, 0.125 mmol) in 1 to 1 mixture of EtOH and THF is stirred at room temperature for 13 hours. The mixture is concentrated under reduced pressure, acidified with aqueous 1M HCl solution, and extracted with ethyl acetate. The mixture is concentrated under reduced pressure to give 2-benzyl-4-{(3,5-bis-trifluoromethyl-benzyl)-[5-(4-carboxy-piperidin-1-yl)-pyrimidin-2-yl]-amino}-6-ethyl-piperidine-1-carboxylic acid tert-butyl ester (26 mg); ESI-MS m/z: 750 [M+1]⁺, Retention time 2.39 min (condition A). The following compound is prepared following the procedure of **Example 66.**

| No. | Product | ESI-MS m/z [M+1]⁺ | Retention time (min) | Starting Material |
|---|---|---|---|---|
| 1 | 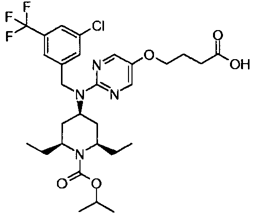 | 595 | 5.02 (condition B) | 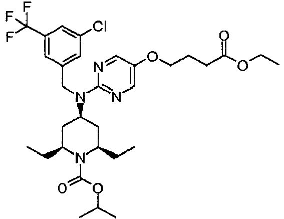 |

### Example 67: Preparation of (2S,4S,6R)-2-benzyl-4-[(3,5-bis-trifluoromethyl-benzyl)-methoxycarbonyl-amino]-6-ethyl-piperidine-1-carboxylic acid tert-butyl ester (enantiomer)

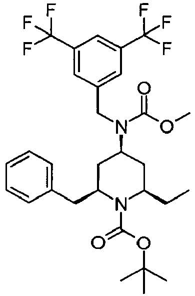

(2S,4S,6R)-2-benzyl-4-[(3,5-bis-trifluoromethyl-benzyl)-methoxycarbonyl-amino]-6-ethyl-piperidine-1-carboxylic acid tert-butyl ester is isolated from a racemic 2-benzyl-4-[(3,5-bis-trifluoromethyl-benzyl)-methoxycarbonyl-amino]-6-ethyl-piperidine-1-carboxylic acid tert-butyl ester using preparatory SFC (column: OCl 7 um, 21.2 x 250 mm; flow rate: 50 g/min; mobile phase: CO₂, 10% DMME/IPA 100:7 isocratic); ESI-MS m/z: 602 [M+1]⁺, Retention time 2.57 min (condition A), 5.16 min (condition D).

### Example 68: Preparation of (2S,4S,6R)-2-benzyl-4-[(3,5-bis-trifluoromethyl-benzyl)-(5-morpholin-4-yl-pyrimidin-2-yl)-amino]-6-ethyl-piperidine-1-carboxylic acid tert-butyl ester (enantiomer)

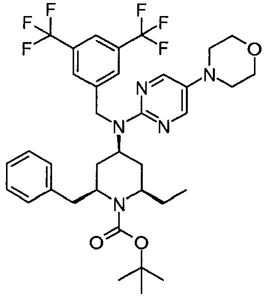

(2S,4S,6R)-2-benzyl-4-[(3,5-bis-trifluoromethyl-benzyl)-(5-morpholin-4-yl-pyrimidin-2-yl)-amino]-6-ethyl-piperidine-1-carboxylic acid tert-butyl ester is isolated from a racemic 2-benzyl-4-[(3,5-bis-trifluoromethyl-benzyl)-(5-morpholin-4-yl-pyrimidin-2-yl)-amino]-6-ethyl-piperidine-1-carboxylic acid tert-butyl ester using preparatory chiral HPLC (column: Chiralpak AD 20 um 5x5 cm; flow rate: 100 ml/min; mobile phase: n-hexane / IPA 95 : 5); ESI-MS m/z: 708 [M+1]⁺, Retention time 2.57 min (condition A), 8.06 min (condition E).

### Example 69: Preparation of (2S,4S,6S)-4-{(3-chloro-5-trifluoromethyl-benzyl)-[5-(1-methyl-1H-pyrazol-4-yl)-pyrimidin-2-yl]-amino}-2,6-diisopropyl-piperidine-1-carboxylic acid isopropyl ester (enantiomer)

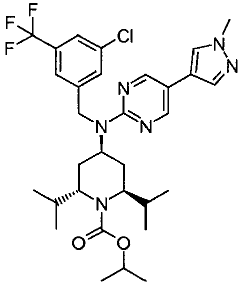

(2S,4S,6S)-4-{(3-chloro-5-trifluoromethyl-benzyl)-[5-(1-methyl-1H-pyrazol-4-yl)-pyrimidin-2-yl]-amino}-2,6-diisopropyl-piperidine-1-carboxylic acid isopropyl ester is isolated from a racemic 4-{(3-chloro-5-trifluoromethyl-benzyl)-[5-(1-methyl-1H-pyrazol-4-yl)-pyrimidin-2-yl]-amino}-2,6-diisopropyl-piperidine-1-carboxylic acid isopropyl ester using preparatory chiral HPLC (column: Chiralpak AD 20 um 5x50 cm; flow rate: 70 ml/min; mobile phase: heptane/ 2-propanol 90 : 10); ESI-MS m/z: 621 [M+1]⁺, Retention time 2.59 min (condition A), 16.2 min (condition F).

### Example 70: Preparation of (2S,4R,6S)-4-{(3-chloro-5-trifluoromethyl-benzyl)-[5-(1-methyl-1 H-pyrazol-4-yl)-pyrimidin-2-yl]-amino}-2,6-diisopropyl-piperidine-1-carboxylic acid isopropyl ester (enantiomer)

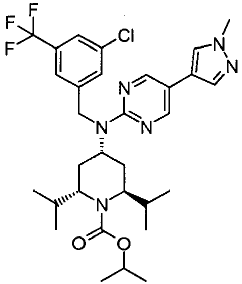

(2S,4R,6S)-4-{(3-chloro-5-trifluoromethyl-benzyl)-[5-(1-methyl-1H-pyrazol-4-yl)-pyrimidin-2-yl]-amino}-2,6-diisopropyl-piperidine-1-carboxylic acid isopropyl ester is isolated from a racemic 4-{(3-chloro-5-trifluoromethyl-benzyl)-[5-(1-methyl-1H-pyrazol-4-yl)-pyrimidin-2-yl]-amino}-2,6-diisopropyl-piperidine-1-carboxylic acid isopropyl ester using preparatory chiral HPLC (column: Chiralpak AD 20 um 5x50 cm; flow rate: 70 ml/min; mobile phase: heptane/ 2-propanol 90 : 10); ESI-MS m/z: 621 [M+1]⁺, Retention time 2.59 min (condition A), 9.91 min (condition F).

### Example 71, Preparation of the starting materials can be done as follows. 1) Synthesis of 2-ethyl-4-oxo-3,4-dihydro-2H-pyridine-1-carboxylic acid tert-butyl ester

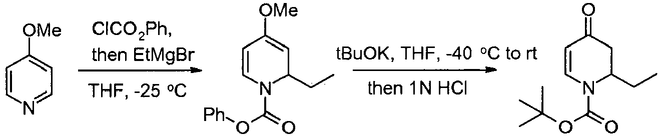

To a solution of 4-aminopiperidine (15.6 g, 143 mmol) in dry THF (1 L) cooled to -35 °C is added ClCO₂Ph (22.7 g, 144 mmol). After stirring the slurry for 1 hour, EtMgBr (150 mL, 150 mmol) is added slowly over 30 min. The mixture is warmed to 10 °C over 2 hours then quenched with H₂O. The reaction mixture is extracted twice with Et₂O (1 L), combined organic layer is dried over Na₂SO₄, and the solvent is removed under reduced pressure. To a solution of the resultant colorless oil in dry THF (500 mL) at -78 °C is added *t*-BuOK (64 g, 572 mmol). The reaction mixture is stirred overnight and warmed to room temperature. The reaction mixture is diluted with Et₂O, quenched with ice, partitioned, and the organic layer is washed three times with 1.5 N aqueous NaOH and then with brine, dried over MgSO₄ and concentrated in reduced pressure to afford 2-ethyl-4-oxo-3,4-dihydro-2H-pyridine-1-carboxylic acid tert-butyl ester as a pale yellow oil (27.8 g, 86% yield); ESI-MS m/z: 226 [M+1]⁺, Retention time 1.64 min (condition A).

The following material is prepared following the above procedure.

| Name | Structure | Reagent |
|---|---|---|
| 2-Ethyl-4-oxo-3,4-dihydro-2H-pyridine-1-carboxilic acid isopropyl ester | 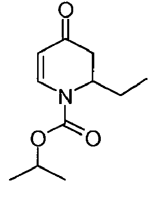 | ClCO₂/Pr instead of ClCO₂Ph |
| 2-Ethyl-4-oxo-3,4-dihydro-2H-pyridine-1-carboxilic acid isopropyl ester | 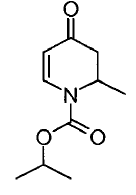 | ClCO₂/Pr instead of ClCO₂Ph, MeMgBr instead of EtMgBr |

### 2). Synthesis of 2-benzyl-4-(3,5-bis-trifluoromethyl-benzylamino)-6-ethyl-piperidine-1-carboxylic acid benzyl ester

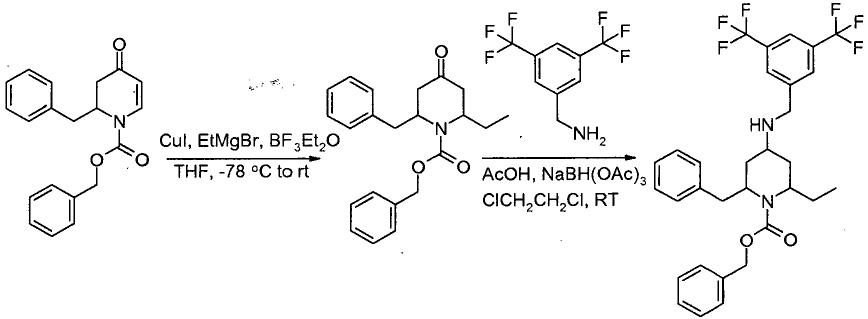

To Cul (1.0 mmol, 190.5 mg) in a flask purged with N₂ is added 0.86 M tetrahydrofuran solution of EtMgBr (1.0 mmol, 1.2 ml) at -78 °C. After stirring the suspension for 10 min, BF₃·Et₂O (0.5 mmol, 70.6 mg) is added and stirred for 10 min at the same temperature. To the suspension is added tetrahydrofuran solution (3.8 ml) of 2-benzyl-4-oxo-3,4-dihydro-2*H*-pyridine-1-carboxylic acid benzyl ester (0.5 mmol, 160.7 mg) at -78 °C, then the mixture is allowed to stir for 1 hour and then gradually allow to warm to room temperature and stir for 13 hours. This mixture is quenched with saturated aqueous NH₄Cl and extracted with EtOAc. The combined organic layers are washed with brine, dried over MgSO₄, filtered, concentrated under reduced pressure, and purified by silica gel column chromatography (eluent: hexane / EtOAc = 4 / 1) to give 2-benzyl-6-ethyl-4-oxo-piperidine-1-carboxylic acid benzyl ester (57mg, 32%); ESI-MS m/z: 352 [M+1]⁺, Retention time 2.12 min (condition A).

To a solution of 2-benzyl-6-ethyl-4-oxo-piperidine-1-carboxylic acid benzyl ester (0.16 mmol, 57.0 mg) and 3,5-bis(trifluoromethyl)benzylamine (0.2 mmol, 48.6 mg) in 1,2-dichloroethane (0.3 ml) is added acetic acid (0.2 mmol, 12 mg) and NaBH(OAc)₃ (0.4 mmol, 84.8 mg) under N₂ at room temperature. The mixture is allowed to stir for 15 hours and then basified with aq. 1 N NaOH to about pH 10. The layers are separated, and the aqueous layer is extracted with CH₂Cl₂. The combined organic layer is washed with brine, dried over Na₂SO₄, filtrated, and concentrated under reduced pressure and purified with silica gel column chromatography (eluent: hexane / EtOAc = 4:1) to give 2-benzyl-4-(3,5-bis-trifluoromethyl-benzylamino)-6-ethyl-piperidine-1-carboxylic acid benzyl ester (47.4 mg, 51%); ESI-MS m/z: 579 [M+1]⁺, Retention time 2.01 min (condition A).

### 3). Synthesis of 2,6-diethyl-4-oxo-piperidine-1-carboxylic acid tert-butyl ester (racemic)

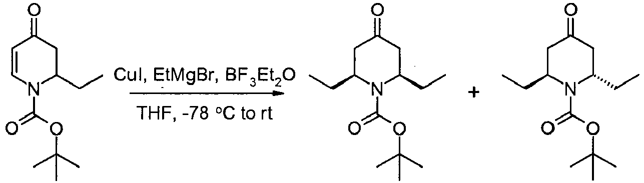

To Cul (0.82 mmol, 156 mg) in a flask purged with N₂ is added 1.00 M tetrahydrofuran solution of EtMgBr (0.82 mmol, 0.82 ml) at -78 °C. After stirring the suspension for 30 min, BF₃·Et₂O (0.41 mmol, 57.9 mg) is added and stirred for 10 min at the same temperature. To the suspension is added tetrahydrofuran solution (3.3 mL) of 2-ethyl-4-oxo-3,4-dihydro-2H-pyridine-1-carboxylic acid tert-butyl ester (0.41 mmol, 92.7 mg) at -78 °C, then the mixture is allowed to stir for 1.5 hours and then allow to stir at -40 °C for 2 hours. The mixture is warmed to room temperature and quenched with saturated aqueous NH₄Cl and extracted with EtOAc. The combined organic layers are washed with brine, dried over MgSO₄, filtered, concentrated under reduced pressure, and purified by silica gel column chromatography (eluent: hexane / EtOAc = 10 / 1) and separated the *cis* and *trans* isomers of racemic 2,6-diethyl-4-oxo-piperidine-1-carboxylic acid tert-butyl ester (*cis:* 50 mg, 50%); ESI-MS m/z: 200 [M-*t*Bu+2]⁺, Retention time 3.51 min. (*trans:* 13 mg, 13%); ESI-MS m/z: 200 [M-*t*Bu+2]⁺, Retention time 3.53 min (condition A).

The following material is prepared following the above procedure.

| Name | Structure | Starting Material |
|---|---|---|
| (2R,6S)-2,6-Diethyl-4-oxo-piperidine-1-carboxylic acid isopropyl ester | 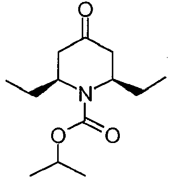 | 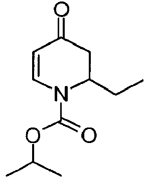 |
| (2R,6S)-2,6-Dimethyl-4-oxo-piperidine-1-carboxylic acid isopropyl ester | 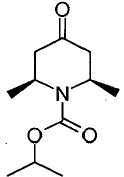 | 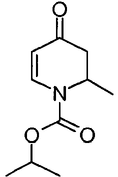 |
| | | MeMgBr instead of EtMgBr |
| 2,6-*trans*-Dimethyl-4-oxo-piperidine-1-carboxylic acid isopropyl ester | 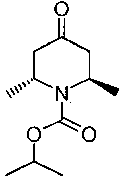 | 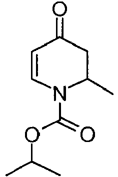 |
| | | MeMgBr instead of EtMgBr |
| (2S,3S,6R)-2,6-Diethyl-3-methyl-4-oxo-piperidine-1-carboxylic acid isopropyl ester | 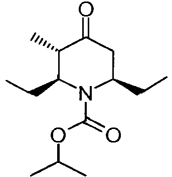 | 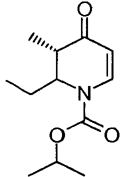 |

### 4). Synthesis of 4-(3,5-bis-trifluoromethyl-benzylamino)-2,6-diethyl-piperidine-1-carboxylic acid tert-butyl ester (2,6-cis-isomer)

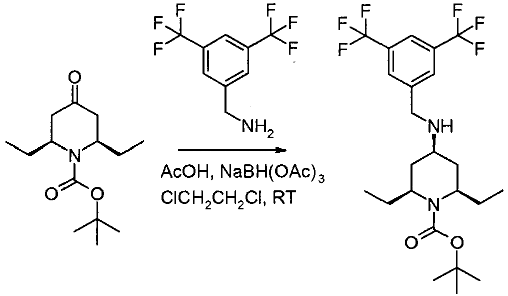

To a solution of a racemic mixture of (*cis*)-2,6-diethyl-4-oxo-piperidine-1-carboxylic acid tert-butyl ester (0.21 mmol, 53 mg) and 3,5-bis(trifluoromethyl)benzylamine (0.25 mmol, 61.3 mg) in 1,2-dichloroethane (0.5 ml) is added acetic acid (0.25 mmol, 15 mg) and NaBH(OAc)₃ (0.42 mmol, 89 mg) under N₂ at room temperature. The mixture is allowed to stir for 20 hours and then basified with aq.1 N NaOH to approximately pH 10. The layers are separated, and the aqueous layer is extracted with CH₂Cl₂. The combined organic layer is washed with brine, dried over Na₂SO₄, filtrated, and concentrated under reduced pressure and purified on preparative TLC (eluent: hexane / EtOAc = 2:1) to give 4-(3,5-bis-trifluoromethyl-benzylamino)-2,6-diethyl-piperidine-1-carboxylic acid tert-butyl ester (20.8 mg, 20%); ESI-MS m/z: 483 [M+1]⁺, Retention time 1.92 min (condition A).

### 5). Synthesis of 4-(3,5-bis-trifluoromethyl-benzylamino)-2,6-diethyl-piperidine-1-carboxylic acid tert-butyl ester (2, 6-trans-isomer, racemic)

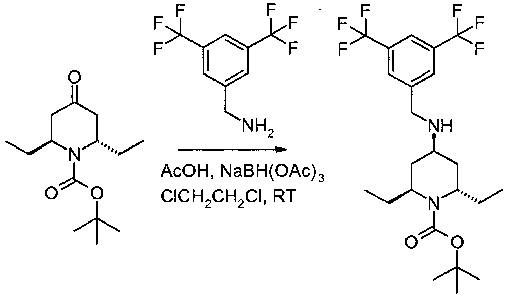

To a solution of a racemic mixture of (*trans*)-2,6-diethyl-4-oxo-piperidine-1-carboxylic acid tert-butyl ester (0.052 mmol, 13.3 mg) and 3,5-bis(trifluoromethyl)benzylamine (0.062 mmol, 15.1 mg) in 1,2-dichloroethane (0.1 ml) is added acetic acid (0.062 mmol, 3.7 mg) and NaBH(OAc)₃ (0.104 mmol, 22 mg) under N₂ at room temperature. The mixture is allowed to stir for 20 hours and then basified with aq.1 N NaOH to approximately pH 10. The layers are separated, and the aqueous layer is extracted with CH₂Cl₂. The combined organic layer is washed with brine, dried over Na₂SO₄, filtrated, and concentrated under reduced. The residue is purified on preparative TLC (eluent: hexane / EtOAc = 2:1) to give a racemic mixture of 4-(3,5-bis-trifluoromethyl-benzylamino)-2,6-diethyl-piperidine-1-carboxylic acid tert-butyl ester (8.6 mg, 34%); ESI-MS m/z: 483 [M+1]⁺, Retention time 1.93 min (condition A).

### 6). Synthesis of (2S,3R,4S,6R)-4-(3,5-Bis-trifluoromethyl-benzylamino)-2,6-diethyl-3-methyl-piperidine-1-carboxylic acid tert-butyl ester (2,6-cis-isomer, racemic)

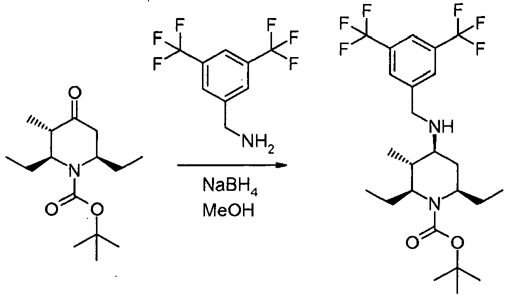

To a solution of a racemic mixture of (2S,3S,6R)-2,6-diethyl-3-methyl-4-oxo-piperidine-1-carboxylic acid isopropyl ester (0.193 mmol, 52 mg) and 3,5-bis(trifluoromethyl)benzylamine (0.21 mmol, 66 mg) in toluene (7 ml) is added BF₃OEt, and stirred at 130 °C for 30 minutes. After removing toluene under reduced pressure, the residue is dissolved in methanol (2 mL). To the mixture is added NaBH₄ (6.1 mg, 0.16 mmol) and stirred at 100 °C for 1 hour. After cooling to room temperature, water is added to the mixture and extracted with dichloromethane. The organic layer is washed with saturated sodium bicarbonate, dried over MgSO₄, filtrated, and concentrated under reduced. The residue is purified by RP-HPLC to give a racemic mixture of (2S,3R,4S,6R)-4-(3,5-Bis-trifluoromethyl-benzylamino)-2,6-diethyl-3-methyl-piperidine-1-carboxylic acid tert-butyl ester (14 mg); ESI-MS m/z: 497 [M+1]⁺, Retention time 2.19 min (condition A).

### 7). Synthesis of 2-benzyl-4-(5-bromo-pyrimidin-2-ylamino)-6-ethyl-piperidine-1-carboxylic acid tert-butyl ester

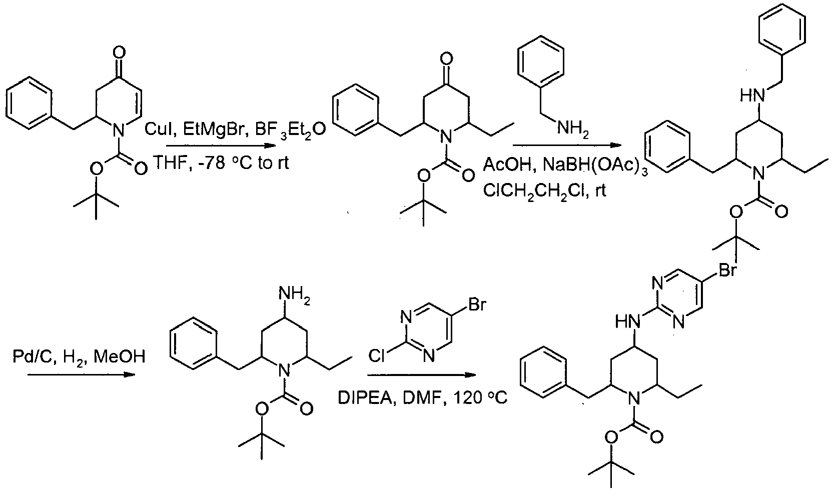

To Cul (83.6 mmol, 15.9 g) in a flask purged with N₂ is added 1.00 M EtMgBr (83.6 mmol, 83.6 ml) in THF at -78 °C. After stirring the suspension for 10 min, BF₃·Et₂O (41.8 mmol, 5.9 g) is added and stirred for 10 min at the same temperature. To the suspension is added tetrahydrofuran solution (125.4 ml) of 2-benzyl-4-oxo-3,4-dihydro-2H-pyridine-1-carboxylic acid tert-butyl ester (41.8 mmol, 12.0 g) at -78 °C, then the mixture is allowed to stir for 2 hours. The mixture is quenched with saturated aqueous NH₄Cl and extracted with EtOAc. The combined organic layers are washed with brine, dried over Na₂SO₄, filtered, concentrated under reduced pressure, and purified by silica gel column chromatography (eluent: hexane / EtOAc) to give 2-benzyl-6-ethyl-4-oxo-piperidine-1-carboxylic acid tert-butyl ester (8.19 g, 62%); ESI-MS m/z: 262 [M -*t*Bu+1]⁺, Retention time 2.14 min (condition A).

To a solution of 2-benzyl-6-ethyl-4-oxo-piperidine-1-carboxylic acid tert-butyl ester (25.8 mmol, 8.19 g) and benzylamine (28.4 mmol, 3.04 g) in 1,2-dichloroethane (65 ml) is added acetic acid (28.4 mmol, 1.76 g) and NaBH(OAc)₃ (51.6 mmol, 10.9 g) under N₂ at room temperature. The mixture is allowed to stir for 12 hours and then basified with aq.1N NaOH to approximately pH 10. The mixture is extracted with CH₂Cl₂. The combined organic layer is washed with brine, dried over Na₂SO₄, filtrated, and concentrated under reduced pressure. The residue is purified by silica gel column chromatography (eluent: hexane / EtOAc) to give 2-benzyl-4-benzylamino-6-ethyl-piperidine-1-carboxylic acid tert-butyl ester (9.55 g, 91%); ESI-MS m/z: 409 [M+1]⁺, Retention time 1.85 min (condition A).

The flask is charged with a solution of 2-benzyl-4-benzylamino-6-ethyl-piperidine-1-carboxylic acid tert-butyl ester (23 mmol, 9.4 g in MeOH (94 ml) and then purged with nitrogen. To the solution is added 10% Pd on carbon (50% wet) (2.3 mmol, 940 mg). After stirring for 30 min, the flask is purged with hydrogen. The mixture is allowed to stir for 4 hours at room temperature and then for 3 hours at 40 °C. The mixture is filtrated and concentrated under reduced pressure. 4-Amino-2-benzyl-6-ethyl-piperidine-1-carboxylic acid tert-butyl ester (6.8 g, 93%) is used in the next step without further purification; ESI-MS m/z: 319 [M+1]⁺, Retention time 2.88 min (condition B).

A solution of 4-amino-2-benzyl-6-ethyl-piperidine-1-carboxylic acid tert-butyl ester (0.1 mmol, 31.9 mg), 5-bromo-2-chloropyrimidine (0.15 mmol, 29.0 mg) and *N,N-*diisopropylethylamine (0.2 mmol, 25.8 mg) in DMF (0.3 ml) is allowed to warm to 120 °C and stir for 4hours. The mixture is cooled to room temperature, then quenched with SiO₂ and passed through a pad of silica gel. The mixture is concentrated under reduced pressure. The obtained residue is purified by silica gel column chromatography (eluent: hexane / EtOAc) to give 2-benzyl-4-(5-bromo-pyrimidin-2-ylamino)-6-ethyl-piperidine-1-carboxylic acid tert-butyl ester (36 mg, 76%); ESI-MS m/z: 475 [M+1]⁺, Retention time 2.40, 2.45 min (condition B).

The following material is prepared following the above procedure.

| Name | Product | ESI-MS m/z [M+1]⁺ | Retention time (min) | Starting Material |
|---|---|---|---|---|
| 2,4,6-*cis*-2-Benzyl-6-ethyl-4-(4-methoxy-pyrimidin-2-ylamino)-piperidine-1-carboxylic acid tert-butyl ester | 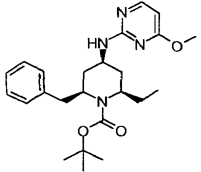 | 427 | 2.13 (condition A) | 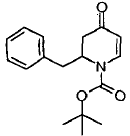 |
| | | | | 2-Chloro-4-methoxy-pyrimidine instead of 5-bromo-2-chloropyrimidine |

### 8). Synthesis of 4-(3,5-bis-trifluoromethyl-benzylamino)-2-ethyl-6-(4-fluoro-benzyl)-piperidine-1-carboxylic acid tert-butyl ester (2,4-cis and 2,4-trans isomers)

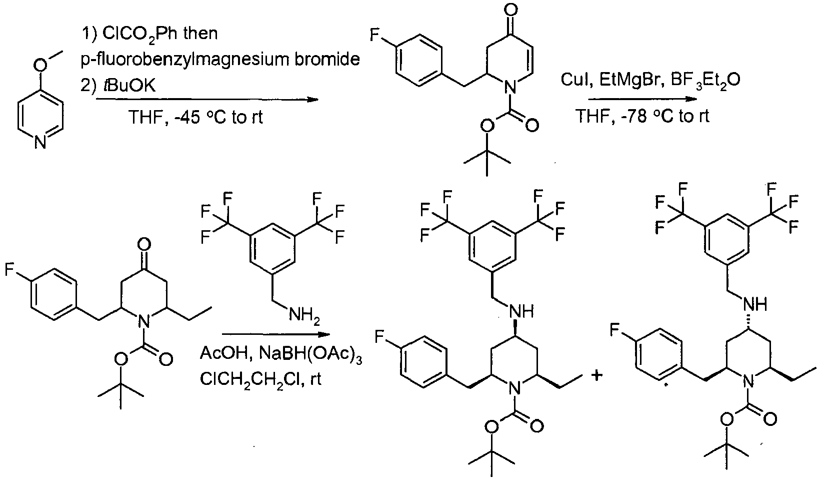

To a solution of 4-methoxypyridine (12 mmol, 1.31 g) in THF which is cooled to -45 °C is added phenyl chloroformate (12.1 mmol, 1.89 g) under nitrogen. The solution is allowed to warm to -25 °C and stirred for 30 min. To the solution cooled at -45 °C, a solution of 0.25 M *p*-fluorobenzylmagnesium bromide in THF (12.5 mmol, 50 ml) is added slowly and stirred for 1 hour. The mixture is allowed to warm to room temperature, and after stirring for 2 hours, the mixture is quenched with ice and then with H₂O and extracted with Et₂O. The combined organic layer is washed with brine, dried over Na₂SO₄, filtrated, and concentrated under reduced pressure. To the residue is added THF (100 ml) and the solution is allowed to cool to -45 °C and *t*BuOK (48 mmol, 5.39 g) is added. The solution is allowed to warm to room temperature gradually and stir for 15 hours. The mixture is quenched with ice and then with H₂O and extracted with Et₂O. The combined organic layer is washed three times with aq.1 N NaOH and then three times with aq.1 N HCl. The mixture is dried over Na₂SO₄, filtrated, and concentrated under reduced pressure. The residue is purified by silica gel column chromatography (eluent: hexane / EtOAc then MeOH / CH₂Cl₂) to give 2-(4-fluoro-benzyl)-4-oxo-3,4-dihydro-2H-pyridine-1-carboxylic acid tert-butyl ester (4%, 150 mg); ESI-MS m/z: 306 [M+1]⁺, Retention time 1.97 min (condition A).

To Cul (0.556 mmol, 106 mg) in a flask purged with N₂ is added 1.00 M tetrahydrofuran solution of EtMgBr (0.556 mmol, 0.556 ml) at -78 °C. After stirring the suspension for 15 min, BF₃.Et₂O (0.278 mmol, 39.2 mg) is added and stirred for 10 min at the same temperature. To the suspension is added tetrahydrofuran solution (0.2 ml) of 2-(4-fluoro-benzyl)-4-oxo-3,4-dihydro-2H-pyridine-1-carboxylic acid tert-butyl ester (0.278 mmol, 93.4 mg) at -78 °C, then the mixture is allowed to stir for 1 hour. After the mixture is warmed to room temperature and stirred for 2 hours, it is quenched with saturated aqueous NH₄Cl and extracted with EtOAc. The combined organic layer is washed with brine, dried over MgSO₄, filtered, and concentrated under reduced pressure. The obtained residue is purified by silica gel column chromatography (eluent: hexane / EtOAc) to give 2-ethyl-6-(4-fluoro-benzyl)-4-oxo-piperidine-1-carboxylic acid tert-butyl ester (63.8 mg, 68%); ESI-MS m/z: 280 [M-*t*Bu+2]⁺, Retention time 2.14 min (condition A).

To a solution of 2-ethyl-6-(4-fluoro-benzyl)-4-oxo-piperidine-1-carboxylic acid tert-butyl ester (0.19 mmol, 63.8 mg) and 3,5-bis(trifluoromethyl)benzylamine (0.266 mmol, 64.7 mg) in 1,2-dichloroethane (0.4 ml) is added acetic acid (0.266 mmol, 16 mg) and NaBH(OAc)₃ (0.40 mmol, 84.8 mg) under N₂ at room temperature. The mixture is allowed to stir for 22 hours and then basified with aq.1N NaOH to approximately pH 10. The layers are separated, and the aqueous layer is extracted with CH₂Cl₂. The combined organic layer is washed with brine, dried over Na₂SO₄, filtrated, and concentrated under reduced pressure. The residue is purified by silica gel column chromatography (eluent: hexane / EtOAc) to give a racemic mixture of 4-(3,5-bis-trifluoromethyl-benzylamino)-2-ethyl-6-(4-fluoro-benzyl)-piperidine-1-carboxylic acid tert-butyl ester (2,4-*cis* isomer: 35.3 mg, 33%); ESI-MS m/z: 563 [M+1]⁺, Retention time 2.03 min (condition A), (2,4-*trans* isomer: 14.0 mg, 13%); ESI-MS m/z: 563 [M+1]⁺, Retention time 2.04 min (condition A).

The following materials are prepared following the above procedure.

| Name | Structure | ESI-MS m/z [M+1]⁺ | Retention time |
|---|---|---|---|
| 4-(3,5-bis-trifluoromethyl-benzylamino)-2-ethyl-6-(2-fluoro-benzyl)-piperidine-1-carboxylic acid tert-butyl ester | 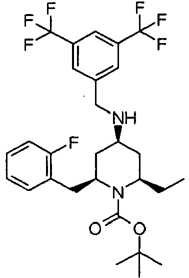 | 621 | 2.54 min (condition A) |
| 4-(3,5-bis-trifluoromethyl-benzylamino)-2-ethyl-6-(3-fluoro-benzyl)-piperidine-1-carboxylic acid tert-butyl ester | 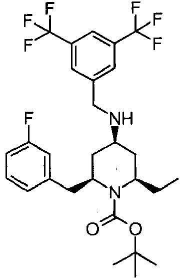 | 621 | 2.63 min (condition A) |
| 4-(3,5-bis-trifluorometh yl-benzylamino)-2-ethyl-6-(3-methyl-benzyl)-piperidine-1-carboxylic acid tert-butyl ester | 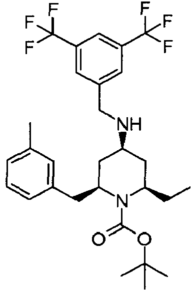 | 617 | 2.69 min (condition A) |
| 4-(3,5-bis-trifluoromethyl-benzylamino)-2-(4-chloro-benzyl)-6-ethyl-piperidine-1-carboxylic acid tert-butyl ester | 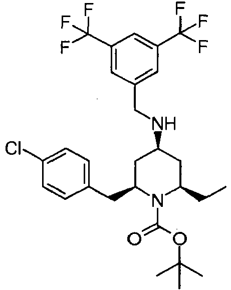 | 637 | 2.70 min (condition A) |
| 4-(3,5-bis-trifluoromethyl-benzylamino)-2-(3,4-difluoro-benzyl)-6-ethyl-piperidine-1-carboxylic acid tert-butyl ester | 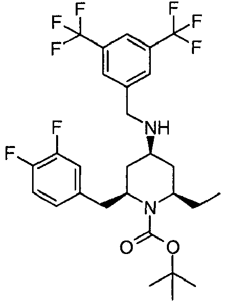 | 639 | 2.62 min (condition A) |
| 4-(3,5-bis-trifluoromethyl-benzylamino)-2-ethyl-6-(2-methyl-benzyl)-piperidine-1-carboxylic acid tert-butyl ester | 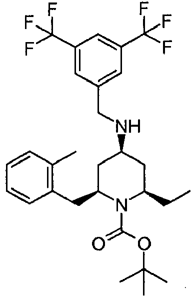 | 617 | 2.77 min (condition A) |
| 4-(3,5-Bis-trifluoromethyl-benzylamino)-2-(2,6-dimethyl-benzyl)-6-ethyl-piperidine-1-carboxylic acid tert-butyl ester | 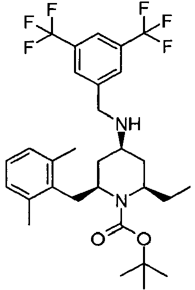 | 631 | 2.71 min (condition A) |
| 4-(3,5-bis-trifluoromethyl-benzylamino)-2-ethyl-6-(2-methoxy-benzyl)-piperidine-1-carboxylic acid tert-butyl ester | 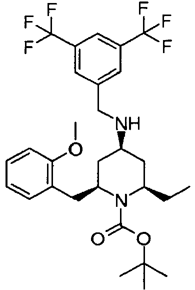 | 633 | 2.63 min (condition A) |

### 9). Synthesis of 4-(3,5-bis-trifluoromethyl-benzylamino)-2-ethyl-piperidine-1-carboxylic acid tert-butyl ester

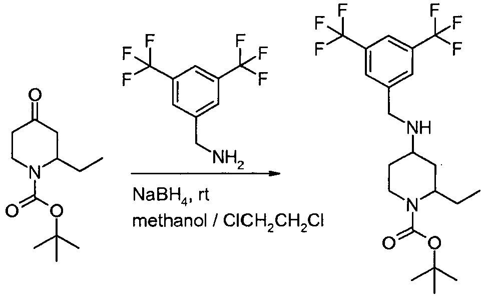

A solution of 2-ethyl-4-oxo-piperidine-1-carboxylic acid tert-butyl ester (1 g, 4.4 mmol), 3,5-bis(trifluoromethyl)benzylamine (1.4g, 4.84 mmol), titanium isopropoxide (catalytic, 3 drops) in 7.5 mL of methanol and 7.5 mL of dichloroethane are stirred with NaBH₄ (183 mg, 4.84 mmol) at room temperature for 6 hours under nitrogen atmosphere. The reaction is quenched by addition of saturated aqueous ammonium chloride and filtered, and the cake is washed with ethyl acetate. The mixture is extracted with ethyl acetate (2 X 50mL). The organic layer is washed with brine, dried over anhydrous sodium sulfate, and then concentration give 1.7 g (76%) of 4-(3,5-bis-trifluoromethyl-benzylamino)-2-ethyl-piperidine-1-carboxylic acid tert-butyl ester; ESI-MS m/z: 455 [M+1]⁺, Retention time 1.75 min (condition A).

### 10). Synthesis of (3,5-bis-trifluoromethyl-benzyl)-(2-ethyl-piperidin-4-yl)-carbamic acid methyl ester

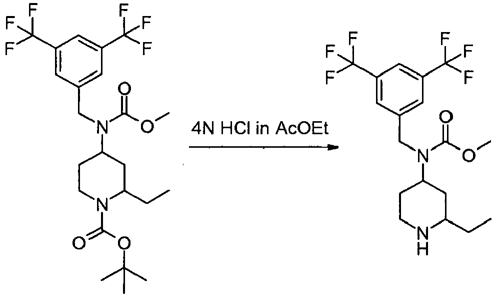

To a solution of 4-[(3,5-bis-trifluoromethyl-benzyl)-methoxycarbonyl-amino]-2-ethyl-piperidine-1-carboxylic acid tert-butyl ester (1.2g, 2.3mmol) in 2 mL of ethyl acetate under N₂, is added 4N HCl in ethyl acetate (10ml), 40mmol) dropwise over 5 minutes. The reaction mixture is stirred for 4 hours. The reaction is subsequently concentrated and then quenched by addition of saturated aqueous sodium bicarbonate (50 mL), and the aqueous layer is extracted with ethyl acetate (2X 50 mL). The organic layers are combined and washed with brine, dried with anhydrous sodium sulfate, and then concentration give 0.8 g (84%) of (3,5-bis-trifluoromethyl-benzyl)-(2-ethyl-piperidin-4-yl)-carbamic acid methyl ester; ESI-MS m/z: 413 [M+1]⁺, Retention time 1.67 min (condition A).

### 11). Synthesis of 5-benzyl-4-(3,5-bis-trifluoromethyl-benzylamino)-2-ethyl-piperidine-1-carboxylic acid tert-butyl ester

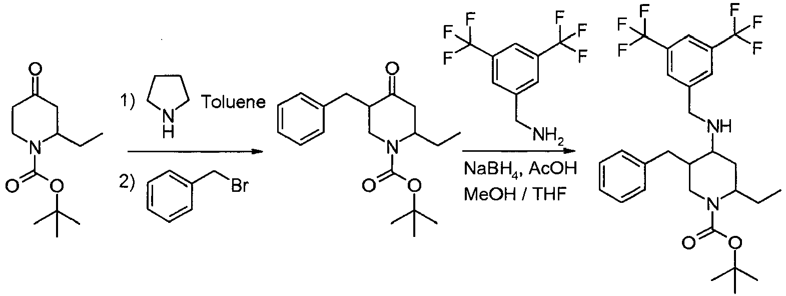

A mixture of 2-ethyl-4-oxo-piperidine-1-carboxylic acid tert-butyl ester (610 mg, 2.7 mmol), pyrrolidine (287 mg, 4.0 mmol) and 5 mL of toluene are refluxed overnight, using a Dean-Stark apparatus. The mixture is evaporated to give a dark residue, which is used directly in the next step. To this dark residue is added benzyl bromide (2 mL, 17 mmol) and stirred at room temperature for 2 days. The reaction is quenched with saturated aqueous ammonium chloride (40 mL), and the mixture is extracted with ethyl acetate (2 X 50 mL), dried with anhydrous sodium sulfate, and concentrated under reduced pressure. The obtained residue is purified by flash silica gel chromatography, eluting with hexane-ethyl acetate mixtures to give 5-benzyl-2-ethyl-4-oxo-piperidine-1-carboxylic acid tert-butyl ester in 20% yields; ESI-MS m/z: 262 [M-55]⁺, Retention time 2.10 min (condition A).

A solution of 5-benzyl-2-ethyl-4-oxo-piperidine-1-carboxylic acid tert-butyl ester (127 mg, 0.4 mmol), 3,5-bis(trifluoromethyl)benzylamine (117mg, 0.484 mmol), titanium isopropoxide (catalytic, 3 drops) in 0.5 mL of acetic acid, 1 mL of methanol and 1 mL of tetrahydrofuran are stirred with NaBH₄ (18 mg, 0.484 mmol) at room temperature for 1 day under nitrogen atmosphere. The reaction is quenched by addition of saturated aqueous ammonium chloride, filtered, and washed with ethyl acetate. The mixture is extracted with ethyl acetate (2 X 50 mL). The organic layers are washed with brine, dried over anhydrous sodium sulfate, and then concentration give the crude product. The obtained residue is purified by flash silica gel chromatography, eluting with hexane-ethyl acetate mixtures to give 5-benzyl-4-(3,5-bis-trifluoromethyl-benzylamino)-2-ethyl-piperidine-1-carboxylic acid tert-butyl ester in 15% yields; ESI-MS m/z: 545 [M+1]⁺, Retention time 2.00 min (condition A).

### 12). Synthesis of 4-(4-bromo-phenylamino)-2-ethyl-piperidine-1-carboxylic acid tert-butyl ester

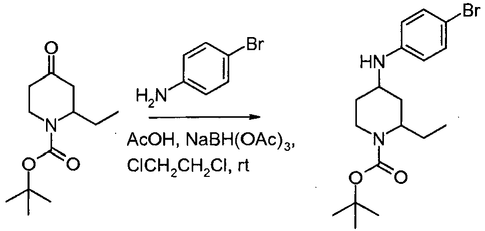

A mixture of 2-ethyl-4-oxo-piperidine-1-carboxylic acid tert-butyl ester (114 mg, 0.5 mmol), *p*-bromoaniline (95 mg, 0.55 mmol), sodium triacetoxyborohydride (111 mg, 0.525 mmol) and acetic acid (34 *µ* L, 0.6 mmol) in 1,2-dichloroethane (0.8 mL) is stirred at room temperature for 5 hours. To the mixture is added sodium triacetoxyborohydride (111 mg, 0.525 mmol). Then, the mixture is stirred at room temperature for 20 hours. The mixture is basified with aq. 1 N sodium hydroxide solution until pH 10, then extracted with 1,2-dichloroethane. The organic layer is concentrated under reduced pressure. The residue is purified by silica gel column chromatography (eluent: ethyl acetate / n-hexane = 1/4) to give 93 mg of 4-(4-bromo-phenylamino)-2-ethyl-piperidine-1-carboxylic acid tert-butyl ester; ESI-MS m/z: 383 [M+1]⁺, Retention time 2.18 min (condition A).

### 13). Synthesis of 4-cyclohexylamino-2-ethyl-piperidine-1-carboxylic acid tert-butyl ester

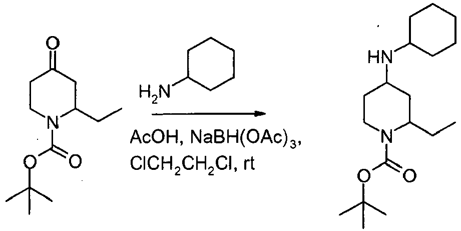

A mixture of 2-ethyl-4-oxo-piperidine-1-carboxylic acid tert-butyl ester (114 mg, 0.5 mmol), cyclohexylamine (63 *µ* L, 0.55 mmol), sodium triacetoxyborohydride (111 mg, 0.525 mmol) and acetic acid (34 *µ* L, 0.6 mmol) in 1,2-dichloroethane (0.8 mL) is stirred at room temperature for 5 hours. To the mixture is added sodium triacetoxyborohydride (111 mg, 0.525 mmol). Then, the mixture is stirred at room temperature for 20 hours. The mixture is basified with aq. 1N sodium hydroxide solution until pH 10, then extracted with 1,2-dichloroethane. The organic layer is concentrated under reduced pressure. The residue is purified by silica gel column chromatography (eluent: methanol / dichloromethane = 1/10) to give 124 mg of 4-cyclohexylamino-2-ethyl-piperidine-1-carboxylic acid tert-butyl ester; ESI-MS m/z: 311 [M+1]⁺, Retention time 1.63 min (condition A).

### 14). Synthesis of (3-chloromethyl-5-trifluoromethyl-phenyl)-dimethylamine

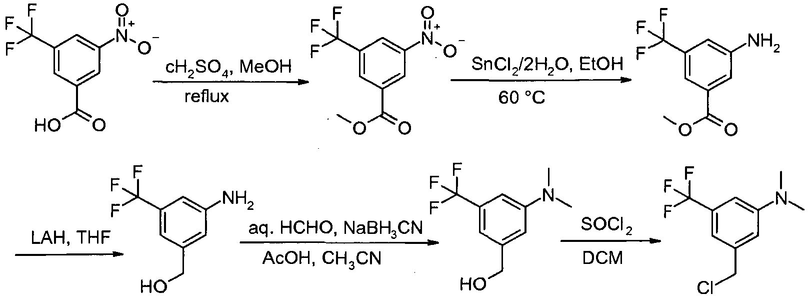

A solution of 5-nitro-3-(trifluoromethyl)benzoic acid (2.35 g, 10 mmol) and H₂SO₄ (53 µL, 1 mmol) in methanol (30 mL) is allowed to warm to reflux and stir for 20 hours. The mixture is cooled to room temperature, then the mixture is concentrated under reduced pressure. The obtained residue is neutralized with saturated aq. NaHCO₃. The mixture is extracted with CH₂Cl₂. The combined organic layer is washed with brine, dried over Na₂SO₄, filtrated, and concentrated under reduced pressure to give 3-nitro-5-trifluoromethyl-benzoic acid methyl ester (2.15 g, 86%), which is used in the next step without further purification.

A solution of 3-nitro-5-trifluoromethyl-benzoic acid methyl ester (8.6 mmol, 2.15 g) and tin chloride dihydrate (25.8 mmol, 5.82 in ethanol (40 ml) is allowed to warm to 60 °C and stir for 4 hours. The mixture is cooled to room temperature, then concentrated under reduced pressure. The obtained residue is neutralized with saturated aq. NaHCO₃. The mixture is filtered and concentrated under reduced pressure. The obtained residue is purified by silica gel column chromatography (eluent: hexane / EtOAc) to give 3-amino-5-trifluoromethyl-benzoic acid methyl ester (74%, 1.4 g); ESI-MS m/z: 220 [M+1]⁺, Retention time 1.81 min (condition A).

To a suspension of lithium aluminum hydride (4 mmol, 152 mg) in THF (5 mL) which is cooled to 0 °C is dropwise added a solution of 3-amino-5-trifluoromethyl-benzoic acid methyl ester (2 mmol, 438 mg) in THF (1 mL) under nitrogen. The solution is allowed to warm to rt and stirred for 2 hours. To the solution is added diethyl ether (6 mL), then quenched with sodium sulfate decahydrate and brine. After decantation, the solution is dried over Na₂SO₄, filtrated, and concentrated under reduced pressure to give (3-amino-5-trifluoromethyl-phenyl)-methanol (quantitative yield), which is used in the next step without further purification); ESI-MS m/z: 192 [M+1]⁺, Retention time 1.30 min (condition A).

To a solution of (3-amino-5-trifluoromethyl-phenyl)-methanol (1 mmol, 191 mg) and 37% aq. formaldehyde (5 mmol, 372 uL) in acetonitril (2.5 ml) is added acetic acid (5.5 mmol, 315 uL) and NaBH(OAc)₃ (0.40 mmol, 84.8 mg) under nitrogen at 0 °C. The mixture is allowed to stir for 30 minutes at 0 °C and then basified with aq.1 N NaOH to approximately pH 10. The layers are separated, and the aqueous layer is extracted with dichloromethane. The combined organic layer is washed with brine, dried over Na₂SO₄, filtrated, and concentrated under reduced pressure. The residue is purified by silica gel column chromatography (eluent: hexane / EtOAc) to give (3-dimethylamino-5-trifluoromethyl-phenyl)-methanol (128 mg, 58%); ESI-MS m/z: 220 [M+1]⁺, Retention time 1.55 min (condition A).

To a solution of (3-dimethylamino-5-trifluoromethyl-phenyl)-methanol (0.15 mmol, 33 mg) in dichloromethane (1 mL) which is cooled to 0 °C is dropwise added thionyl chloride (0.3 mmol, 21 uL) under nitrogen. The solution is allowed to warm to rt and stirred for 1 hour. The solution is added saturated aq.NaHCO₃. The layers are separated, and the aqueous layer is extracted with dichloromethane. The combined organic layer is washed with brine, dried over Na₂SO₄, filtrated, and concentrated under reduced pressure to give (3-chloromethyl-5-trifluoromethyl-phenyl)-dimethylamine (quantitative); ESI-MS m/z: 238 [M+1]⁺, Retention time 2.14 min (condition A).

### 15). Synthesis of 1-bromomethyl-3-methoxy-5-trifluoromethyl-benzene

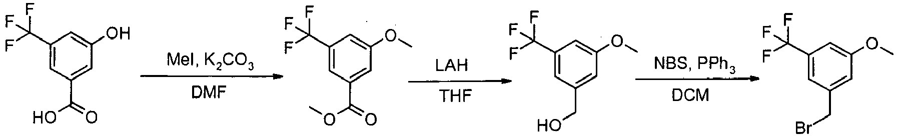

A solution of 3-hydroxy-5-(trifluoromethyl)benzoic acid (1.03 g, 5 mmol) and K₂CO₃ (2.07 g, 15 mmol) in DMF (50 mL) is added methyl iodide (1.09 mL, 17.5 mmol) allowed to warm to 40 °C and stir for 20 hours. The mixture is cooled to room temperature, then added water. The mixture is extracted with ethyl acetate. The combined organic layer is washed with brine, dried over Na₂SO₄, filtrated, and concentrated under reduced pressure. The residue is purified by silica gel column chromatography (eluent: hexane / EtOAc) to give 3-methoxy-5-trifluoromethyl-benzoic acid methyl ester (1.11 g, 95%); ESI-MS m/z: 235 [M+1]⁺, Retention time 2.06 min (condition A).

To a suspension of lithium aluminum hydride (9.48 mmol, 360 mg) in THF which is cooled to 0 °C is dropwise added a solution of 3-methoxy-5-trifluoromethyl-benzoic acid methyl ester (4.74 mmol, 1.11 g) in THF under nitrogen. The solution is allowed to warm to rt and stirred for 2 hours. The solution is added diethyl ether, then quenched with sodium sulfate decahydrate and brine. After decantation, the solution is dried over Na₂SO₄, filtrated, and concentrated under reduced pressure to give (3-methoxy-5-trifluoromethyl-phenyl)-methanol (960 mg, 98%), which is used in the next step without further purification.

To a solution of (3-methoxy-5-trifluoromethyl-phenyl)-methanol (1 mmol, 206 mg) and triphenylphosphine (1.5 mmol, 392 mg) in dichloromethane (10 mL) which is cooled to 0 °C is added NBS (1.5 mmol, 266 mg) under nitrogen. The solution is allowed to warm to rt and stirred for 1 hour. The solution is added water, then extracted with ethyl acetate. The combined organic layer is washed with brine, dried over Na₂SO₄, filtrated, and concentrated under reduced pressure. The residue is purified by silica gel column chromatography (eluent: hexane / EtOAc) to give 1-bromomethyl-3-methoxy-5-trifluoromethyl-benzene (245 mg, 91%); ESI-MS m/z: 189 [M-Br+1]⁺, Retention time 1.76 min (condition A).

### 16). Synthesis of 1-bromomethyl-3-methanesulfonyl-5-trifluoromethyl-benzene

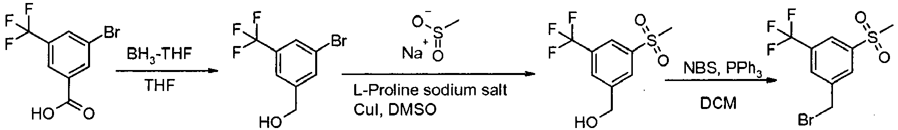

To a solution of 3-bromo-5-(trifluoromethyl)benzoic acid (5 mmol, 1.35 g) in THF (8 mL) is added 1M boran in THF (16 mmol, 16 mL) under nitrogen. The solution is allowed to warm to 65 °C and stirred for 2 hours. The mixture is cooled to room temperature, then poured into saturated aq. NaHCO₃. The mixture is extracted with EtOAc. The combined organic layer is washed with brine, dried over Na₂SO₄, filtrated, and concentrated under reduced pressure. The residue is purified by silica gel column chromatography (eluent: hexane / EtOAc) to give (3-bromo-5-trifluoromethyl-phenyl)-methanol (418 mg, 69%).

To a solution of (3-bromo-5-trifluoromethyl-phenyl)-methanol (2 mmol, 613 mg), L-proline sodium salt (0.4 mmol, 55 mg), copper iodide (0.2 mmol, 38 mg) in DMSO (4 mL) is added sodium methanesulfinate (2.4 mmol, 245 mg) under nitrogen. The solution is allowed to warm to 95 °C and stirred for 20 hours. The mixture is cooled to room temperature, then added water. The mixture is extracted with dichloromethane. The combined organic layer is washed with brine, dried over Na₂SO₄, filtrated, and concentrated under reduced pressure. The residue is purified by silica gel column chromatography (eluent: hexane / EtOAc) to give (3-methanesulfonyl-5-trifluoromethyl-phenyl)-methanol (418 mg, 69%); ESI-MS m/z: 255 [M+1]⁺, Retention time 1.64 min (condition A).

To a solution of (3-methanesulfonyl-5-trifluoromethyl-phenyl)-methanol (0.8 mmol, 203 mg) and triphenylphosphine (1.2 mmol, 315 mg) in dichloromethane (8 mL) which is cooled to 0 °C is added NBS (1.2 mmol, 214 mg) under nitrogen. The solution is allowed to warm to rt and stirred for 1 hour. The solution is added water, then extracted with dichloromethane. The combined organic layer is washed with brine, dried over Na₂SO₄, filtrated, and concentrated under reduced pressure. The residue is purified by silica gel column chromatography (eluent: hexane / EtOAc) to give 1-bromomethyl-3-methanesulfonyl-5-trifluoromethyl-benzene (228 mg, 90%).

### 17). Synthesis of 1-(3-bromomethyl-5-trifluoromethyl-phenyl)-5-methyl-1H-tetrazole

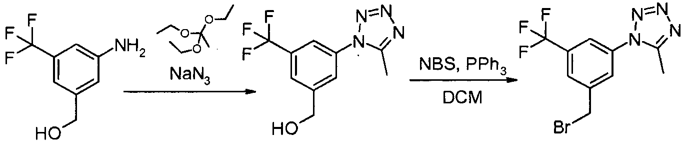

To a solution of (3-amino-5-trifluoromethyl-phenyl)-methanol (210 mg, 1,1 mmol) in acetic acid (1.6 mL) is added triethylorthoacetate (282 µL, 1.54 mmol). The mixture is allowed to warm to 75 °C and stir for 45 minutes, then added sodium azide (215 mg, 3.3 mmol) at 75 °C and stir for 3 hours. The mixture is cooled to room temperature, then added water. The mixture is basified with 1 N aq. NaOH to approximately pH 10, then extracted with dichloromethane. The combined organic layer is washed with brine, dried over Na₂SO₄, filtrated, and concentrated under reduced pressure. The residue is purified by silica gel column chromatography (eluent: hexane / EtOAc) to give [3-(5-methyl-tetrazol-1-yl)-5-trifluoromethyl-phenyl]-methanol (94 mg, 33%); ESI-MS m/z: 259 [M+1]⁺, Retention time 1.70 min (condition A).

To a solution of [3-(5-methyl-tetrazol-1-yl)-5-trifluoromethyl-phenyl]-methanol (0.23 mmol, 60 mg) and triphenylphosphine (0.35 mmol, 92 mg) in dichloromethane (2.5 mL) which is cooled to 0 °C is added NBS (0.35 mmol, 62 mg) under nitrogen. The solution is allowed to warm to rt and stirred for 4 hour. The solution is added water, then extracted with dichloromethane. The combined organic layer is washed with brine, dried over Na₂SO₄, filtrated, and concentrated under reduced pressure. The residue is purified by silica gel column chromatography (eluent: hexane / EtOAc) to give 1-(3-bromomethyl-5-trifluoromethyl-phenyl)-5-methyl-1*H*-tetrazole (72 mg, 96%); ESI-MS m/z: 320 [M+1]⁺, Retention time 2.08 min (condition A).

### 18). Synthesis of 1-bromomethyl-3-chloro-5-trifluoromethoxy-benzene

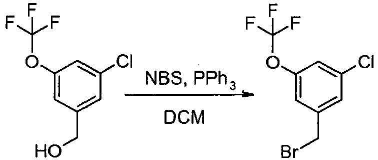

To a solution of (3-chloro-5-trifluoromethoxy-phenyl)-methanol (1 mmol, 226 mg) and triphenylphosphine (1.5 mmol, 392 mg) in dichloromethane (10 mL) which is cooled to 0 °C is added NBS (1.5 mmol, 266 mg) under nitrogen. The solution is allowed to warm to RT and stirred for 1 hour. The solution is added water, then extracted with dichloromethane. The combined organic layer is washed with brine, dried over Na₂SO₄, filtrated, and concentrated under reduced pressure. The residue is purified by silica gel column chromatography (eluent: hexane / EtOAc) to give 1-bromomethyl-3-chloro-5-trifluoromethoxy-benzene (257 mg, 89%).

### 19). Synthesis of 4-(3,5-bis-trifluoromethyl-benzylamino)-2-cyclohexylmethyl-6-ethyl-piperidine-1-carboxylic acid tert-butyl ester (2,4,6-cis isomer)

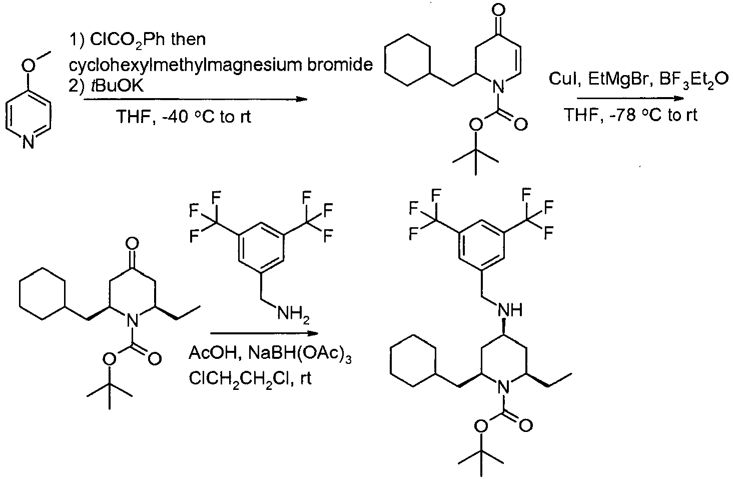

To a solution of 4-methoxypyridine (9.6 mmol, 1.05 g) in THF (75 mL) which is cooled to -40 °C is added phenyl chloroformate (10.6 mmol, 1.33 mL) under nitrogen. After stirred for 20 minutes, a solution of 0.7 M cyclohexylmethylmagnesium bromide in THF (10.08 mmol) is added slowly. The mixture is allowed to warm to room temperature, and after stirring for 2 hours, the mixture is quenched with ice and then with H₂O and extracted with Et₂O. The combined organic layer is washed with brine, dried over Na₂SO₄, filtrated, and concentrated under reduced pressure. To the residue is added THF (30 ml) and the solution is allowed to cool to -78 °C and *t*BuOK (38.4 mmol, 4.3 g) is added. The solution is allowed to warm to room temperature gradually. The mixture is quenched with ice and then with H₂O and extracted with Et₂O. The combined organic layer is washed three times with aq.1 N NaOH and then three times with aq.1 N HCl. The mixture is dried over MgSO₄, filtrated, and concentrated under reduced pressure. The obtained residue is purified by silica gel column chromatography (eluent: n-hexane / ethyl acetate) to give 2-cyclohexylmethyl-4-oxo-3,4-dihydro-2H-pyridine-1-carboxylic acid tert-butyl ester (15%, 0.43 g); ESI-MS m/z: 294 [M+1]⁺, Retention time 2.29 min (condition A).

To Cul (1.0 mmol, 191 mg) and THF (4 mL) in a flask purged with N₂ is added 1.00 M tetrahydrofuran solution of EtMgBr (1.0 mmol, 1.0 ml) at -78 °C. After stirring the suspension for 10 min, BF₃·Et₂O (1.0 mmol, 1.0 mg) is added and stirred for 10 min at the same temperature. To the suspension is added tetrahydrofuran solution (5 mL) of 2-cyclohexylmethyl-4-oxo-3,4-dihydro-2H-pyridine-1-carboxylic acid tert-butyl ester (0.5 mmol, 119.7 mg) at -78 °C, then the mixture is allowed to stir for 1 hour. After the mixture is warmed to room temperature and stirred for 2 hours, it is quenched with saturated aqueous NH₄Cl and extracted with EtOAc. The combined organic layer is washed with brine, dried over Na₂SO₄, filtered, and concentrated under reduced pressure. The obtained residue is purified by silica gel column chromatography (eluent: n-hexane / ethyl acetate) to *cis*-2-cyclohexylmethyl-6-ethyl-4-oxo-piperidine-1-carboxylic acid tert-butyl ester (108 mg, 67%); ESI-MS m/z: 268 [M-*t*Bu+2]⁺, Retention time 2.44 min (condition A).

To a solution of *cis*-2-cyclohexylmethyl-6-ethyl-4-oxo-piperidine-1-carboxylic acid tert-butyl ester (0.21 mmol, 68 mg) and 3,5-bis(trifluoromethyl)benzylamine (0.25 mmol, 60.8 mg) in 1,2-dichloroethane (0.4 ml) is added acetic acid (0.25 mmol, 13.7 uM) and NaBH(OAc)₃ (0.42 mmol, 89 mg) at room temperature. The mixture is allowed to stir for 7.5 hours and then basified with aq.1 N NaOH to approximately pH 10. The mixture is extracted with CH₂Cl₂ and the combined organic layer is concentrated under reduced pressure. The residue is purified by silica gel column chromatography (eluent: n-hexane / ethyl acetate) to give a racemic mixture of 4-(3,5-bis-trifluoromethyl-benzylamino)-2-cyclohexylmethyl-6-ethyl-piperidine-1-carboxylic acid tert-butyl ester (2,4,6-cis isomer: 88.0 mg, 76%); ESI-MS m/z: 551 [M+1]⁺, Retention time 2.21 min (condition A).

### 20). Synthesis of 2-benzyl-4-[(3,5-bis-trifluoromethyl-benzyl)-methoxycarbonyl-amino]-6-hydroxymethyl-piperidine-1-carboxylic acid tert-butyl ester (2,4,6-cis isomer)

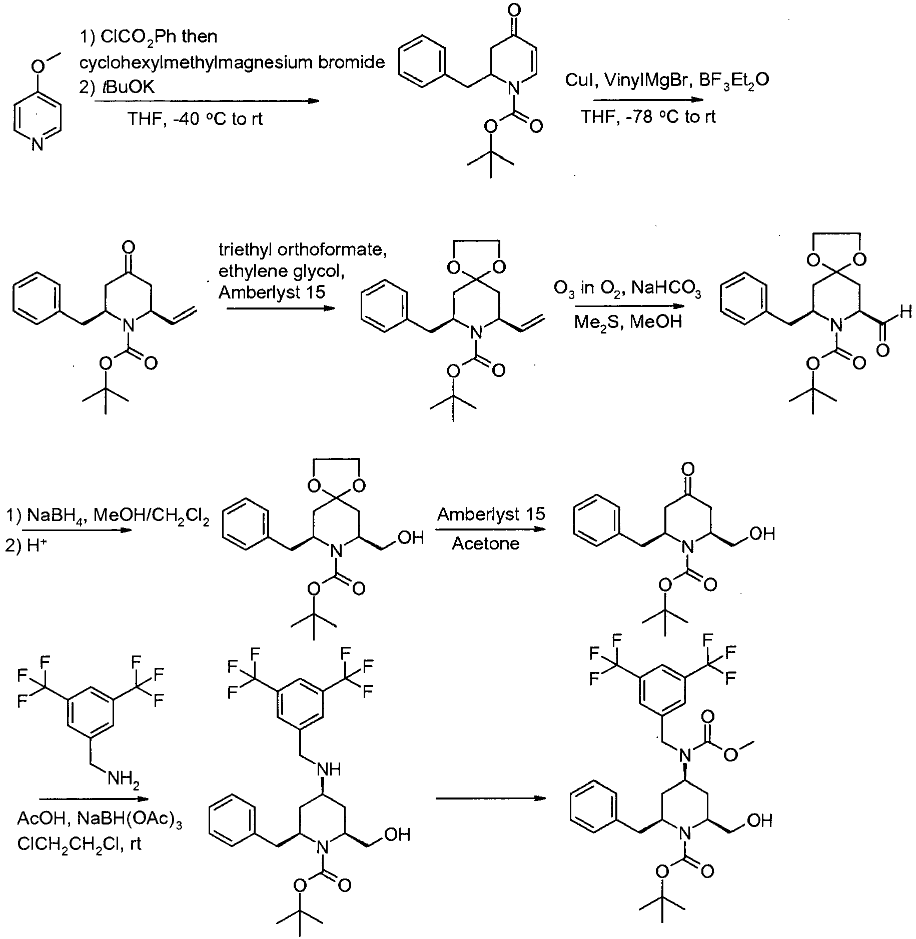

To a solution of 4-methoxypyridine (100 mmol, 10 mL) in THF (390 mL) which is cooled to -40 °C is added phenyl chloroformate (105 mmol, 13 mL) under nitrogen. After stirred for 20 minutes, a solution of 1.0 M benzyllmethylmagnesium bromide in THF (110 mmol) is added slowly. The mixture is allowed to warm to room temperature, and after stirring for 2 hours, the mixture is cooled to -40 °C and *t*BuOK (250 mmol, 28.1 g) is added. The solution is allowed to warm to room temperature gradually and stirred for 14 hours. The mixture is quenched with ice and then with H₂O and extracted with Et₂O. The combined organic layer is washed three times with aq.1 N NaOH and then three times with aq.1 N HCl. The mixture is dried over MgSO₄, filtrated, and concentrated under reduced pressure. The obtained residue is passed through a silica gel pad and concentrated under reduced pressure to give the crude product, which is used in next step without further purification.

To Cul (98.8 mmol, 18.8 g) and THF (200 mL) in a flask purged with N₂ is added 1.00 M tetrahydrofuran solution of vinylmagnesium bromide (98.8 mmol, 98.8 ml) over 30 minutes at -60 °C. After stirring the suspension for 15 min, BF₃ Et₂O (49.4 mmol, 6.2 mL) is added and stirred for 15 min at the same temperature. To the suspension is added tetrahydrofuran solution (200 mL) of the crude product at -40 °C, then the mixture is allowed to stir for 2 hours. After the mixture is warmed to room temperature and stirred for 2 hours, it is quenched with saturated aqueous NH₄Cl and extracted with EtOAc. The combined organic layer is washed with brine, dried over MgSO₄, filtered, and concentrated under reduced pressure. The obtained residue is purified by silica gel column chromatography (eluent: n-hexane / ethyl acetate) to give a diastereomeric mixture of 2-benzyl-4-oxo-6-vinyl-piperidine-1-carboxylic acid tert-butyl ester (4.29 g, 14% in 2 steps); ESI-MS m/z: 260 [M-*^{t}*Bu+2]⁺, Retention time 2.17 min (condition A).

A mixture of 2-benzyl-4-oxo-6-vinyl-piperidine-1-carboxylic acid tert-butyl ester (13.6 mmol, 4.29 g), Amberlyst ^{®} 15 (680 mg), triethyl orthoformate (109 mmol, 18 mL), ethylene glycol (136 mmol, 7.5 mL) is stirred for 24 hours at room temperature. After stirring with an additional Amberlyst ^{®} 15 (136 mg) for 4 hours, the mixture is passed through a pad of silica gel and washed with ethyl acetate. The mixture is washed with 0.1 M aq. HCl, saturated aq. NaHCO₃, and brine. The organic layer is dried over Na₂SO₄, filtered, and concentrated under reduced pressure to give a diastereomeric mixture of 7-benzyl-9-vinyl-1,4-dioxa-8-azaspiro[4.5]decane-8-carboxylic acid tert-butyl ester (4.72 g, 97%); ESI-MS m/z: 304 [M-*^{t}*Bu+2]⁺, Retention time 2.33 min (condition A).

To a mixture of 7-benzyl-9-vinyl-1,4-dioxa-8-aza-spiro[4.5]decane-8-carboxylic acid tert-butyl ester (13.1 mmol, 4.72 g) and NaHCO₃ (26.2 mmol, 2.20 g) in methanol (70 mL) at -78 °C is added O₃ in O₂ (100 NL/hour, 60v) for 1 hour. After O₂ flow for 30 minutes, dimethylsulfide (78.6 mmol, 5.8 mL) is added and the reaction mixture is stirred for 30 minutes at the same temperature and allowed to warm up to room temperature. To the mixture is added K₂CO₃ (15.7 mmol, 2.17 g) and stirred for 11 hours. To the mixture is added water and the organic product is extracted with diethyl ether. The organic layer is washed with saturated aq. NH₄Cl and brine, dried over Na₂SO₄, filtered, and concentrated under reduced pressure. The obtained residue is purified by silica gel column chromatography (eluent: n-hexane / ethyl acetate) to give a diastereomeric mixture of 7-benzyl-9-formyl-1,4-dioxa-8-aza-spiro[4.5]decane-8-carboxylic acid tert-butyl ester (2.06 g, 44%); ESI-MS m/z: 306 [M-*^{t}*Bu+2]⁺, Retention time 2.18 min (condition A).

To a mixture of 7-benzyl-9-formyl-1,4-dioxa-8-aza-spiro[4.5]decane-8-carboxylic acid tert-butyl ester (3.0 mmol, 1.08 g) in methanol (30 mL) and dichloromethane (10 mL) at 0 °C is added NaBH₄ (9.0 mmol, 340 mg). The mixture is stirred for 1.5 hours at room temperature, quenched with saturated aq. NH₄Cl, and extracted with dichloromethane. The organic layer is washed with brine, dried over Na₂SO₄, filtered, and concentrated under reduced pressure. The obtained residue is purified by silica gel column chromatography (eluent: n-hexane / ethyl acetate) to give a diastereomeric mixture of 7-benzyl-9-hydroxymethyl-1,4-dioxa-8-aza-spiro[4.5]decane-8-carboxylic acid tert-butyl ester (890 mg, 82%); ESI-MS m/z: 364 [M+1]⁺, Retention time 3.73 (2,6-*cis*), 3.85 (2,6-*trans*) min (condition B).

A mixture of 7-benzyl-9-hydroxymethyl-1,4-dioxa-8-aza-spiro[4.5]decane-8-carboxylic acid tert-butyl ester (2.45 mmol, 890 mg) and Amberlyst ^{®} 15 (2.9 g) in acetone (25 mL) and water (5 mL) is stirred at 60 °C. The mixture is stirred for 2 days at 60 °C and filtered. After washed with ethyl acetate and dichloromethane, the collected mixture is concentrated under reduced pressure. The obtained residue is mixed with ethyl acetate, washed with brine, dried over MgSO₄, filtered, and concentrated under reduced pressure. The obtained residue is passed through a silica gel pad (eluent: dichloromethane / methanol) to give the crude product (360 mg), which is used in next step without further purification.

To a mixture of the crude product (360 mg), 3,5-bis(trifluoromethyl)benzylamine (1.70 mmol, 413 mg) and acetic acid (1.70 mmol, 93 uL) in dichloroethane (5 mL) is added NaBH(OAc)₃ (3.40 mmol, 721 mg) at room temperature. The mixture is stirred for 13 hours and then quenched with aq. 1 M NaOH. The mixture is extracted with dichloromethane, and the organic layer is concentrated under reduced pressure. The obtained residue is purified by silica gel column chromatography (eluent: dichloromethane / methanol, then n-hexane / ethyl acetate) to give *cis*-2-benzyl-4-(3,5-bis-trifluoromethyl-benzylamino)-6-hydroxymethyl-piperidine-1-carboxylic acid tert-butyl ester (216 mg, 11 % in 2 steps); ESI-MS m/z: 547 [M+1]⁺, Retention time 3.73 min (condition B).

To a mixture of 2-benzyl-4-(3,5-bis-trifluoromethyl-benzylamino)-6-hydroxymethyl-piperidine-1-carboxylic acid tert-butyl ester (0.05 mmol, 28.0 mg) and K₂CO₃ (0.50 mmol, 69 mg) in THF (0.5 mL) is added methylchloroformate (0.15 mmol, 10.6 uL) at room temperature. After stirred for 2 hours at room temperature, water is added, and the mixture is extracted with EtOAc. The organic layer is washed with brine, dried over Na₂SO₄, filtered, and concentrated under reduced pressure. The obtained residue is purified by silica gel column chromatography (eluent: n-hexane / ethyl acetate) to give 2-benzyl-4-[(3,5-bis-trifluoromethyl-benzyl)-methoxycarbonyl-amino]-6-hydroxymethyl-piperidine-1-carboxylic acid tert-butyl ester (17.3 mg, 57%); ESI-MS m/z: 605 [M+1]⁺, Retention time 2.41 min (condition A).

### 21). Synthesis of 4-(3,5-bis-trifluoromethyl-benzylamino)-2-ethyl-6-((R)-hydroxyphenyl-methyl)-piperidine-1-carboxylic acid tert-butyl ester (2,4,6-cis isomer)

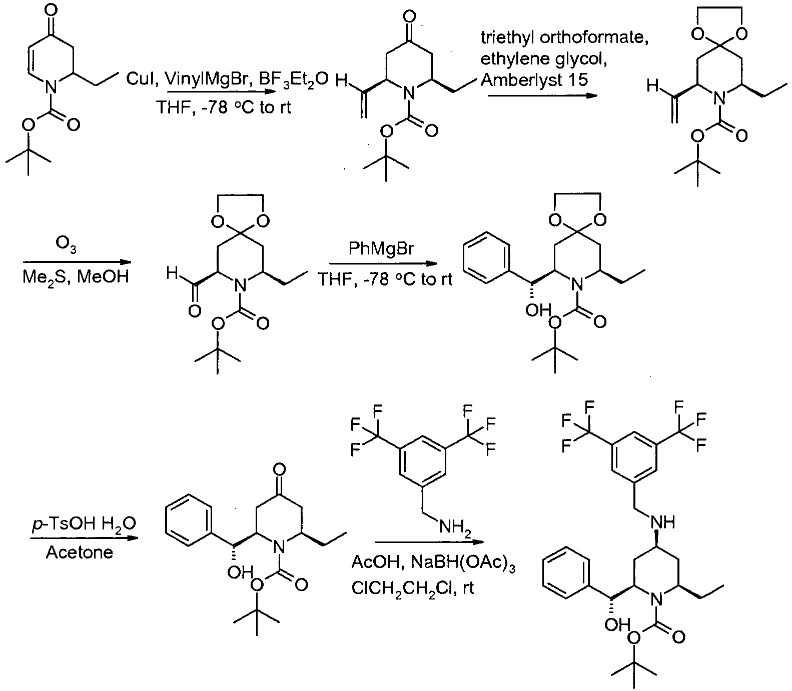

To Cul (10.0 mmol, 1.90 g) in THF (20 mL) is added 1M vinylmagnesium bromide in THF (10.0 mmol, 10 mL) slowly at -78 °C under nitrogen. To the resulting mixture is added BF₃ Et₂O (5.0 mmol, 624 uL), then a solution of 2-ethyl-4-oxo-3,4-dihydro-2H-pyridine-1-carboxylic acid tert-butyl ester (5.0 mmol, 1.13 g) in THF (20 mL). After stirred for 3 hours at room temperature, saturated aq. NH₄Cl is added, and the mixture is extracted with ethyl acetate. The organic layer is washed with brine, dried over Na₂SO₄, filtered, and concentrated under reduced pressure. The obtained residue is purified by silica gel column chromatography (eluent: n-hexane / ethyl acetate) to give a diastereomeric mixture of 2-ethyl-6-isopropenyl-4-oxo-piperidine-1-carboxylic acid tert-butyl ester (1.15 g, 91%); ESI-MS m/z: 198 [M-*^{t}*Bu+2]⁺, Retention time 2.06 min (condition A).

A mixture of 2-ethyl-6-isopropenyl-4-oxo-piperidine-1-carboxylic acid tert-butyl ester (17.4 mmol, 4.41 g), Amberlyst ^{®} 15 (1 g), triethyl orthoformate (122 mmol, 20.3 mL), ethylene glycol (174 mmol, 9.6 mL) is stirred for 18 hours at room temperature. The mixture is passed through a pad of silica gel and washed with ethyl acetate, and the collected solution is washed with 0.1M aq. HCl and saturated aq. NaHCO₃. The organic layer is dried over Na₂SO₄, filtered, and concentrated under reduced pressure to give 7-ethyl-9-isopropenyl-1,4-dioxa-8-aza-spiro[4.5]decane-8-carboxylic acid tert-butyl ester (4.83 g, 93%); ESI-MS m/z: 242 [M-*^{t}*Bu+2]⁺, Retention time 2.31 min (condition A).

To a solution of 7-ethyl-9-isopropenyl-1,4-dioxa-8-aza-spiro[4.5]decane-8-carboxylic acid tert-butyl ester (3.36 mmol, 1.00 g) in methanol (34 mL) at -78 °C is added O₃ (100 NL/hour, 60v) for 45 minutes. After O₂ flow for 15 minutes, dimethylsulfide (27.2 mmol, 2 mL) is added at the same temperature and the reaction mixture is allowed to warm up to room temperature and stirred for 1 hour. The mixture is concentrated, and diethyl ether is added. The mixture is washed with water and brine, and the organic layer is dried over Na₂SO₄, filtered, and concentrated under reduced pressure. The obtained residue is purified by silica gel column chromatography (eluent: n-hexane / ethyl acetate) to give 7-ethyl-9-formyl-1,4-dioxa-8-aza-spiro[4.5]decane-8-carboxylic acid tert-butyl ester (517 mg, 51%); ESI-MS m/z: 244 [M-*^{t}*Bu+2]⁺, Retention time 2.07 min (condition A).

To a solution of 7-ethyl-9-formyl-1,4-dioxa-8-aza-spiro[4.5]decane-8-carboxylic acid tert-butyl ester (1.0 mmol, 299 mg) in THF (9 mL) at -78 °C is added 1 M phenylmagnesium bromide in THF (2.0 mmol, 2.0 mL). The mixture is stirred at room temperature for 1 hour, and then stirred with additional 1 M phenylmagnesium bromide in THF (1.0 mmol, 1.0 mL) for 1 hour. To the mixture is added saturated aq. NH₄Cl, and extracted with ethyl acetate. The mixture is washed with water and brine, and the organic layer is dried over Na₂SO₄, filtered, and concentrated under reduced pressure. The obtained residue is purified by silica gel column chromatography (eluent: *n*-hexane / ethyl acetate) to give 7-ethyl-9-((R)-hydroxy-phenyl-methyl)-1,4-dioxa-8-aza-spiro[4.5]decane-8-carboxylic acid tert-butyl ester (131.7 mg, 35%); ESI-MS m/z: 378 [M+1]⁺, Retention time 4.35 min (condition B).

A mixture of 7-ethyl-9-((R)-hydroxy-phenyl-methyl)-1,4-dioxa-8-aza-spiro[4.5]decane-8-carboxylic acid tert-butyl ester (0.25 mmol, 95.1 mg) and *p*-toluenesulfonic acid monohydrate (0.25 mL, 43 mg) in acetone (9 mL) at 50 °C is stirred for 14 hours, and then saturated aq. NaHCO₃ is added. The mixture is extracted with ethyl acetate, and the organic layer is dried over Na₂SO₄, filtered, and concentrated under reduced pressure. The obtained residue is purified by silica gel column chromatography (eluent: *n*-hexane / ethyl acetate) to give 2-ethyl-6-((R)-hydroxy-phenyl-methyl)-4-oxo-piperidine-1-carboxylic acid tert-butyl ester (36 mg, 43%); ESI-MS m/z: 278 [M-*^{t}*Bu+2]⁺, Retention time 2.08 min (condition A).

To a mixture of 2-ethyl-6-((R)-hydroxy-phenyl-methyl)-4-oxo-piperidine-1-carboxylic acid tert-butyl ester (0.11 mmol, 36 mg), 3,5-bis(trifluoromethyl)benzylamine (0.17 mmol, 41 mg) and acetic acid (0.19 mmol, 10.4 uL) in dichloroethane (1.1 mL) is added NaBH(OAc)₃ (0.34 mmol, 72 mg) at room temperature. The mixture is stirred for 13 hours and then quenched with aq. 1M NaOH. The mixture is extracted with dichloromethane, and the organic layer is concentrated under reduced pressure. The obtained residue is purified by silica gel column chromatography (eluent: n-hexane / ethyl acetate) to give a diastereomeric mixture of 4-(3,5-bis-trifluoromethyl-benzylamino)-2-ethyl-6-((R)-hydroxy-phenyl-methyl)-piperidine-1-carboxylic acid tert-butyl ester (41.4 mg, 67%, 2,4,6-cis / 2,6*-cis*-4-*trans* = 1 / 6); ESI-MS m/z: 561 [M+1]⁺, Retention time 2.06 min (condition A).

### 22). Synthesis of 4-(3,5-bis-trifluoromethyl-benzylamino)-2-ethyl-6-methoxymethyl-piperidine-1-carboxylic acid tert-butyl ester (2,4,6-cis isomer)

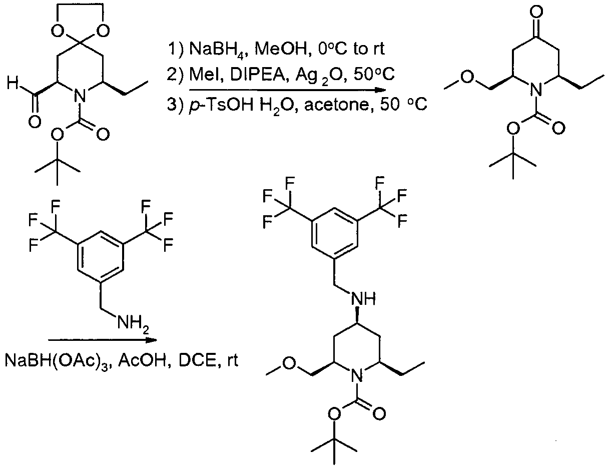

To a solution of 7-ethyl-9-formyl-1,4-dioxa-8-aza-spiro[4.5]decane-8-carboxylic acid tert-butyl ester (1.0 mmol, 299 mg) in methanol (10 mL) is added NaBH₄ (3.0 mmol, 113 mg) at 0 °C. After stirred for 1.5 hours at room temperature, saturated aq. NH₄Cl is added, and the mixture is extracted with ethyl acetate. The organic layer is washed with brine, dried over MgSO₄, filtered, and concentrated under reduced pressure. To the obtained residue in DMF (5 mL) is added DIPEA (3.0 mmol, 523 uL), Ag₂O (3.0 mmol, 493 mg), and Mel (19 mmol, 1.2 mL) at room temperature. After stirred for 24 hours at 50 °C, the mixture is filtered and washed with ethyl acetate. To the collected solution is added saturated aq. NH₄Cl, and the mixture is extracted with ethyl acetate. The organic layer is washed with brine, dried over MgSO₄, filtered, and concentrated under reduced pressure. To the obtained residue in acetone (10 mL) is added p-toluenesulfonic acid monohydrate (1.0 mmol, 172 mg), and the mixture is stirred 50 °C for 14 hours. To the mixture is added saturated aq. NaHCO₃, and then extracted with ethyl acetate. The organic layer is washed with brine, dried over MgSO₄, filtered, and concentrated under reduced pressure. The obtained residue is purified by silica gel column chromatography (eluent: n-hexane / ethyl acetate) to give 2-ethyl-6-methoxymethyl-4-oxo-piperidine-1-carboxylic acid tert-butyl ester (30 mg, 11 % in 3 steps); ESI-MS m/z: 216 [M-*^{t}*Bu+2]⁺, Retention time 1.95 min (condition A).

To a mixture of 2-ethyl-6-methoxymethyl-4-oxo-piperidine-1-carboxylic acid tert-butyl ester (0.11 mmol, 30 mg), 3,5-bis(trifluoromethyl)benzylamine (0.16 mmol, 38.9 mg) and acetic acid (0.16 mmol, 8.7 uL) in dichloroethane (0.3 mL) is added NaBH(OAc)₃ (0.32 mmol, 67.8 mg) at room temperature. The mixture is stirred for 13 hours and then quenched with aq. 1 M NaOH. The mixture is extracted with dichloromethane, and the organic layer is concentrated under reduced pressure. The obtained residue is purified by silica gel column chromatography (eluent: n-hexane / ethyl acetate) to give 4-(3,5-bis-trifluoromethyl-benzylamino)-2-ethyl-6-methoxymethyl-piperidine-1-carboxylic acid tert-butyl ester (2,4,6-*cis* isomer) (17.5 mg, 32%); ESI-MS m/z: 499 [M+1]⁺, Retention time 1.95 min (condition A).

### 23). Synthesis of 2-benzyl-6-ethyl-piperidin-4-yl)-(3,5-bis-trifluoromethyl-benzyl)-(5-bromo-pyrimidin-2-yl)-amine hydrochloric acid salt (2,4,6-cis isomer)

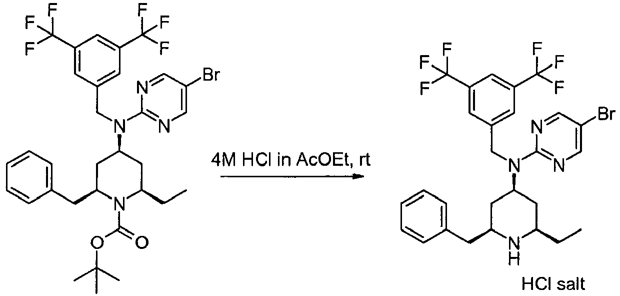

2-Benzyl-4-[(3,5-bis-trifluoromethyl-benzyl)-(5-bromo-pyrimidin-2-yl)-amino]-6-ethyl-piperidine-1-carboxylic acid tert-butyl ester (2,4,6-cis isomer) (1.29 mmol, 900 mg) is dissolved in a solution of 4M HCl in ethyl acetate. The mixture is stirred for 4 hours at room temperature, then concentrated under reduced pressure. To the obtained residue is added diethyl ether and filtered to give 2-benzyl-6-ethyl-piperidin-4-yl)-(3,5-bis-trifluoromethyl-benzyl)-(5-bromo-pyrimidin-2-yl)-amine hydrochloric acid salt (2,4,6-cis isomer) (771 mg); ESI-MS m/z: 601 [M+H]⁺, Retention time 2.14 min (condition A).

The following material is prepared following the above procedure.

| Name | Structure | ESI-MS m/z [M+1]⁺ | Retention time |
|---|---|---|---|
| 2-Benzyl-6-ethyl-piperidin-4-yl)-(3,5-bis-trifluoromethyl-benzyl)-(5-morpholin-4-yl-pyrimidin-2-yl)-amine | 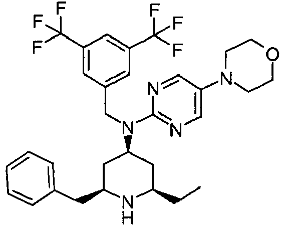 | 608 | 1.99 min (condition A) |

### 24). Synthesis of (2R,4R,6S)-4-(3,5-bis-trifluoromethyl-benzylamino)-2,6-dipropyl-piperidine-1-carboxylic acid tert-butyl ester

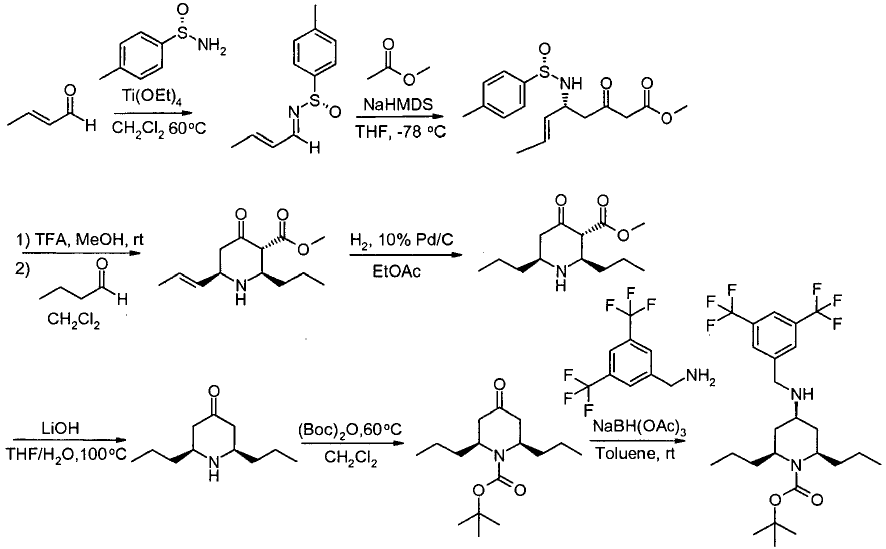

To a mixture of (S)-(+)-p-toluenesulfinamide (6.0 mmol, 931 mg) and (E)-but-2-enal (7.2 mmol, 597 uL) in dichloromethane (80 mL) is added Ti(OEt)₄ (30 mmol, 6.3 mL) at room temperature. The mixture is stirred for 4.5 hours at 50 °C, and then cooled to room temperature. The mixture is poured into water and filtered. The filter cake is rinsed with dichloromethane. The filtrate is washed with water and brine, dried over MgSO₄, filtered, and concentrated under reduced pressure to give 4-methyl-benzenesulfinic acid [(E)-but-2-en-(E)-ylidene]amide (1.16 g, 94%), which can be used without further purification; ESI-MS m/z: 208.00 [M+1]⁺, Retention time 2.17 min (condition A).

To a solution of 1M NaHMDS (28 mmol, 28 mL) in THF (20 mL) is added methyl acetate (28 mmol, 2.23 mL) at -78 °C. The mixture is stirred for 1 hour, and 4-methyl-benzenesulfinic acid [(E)-but-2-en-(E)-ylidene]amide (5.6 mmol, 1.16 g) is added at -78 °C. The temperature is raised slowly while stirring. After ca. 5 hours, saturated aq. NH₄Cl is added at -18 °C. The mixture is extracted with ethyl acetate, and the organic layer is washed with water and brine, dried over MgSO₄, filtered, and concentrated under reduced pressure. The obtained residue is purified by silica gel column chromatography (eluent: hexane / ethyl acetate = 1 / 1) to give (E)-(R)-3-oxo-5-(toluene-4-sulfinylamino)-oct-6-enoic acid methyl ester (0.92 g, 51%); ESI-MS m/z: 323.93 [M+1]⁺, Retention time 2.07 min (condition A).

To a solution of (E)-(R)-3-oxo-5-(toluene-4-sulfinylamino)-oct-6-enoic acid methyl ester (1.3 mmol, 420 mg) in methanol (13 mL) is added TFA (0.48 mmol, 6.5 mL) at room temperature and stirred for 30 minutes. The mixture is concentrated under reduced pressure, and the obtained residue is purified by silica gel column chromatography (eluent:hexane / ethyl acetate 4/1 then methanol). The obtained material is dissolved in dichloromethane (25 mL), and butyraldehyde (13.3 mmol, 1.2 mL) is added. The mixture is stirred for 1 hour at room temperature, diluted with ethyl acetate, then added saturated aq. NaHCO₃. The organic layer is washed with brine, dried over MgSO₄, filtered, and concentrated under reduced pressure to give (2R,3R,6R)-4-oxo-6-((E)-propenyl)-2-propyl-piperidine-3-carboxylic acid methyl ester (280 mg, 80%); ESI-MS m/z: 240.08 [M+1]⁺, Retention time 1.81 min (condition A).

A mixture of (2R,3R,6R)-4-oxo-6-((E)-propenyl)-2-propyl-piperidine-3-carboxylic acid methyl ester (137 mmol, 327 mg) and 10% Pd/C (145 mg) in ethyl acetate (13 mL) is stirred under hydrogen for 1.5 hours at room temperature. The mixture is filtered and concentrated under reduced pressure to give (2R,3R,6S)-4-oxo-2,6-dipropyl-piperidine-3-carboxylic acid methyl ester (361 mg); ESI-MS m/z: 242.08 [M+1]⁺, Retention time 1.55 min (condition A).

To a solution of (2R,3R,6S)-4-oxo-2,6-dipropyl-piperidine-3-carboxylic acid methyl ester (0.62 mmol, 150 mg) in THF (4 mL) is added a solution of LiOH monohydrate (6.2 mmol, 236 mg) in H₂O (20 mL) at room temperature. The mixture is stirred at 100 °C for 2 hours and cooled to room temperature. To the mixture is added saturated aq. NH₄Cl and extracted with dichloromethane. The organic layer is dried over MgSO₄, filtered, and concentrated under reduced pressure to give (2R,6S)-2,6-dipropyl-piperidin-4-one (93 mg), which is used for next step without further purification.

A mixture of (2R,6S)-2,6-dipropyl-piperidin-4-one (0.51 mmol, 93 mg) and BOC anhydride (166 mg) in dichloromethane (2 mL) is stirred at 60 °C for 1.5 hours and cooled to room temperature. To the mixture is added water and extracted with dichloromethane. The organic layer is dried over MgSO₄, filtered, and concentrated under reduced pressure. The obtained residue is purified by silica gel column chromatography (eluent: hexane / ethyl acetate) to give (2R,6S)-4-oxo-2,6-dipropyl-piperidine-1-carboxylic acid tert-butyl ester (46 mg); ESI-MS m/z: 228.10 [M+1]⁺, Retention time 2.25 min (condition A).

A mixture of (2R,6S)4-oxo-2,6-dipropyl-piperidine-1-carboxylic acid tert-butyl ester (0.091 mmol, 26 mg), 3,5-bis(trifluoromethyl)benzylamine (0.11 mmol, 33.5 mg), NaBH(OAc)₃ (0.184 mmol, 39 mg), and acetic acid (0.11 mmol, 6 uL) in dichloromethane (0.4 mL) is stirred at room temperature for 16 hours under nitrogen. To the mixture is added aq. 0.5M NaOH solution and extracted with dichloromethane. The organic layer is dried over Na₂SO₄, filtered, and concentrated under reduced pressure. The obtained residue is purified by silica gel column chromatography (eluent: hexane / ethyl acetate) to give (2R,4R,6S)-4-(3,5-bis-trifluoromethyl-benzylamino)-2,6-dipropyl-piperidine-1-carboxylic acid tert-butyl ester (16.2 mg, 34%); ESI-MS m/z: 511.01 [M+1]⁺, Retention time 2.21 min (condition A).

### 25). Synthesis of 4-[(3,5-bis-trifluoromethyl-benzyl)-methoxycarbonyl-amino]-2-carboxymethyl-6-ethyl-piperidine-1-carboxylic acid tert-butyl ester (racemate).

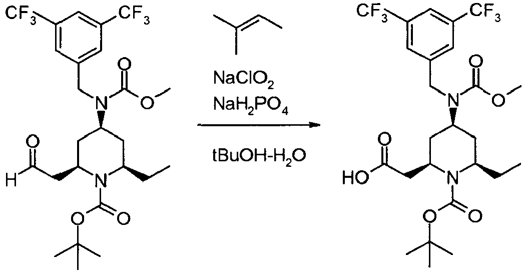

To a solution of 4-[(3,5-bis-trifluoromethyl-benzyl)-methoxycarbonyl-amino]-2-ethyl-6-(2-oxo-ethyl)-piperidine-1-carboxylic acid tert-butyl ester (104 mg, 0.187 mmol) in t-BuOH (3.5 ml) and 2-methylpropene (0.5 ml) at 0 °C is added a chilled solution of NaClO₂ (34 mg, 0.375mmol) and NaH₂PO₄ (0.112 mg, 0.935 mmol) in water (3.5 ml). The mixture is stirred at 0 °C for 5 min and quenched with a saturated solution of Na₂S₂O₄. The mixture is extracted twice with ethyl acetate and the combined organic layer is washed with brine, dried over magnesium sulfate, filtered, and concentrated in vacuo to give 4-[(3,5-bis-trifluoromethyl-benzyl)-methoxycarbonyl-amino]-2-carboxymethyl-6-ethyl-piperidine-1-carboxylic acid tert-butyl ester as a colorless foam (119 mg). ESI-MS m/z: 571 [M+1]⁺, Retention time 2.42 min (condition A).

### 26). Synthesis of 4-[(3,5-bis-trifluoromethyl-benzyl)-methoxycarbonyl-amino]-2-ethyl-6-(2-oxo-but-3-ynyl)-piperidine-1-carboxylic acid tert-butyl ester (racemic).

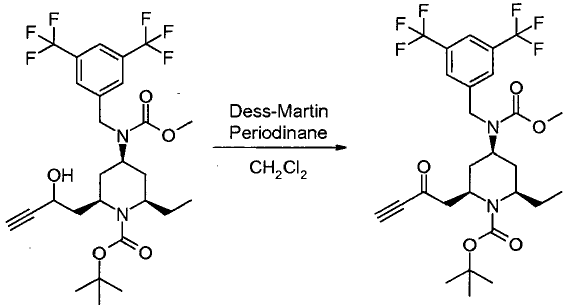

To a solution of 4-[(3,5-bis-trifluoromethyl-benzyl)-methoxycarbonyl-amino]-2-ethyl-6-(2-hydroxy-but-3-ynyl)-piperidine-1-carboxylic acid tert-butyl ester (145 mg, 0.250 mmol) in methylene chloride (3 ml) at 0 °C is added Dess-Martin periodinane (265 mg, 0.624 mmol) and stirred at rt for 1 h. An additional Dess-Martin periodinane (86 mg, 0.202 mmol) is added and the mixture is stirred for further 1 h. To the mixture is added water and sat. aq. Na₂S₂O₄. The mixture is extracted twice with diethyl ether, washed with brine and combined organic layer is dried over magnesium sulfate and purification by silica gel column chromatography to give 4-[(3,5-bis-trifluoromethyl-benzyl)-methoxycarbonyl-amino]-2-ethyl-6-(2-oxo-but-3-ynyl)-piperidine-1-carboxylic acid tert-butyl ester as a colorless oil (194 mg). ESI-MS m/z: 579 [M+1]⁺, Retention time 1.57 min (condition A).

The following materials are prepared following the above procedure.

| Name | Structure |
|---|---|
| 4-[(3,5-Bis-trifluoromethyl-benzyl)-methoxycarbonyl-amino]-2-ethyl-6-(2-oxo-pent-3-ynyl)-piperidine-1-carboxylic acid tert-butyl ester | 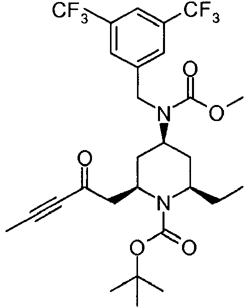 |
| 4-[(3-Chloro-5-trifluoromethyl-benzyl)-methoxycarbonyl-amino]-2-ethyl-6-(2-oxo-but-3-ynyl)-piperidine-1-carboxylic acid isopropyl ester | 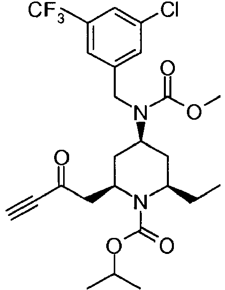 |

### 27). Synthesis of 4-[(3,5-bis-trifluoromethyl-benzyl)-methoxycarbonyl-amino]-2-ethyl-6-(2-oxo-ethyl)-piperidine-1-carboxylic acid tert-butyl ester (racemic).

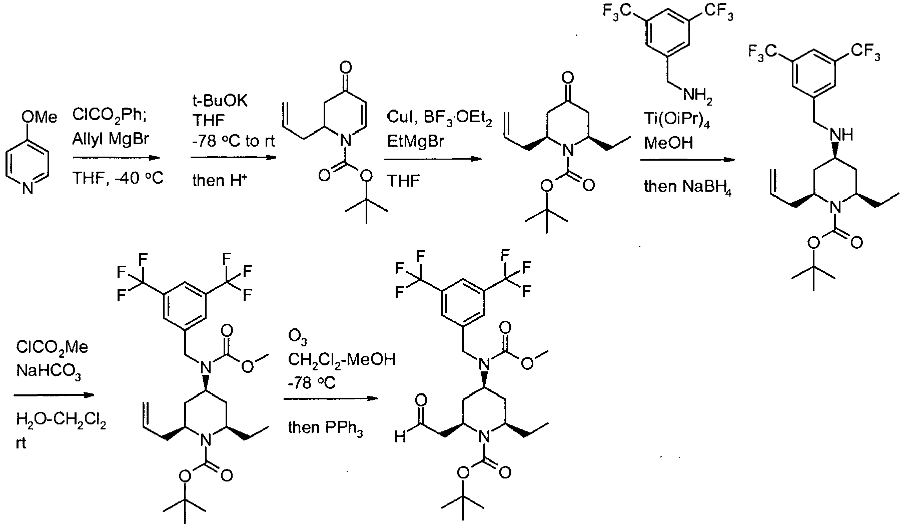

To a solution of 2-allyl-4-(3,5-bis-trifluoromethyl-benzylamino)-6-ethyl-piperidine-1-carboxylic acid tert-butyl ester (1.1 g, 2.22 mmol) in methylene chloride (5 ml) is added sat. aq. NaHCO₃ (10 ml), followed by methyl chloroformate (0.190 ml, 2.44 mmol) and stirred at rt for 1 h. The methylene chloride layer is extracted with phase separator and solvent is removed in vacuo to give 2-allyl-4-[(3,5-bis-trifluoromethyl-benzyl)-methoxycarbonyl-amino]-6-ethyl-piperidine-1-carboxylic acid tert-butyl ester as a colorless oil (989 mg, 81% yield). ESI-MS m/z: 553 [M+1]⁺, Retention time 2.65 min (condition A).

A solution of 2-allyl-4-[(3,5-bis-trifluoromethyl-benzyl)-methoxycarbonyl-amino]-6-ethyl-piperidine-1-carboxylic acid tert-butyl ester (677 mg, 1.22 mmol) in methylene chloride (8 ml) and methanol (2 ml) at -78 °C is treated with ozone gas (bubbling) for 5 min. After purging the solution with oxygen, triphenylphosphine (962 mg, 3.67 mmol) is added. The solution is let warm to rt, concentrated in vacuo and purified by silica gel column chromatography to give 4-[(3,5-bis-trifluoromethyl-benzyl)-methoxycarbonyl-amino]-2-ethyl-6-(2-oxo-ethyl)-piperidine-1-carboxylic acid tert-butyl ester as a colorless oil (642 mg, 95% yield). ESI-MS m/z: 499 [M-tBu+1]⁺, Retention time 2.48 min (condition A).

### 28). Synthesis of 4-[(3-chloro-5-trifluoromethyl-benzyl)-methoxycarbonyl-amino]-2-ethyl-6-(2-oxo-ethyl)-piperidine-1-carboxylic acid isopropyl ester (racemic).

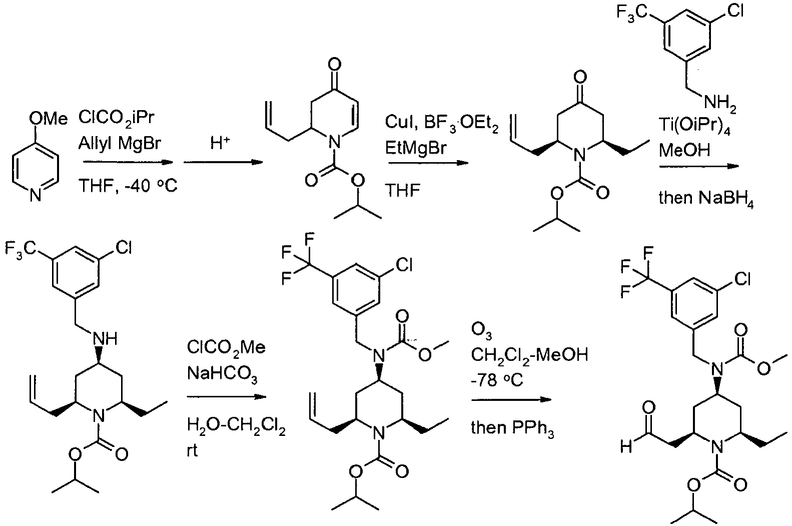

To a solution of 2-allyl-4-(3-chloro-5-trifluoromethyl-benzylamino)-6-ethyl-piperidine-1-carboxylic acid isopropyl ester (0.846 mg, 1.89 mmol) in methylene chloride (5 ml) is added sat. aq. NaHCO₃ (10 ml), followed by methyl chloroformate (0.161 ml, 2.08 mmol) and stirred at rt for 15 min. The methylene chloride layer is extracted with phase separator and solvent is removed in vacuo to give as a colorless oil (954 mg, quant). ESI-MS m/z: 505 [M+1]⁺, Retention time 2.47 min (condition A).

A solution of 2-allyl-4-[(3-chloro-5-trifluoromethyl-benzyl)-methoxycarbonyl-amino]-6-ethyl-piperidine-1-carboxylic acid isopropyl ester (954 mg, 1.89 mmol) in methylene chloride (8 ml) and methanol (2 ml) at -78 °C is treated with ozone gas (bubbling) for 7 min. After purging the solution with oxygen, triphenylphosphine (1.48 g, 5.57 mmol) is added. The solution is let warm to rt, concentrated in vacuo and purified by silica gel column chromatography to give a colorless oil (834 mg, 87% yield). ESI-MS m/z: 506 [M+1]⁺, Retention time 2.28 min (condition A).

### 29). Synthesis of 4-(3,5-bistrifluoromethyl-benzylamino)-2,6- trans-diisopropyl-piperidine-1-carboxylic acid tert-butyl ester (racemic)

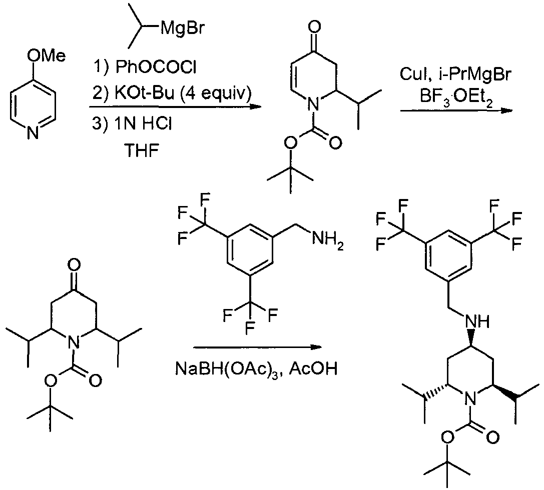

To a solution of 4-methoxypyridine (10 mmol, 1.02 g) in THF (39 mL) at -40 °C is added slowly 0.76 M THF solution of isopropyl magnesium bromide (11 mmol, 14.5 ml) under nitrogen. After stirred for 20 minutes at -40 °C, phenyl chloroformate (10.5 mmol, 1.33 mL) is added and resulted in slightly gray suspension. The cool bath is removed and after stirring for 2 hours, the mixture is cooled to -40 °C, *t*BuOK (40 mmol, 4.5 g) is added in one portion which gives a yellow suspension. After stirring for 10 min at -40 °C, cool bath is removed and the mixture is allowed to stir vigorously for another 4 hrs. The reaction is quenched with 31 ml of H₂O and extracted with Et₂O. The combined organic layer is washed once with aq.1 N NaOH and then three times with aq.1 N HCl. After washing with brine, the organic layer is dried over MgSO₄, filtrated, and concentrated under reduced pressure to give 2-Isopropyl-4-oxo-3,4-dihydro-2H-pyridine-1-carboxylic acid tert-butyl ester (957 mg, 40%) which is used for next step without further purification. ESI-MS m/z: 240 [M+1]⁺, Retention time 2.27 min (condition A).

To a suspension of Cul (8 mmol, 1.52 g) in THF (40 mL) is added slowly a 0.76 M THF solution of isopropyl magnesium bromide (8 mmol, 10.5 ml) under nitrogen at -78 °C. After stirring for 10 min, BF₃·Et₂O (4.8 mmol, 0.48 ml) is added dropwise at -70 °C and the gray suspension stirred vigorously for 1 h at the same temperature. To the suspension is added THF solution (5 mL) of 2-isopropyl-4-oxo-3,4-dihydro-2H-pyridine-1-carboxylic acid tert-butyl ester (4 mmol, 957 mg) at -78 °C. The mixture is allowed to raise to room temperature overnight and quenched with ice, saturated aqueous NH₄Cl and extracted with EtOAc. The combined organic layer is washed with brine, dried over MgSO₄, filtered, and concentrated under reduced pressure. The obtained residue is purified by silica gel column chromatography (eluent: n-hexane / ethyl acetate) to give 2,6-*trans* diisopropyl-4-oxo-piperidine-1-carboxylic acid tert-butyl ester (164 mg, 20%); ESI-MS m/z: 228.04 [M-*^{t}*Bu+2]⁺, Retention time 2.44 min (condition A).

To a solution of 2,6-trans -diisopropyl-4-oxo-piperidine-1-carboxylic acid tert-butyl ester (0.15 mmol, 42 mg) and 3,5-bis(trifluoromethyl)benzylamine (0.18 mmol, 55 mg) in 1,2-dichloroethane (1 ml) is added acetic acid (0.3 mmol, 17 uM) and NaBH(OAc)₃ (0.3 mmol, 64 mg) at room temperature. The mixture is allowed to stir overnight and then diluted with H₂O, basified with aq.1N NaOH, neutralized to PH 7 with Sat. NH₄Cl. The mixture is extracted with CH₂Cl₂ and the combined organic layer is concentrated under reduced pressure. The residue is purified by preparative TLC (eluent: n-hexane / ethyl acetate) to give a racemic mixture of 4-(3,5-bistrifluoromethyl-benzylamino)-2,6-*trans*-diisopropyl-piperidine-1-carboxylic acid tert-butyl ester (2,6-*trans* isomer: 18 mg, 24%); ESI-MS m/z: 551.01 [M+1]⁺, Retention time 2.33 min (condition A).

### 30). Synthesis of 4-[(5-bromo-pyrimidin-2-yl)-(3-chloro-5-trifluoromethyl-benzyl)-amino]-2,6-trans-diisopropyl-piperidine-1-carboxylic acid isopropyl ester (racemic).

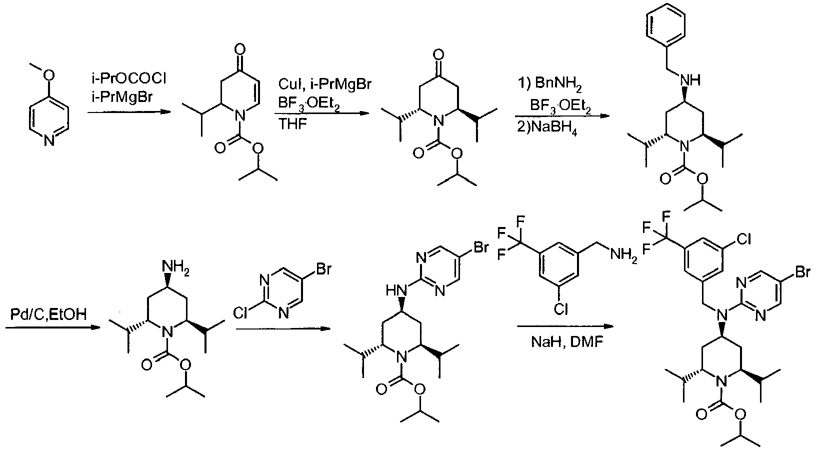

To a solution of 4-methoxypyridine (70 mmol, 7 ml) in THF (300 mL) at -40 °C is added slowly 0.76 M THF solution of isopropyl magnesium bromide (76 mmol, 100 ml) via cannula under nitrogen. After stirred for 20 minutes at -50 °C, isopropyl chloroformate (73 mmol, 8.36 mL) is added and resulted in slightly gray suspension. The mixture is allowed to warm to room temperature, and after stirring for additional 2 hrs, the reaction is quenched with 90 ml H₂O and extracted with Et₂O. The combined organic layer is washed three times with 50 ml of aq.1 N HCl. After washing with brine, the organic layer is dried over MgSO₄, filtrated, and concentrated under reduced pressure to give 2-isopropyl-4-oxo-3,4-dihydro-2H-pyridine-1-carboxylic acid isopropyl ester (15 g, 95%) which is used for next step without further purification. ESI-MS m/z: 226.04 [M+1]⁺, Retention time 1.84 min (condition A).

To a suspension of Cul (70 mmol, 13.3 g) in THF (250 mL) is added slowly 0.76 M THF solution of isopropyl magnesium bromide (70 mmol, 92 ml) under nitrogen at -78 °C. After stirring for 10 min, BF_{3·}Et₂O (42 mmol, 4 ml) is added dropwise at -70 °C, and the gray suspension is stirred vigorously for 1 h at the same temperature. To the suspension is added THF solution (15 mL) of 2-isopropyl-4-oxo-3,4-dihydro-2H-pyridine-1-carboxylic acid isopropyl ester (35 mmol, 8.56 g) at -78 °C. The mixture is allowed to raise to room temperature overnight with vigorously stirring and quenched with ice, saturated aqueous NH₄Cl and extracted with EtOAc. The combined organic layer is washed with brine, dried over MgSO₄, filtered, and concentrated under reduced pressure. The obtained residue is purified by silica gel column chromatography (eluent: n-hexane / ethyl acetate) to give 2,6-*trans*-diisopropyl-4-oxo-piperidine-1-carboxylic acid isopropyl ester (1.75 g, 19%); ESI-MS m/z: 270.12 [M+1]⁺, Retention time 2.13 min (condition A).

To a mixture of 2,6-*trans*-diisopropyl-4-oxo-piperidine-1-carboxylic acid isopropyl ester (6.5 mmol, 1.75 g) in toluene (85 ml) is added benzyl amine (9.75 mmol, 1.07 ml) and boron trifluoride ethyl etherate (0.065 mmol, 8 ul) at rt, and the resulted mixture is refluxed for 30 min at 137 °C. After cooled to rt, toluene is removed under reduced pressure and the crude imine is used without further purification. To the crude imide in 85 ml MeOH is added sodium borohydride (4.7 mmol, 173 mg) under N₂. After warming to rt, MeOH is removed under reduced pressure and sat. aqueous NH₄Cl is added. Aqueous layer is extracted with dichloromethane. The organic layer is washed with H₂O, brine, dried over Na₂SO₄, filtrated and evaporated. The obtained residue is purified by silica gel column chromatography (eluent: *n*-hexane / ethyl acetate) to give 4-benzylamino-2,6-*trans*-diisopropyl-piperidine-1-carboxylic acid isopropyl ester (2.09 g, 89%). ESI-MS m/z: 361.10 [M+1]⁺, Retention time 1.88 min (condition A).

To a mixture of 4-benzylamino-2,6-*trans*-diisopropyl-piperidine-1-carboxylic acid isopropyl ester (2.77 mmol, 1 g) in ethanol (80 ml) is added Pd/C (100 mg) under N₂. Then the flask is replaced with hydrogen gas (1 atm) and the reaction mixture is stirred for 7 hrs at 60 °C. After cooled to rt, filtration and evaporation gives 4-amino-2,6-*trans*-diisopropyl-piperidine-1-carboxylic acid isopropyl ester (0.75 g, 100%) which is used without further purificaiton. ESI-MS m/z: 271.12 [M+1]⁺, Retention time 1.68 min (condition A).

To a mixture of 4-amino-2,6-*trans*-diisopropyl-piperidine-1-carboxylic acid isopropyl ester (2.6 mmol, 716 mg) in DMF (8 ml) is added 2-chloro-5-bromo-pyrimidine (3.9 mmol, 754 mg) and diisopropyl ethyl amine (5.2 mmol, 0.91 ml) under N₂. The reaction mixture is stirred for 4 hrs at 120 °C. After cooling to rt, brine and H₂O are added and the aqueous layer is extracted with ethyl acetate. The organic layer is washed with sat. aqueous ammonium chloride and then dried over MgSO₄. The obtained residue is purified by silica gel column chromatography (eluent: n-hexane / ethyl acetate) to give 4-(5-bromo-pyrimidin-2-ylamino)-2,6- *trans*-diisopropyl-piperidine-1-carboxylic acid isopropyl ester (0.66 g, 60%). ESI-MS m/z: 426.99 [M+1]⁺, Retention time 2.45 min (condition A).

To a mixture of 4-(5-bromo-pyrimidin-2-ylamino)-2,6-*trans*-diisopropyl-piperidine-1-carboxylic acid isopropyl ester (1.54 mmol, 660 mg) in DMF is added sodium hydride (3.08 mmol, 123 mg) under N₂, then an ice bath is removed. After stirring for 20 min at rt, the reaction mixture is cooled down to 4 °C and 3-chloro-5-trifluoromethyl-benzyl bromide (2.31 mmol, 375 ul) is added. After removing an ice bath, reaction mixture is stirred for 2 hrs at rt. H₂O is added and the aqueous layer is extracted with ethyl acetate. The combined organic layer is dried over Na₂SO₄, filtrated and evaporated under reduced pressure. The obtained residue is purified by silica gel column chromatography (eluent: *n*-hexane / ethyl acetate) to give 4-[(5-bromo-pyrimidin-2-yl)-(3-chloro-5-trifluoromethyl-benzyl)-amino]-2,6-*trans-*diisopropyl-piperidine-1-carboxylic acid isopropyl ester (435 mg, 46%). ESI-MS m/z: 620.87 [M+1]⁺, Retention time 2.85 min (condition A).

### 31). Synthesis of 2-benzyl-4-(4-ethoxycarbonyl-oxazol-2-ylamino)-6-ethyl-piperidine-1-carboxylic acid tert-butyl ester

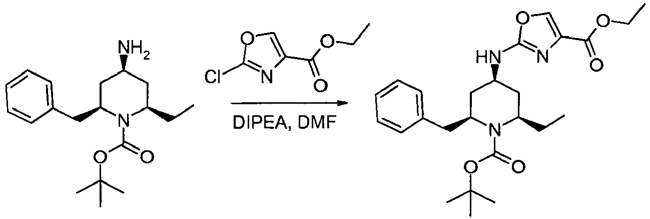

A solution of 4-amino-2-benzyl-6-ethyl-piperidine-1-carboxylic acid tert-butyl ester (1 mmol, 318 mg), 2-chloro-oxazole-4-carboxylic acid ethyl ester (3 mmol, 527 mg) and *N*,*N*-diisopropylethylamine (2 mmol, 348 uL) in DMF (4 ml) is allowed to warm to 110 °C and stirred for 24 hours. The mixture is cooled to room temperature, then added with water. The mixture is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-(4-ethoxycarbonyl-oxazol-2-ylamino)-6-ethyl-piperidine-1-carboxylic acid tert-butyl ester (288 mg, 63%); ESI-MS m/z: 458 [M+1]⁺, Retention time 2.46 min (condition A).

The following material is prepared following the above procedure.

| Name | Structure |
|---|---|
| 4-(4-Ethoxycarbonyl-oxazol-2-ylamino)-2,6-diethyl-piperidine-1-carboxylic acid isopropyl ester | 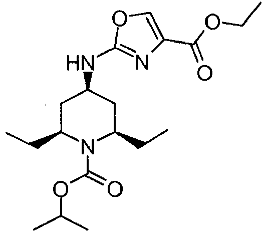 |

### 32). Synthesis of 4-[(3,5-bis-trifluoromethyl-benzyl)-cyano-amino]-2,6-diethyl-piperidine-1-carboxylic acid isopropyl ester

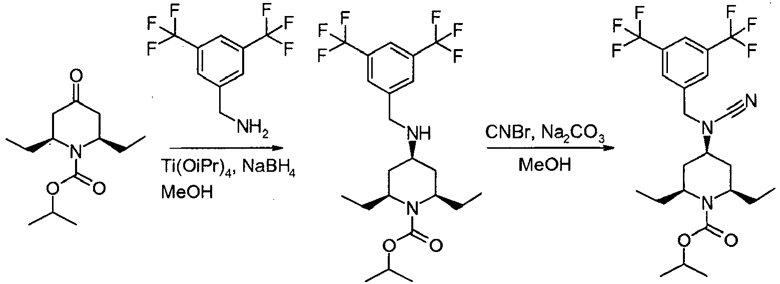

A solution of 2,6-diethyl-4-oxo-piperidine-1-carboxylic acid isopropyl ester (10 mmol, 2.41g), 3,5-bis(trifluoromethyl)benzylamine (15 mmol, 3.65g), titanium isopropoxide (12 mmol, 3.55 mL) in 20 mL of methanol is stirred at room temperature for 17 hours. The mixture is added NaBH₄ (15 mmol, 570 mg) portionwise at 0 °C and stir for 3 hours. The reaction is quenched by addition of water and stirred at room temperature for 1 hour. The suspension is filtered and washed with ethyl acetate. The solution is concentrated under reduced pressure. The obtained residue is purified by silica gel column chromatography (eluent: hexane / EtOAc) to give 4-(3,5-Bis-trifluoromethyl-benzylamino)-2,6-diethyl-piperidine-1-carboxylic acid isopropyl ester (3.02 g, 64%)

To a mixture of 4-(3,5-bis-trifluoromethyl-benzylamino)-2,6-diethyl-piperidine-1-carboxylic acid isopropyl ester (0.6 mmol, 280 mg) and sodium carbonate (1.2 mmol, 127 mg) in methanol is added cyanogen bromide (0.9 mmol, 95 mg) at room temperature and stirred for 3 hours. The mixture is filtrated and the resultant solution is concentrated under reduced pressure. The obtained residue is purified by silica gel column chromatography (eluent: hexane / EtOAc) to give 4-[(3,5-bis-trifluoromethyl-benzyl)-cyano-amino]-2,6-diethyl-piperidine-1-carboxylic acid isopropyl ester (265 mg, 90%); ESI-MS m/z: 494 [M+1]⁺, Retention time 2.52 min (condition A).

### 33). Synthesis of 4-[(3,5-bis-trifluoromethyl-benzoyl)-(1-methyl-1H-[1,2,4]triazol-3-yl)-amino]-2,6-diethyl-piperidine-1-carboxylic acid isopropyl ester

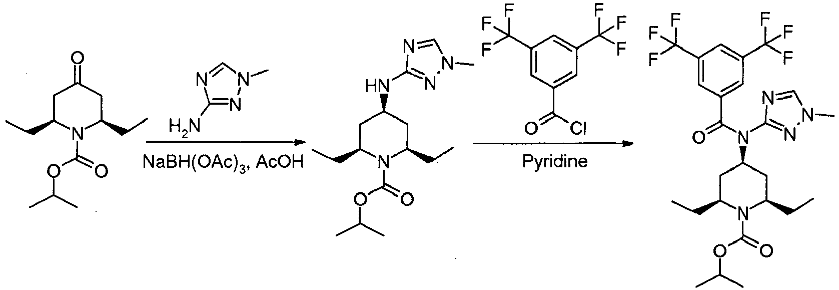

To a solution of 2,6-diethyl-4-oxo-piperidine-1-carboxylic acid isopropyl ester (1 mmol, 240 mg), 1-methyl-1H-[1,2,4]triazol-3-ylamine (2 mmol, 200 mg) and acetic acid (2 mmol, 114 uL) in dichloroethane (2 mL) is added sodium triacetoxyborohydride (2 mmol, 424 mg) at room temperature and stirred for 15 hours. The reaction is quenched by addition of saturated aq. sodium hydrogen carbonate. The mixture is extracted with dichloromethane and the combined organic layer is concentrated under reduced pressure. The obtained residue is purified by silica gel column chromatography (eluent: DCM / MeOH) to give 2,6-diethyl-4-(1-methyl-1H-[1,2,4]triazol-3-ylamino)-piperidine-1-carboxylic acid isopropyl ester (168 mg, 52%); ESI-MS m/z: 324 [M+1]⁺, Retention time 2.06 min (condition A).

To a solution of 2,6-diethyl-4-(1-methyl-1H-[1,2,4]triazol-3-ylamino)-piperidine-1-carboxylic acid isopropyl ester (0.28 mmol, 90 mg) in pyridine (1 mL) is added 3,5-bis-trifluoromethyl-benzoyl chloride (0.42 mmol, 77 uL) at 0 °C and stirred for 1 hour at room temperature. The mixture is added 3,5-bis-trifluoromethyl-benzoyl chloride (0.42 mmol, 77 uL) at 0 °C and stirred for 17 hours at room temperature. The mixture is added saturated aq. NH₄Cl and then extracted with DCM. The combined organic solution is concentrated under reduced pressure. The obtained residue is purified by silica gel column chromatography (eluent: hexane / EtOAc) to give 4-[(3,5-bis-trifluoromethyl-benzoyl)-(1-methyl-1H-[1,2,4]triazol-3-yl)-amino]-2,6-diethyl-piperidine-1-carboxylic acid isopropyl ester (160 mg, quant); ESI-MS m/z: 564 [M+1]⁺, Retention time 2.48 min (condition A).

The following material is prepared following the above procedure.

| Name | Structure | ESI-MS m/z [M+1]⁺ | Retention time (min) |
|---|---|---|---|
| 4-[(3,5-Bis-trifluoromethyl-benzoyl)-(2-methyl-2H-tetrazol-5-yl)-amino]-2,6-diethyl-piperidine-1-carboxylic acid isopropyl ester | 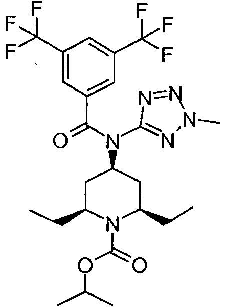 | 565 | 2.59 (condition A) |
| 4-[(3,5-Bis-trifluoromethyl-benzoyl)-(5-methyl-isoxazol-3-yl)-amino]-2,6-diethyl-piperidine-1-carboxylic acid isopropyl ester | 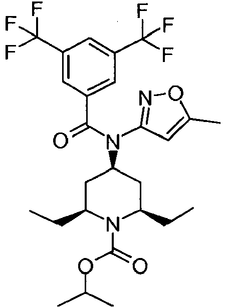 | 564 | 2.59 (condition A) |

### 34). Synthesis of 2-chloro-5-(4,4,5,5-tetramethyl-[1,3,2]dioxaborolan-2-yl)-pyrimidine

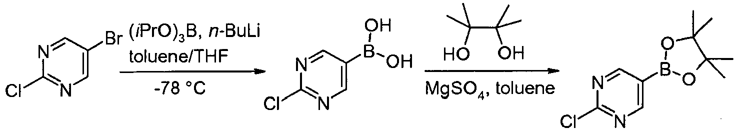

To a solution of 5-bromo-2-chloro-pyrimidine (10 mmol, 1.93 g) and triisopropyl' borate (12 mmol, 2.8 mL) in toluene (16 ml) and THF (4 mL) is added n-butyl lithium in hexane (1.58 M, 12 mmol, 7.6 mL) dropwise at -78 °C over 45 min and stirred at -78 °C for 1 hour. The mixture is warmed to -20 °C, then added aq. hydrogen chloride (1M, 20 mL). The mixture is warmed to room temperature. The precipitate is collected and washed with hexane to give a colorless powder (808 mg, 51%). A mixture of the powder (3.63 mmol, 575 mg), pinacol (3.81 mmol, 450 mg) and MgSO₄ (18.15 mmol, 2.2 g) in toluene (10 mL) is stirred at room temperature for 15 hour. The mixture is filtrated and the solution is concentrated under reduced pressure. The resultant solid is washed with water to give 2-chloro-5-(4,4,5,5-tetramethyl-[1 ,3,2]dioxaborolan-2-yl)-pyrimidine (875 mg, quant); ESI-MS m/z: 159 [M+1-pinacol]⁺, Retention time 1.75 min (condition A).

### 35). Synthesis of 4-[(5-benzyloxy-pyrimidin-2-yl)-(3-chloro-5-trifluoromethyl-benzyl)-amino]-2,6-diethyl-piperidine-1-carboxylic acid isopropyl ester

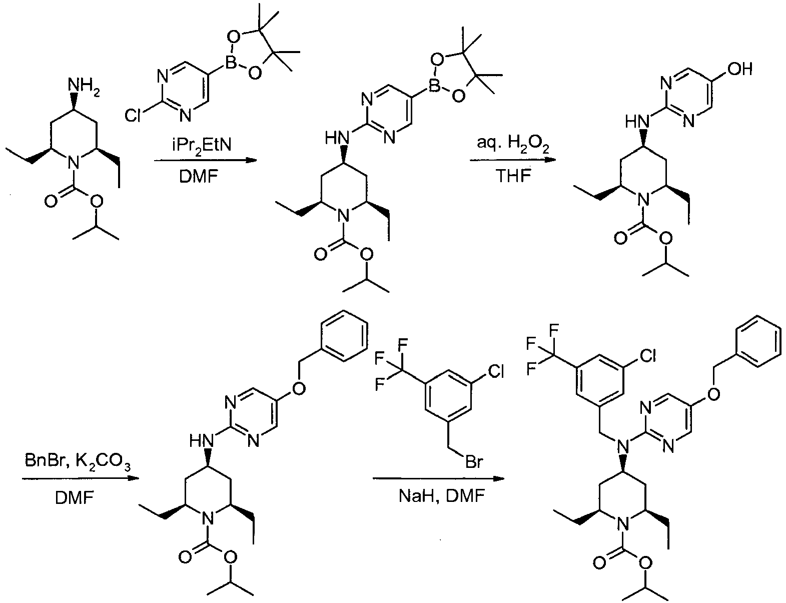

A solution of 4-amino-2,6-diethyl-piperidine-1-carboxylic acid isopropyl ester (3.3 mmol, 800 mg), 2-chloro-5-(4,4,5,5-tetramethyl-[1,3,2]dioxaborolan-2-yl)-pyrimidine (3.74 mmol, 900 mg) and *N*,*N*-diisopropylethylamine (6.6 mmol, 1.15 mL) in DMF (10 ml) is allowed to warm to 120 °C and stir for 3 hours. The mixture is cooled to room temperature, then added with water. The mixture is extracted with EtOAc. The combined organic layer is dried over Na₂SO₄, filtrated, and concentrated under reduced pressure.

The obtained residue is dissolved in THF (12 mL) and added aq. H₂O₂ (35%, 3.8 mL) at room temperature. The mixture is stirred at room temperature for 2 hours. The mixture is cooled down until 0 °C and quenched with saturated aq. sodium thiosulfate. The mixture is extracted with EtOAc. The combined organic layer is dried over Na₂SO₄, filtrated, and concentrated under reduced pressure. The obtained residue is purified by silica gel column chromatography (eluent: hexane / EtOAc) to give 2,6-diethyl-4-(5-hydroxy-pyrimidin-2-ylamino)-piperidine-1-carboxylic acid isopropyl ester (580 mg, 53%); ESI-MS m/z: 337 [M+1]⁺, Retention time 3.94 min (condition B).

To a mixture of 2,6-diethyl-4-(5-hydroxy-pyrimidin-2-ylamino)-piperidine-1-carboxylic acid isopropyl ester (1.72 mmol, 580 mg) and potassium carbonate (3.44 mmol, 475 mg) in DMF (6 mL) is added benzylamine (1.89 mmol, 225 uL) at room temperature and stirred for 13 hours. The mixture is added water, then extracted with EtOAc. The combined organic layer is dried over Na₂SO₄, filtrated, and concentrated under reduced pressure. The obtained residue is washed with n-hexane to give 4-(5-benzyloxy-pyrimidin-2-ylamino)-2,6-diethyl-piperidine-1-carboxylic acid isopropyl ester (635 mg, 87%); ESI-MS m/z: 427 [M+1]⁺, Retention time 2.33 min (condition A).

To a solution of 4-(5-benzyloxy-pyrimidin-2-ylamino)-2,6-diethyl-piperidine-1-carboxylic acid isopropyl ester (1.49 mmol, 635 mg) in DMF (15 mL) is added sodium hydride (60% oil suspension, 3 mmol, 120 mg) at 0 °C and stirred at room temperature for 20 min. To the mixture is added 1-bromomethyl-3-chloro-5-trifluoromethyl-benzene (2.25 mmol, 370 uL) at 0 °C and stirred at room temperature for 5 hours. The mixture is added water and extracted with EtOAc. The combined organic layer is dried over Na₂SO₄, filtrated, and concentrated under reduced pressure. The obtained residue is purified by silica gel column chromatography (eluent: hexane / EtOAc) to give 4-[(5-benzyloxy-pyrimidin-2-yl)-(3-chloro-5-trifluoromethyl-benzyl)-amino]-2,6-diethyl-piperidine-1-carboxylic acid isopropyl ester (705 mg, 76%); ESI-MS m/z: 619 [M+1]⁺, Retention time 2.78 min (condition A).

The following material is prepared following the above procedure.

| Name | Product | ESI-MS m/z [M+1]⁺ | Retention time (min) | Starting Material |
|---|---|---|---|---|
| 2-Benzyl-4-[(5-benzyloxy-pyrimidin-2-yl)-(3,5-bis-trifluoromethylbenzyl)-amino]-6-ethyl-piperidine-1-carboxylic acid tert-butyl ester | 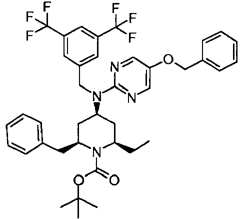 | 729 | 6.00 (condition B) | 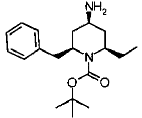 |
| | | | | 1-bromomethyl-3,5-bistrifluorome thyl-benzene instead of 1-bromomethyl-3-chloro-5-trifluoromethy l-benzene |
| 2-Benzyl-4-[(5-benzyloxy-pyrimidin-2-yl)-(3-chloro-5-trifluoromethyl-benzyl)-amino]-6-ethyl-piperidine-1-carboxylic acid tert-butyl ester | 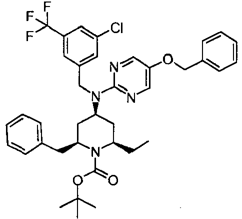 | 695 | 6.06 (condition B) | 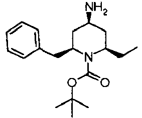 |

### 36). Synthesis of 2-benzyl-4-[(5-benzyloxy-pyrimidin-2-yl)-(3-chloro-5-trifluoromethyl-benzyl)-amino]-6-ethyl-piperidine-1-carboxylic acid isopropyl ester

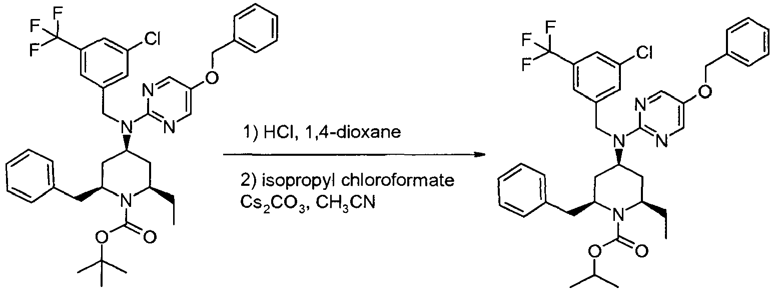

To a solution of 2-benzyl-4-[(5-benzyloxy-pyrimidin-2-yl)-(3-chloro-5-trifluoromethyl-benzyl)-amino]-6-ethyl-piperidine-1-carboxylic acid tert-butyl ester (0.974 mmol, 677 mg) in 1,4-dioxane (5 mL) is added hydrogen chloride solution (4N, 1,4-dioxane solution, 5 mL) at 0 °C and stirred for 1 hour. The mixture is concentrated under reduced pressure. To the residue is added aq. sodium hydroxide solution (1 N) and extracted with dichloromethane. The combined organic layer is concentrated under reduced pressure. To a mixture of the obtained residue and cesium carbonate (9.74 mmol, 3.2 g) in acetonitrile (5 mL) is added isopropyl chloroformate (9.74 mmol, 1.12 mL) at room temperature. The mixture is warmed to 60 °C and stirred for 3 hours. The mixture is cooled down to room temperature, and then added water. The mixture is extracted with EtOAc. The combined organic layer is dried over Na₂SO₄, filtrated, and concentrated under reduced pressure. The obtained residue is purified by silica gel column chromatography (eluent: hexane / EtOAc) to give 2-benzyl-4-[(5-benzyloxy-pyrimidin-2-yl)-(3-chloro-5-trifluoromethyl-benzyl)-amino]-6-ethyl-piperidine-1-carboxylic acid isopropyl ester (540 mg, 81%); ESI-MS m/z: 681 [M+1]⁺, Retention time 5.94 min (condition B).

### 37). Synthesis of 2-ethyl-6-(hydroxy-phenyl-methyl)-4-oxo-piperidine-1-carboxylic acid tert-butyl ester

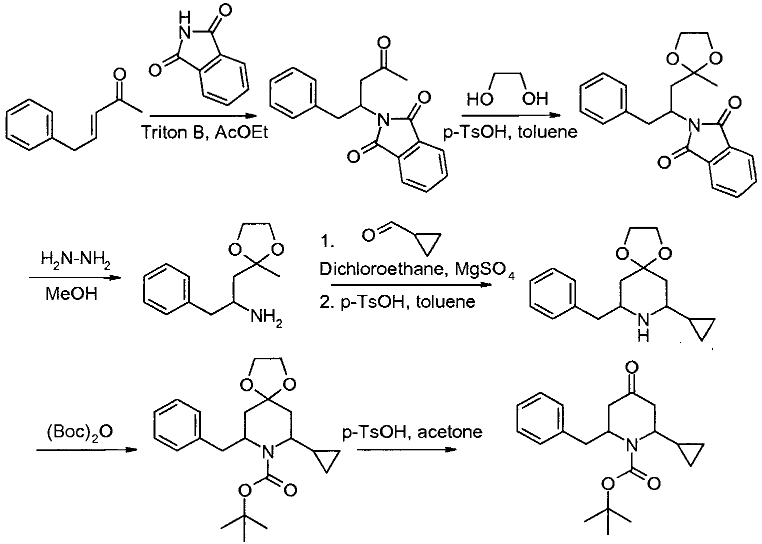

To a stirred solution of 5-phenyl-3-penten-2-one (4.8 g, 30 mmol) in ethyl acetate (50 mL) is added phthalimide (4.4 g, 30 mmol) and a 40% solution of Triton^{®} B in methanol (2 mL). The resulting solution is heated at reflux until complete disappearance of the phthalimide. The mixture is then allowed to cool to room temperature. Evaporation of the solvent followed by recrystallization from ethanol afforded 2-(1-benzyl-3-oxo-butyl)-isoindole-1,3-dione (4.0 g, 43%) as a white solid. ESI-MS m/z: 308.01 [M+1]⁺, Retention time 3.59 min (condition B).

In a round bottomed flask, fitted with a Dean-Stark apparatus, to a solution of 2-(1-benzyl-3-oxo-butyl)-isoindole-1,3-dione (4.0 g, 13.0mmol) in toluene (50 mL), freshly distilled ethylene glycol (0.87mL, 15.6 mmol) and p-TsOH (495mg, 2.6mmol) are added. The mixture is refluxed for 5 h, then cooled to rt and treated with saturated NaHCO₃ solution. The two layers are separated, and the aqueous phase is extracted several times with ethyl acetate. The combined organic layers are washed with brine solution and then dried on Na₂SO₄. After evaporation of the solvent, the residue is purified by silica gel chromatography (eluent : hexane/ethyl acetate, 5/1) and furnished 2-[1-benzyl-2-(2-methyl-[1,3]dioxolan-2-yl)-ethyl]-isoindole-1,3-dione (4.3 g) as a pale brown oil. Rf:0.68 (hexane/ethyl acetate, 5/1).

To a solution of 2-[1-benzyl-2-(2-methyl-[1,3]dioxolan-2-yl)-ethyl]-isoindole-1,3-dione (4.3g, 13 mmol) in methanol (14 mL) is added 98% hydrazine monohydrate (14mL, 260 mmol). The mixture is refluxed for 7 hours. After cooling the reaction mixture to rt, 5M KOH solution (20 mL) is added. The aqueous layer is extracted three times with dichloromethane (50mL). The combined organic layers are washed with saturated brine solution and dried on Na₂SO₄. After evaporation of the solvent under reduce pressure, 1-benzyl-2-(2-methyl-[1,3]dioxolan-2-yl)-ethylamine (2.2 g, 77%) is obtained as a yellow oil and is used further without purification. ESI-MS m/z: 222 [M+1]⁺, Retention time 2.27 min (condition B).

To a stirred solution of cyclopropanecarboxaldehyde (84 mg, 1.2 mmol) in dichloroethane (2 mL) is added MgSO₄ (1 g) followed by a solution of 1-benzyl-2-(2-methyl-[1,3]dioxolan-2-yl)-ethylamine (221 mg, 1.0 mmol) in CH₂Cl₂ (1 mL). The resulting solution is heated at reflux until complete disappearance of the amine (3-4 h), then cooled to rt and transferred via cannula to a solution of dry *p*-TsOH (380 mg, 2.0 mmol) in toluene (3 mL). The resulting mixture is heated at 70°C for 4 h. After cooled to rt, saturated aqueous NaHCO₃ (15mL) is added and the mixture is extracted with ethyl acetate (20 mL). The combined extracts are dried over Na₂SO₄ and evaporated under reduce pressure, 7-benzyl-9-cyclopropyl-1,4-dioxa-8-aza-spiro[4.5]decane (112 mg) is obtained as a brown oil and is used further without purification. ESI-MS m/z: 274.11 [M+1]⁺, Retention time 2.65 min (condition B).

A mixture of 7-benzyl-9-cyclopropyl-1,4-dioxa-8-aza-spiro[4.5]decane (100mg, 0.37mmol) and BOC anhydride (1g, 4.6mmol) is stirred at 70 °C for 9 hours and cooled to room temperature. To the mixture is added water and extracted with ethyl acetate. The organic layer is dried over Na₂SO₄, filtered, and concentrated under reduced pressure. 7-Benzyl-9-cyclopropyl-1,4-dioxa-8-aza-spiro[4.5]decane-8-carboxylic acid tert-butyl ester is obtained as a brown oil and is used further without purification. ESI-MS m/z: 374.02 [M+1]⁺, Retention time 5.02 min (condition B).

A mixture of 7-benzyl-9-cyclopropyl-1,4-dioxa-8-aza-spiro[4.5]decane-8-carboxylic acid tert-butyl ester (0.25 mmol, 95.1 mg) and p-toluenesulfonic acid monohydrate (0.25 mL, 43 mg) in acetone (9 mL) at 50 °C is stirred for 14 hours, and then saturated aq. NaHCO₃ is added. The mixture is extracted with ethyl acetate, and the organic layer is dried over Na₂SO₄, filtered, and concentrated under reduced pressure. The obtained residue is purified by silica gel column chromatography (eluent: n-hexane / ethyl acetate) to give 2-ethyl-6-(hydroxy-phenyl-methyl)-4-oxo-piperidine-1-carboxylic acid tert-butyl ester (36 mg, 43%). Rf:0.68 (hexane/ethyl acetate, 5/1).

### 38). Synthesis of 4-[(5-bromo-pyrimidin-2-yl)-(3-chloro-5-trifluoromethyl-benzyl)-amino]-2,6-diethyl-piperidine-1-carboxylic acid isopropyl ester

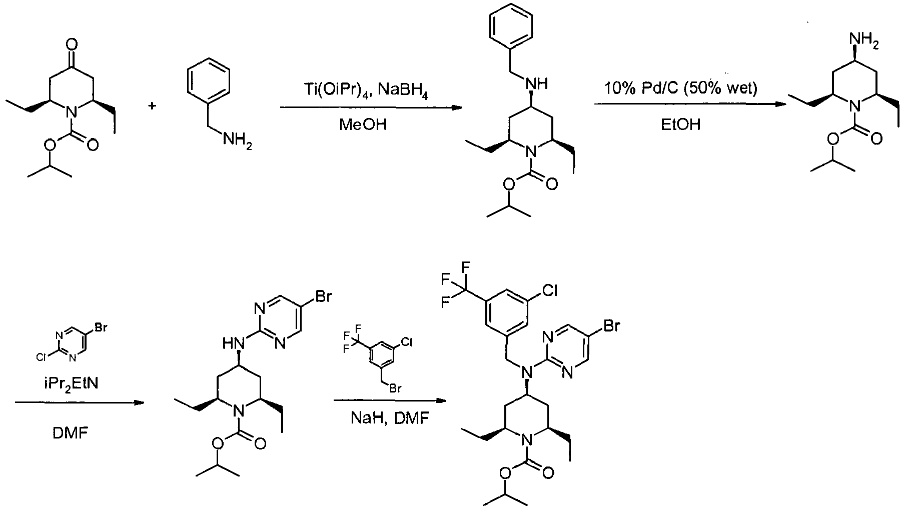

A mixture of 2,6-diethyl-4-oxo-piperidine-1-carboxylic acid isopropyl ester (41.5 mmol, 10 g), benzylamine (62.3 mmol, 6.8 mL), and titanium isopropoxide (50 mmol, 15 mL) in 100 mL of methanol is stirred at room temperature for 17 hours. To the mixture is added NaBH₄ (62.3 mmol, 2.35 g) portionwise at 0 °C and stirred for 3.5 hours. The reaction is quenched by addition of water and stirred at room temperature for 1.5 hour. The suspension is filtered and washed with ethyl acetate. The filtrate is concentrated under reduced pressure. The obtained residue is purified by silica gel column chromatography (eluent: hexane / EtOAc) to give 4-benzylamino-2,6-diethyl-piperidine-1-carboxylic acid isopropyl ester (8.94 g, 65%)

To a solution of 4-benzylamino-2,6-diethyl-piperidine-1-carboxylic acid isopropyl ester (27 mmol, 8.94 g) in ethanol (90 mL) is added 10% Pd/C (50% wet). The mixture is stirred at 40 °C under hydrogen atmosphere for 5 hours. The suspension is filtered over and the filtrate is concentrated under reduced pressure to give 4-Amino-2,6-diethyl-piperidine-1-carboxylic acid isopropyl ester (6.34 g, 97%)

A solution of 4-amino-2,6-diethyl-piperidine-1-carboxylic acid isopropyl ester (26.2 mmol, 6.34 g), 5-bromo-2-Chloro-pyrimidine (31.4 mmol, 6.1 g) and *N*,*N-*diisopropylethylamine (31.4 mmol, 5.5 mL) in DMF (80 ml) is allowed to warm to 120 °C and stir for 3.5 hours. The mixture is cooled to room temperature, then added with water. The mixture is extracted with EtOAc. The combined organic layer is dried over Na₂SO₄, filtrated, and concentrated under reduced pressure. The obtained residue is purified by silica gel column chromatography (eluent: hexane / EtOAc) to give 4-(5-bromo-pyrimidin-2-ylamino)-2,6-diethyl-piperidine-1-carboxylic acid isopropyl ester (6.2 g, 59%); ESI-MS m/z: 399 [M+1]⁺, Retention time 2.79 min (condition A).

To a solution of 4-(5-bromo-pyrimidin-2-ylamino)-2,6-diethyl-piperidine-1-carboxylic acid isopropyl ester (14.85 mmol, 5.93 g) in DMF (75 mL) is added sodium hydride (60% oil suspension, 30 mmol, 1.2 g) at 0 °C and stirred at room temperature for 30 min. To the mixture is added 1-bromomethyl-3-chloro-5-trifluoromethyl-benzene (22.3 mmol, 3.6 mL) at 0 °C and stirred at room temperature for 3 hours. The mixture is added water and extracted with EtOAc. The combined organic layer is dried over Na₂SO₄, filtrated, and concentrated under reduced pressure. The obtained residue is purified by silica gel column chromatography (eluent: hexane / EtOAc) to give 4-[(5-bromo-pyrimidin-2-yl)-(3-chloro-5-trifluoromethyl-benzyl)-amino]-2,6-diethyl-piperidine-1-carboxylic acid isopropyl ester (7.2 g, 82%); ESI-MS m/z: 591 [M+1]⁺, Retention time 5.93 min (condition B).

The following material is prepared following the above procedure.

| Name | Product | ESI-MS m/z [M+1]⁺ | Retention time (min) | Starting Material |
|---|---|---|---|---|
| (2R,4R,6S)-4-[(3,5-Bis-trifluoromethylbenzyl)-(5-bromo-pyrimidin-2-yl)-amino]-2,6-diethyl-piperidine-1-carboxylic acid isopropyl ester | 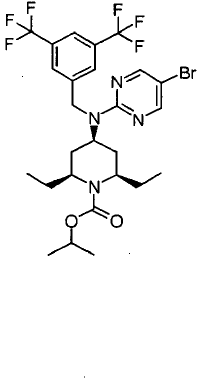 | 624,626 | 2.59 (condition A) | 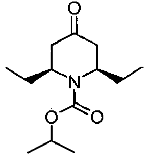 |
| | | | | 1-bromometh yl-3,5-bistrifluorom ethylbenzene instead of 1-bromometh yl-3-chloro-5-trifluoromet hyl-benzene |
| (2R,4R,6S)-4-[(5-Bromo-pyrimidin-2-yl)-(3-chloro-5-trifluoromethylbenzyl)-amino]-2,6-dimethyl-piperidine-1-carboxylic acid isopropyl ester | 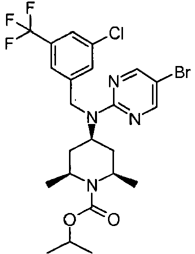 | 563 | 2.70 (condition A) | 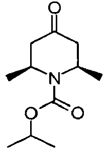 |
| (2R,6R)-4-[(5-Bromo-pyrimidin-2-yl)-(3-chloro-5-trifluoromethyl-benzyl)-amino]-2,6-dimethyl-piperidine-1-carboxylic acid isopropyl ester | 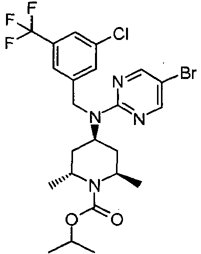 | 564 | 2.77 (condition A) | 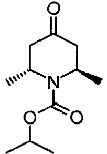 |

### 39). Synthesis of (2S,4R,6R)-4-{(3-chloro-5-trifluoromethyl-benzyl)-[5-(1-trityl-1H-imidazol-4-yl)-pyrimidin-2-yl]-amino}-2,6-diethyl-piperidine-1-carboxylic acid isopropyl ester

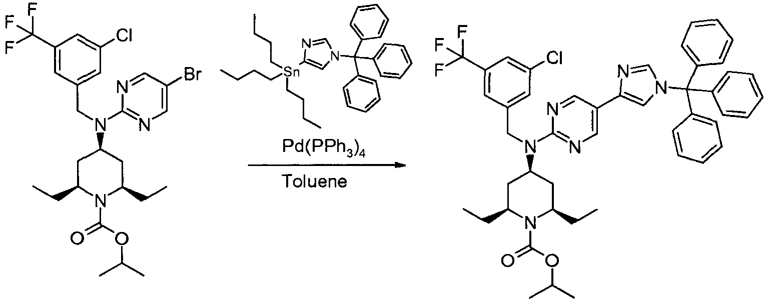

A mixture of (2S,4R,6R)-4-[(5-bromo-pyrimidin-2-yl)-(3-chloro-5-trifluoromethyl-benzyl)-amino]-2,6-diethyl-piperidine-1-carboxylic acid isopropyl ester (118 mg, 0.20 mmol), 1-trityl-4-tributylstannanyl-1 H-imidazole (144 mg, 0.24 mmol), and Pd(PPh₃)₄ (23 mg, 0.02 mmol) in toluene (3 mL) under nitrogen is stirred for 4.5 hours at 130 °C. The mixture is cooled to room temperature quenched with saturated ammonium chloride solution. The mixture is extracted with ethyl acetate, and the organic layer is washed with brine, dried over Na₂SO₄, filtered, and concentrated under reduced pressure. The obtained residue is purified by silica gel column chromatography (hexane-ethyl acetate) to give (2S,4R,6R)-4-{(3-chloro-5-trifluoromethyl-benzyl)-[5-(1-trityl-1H-imidazol-4-yl)-pyrimidin-2-yl]-amino}-2,6-diethyl-piperidine-1-carboxylic acid isopropyl ester (170 mg); ESI-MS m/z: 821 [M+1]⁺, Retention time 5.02 min (condition B).

## Claims

1. A compound of formula (I): 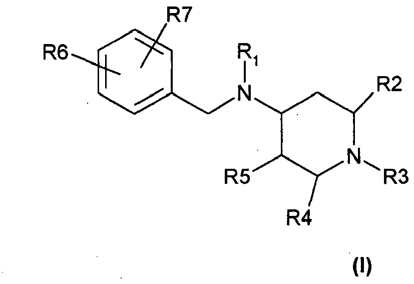 wherein R1 is cycloalkyl, heterocyclyl, aryl, alkyl-O-C(O)-, alkanoyl, or alkyl, wherein each cycloalkyl, heterocyclyl, or aryl is optionally substituted with one to three substituents selected from alkyl, aryl, haloalkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamoyl, alkyl-S-, alkyl-SO-, alkyl-SO₂-, amino, mono- or di-substituted (alkyl, cycloalkyl, aryl and/or aryl-alkyl-) amino, H₂N-SO₂-, or heterocyclyl, and wherein each alkanoyl, alkyl-O-C(O)-, alkyl, alkoxy, or heterocyclyl is further optionally substituted with one to three substituents selected from hydroxy, alkyl, halogen, nitro, carboxy, thiol, cyano, HSO₃-, cycloalkyl, alkenyl, alkoxy, cycloalkoxy, alkenyloxy, alkyl-O-C(O)-, alkanoyl, carbamoyl, alkyl-S-, alkyl-SO-, alkyl-SO₂-, amino, mono- or di-substituted (alkyl, cycloalkyl, aryl and/or aryl-alkyl-) amino, H₂N-SO₂-, or heterocyclyl;
R2 is alkyl, cycloalkyl, cycloalkyl-alkyl-, or alkoxy, wherein each alkyl, cycloalkyl or alkoxy is optionally substituted with one to three substituents selected from alkyl, alkoxy or halogen;
R3 is R8-O-C(O)-, (R8)(R9)N-C(O)-, R8-C(O)-, R8-S(O)₂-, alkyl, cycloalkyl, or aryl-alkyl-, wherein each alkyl, cycloalkyl or aryl-alkyl- is optionally substituted with one to three substituents selected from alkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamimidoyl, alkyl-S-, alkyl-SO-, alkyl-SO₂-, amino, H₂N-SO₂-, heterocyclyl,
wherein R8 and R9 are independently hydrogen, alkyl, cycloalkyl, aryl, aryl-alkyl- or cycloalkyl-alkyl-, wherein each alkyl, cycloalkyl, aryl, aryl-alkyl- or cycloalkyl-alkyl- is optionally substituted with one to three substituents selected from alkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamimidoyl, alkyl-S-, alkyl-SO-, alkyl-SO₂-, amino, H₂N-SO₂-, heterocyclyl;
R4 and R5 are independently hydrogen, alkyl, alkoxy, cycloalkyl, aryl, heteroaryl, aryl-alkyl-, cycloalkyl-alkyl-, or heteroaryl-alkyl-, wherein each alkyl, cycloalkyl, aryl, heteroaryl, aryl-alkyl-, cycloalkyl-alkyl-, or heteroaryl-alkyl- is optionally substituted with one to three substituents selected from alkyl, hydroxy, halogen, haloalkyl, nitro, carboxy, thiol, cyano, HSO₃-, cycloalkyl, alkenyl, alkoxy, cycloalkoxy, haloalkoxy, alkenyloxy, alkyl-O-C(O)-, alkanoyl, carbamimidoyl, alkyl-S-, alkyl-SO-, alkyl-SO₂-, amino, mono- or di-substituted (alkyl, cycloalkyl, aryl and/or aryl-alkyl-) amino, H₂N-SO₂-, heterocyclyl, with the proviso that R4 and R5 cannot be hydrogen simultaneously;
R6 and R7 are independently hydrogen, alkyl, haloalkyl, halogen, cyano, nitro, hydroxy, amino, dialkylamino, or alkoxy, haloalkoxy; or
R6 is aryl, heteroaryl, or alkyl-S(O)₂-, wherein each aryl or heteroaryl is optionally substituted with one to three substituents selected from alkyl, hydroxy, halogen, nitro, carboxy, thiol, cyano, HSO₃-, cycloalkyl, alkenyl, alkoxy, cycloalkoxy, alkenyloxy, alkyl-O-C(O)-, alkanoyl, carbamimidoyl, alkyl-S-, alkyl-SO-, alkyl-SO₂-, amino, H₂N-SO₂- heterocyclyl; or a pharmaceutically acceptable salt thereof; or an optical isomer thereof; or a mixture of optical isomers, with the proviso that when R2 and R5 are independently alkyl and R4 is hydrogen, R6 or R7 cannot be hydrogen or alkoxy.

2. The compound according to claim 1 wherein R1 is alkyl-O-C(O)-, or heteroaryl that is optionally substituted by one to three substituents selected from halogen, heteroaryl, hydroxyl, alkoxy, non-aromatic heterocyclyl, alkyl, or dialkylamino, wherein each of heteroaryl, alkoxy, alkyl and non-aromatic heterocyclyl is further optionally substituted by one to three substituents selected from alkyl, hydroxyl, alkyl-O-C(O)-, carboxy, alkyl-SO₂-, alkoxy, dialkylamino, or non-aromatic heterocyclyl, or alkanoyl;
R2 is alkyl;
R3 is R8-C(O)-, or R8-O-C(O)-, wherein R8 is alkyl, non-aromatic heterocyclyl or cycloalkyl, each of alkyl, non-aromatic heterocyclyl or cycloalkyl is optionally substituted by one to three substituents selected from alkanoyl, alkyl-C(O)-O-, or hydroxyl;
R4 is aryl-alkyl-, alkyl, or heteroaryl, each of which is optionally substituted by one to three substituents selected from alkyl, halogen, or hydroxyl;
R5 is hydrogen or alkyl;
R6 and R7 are independently haloalkyl, halogen or alkoxy ; or
a pharmaceutically acceptable salt thereof; or an optical isomer thereof; or a mixture of optical isomers.

3. The compound according to claim 1 wherein R1 is (C1-C4) alkyl-O-C(O)-, or 5- to 7-membered heteroaryl that is optionally substituted by one to three substituents selected from halogen, 5- to 7-membered heteroaryl, (C1-C4) alkoxy, 5- to 7-membered non-aromatic heterocyclyl, (C1-C4) alkyl, or (C1-C4) dialkylamino, wherein (C1-C4) alkyl is optionally substituted by one to three hydroxyl groups, 5- to 7-membered non-aromatic heterocyclyl is optionally substituted by one to three alkanoyl groups, and wherein each of 5- to 7-membered heteroaryl and (C1-C4) alkoxy is further optionally substituted by one to three substituents selected from (C1-C4) alkyl, hydroxy, (C1-C4) alkyl-O-C(O)-, (C1-C4) alkyl-SO₂-(C1-C4) alkoxy, (C1-C4) dialkylamino, or 5- to 7-membered non-aromatic heterocyclyl;
R2 is (C1-C4) alkyl;
R3 is R8-C(O)-, or R8-O-C(O)-, wherein R8 is (C1-C4) alkyl, 5- to 7-membered non-aromatic heterocyclyl or (C5-C7) cycloalkyl, each of (C1-C4) alkyl, 5- to 7-membered non-aromatic heterocyclyl and (C5-C7) cycloalkyl is optionally substituted by one to three substituents selected from (C1-C4) alkanoyl, (C1-C4) alkyl-C(O)-O-, or hydroxy;
R4 is (C5-C9)aryl-(C1-C4) alkyl-, (C1-C4) alkyl, or 5- to 7-membered heteroaryl, each of which is optionally substituted by one to three substituents selected from (C1-C4) alkyl, halogen, or hydroxy;
R5 is hydrogen or (C1-C4) alkyl;
R6 and R7 are independently (C1-C4) haloalkyl, halogen or (C1-C4) alkoxy; or
a pharmaceutically acceptable salt thereof; or an optical isomer thereof; or a mixture of optical isomers.

4. A compound of formula (I) according to any of claims 1 to 3 for use in inhibiting CETP activity in a subject.

5. A compound of formula (I) according to any of claims 1 to 3 for use in treating a disorder or a disease in a subject mediated by CETP or responsive to inhibition of CETP.

6. A compound for use according to claim 5, wherein the disorder or the disease is selected from hyperlipidemia, arteriosclerosis, atherosclerosis, peripheral vascular disease, dyslipidemia, hyperbetalipoproteinemia, hypoalphalipoproteinemia, hypercholesterolemia, hypertriglyceridemia, familial hypercholesterolemia, cardiovascular disorder, coronary heart disease, coronary artery disease, coronary vascular disease, angina, ischemia, heart ischemia, thrombosis, cardiac infarction such as myocardial infarction, stroke, peripheral vascular disease, reperfusion injury, angioplasty restenosis, hypertension, congestive heart failure, diabetes such as type II diabetes mellitus, diabetic vascular complications, obesity or endotoxemia.

7. A pharmaceutical composition comprising a therapeutically effective amount of a compound of formula (I) according to any of claims 1 to 3 and one or more pharmaceutically acceptable carriers.

8. A pharmaceutical composition comprising a therapeutically effective amount of the compound according to any of claims 1 to 3 and one or more therapeutically active agents selected from the group consisting of a:
(i) HMG-Co-A reductase inhibitor or a pharmaceutically acceptable salt thereof,
(ii) angiotensin II receptor antagonist or a pharmaceutically acceptable salt thereof,
(iii) angiotensin converting enzyme (ACE) Inhibitor or a pharmaceutically acceptable salt thereof,
(iv) calcium channel blocker or a pharmaceutically acceptable salt thereof,
(v) aldosterone synthase inhibitor or a pharmaceutically acceptable salt thereof,
(vi) aldosterone antagonist or a pharmaceutically acceptable salt thereof,
(vii) dual angiotensin converting enzyme/neutral endopeptidase (ACE/NEP) inhibitor or a pharmaceutically acceptable salt thereof,
(viii) endothelin antagonist or a pharmaceutically acceptable salt thereof,
(ix) renin inhibitor or a pharmaceutically acceptable salt thereof,
(x) diuretic or a pharmaceutically acceptable salt thereof;
(xi) an ApoA-I mimic; and
(Xii) a DGAT inhibitor.

9. A compound of formula (I) according to any of claims 1 to 3 for use as a medicament.

10. Use of a compound of formula (I) according to any of claims 1 to 3, for the preparation of a medicament for the treatment of a disorder or disease in a subject mediated by CETP or responsive to inhibition of CETP.

11. Use of claim 10, wherein the disorder or the disease is selected from hyperlipidemia, arteriosclerosis, atherosclerosis, peripheral vascular disease, dyslipidemia, hyperbetalipoproteinemia, hypoalphalipoproteinemia, hypercholesterolemia, hypertriglyceridemia, familial hypercholesterolemia, cardiovascular disorder, coronary heart disease, coronary artery disease, coronary vascular disease, angina, ischemia, heart ischemia, thrombosis, cardiac infarction such as myocardial infarction, stroke, peripheral vascular disease, reperfusion injury, angioplasty restenosis, hypertension, congestive heart failure, diabetes such as type II diabetes mellitus, diabetic vascular complications, obesity or endotoxemia.

## Patentansprüche

1. Eine Verbindung der Formel (I): 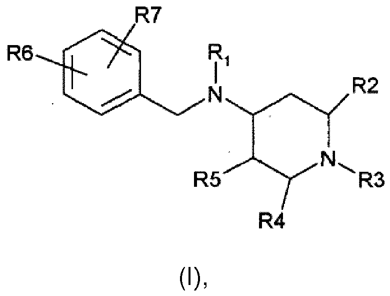 wobei gilt: R1 ist Cycloalkyl, Heterocyclyl, Aryl, Alkyl-O-C(O)-, Alkanoyl oder Alkyl, wobei jedes Cycloalkyl, Heterocyclyl oder Aryl wahlweise substituiert ist mit einem bis drei Substituenten, ausgewählt aus Alkyl, Aryl, Haloalkyl, Hydroxy, Halogen, Nitro, Carboxy, Thiol, Cyano, HSO₃-, Cycloalkyl, Alkenyl, Alkoxy, Cycloalkoxy, Alkenyloxy, Alkyl-O-C(O)-, Alkanoyl, Carbamoyl, Alkyl-S-, Alkyl-SO-, Alkyl-SO₂-, Amino, mono- oder disubstituiertem (Alkyl, Cycloalkyl, Aryl und/oder Arylalkyl)-amino, H₂N-SO₂- oder Heterocyclyl, und wobei jedes Alkanoyl, Alkyl-O-C(O)-, Alkyl, Alkoxy oder Heterocyclyl ferner wahlweise substituiert ist mit einem bis drei Substituenten, ausgewählt aus Hydroxy, Alkyl, Halogen, Nitro, Carboxy, Thiol, Cyano, HSO₃-, Cycloalkyl, Alkenyl, Alkoxy, Cycloalkoxy, Alkenyloxy, Alkyl-O-C(O)-, Alkanoyl, Carbamoyl, Alkyl-S-, Alkyl-SO-, Alkyl-SO₂-, Amino, mono- oder disubstituiertem (Alkyl, Cycloalkyl, Aryl und/oder Arylalkyl)-amino, H₂N-SO₂- oder Heterocyclyl;
R2 ist Alkyl, Cycloalkyl, Cycloalkyl-alkyl- oder Alkoxy, wobei jedes Alkyl, Cycloalkyl oder Alkoxy wahlweise substituiert ist mit einem bis drei Substituenten, ausgewählt aus Alkyl, Alkoxy oder Halogen;
R3 ist R8-O-C(O)-, (R8)(R9)N-C(O)-, R8-C(O)-, R8-S(O)₂-, Alkyl, Cycloalkyl oder Aryl-alkyl-, wobei jedes Alkyl, Cycloalkyl oder Aryl-alkyl- wahlweise substituiert ist mit einem bis drei Substituenten, ausgewählt aus Alkyl, Hydroxy, Halogen, Nitro, Carboxy, Thiol, Cyano, HSO₃-, Cycloalkyl, Alkenyl, Alkoxy, Cycloalkoxy, Alkenyloxy, Alkyl-O-C(O)-, Alkanoyl, Carbamimidoyl, Alkyl-S-, Alkyl-SO-, Alkyl-SO₂-, Amino, H₂N-SO₂-, Heterocyclyl,
wobei gilt: R8 und R9 sind unabhängig Wasserstoff, Alkyl, Cycloalkyl, Aryl, Aryl-alkyl- oder Cycloalkyl-alkyl-, wobei jedes Alkyl, Cycloalkyl, Aryl, Aryl-alkyl- oder Cycloalkyl-alkyl- wahlweise substituiert ist mit einem bis drei Substituenten, ausgewählt aus Alkyl, Hydroxy, Halogen, Nitro, Carboxy, Thiol, Cyano, HSO₃-, Cycloalkyl, Alkenyl, Alkoxy, Cycloalkoxy, Alkenyloxy, Alkyl-O-C(O)-, Alkanoyl, Carbamimidoyl, Alkyl-S-, Alkyl-SO-, Alkyl-SO₂-, Amino, H₂N-SO₂-, Heterocyclyl;
R4 und R5 sind unabhängig Wasserstoff, Alkyl, Alkoxy, Cycloalkyl, Aryl, Heteroaryl, Aryl-alkyl-, Cycloalkyl-alkyl- oder Heteroaryl-alkyl-, wobei jedes Alkyl, Cycloalkyl, Aryl, Heteroaryl, Aryl-alkyl-, Cycloalkyl-alkyl- oder Heteroaryl-alkyl- wahlweise substituiert ist mit einem bis drei Substituenten, ausgewählt aus Alkyl, Hydroxy, Halogen, Haloalkyl, Nitro, Carboxy, Thiol, Cyano, HSO₃-, Cycloalkyl, Alkenyl, Alkoxy, Cycloalkoxy, Haloalkoxy, Alkenyloxy, Alkyl-O-C(O)-, Alkanoyl, Carbamimidoyl, Alkyl-S-, Alkyl-SO-, Alkyl-SO₂-, Amino, mono- oder disubstituiertem (Alkyl, Cycloalkyl, Aryl und/oder Arylalkyl)-amino, H₂N-SO₂-, Heterocyclyl, mit der Maßgabe, dass R4 und R5 nicht gleichzeitig Wasserstoff sein können;
R6 und R7 sind unabhängig Wasserstoff, Alkyl, Haloalkyl, Halogen, Cyano, Nitro, Hydroxy, Amino, Dialkylamino oder Alkoxy, Haloalkoxy; oder
R6 ist Aryl, Heteroaryl oder Alkyl-S(O)₂-, wobei jedes Aryl oder Heteroaryl wahlweise substituiert ist mit einem bis drei Substituenten, ausgewählt aus Alkyl, Hydroxy, Halogen, Nitro, Carboxy, Thiol, Cyano, HSO₃-, Cycloalkyl, Alkenyl, Alkoxy, Cycloalkoxy, Alkenyloxy, Alkyl-O-C(O)-, Alkanoyl, Carbamimidoyl, Alkyl-S-, Alkyl-SO-, Alkyl-SO₂-, Amino, H₂N-SO₂-Heterocyclyl; oder ein pharmazeutisch verträgliches Salz davon; oder ein optisches Isomer davon; oder ein Gemisch von optischen Isomeren, mit der Maßgabe, dass, wenn R2 und R5 unabhängig Alkyl sind und R4 Wasserstoff ist, R6 oder R7 nicht Wasserstoff oder Alkoxy sein können.

2. Die Verbindung nach Anspruch 1, wobei gilt: R1 ist Alkyl-O-C(O)- oder Heteroaryl, das wahlweise substituiert ist mit einem bis drei Substituenten, ausgewählt aus Halogen, Heteroaryl, Hydroxyl, Alkoxy, nicht-aromatischem Heterocyclyl, Alkyl oder Dialkylamino, wobei jedes Heteroaryl, Alkoxy, Alkyl und nicht-aromatisches Heterocyclyl ferner wahlweise substituiert ist mit einem bis drei Substituenten, ausgewählt aus Alkyl, Hydroxyl, Alkyl-O-C(O)-, Carboxy, Alkyl-SO₂-, Alkoxy, Dialkylamino oder nicht-aromatischem Heterocyclyl oder Alkanoyl;
R2 ist Alkyl;
R3 ist R8-C(O)- oder R8-O-C(O)-, wobei gilt: R8 ist Alkyl, nicht-aromatisches Heterocyclyl oder Cycloalkyl, wobei jedes Alkyl, nicht-aromatisches Heterocyclyl oder Cycloalkyl wahlweise substituiert ist mit einem bis drei Substituenten, ausgewählt aus Alkanoyl, Alkyl-C(O)-O- oder Hydroxyl;
R4 ist Aryl-alkyl-, Alkyl oder Heteroaryl, wobei jedes wahlweise substituiert ist mit einem bis drei Substituenten, ausgewählt aus Alkyl, Halogen oder Hydroxyl;
R5 ist Wasserstoff oder Alkyl;
R6 und R7 sind unabhängig Haloalkyl, Halogen oder Alkoxy; oder
ein pharmazeutisch verträgliches Salz davon; oder ein optisches Isomer davon; oder ein Gemisch von optischen Isomeren.

3. Die Verbindung nach Anspruch 1, wobei gilt: R1 ist (C1-C4)Alkyl-O-C(O)- oder 5- bis 7-gliedriges Heteroaryl, das wahlweise substituiert ist mit einem bis drei Substituenten, ausgewählt aus Halogen, 5- bis 7-gliedrigem Heteroaryl, (C1-C4)Alkoxy, 5- bis 7-gliedrigem nicht-aromatischen Heterocyclyl, (C1-C4)Alkyl oder (C1-C4)Dialkylamino, wobei (C1-C4)Alkyl wahlweise substituiert ist mit einem bis drei Hydroxyl-Gruppen, 5- bis 7-gliedriges nicht-aromatisches Heterocyclyl wahlweise substituiert ist mit einem bis drei Alkanoyl-Gruppen, und wobei jedes 5- bis 7-gliedriges Heteroaryl und (C1-C4)Alkoxy ferner wahlweise substituiert ist mit einem bis drei Substituenten, ausgewählt aus (C1-C4)Alkyl, Hydroxy, (C1-C4)Alkyl-O-C(O)-, (C1-C4)Alkyl-SO₂-, (C1-C4)Alkoxy, (C1-C4)Dialkylamino oder 5- bis 7-gliedrigem nicht-aromatischen Heterocyclyl;
R2 ist (C1-C4)Alkyl;
R3 ist R8-C(O)- oder R8-O-C(O)-, wobei gilt: R8 ist (C1-C4)Alkyl, 5- bis 7-gliedriges nicht-aromatisches Heterocyclyl oder (C5-C7)Cycloalkyl, wobei jedes (C1-C4)Alkyl, 5- bis 7-gliedriges nicht-aromatisches Heterocyclyl und (C5-C7)Cycloalkyl wahlweise substituiert ist mit einem bis drei Substituenten, ausgewählt aus (C1-C4)Alkanoyl, (C1-C4)Alkyl-C(O)-O- oder Hydroxy;
R4 ist (C5-C9)Aryl-(C1-C4)alkyl-, (C1-C4)Alkyl oder 5- bis 7-gliedriges Heteroaryl, wobei jedes wahlweise substituiert ist mit einem bis drei Substituenten, ausgewählt aus (C1-C4)Alkyl, Halogen oder Hydroxy;
R5 ist Wasserstoff oder (C1-C4)Alkyl;
R6 und R7 sind unabhängig (C1-C4)Haloalkyl, Halogen oder (C1-C4)Alkoxy; oder
ein pharmazeutisch verträgliches Salz davon; oder ein optisches Isomer davon; oder ein Gemisch von optischen Isomeren.

4. Eine Verbindung der Formel (I) nach einem der Ansprüche 1 bis 3 zur Verwendung bei der Hemmung der CETP-Aktivität bei einer Testperson.

5. Eine Verbindung der Formel (I) nach einem der Ansprüche 1 bis 3 zur Verwendung bei der Behandlung einer Störung oder einer Erkrankung bei einer Testperson, die durch CETP vermittelt wird oder die auf die Hemmung von CETP anspricht.

6. Eine Verbindung zur Verwendung nach Anspruch 5, wobei die Störung oder die Erkrankung ausgewählt ist aus Hyperlipidämie, Arteriosklerose, Atherosklerose, peripherer Gefäßerkrankung, Dyslipidämie, Hyperbetalipoproteinämie, Hypoalphalipoproteinämie, Hypercholesterinämie, Hypertriglyzeridämie, familiärer Hypercholesterinämie, kardiovaskulärer Störung, koronarer Herzerkrankung, koronarer Arterienerkrankung, koronarer Gefäßerkrankung, Angina, Ischämie, Herz-Ischämie, Thrombose, Herzinfarkt, wie z.B. Myokardinfarkt, Schlaganfall, peripherer Gefäßerkrankung, Reperfusionsverletzung, Restenose nach Angioplastie, Hypertonie, kongestiver Herzinsuffizienz, Diabetes, wie z.B. Typ-II-Diabetes mellitus, diabetischen vaskulären Komplikationen, Fettleibigkeit oder Endotoxinämie.

7. Eine pharmazeutische Zusammensetzung, umfassend eine therapeutisch wirksame Menge einer Verbindung der Formel (I) nach einem der Ansprüche 1 bis 3 und einen oder mehr pharmazeutisch verträgliche Träger.

8. Eine pharmazeutische Zusammensetzung, umfassend eine therapeutisch wirksame Menge der Verbindung nach einem der Ansprüche 1 bis 3 und einen oder mehr therapeutische Wirkstoffe, ausgewählt aus der Gruppe bestehend aus einem:
(i) HMG-Co-A-Reduktase-Inhibitor oder einem pharmazeutisch verträglichen Salz davon,
(ii) Angiotensin-II-Rezeptor-Antagonisten oder einem pharmazeutisch verträglichen Salz davon,
(iii) Angiotensin-umwandelndes Enzym (ACE)-Inhibitor oder einem pharmazeutisch verträglichen Salz davon,
(iv) Calcium-Kanal-Blocker oder einem pharmazeutisch verträglichen Salz davon,
(v) Aldosteron-Synthase-Inhibitor oder einem pharmazeutisch verträglichen Salz davon,
(vi) Aldosteron-Antagonisten oder einem pharmazeutisch verträglichen Salz davon,
(vii) dualen Angiotensin-umwandelndes Enzym/neutrale Endopeptidase (ACE/NEP)-Inhibitor oder einem pharmazeutisch verträglichen Salz davon,
(viii) Endothelin-Antagonisten oder einem pharmazeutisch verträglichen Salz davon,
(ix) Renin-Inhibitor oder einem pharmazeutisch verträglichen Salz davon,
(x) Diuretikum oder einem pharmazeutisch verträglichen Salz davon,
(xi) ApoA-I-Mimetikum; und
(xii) DGAT-Inhibitor.

9. Eine Verbindung der Formel (I) nach einem der Ansprüche 1 bis 3 zur Verwendung als Medikament.

10. Die Verwendung einer Verbindung der Formel (I) nach einem der Ansprüche 1 bis 3 zur Herstellung eines Medikaments zur Behandlung einer Störung oder Erkrankung bei einer Testperson, die durch CETP vermittelt wird oder die auf die Hemmung von CETP anspricht.

11. Die Verwendung nach Anspruch 10, wobei die Störung oder die Erkrankung ausgewählt ist aus Hyperlipidämie, Arteriosklerose, Atherosklerose, peripherer Gefäßerkrankung, Dyslipidämie, Hyperbetalipoproteinämie, Hypoalphalipoproteinämie, Hypercholesterinämie, Hypertriglyzeridämie, familiärer Hypercholesterinämie, kardiovaskulärer Störung, koronarer Herzerkrankung, koronarer Arterienerkrankung, koronarer Gefäßerkrankung, Angina, Ischämie, Herz-Ischämie, Thrombose, Herzinfarkt, wie z.B. Myokardinfarkt, Schlaganfall, peripherer Gefäßerkrankung, Reperfusionsverletzung, Restenose nach Angioplastie, Hypertonie, kongestiver Herzinsuffizienz, Diabetes, wie z.B. Typ-II-Diabetes mellitus, diabetischen vaskulären Komplikationen, Fettleibigkeit oder Endotoxinämie.

## Revendications

1. Composé de formule (I) : 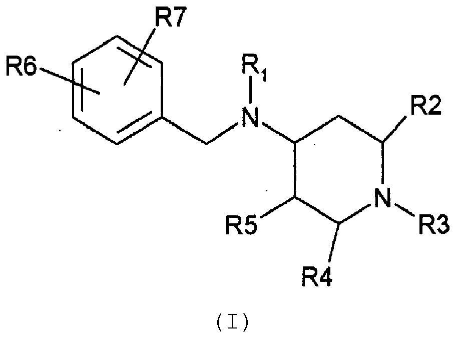 dans laquelle :
- R1 représente cycloalkyle, hétérocyclyle, aryle, alkyl-O-C(O)-, alcanoyle ou alkyle, où chaque cycloalkyle, hétérocyclyle ou aryle est facultativement substitué par un à trois substituants choisis parmi alkyle, aryle, haloalkyle, hydroxy, halogène, nitro, carboxy, thiol, cyano, HSO₃-, cycloalkyle, alcényle, alcoxy, cycloalcoxy, alcényloxy, alkyl-O-C(O)-, alcanoyle, carbamoyle, alkyl-S-, alkyl-SO-, alkyl-SO₂-, amino, mono- ou di- substitué (alkyl, cycloalkyl, aryl et/ou aryl-alkyl-) amino, H₂N-SO₂- ou hétérocyclyle, et où chaque alcanoyle, alkyl-O-C(O)-, alkyle, alcoxy ou hétérocyclyle est encore facultativement substitué par un à trois substituants choisis parmi hydroxy, alkyle, halogène, nitro, carboxy, thiol, cyano, HSO₃-, cycloalkyle, alcényle, alcoxy, cycloalcoxy, alcényloxy, alkyl-O-C(O)-, alcanoyle, carbamoyle, alkyl-S-, alkyl-SO-, alkyl-SO₂-, amino, mono- ou di-substitué (alkyl, cycloalkyl, aryl et/ou aryl-alkyl-) amino, H₂N-SO₂- ou hétérocyclyle ;
- R2 représente alkyle, cycloalkyle, cycloalkyl-alkyl- ou alcoxy, où chaque alkyle, cycloalkyle ou alcoxy est facultativement substitué par un à trois substituants choisis parmi alkyle, alcoxy ou halogène ;
- R3 représente R8-O-C(O)-, (R8)(R9)N-C(O)-, R8-C(O)-, R8-S(O)₂-, alkyle, cycloalkyle ou aryl-alkyl-, où chaque alkyle, cycloalkyle ou aryl-alkyl- est facultativement substitué par un à trois substituants choisis parmi alkyle, hydroxy, halogène, nitro, carboxy, thiol, cyano, HSO₃-, cycloalkyle, alcényle, alcoxy, cycloalcoxy, alcényloxy, alkyl-O-C(O)-, alcanoyle, carbamimidoyle, alkyl-S-, alkyl-SO-, alkyl-SO₂-, amino, H₂N-SO₂-, hétérocyclyle,
où R8 et R9 représentent indépendamment hydrogène, alkyle, cycloalkyle, aryle, aryl-alkyl- ou cycloalkyl-alkyl-, où chaque alkyle, cycloalkyle, aryle, aryl-alkyl-ou cycloalkyl-alkyl- est facultativement substitué par un à trois substituants choisis parmi alkyle, hydroxy, halogène, nitro, carboxy, thiol, cyano, HSO₃-, cycloalkyle, alcényle, alcoxy, cycloalcoxy, alcényloxy, alkyl-O-C(O)-, alcanoyle, carbamimidoyle, alkyl-S-, alkyl-SO-, alkyl-SO₂-, amino, H₂N-SO₂-,hétérocyclyle ;
- R4 et R5 représentent indépendamment hydrogène, alkyle, alcoxy, cycloalkyle, aryle, hétéroaryle, aryl-alkyl-, cycloalkyl-alkyl- ou hétéroaryl-alkyl-, où chaque alkyle, cycloalkyle, aryle, hétéroaryle, aryl-alkyl-, cycloalkyl-alkyl- ou hétéroaryl-alkyl- est facultativement substitué par un à trois substituants choisis parmi alkyle, hydroxy, halogène, haloalkyle, nitro, carboxy, thiol, cyano, HSO₃-, cycloalkyle, alcényle, alcoxy, cycloalcoxy, haloalcoxy, alcényloxy, alkyl-O-C(O)-, alcanoyle, carbamimidoyle, alkyl-S-, alkyl-SO-, alkyl-SO₂-, amino, mono- ou di-substitué (alkyl, cycloalkyl, aryl et/ou aryl-alkyl-) amino, H₂N-SO₂-,hétérocyclyle, à la condition que R4 et R5 ne peuvent pas représenter hydrogène simultanément ;
- R6 et R7 représentent indépendamment hydrogène, alkyle, haloalkyle, halogène, cyano, nitro, hydroxy, amino, dialkylamino, ou alcoxy, haloalcoxy ; ou
- R6 représente aryle, hétéroaryle ou alkyl-S(O)₂-, où chaque aryle ou hétéroaryle est facultativement substitué par un à trois substituants choisis parmi alkyle, hydroxy, halogène, nitro, carboxy, thiol, cyano, HSO₃-, cycloalkyle, alcényle, alcoxy, cycloalcoxy, alcényloxy, alkyl-O-C(O)-, alcanoyle, carbamimidoyle, alkyl-S-, alkyl-SO-, alkyl-SO₂-, amino, H₂N-SO₂-,hétérocyclyle ;
ou un sel pharmaceutiquement acceptable de ce composé ; ou un isomère optique de celui-ci ; ou un mélange d'isomères optiques, à la condition que, lorsque R2 et R5 représentent indépendamment alkyle et R4 représente hydrogène, R6 ou R7 ne peuvent pas représenter hydrogène ou alcoxy.

2. Composé selon la revendication 1, dans lequel :
- R1 représente alkyl-O-C(O)- ou hétéroaryle qui est facultativement substitué par un à trois substituants choisis parmi halogène, hétéroaryle, hydroxyle, alcoxy, hétérocyclyle non aromatique, alkyle ou dialkylamino, où chacun parmi hétéroaryle, alcoxy, alkyle et hétérocyclyle non aromatique est encore facultativement substitué par un à trois substituants choisis parmi alkyle, hydroxyle, alkyl-O-C(O)-, carboxy, alkyl-SO₂-, alcoxy, dialkylamino ou hétérocyclyle non aromatique, ou alcanoyle ;
- R2 représente alkyle ;
- R3 représente R8-C(0)- ou R8-O-C(O)-, où R8 représente alkyle, hétérocyclyle non aromatique ou cycloalkyle, chacun parmi alkyle, hétérocyclyle non aromatique ou cycloalkyle est facultativement substitué par un à trois substituants choisis parmi alcanoyle, alkyl-C(O)-O- ou hydroxyle ;
- R4 représente aryl-alkyl-, alkyle, ou hétéroaryle, dont chacun est facultativement substitué par un à trois substituants choisis parmi alkyle, halogène ou hydroxyle ;
- R5 représente hydrogène ou alkyle ;
- R6 et R7 représentent indépendamment haloalkyle, halogène ou alcoxy ; ou
un sel pharmaceutiquement acceptable de ce composé ou un isomère optique de celui-ci ; ou un mélange d'isomères optiques.

3. Composé selon la revendication 1, dans lequel :
- R1 représente alkyl en C1-C4-O-C(O)- ou hétéroaryle à 5 à 7 chaînons qui est facultativement substitué par un à trois substituants choisis parmi halogène, hétéroaryle à 5 à 7 chaînons, alcoxy en C1-C4, hétérocyclyle non aromatique à 5 à 7 chaînons, alkyle en C1-C4 ou di(alkyl en C1-C4)amino, où alkyle en C1-C4 est facultativement substitué par un à trois groupes hydroxyle, hétérocyclyle non aromatique à 5 à 7 chaînons est facultativement substitué par un à trois groupes alcanoyle, et où chacun parmi hétéroaryle à 5 à 7 chaînons et alcoxy en C1-C4 est encore facultativement substitué par un à trois substituants choisis parmi alkyle en C1-C4, hydroxy, alkyl en C1-C4-O-C(O)-, alkyl en C1-C4-SO₂-, alcoxy en C1-C4, di(alkyl en C1-C4)amino ou hétérocyclyle non aromatique à 5 à 7 chaînons ;
- R2 représente alkyle en C1-C4 ;
- R3 représente R8-C(0)- ou R8-O-C(O)-, où R8 représente alkyle en C1-C4, hétérocyclyle non aromatique à 5 à 7 chaînons ou cycloalkyle en C5-C7, chacun parmi alkyle en C1-C4, hétérocyclyle non aromatique à 5 à 7 chaînons et cycloalkyle en C5-C7 étant facultativement substitué par un à trois substituants choisis parmi alcanoyle en C1-C4, alkyl en C1-C4-C(O)-O- ou hydroxy ;
- R4 représente aryl en C5-C9-alkyl en C1-C4-, alkyle en C1-C4 ou hétéroaryle à 5 à 7 chaînons, dont chacun est facultativement substitué par un à trois substituants choisis parmi alkyle en C1-C4, halogène ou hydroxy ;
- R5 représente hydrogène ou alkyle en C1-C4 ;
- R6 et R7 représentent indépendamment haloalkyle en C1-C4, halogène ou alcoxy en C1-C4 ; ou
un sel pharmaceutiquement acceptable de ce composé ; ou un isomère optique de celui-ci ; ou un mélange d'isomères optiques.

4. Composé de formule (I) selon l'une quelconque des revendications 1 à 3 destiné à être utilisé dans l'inhibition de l'activité de la CETP dans un sujet.

5. Composé de formule (I) selon l'une quelconque des revendications 1 à 3, en vue d'une utilisation dans le traitement d'un trouble ou d'une maladie dans un sujet, à médiation par la CETP ou sensible à l'inhibition de la CETP.

6. Composé en vue d'une utilisation selon la revendication 5, dans lequel le trouble ou la maladie est choisi(e) parmi l'hyperlipidémie, l'artériosclérose, l'athérosclérose, la maladie vasculaire périphérique, la dyslipidémie, l'hyperbêtalipoprotéinémie, l'hypoalphalipoprotéinémie, l'hypercholestérolémie, l'hypertriglycéridémie, l'hypercholestérolémie familiale, le trouble cardiovasculaire, la coronaropathie, la maladie des artères coronaires, la maladie vasculaire coronaire, l'angine de poitrine, l'ischémie, l'ischémie cardiaque, la thrombose, l'infarctus cardiaque tel que l'infarctus du myocarde, l'accident vasculaire cérébral, la maladie vasculaire périphérique, la lésion de reperfusion, la resténose post-angioplastie, l'hypertension, l'insuffisance cardiaque congestive, le diabète tel que le diabète sucré de type II, les complications vasculaires diabétiques, l'obésité ou l'endotoxémie.

7. Composition pharmaceutique comprenant une quantité thérapeutiquement efficace d'un composé de formule (I) selon l'une quelconque des revendications 1 à 3 et un ou plusieurs supports pharmaceutiquement acceptables.

8. Composition pharmaceutique comprenant une quantité thérapeutiquement efficace du composé selon l'une quelconque des revendications 1 à 3 et un ou plusieurs agents thérapeutiquement actifs choisis dans le groupe consistant en :
(i) un inhibiteur de HMG-Co-A réductase ou un sel pharmaceutiquement acceptable de celui-ci ;
(ii) un antagoniste des récepteurs de l'angiotensine II ou un sel pharmaceutiquement acceptable de celui-ci ;
(iii) un inhibiteur de l'enzyme de conversion de l'angiotensine (ACE) ou un sel pharmaceutiquement acceptable de celui-ci ;
(iv) un bloqueur des canaux calciques ou un sel pharmaceutiquement acceptable de celui-ci ;
(v) un inhibiteur de l'aldostérone synthase ou un sel pharmaceutiquement acceptable de celui-ci ;
(vi) un antagoniste de l'aldostérone ou un sel pharmaceutiquement acceptable de celui-ci ;
(vii) un inhibiteur double de l'enzyme de conversion de l'angiotensine/de l'endopeptidase neutre (ACE/NEP) ou un sel pharmaceutiquement acceptable de celui-ci ;
(viii) un antagoniste de l'endothéline ou un sel pharmaceutiquement acceptable de celui-ci ;
(ix) un inhibiteur de la rénine ou un sel pharmaceutiquement acceptable de celui-ci ;
(x) un diurétique ou un sel pharmaceutiquement acceptable de celui-ci ;
(xi) un mimétique d'ApoA-1 ; et
(xii) un inhibiteur de DGAT.

9. Composé de formule (I) selon l'une quelconque des revendications 1 à 3 en vue d'une utilisation comme médicament.

10. Utilisation d'un composé de formule (I) selon l'une quelconque des revendications 1 à 3, pour la préparation d'un médicament pour le traitement d'un trouble ou d'une maladie dans un sujet à médiation par la CETP ou sensible à l'inhibition de la CETP.

11. Utilisation selon la revendication 10, dans lequel le trouble ou la maladie est choisi(e) parmi l'hyperlipidémie, l'artériosclérose, l'athérosclérose, la maladie vasculaire périphérique, la dyslipidémie, l'hyperbêtalipoprotéinémie, l'hypoalphalipoprotéinémie, l'hypercholestérolémie, l'hypertriglycéridémie, l'hypercholestérolémie familiale, le trouble cardiovasculaire, la coronaropathie, la maladie des artères coronaires, la maladie vasculaire coronaire, l'angine de poitrine, l'ischémie, l'ischémie cardiaque, la thrombose, l'infarctus cardiaque tel que l'infarctus du myocarde, l'accident vasculaire cérébral, la maladie vasculaire périphérique, la lésion de reperfusion, la resténose post-angioplastie, l'hypertension, l'insuffisance cardiaque congestive, le diabète tel que le diabète sucré de type II, les complications vasculaires diabétiques, l'obésité ou l'endotoxémie.
